# EUROPÄISCHE PATENTANMELDUNG

(11) **EP 2 314 613 A2**
(43) Veröffentlichungstag der Anmeldung: **27.04.2011**
(21) Anmeldenummer: 10011192.1
(22) Anmeldetag: 10.09.2004
(51) Int. Cl.: C07K 14/47, C07K 16/30, C12Q 1/68

(54) **Differentiell in Tumoren exprimierte Genprodukte und deren Verwendung**

(30) Priorität: 10.09.2003 DE 10341812
(62) Teilanmeldung aus: 04765088.2
(71) Anmelder: Ganymed Pharmaceuticals AG, 55131 Mainz (DE)
(72) Erfinder: Türeci, Özlem, 55116 Mainz (DE); Sahin, Ugur, 55116 Mainz (DE); Koslowski, Michael, 65933 Frankfurt (DE)
(74) Vertreter: Schnappauf, Georg

(57) **Zusammenfassung**

Erfindungsgemäß wurden Tumor-assoziiert exprimierte Genprodukte und die dafür kodierenden Nukleinsäuren identifiziert. Die vorliegende Erfindung betrifft die Therapie und Diagnose von Erkrankungen, bei denen diese Tumor-assoziiert exprimierten Genprodukte aberrant exprimiert werden. Des weiteren betrifft die Erfindung Proteine, Polypeptide und Peptide, die Tumor-assoziiert exprimiert werden und die dafür kodierenden Nukleinsäuren.

SEQUENCE LISTING

<110>  Ganymed Pharmaceuticals AG

<120>  Differentiell in Tumoren exprimierte Genprodukte und deren Verwendung

<130>  342-14 EPT3

<150>  DE 103 41 812.1
<151>  2003-09-10

<160>  822

<170>  PatentIn version 3.1

<210>  1
<211>  1171
<212>  DNA
<213>  Homo sapiens

<400>  1
ctgtcgttgg tgtatttttc tggtgtcact tctgtgcctt ccttcaaagg ttctccaaat      60
gtcaactgtc aaggagcagc taattgagaa gctaattgag gatgatgaaa actcccagtg     120
taaaattact attgttggaa ctggtgccgt aggcatggct tgtgctatta gtatcttact     180
gaaggatttg gctgatgaac ttgcccttgt tgatgttgca ttggacaaac tgaagggaga     240
aatgatggat cttcagcatg gcagtctttt ctttagtact tcaaagatta cttctggaaa     300
agattacagt gtatctgcaa actccagaat agttattgtc acagcaggtg caaggcagca     360
ggagggagaa actcgccttg ccctggtcca acgtaatgtg gctataatga aatcaatcat     420
tcctgccata gtccattata gtcctgattg taaaattctt gttgtttcaa atccagtgga     480
tattttgaca tatatagtct ggaagataag tggcttacct gtaactcgtg taattggaag     540
tggttgtaat ctagactctg cccgtttccg ttacctaatt ggagaaaagt tgggtgtcca     600
ccccacaagc tgccatggtt ggattattgg agaacatggt gattctagtg tgcccttatg     660
gagtggggtg aatgttgctg gtgttgctct gaagactctg gaccctaaat taggaacgga     720
ttcagataag gaacactgga aaaatatcca taaacaagtt attcaaagtg cctatgaaat     780
tatcaagctg aaggggtata cctcttgggc tattggactg tctgtgatgg atctggtagg     840
atccattttg aaaaatctta ggagagtgca cccagtttcc accatggtta agggattata     900

tggaataaaa gaagaactct ttctcagtat cccttgtgtc ttggggcgga atggtgtctc 960
agatgttgtg aaaattaact tgaattctga ggaggaggcc cttttcaaga agagtgcaga 1020
aacactttgg aatattcaaa aggatctaat attttaaatt aaagccttct aatgttccac 1080
tgtttggaga acagaagata gcaggctgtg tattttaaat tttgaaagta ttttcattga 1140
tcttaaaaaa taaaaacaaa ttggagacct g 1171

<210> 2
<211> 1053
<212> DNA
<213> Homo sapiens

<400> 2
ctgtcgttgg tgtatttttc tggtgtcact tctgtgcctt ccttcaaagg ttctccaaat 60
gtcaactgtc aaggagcagc taattgagaa gctaattgag gatgatgaaa actcccagtg 120
taaaattact attgttggaa ctggtgccgt aggcatggct tgtgctatta gtatcttact 180
gaagattaca gtgtatctgc aaactccaga atagttattg tcacagcagg tgcaaggcag 240
caggagggag aaactcgcct tgccctggtc caacgtaatg tggctataat gaaatcaatc 300
attcctgcca tagtccatta tagtcctgat tgtaaaattc ttgttgtttc aaatccagtg 360
gatattttga catatatagt ctggaagata agtggcttac ctgtaactcg tgtaattgga 420
agtggttgta atctagactc tgcccgtttc cgttacctaa ttggagaaaa gttgggtgtc 480
caccccacaa gctgccatgg ttggattatt ggagaacatg gtgattctag tgtgccctta 540
tggagtgggg tgaatgttgc tggtgttgct ctgaagactc tggaccctaa attaggaacg 600
gattcagata aggaacactg gaaaaatatc cataaacaag ttattcaaag tgcctatgaa 660
attatcaagc tgaaggggta tacctcttgg gctattggac tgtctgtgat ggatctggta 720
ggatccattt tgaaaaatct taggagagtg cacccagttt ccaccatggt taagggatta 780
tatggaataa aagaagaact ctttctcagt atcccttgtg tcttggggcg gaatggtgtc 840
tcagatgttg tgaaaattaa cttgaattct gaggaggagg cccttttcaa gaagagtgca 900
gaaacacttt ggaatattca aaaggatcta atattttaaa ttaaagcctt ctaatgttcc 960
actgtttgga gaacagaaga tagcaggctg tgtattttaa attttgaaag tattttcatt 1020
gatcttaaaa aataaaaaca aattggagac ctg 1053

<210> 3
<211> 879
<212> DNA
<213> Homo sapiens

<400> 3
ctgtcgttgg tgtatttttc tggtgtcact tctgtgcctt ccttcaaagg ttctccaaat 60
gtcaactgtc aaggagcagc taattgagaa gctaattgag gatgatgaaa actcccagtg 120
taaaattact attgttggaa ctggtgccgt aggcatggct tgtgctatta gtatcttact 180
gaagtggata ttttgacata tatagtctgg aagataagtg gcttacctgt aactcgtgta 240
attggaagtg gttgtaatct agactctgcc cgtttccgtt acctaattgg agaaaagttg 300
ggtgtccacc ccacaagctg ccatggttgg attattggag aacatggtga ttctagtgtg 360
cccttatgga gtggggtgaa tgttgctggt gttgctctga agactctgga ccctaaatta 420
ggaacggatt cagataagga acactggaaa aatatccata aacaagttat tcaaagtgcc 480
tatgaaatta tcaagctgaa ggggtatacc tcttgggcta ttggactgtc tgtgatggat 540
ctggtaggat ccattttgaa aaatcttagg agagtgcacc cagtttccac catggttaag 600
ggattatatg gaataaaaga agaactcttt ctcagtatcc cttgtgtctt ggggcggaat 660
ggtgtctcag atgttgtgaa aattaacttg aattctgagg aggaggccct tttcaagaag 720
agtgcagaaa cactttggaa tattcaaaag gatctaatat tttaaattaa agccttctaa 780
tgttccactg tttggagaac agaagatagc aggctgtgta ttttaaattt tgaaagtatt 840
ttcattgatc ttaaaaaata aaaacaaatt ggagacctg 879

<210> 4
<211> 811
<212> DNA
<213> Homo sapiens

<400> 4
ctgtcgttgg tgtatttttc tggtgtcact tctgtgcctt ccttcaaagg ttctccaaat 60
gtcaactgtc aaggagcagc taattgagaa gctaattgag gatgatgaaa actcccagtg 120
taaaattact attgttggaa ctggtgccgt aggcatggct tgtgctatta gtatcttact 180
gaagattaca gtgtatctgc aaactccaga atagttattg tcacagcagg tgcaaggcag 240
caggagggag aaactcgcct tgccctggtc caacgtaatg tggctataat gaaatcaatc 300
attcctgcca tagtccatta tagtcctgat tgtaaaattc ttgttgtttc aaatccagtg 360
gatattttga catatatagt ctggaagata agtggcttac ctgtaactcg tgtaattgga 420
agtggttgta atctagactc tgcccgtttc cgttacctaa ttggagaaaa gttgggtgtc 480
caccccacaa gctgccatgg ttggattatt ggagaacatg gtgattctag tgggattata 540
tggaataaaa gaagaactct ttctcagtat cccttgtgtc ttggggcgga atggtgtctc 600
agatgttgtg aaaattaact tgaattctga ggaggaggcc cttttcaaga agagtgcaga 660
aacactttgg aatattcaaa aggatctaat attttaaatt aaagccttct aatgttccac 720

tgtttggaga acagaagata gcaggctgtg tattttaaat tttgaaagta ttttcattga 780
tcttaaaaaa taaaaacaaa ttggagacct g 811

<210> 5
<211> 1047
<212> DNA
<213> Homo sapiens

<400> 5
ctgtcgttgg tgtatttttc tggtgtcact tctgtgcctt ccttcaaagg ttctccaaat 60
gtcaactgtc aaggagcagc taattgagaa gctaattgag gatgatgaaa actcccagtg 120
taaaattact attgttggaa ctggtgccgt aggcatggct tgtgctatta gtatcttact 180
gaaggatttg gctgatgaac ttgcccttgt tgatgttgca ttggacaaac tgaagggaga 240
aatgatggat cttcagcatg gcagtctttt ctttagtact tcaaagatta cttctggaaa 300
agattacagt gtatctgcaa actccagaat agttattgtc acagcaggtg caaggcagca 360
ggagggagaa actcgccttg ccctggtcca acgtaatgtg gctataatga aatcaatcat 420
tcctgccata gtccattata gtcctgattg taaaattctt gttgtttcaa atccagtgga 480
tattttgaca tatatagtct ggaagataag tggcttacct gtaactcgtg taattggaag 540
tggttgtaat ctagactctg cccgtttccg ttacctaatt ggagaaaagt tgggtgtcca 600
ccccacaagc tgccatggtt ggattattgg agaacatggt gattctagtg tgcccttatg 660
gagtggggtg aatgttgctg gtgttgctct gaagactctg gaccctaaat taggaacgga 720
ttcagataag gaacactgga aaaatatcca taaacaagtt attcaaaggg attatatgga 780
ataaaagaag aactctttct cagtatccct tgtgtcttgg ggcggaatgg tgtctcagat 840
gttgtgaaaa ttaacttgaa ttctgaggag gaggcccttt tcaagaagag tgcagaaaca 900
ctttggaata ttcaaaagga tctaatattt taaattaaag ccttctaatg ttccactgtt 960
tggagaacag aagatagcag gctgtgtatt ttaaattttg aaagtatttt cattgatctt 1020
aaaaaataaa aacaaattgg agacctg 1047

<210> 6
<211> 332
<212> PRT
<213> Homo sapiens

<400> 6

Met Ser Thr Val Lys Glu Gln Leu Ile Glu Lys Leu Ile Glu Asp Asp
1               5                   10                  15

Glu Asn Ser Gln Cys Lys Ile Thr Ile Val Gly Thr Gly Ala Val Gly
            20                  25                  30

Met Ala Cys Ala Ile Ser Ile Leu Leu Lys Asp Leu Ala Asp Glu Leu
        35                  40                  45

Ala Leu Val Asp Val Ala Leu Asp Lys Leu Lys Gly Glu Met Met Asp
    50                  55                  60

Leu Gln His Gly Ser Leu Phe Phe Ser Thr Ser Lys Ile Thr Ser Gly
65                  70                  75                  80

Lys Asp Tyr Ser Val Ser Ala Asn Ser Arg Ile Val Ile Val Thr Ala
                85                  90                  95

Gly Ala Arg Gln Gln Glu Gly Glu Thr Arg Leu Ala Leu Val Gln Arg
            100                 105                 110

Asn Val Ala Ile Met Lys Ser Ile Ile Pro Ala Ile Val His Tyr Ser
        115                 120                 125

Pro Asp Cys Lys Ile Leu Val Val Ser Asn Pro Val Asp Ile Leu Thr
    130                 135                 140

Tyr Ile Val Trp Lys Ile Ser Gly Leu Pro Val Thr Arg Val Ile Gly
145                 150                 155                 160

Ser Gly Cys Asn Leu Asp Ser Ala Arg Phe Arg Tyr Leu Ile Gly Glu
                165                 170                 175

Lys Leu Gly Val His Pro Thr Ser Cys His Gly Trp Ile Ile Gly Glu
            180                 185                 190

His Gly Asp Ser Ser Val Pro Leu Trp Ser Gly Val Asn Val Ala Gly
        195                 200                 205

Val Ala Leu Lys Thr Leu Asp Pro Lys Leu Gly Thr Asp Ser Asp Lys
    210                 215                 220

Glu His Trp Lys Asn Ile His Lys Gln Val Ile Gln Ser Ala Tyr Glu
225                 230                 235                 240

Ile Ile Lys Leu Lys Gly Tyr Thr Ser Trp Ala Ile Gly Leu Ser Val
                245                 250                 255

Met Asp Leu Val Gly Ser Ile Leu Lys Asn Leu Arg Arg Val His Pro
            260                 265                 270

Val Ser Thr Met Val Lys Gly Leu Tyr Gly Ile Lys Glu Glu Leu Phe
        275                 280                 285

Leu Ser Ile Pro Cys Val Leu Gly Arg Asn Gly Val Ser Asp Val Val
    290                 295                 300

Lys Ile Asn Leu Asn Ser Glu Glu Glu Ala Leu Phe Lys Lys Ser Ala
305                 310                 315                 320

Glu Thr Leu Trp Asn Ile Gln Lys Asp Leu Ile Phe
                325                 330

<210>  7

<211>  51

<212>  PRT

<213>  Homo sapiens


<400>  7

Met Ser Thr Val Lys Glu Gln Leu Ile Glu Lys Leu Ile Glu Asp Asp
1               5                   10                  15

Glu Asn Ser Gln Cys Lys Ile Thr Ile Val Gly Thr Gly Ala Val Gly
            20                  25                  30

Met Ala Cys Ala Ile Ser Ile Leu Leu Lys Ile Thr Val Tyr Leu Gln
        35                  40                  45

Thr Pro Glu
    50

<210>  8

<211>  216

<212>  PRT

<213>  Homo sapiens


<400>  8

Met Lys Ser Ile Ile Pro Ala Ile Val His Tyr Ser Pro Asp Cys Lys
1               5                   10                  15

Ile Leu Val Val Ser Asn Pro Val Asp Ile Leu Thr Tyr Ile Val Trp
            20                  25                  30

Lys Ile Ser Gly Leu Pro Val Thr Arg Val Ile Gly Ser Gly Cys Asn
        35                  40                  45

Leu Asp Ser Ala Arg Phe Arg Tyr Leu Ile Gly Glu Lys Leu Gly Val
    50                  55                  60

His Pro Thr Ser Cys His Gly Trp Ile Ile Gly Glu His Gly Asp Ser
65                  70                  75                  80

Ser Val Pro Leu Trp Ser Gly Val Asn Val Ala Gly Val Ala Leu Lys
                85                  90                  95

Thr Leu Asp Pro Lys Leu Gly Thr Asp Ser Asp Lys Glu His Trp Lys
            100                 105                 110

Asn Ile His Lys Gln Val Ile Gln Ser Ala Tyr Glu Ile Ile Lys Leu
        115                 120                 125

Lys Gly Tyr Thr Ser Trp Ala Ile Gly Leu Ser Val Met Asp Leu Val
    130                 135                 140

Gly Ser Ile Leu Lys Asn Leu Arg Arg Val His Pro Val Ser Thr Met
145                 150                 155                 160

Val Lys Gly Leu Tyr Gly Ile Lys Glu Glu Leu Phe Leu Ser Ile Pro
                165                 170                 175

Cys Val Leu Gly Arg Asn Gly Val Ser Asp Val Val Lys Ile Asn Leu
            180                 185                 190

Asn Ser Glu Glu Glu Ala Leu Phe Lys Lys Ser Ala Glu Thr Leu Trp
        195                 200                 205

Asn Ile Gln Lys Asp Leu Ile Phe
    210                 215

<210>  9

<211>  45

<212>  PRT

<213>  Homo sapiens


<400>  9

Met Ser Thr Val Lys Glu Gln Leu Ile Glu Lys Leu Ile Glu Asp Asp
1               5                   10                  15

Glu Asn Ser Gln Cys Lys Ile Thr Ile Val Gly Thr Gly Ala Val Gly
            20                  25                  30

Met Ala Cys Ala Ile Ser Ile Leu Leu Lys Trp Ile Phe
        35                  40                  45

<210>  10

<211>  76

<212>  PRT
<213>  Homo sapiens

<400>  10

Met Asp Leu Val Gly Ser Ile Leu Lys Asn Leu Arg Arg Val His Pro
1               5                   10                  15

Val Ser Thr Met Val Lys Gly Leu Tyr Gly Ile Lys Glu Glu Leu Phe
            20                  25                  30

Leu Ser Ile Pro Cys Val Leu Gly Arg Asn Gly Val Ser Asp Val Val
        35                  40                  45

Lys Ile Asn Leu Asn Ser Glu Glu Glu Ala Leu Phe Lys Lys Ser Ala
    50                  55                  60

Glu Thr Leu Trp Asn Ile Gln Lys Asp Leu Ile Phe
65                  70                  75

<210>  11
<211>  109
<212>  PRT
<213>  Homo sapiens

<400>  11

Met Lys Ser Ile Ile Pro Ala Ile Val His Tyr Ser Pro Asp Cys Lys
1               5                   10                  15

Ile Leu Val Val Ser Asn Pro Val Asp Ile Leu Thr Tyr Ile Val Trp
            20                  25                  30

Lys Ile Ser Gly Leu Pro Val Thr Arg Val Ile Gly Ser Gly Cys Asn
        35                  40                  45

Leu Asp Ser Ala Arg Phe Arg Tyr Leu Ile Gly Glu Lys Leu Gly Val
    50                  55                  60

His Pro Thr Ser Cys His Gly Trp Ile Ile Gly Glu His Gly Asp Ser
65                  70                  75                  80

Ser Gly Ile Ile Trp Asn Lys Arg Arg Thr Leu Ser Gln Tyr Pro Leu
                85                  90                  95

Cys Leu Gly Ala Glu Trp Cys Leu Arg Cys Cys Glu Asn
            100                 105

<210>  12

<211>  66

<212>  PRT

<213>  Homo sapiens


<400>  12

Met Val Gly Leu Leu Glu Asn Met Val Ile Leu Val Gly Leu Tyr Gly
1               5                   10                  15

Ile Lys Glu Glu Leu Phe Leu Ser Ile Pro Cys Val Leu Gly Arg Asn
            20                  25                  30

Gly Val Ser Asp Val Val Lys Ile Asn Leu Asn Ser Glu Glu Glu Ala
        35                  40                  45

Leu Phe Lys Lys Ser Ala Glu Thr Leu Trp Asn Ile Gln Lys Asp Leu
    50                  55                  60

Ile Phe
65

<210>  13

<211>  241

<212>  PRT

<213>  Homo sapiens


<400>  13

Met Ser Thr Val Lys Glu Gln Leu Ile Glu Lys Leu Ile Glu Asp Asp
1               5                   10                  15

Glu Asn Ser Gln Cys Lys Ile Thr Ile Val Gly Thr Gly Ala Val Gly
            20                  25                  30

Met Ala Cys Ala Ile Ser Ile Leu Leu Lys Asp Leu Ala Asp Glu Leu
        35                  40                  45

Ala Leu Val Asp Val Ala Leu Asp Lys Leu Lys Gly Glu Met Met Asp
    50                  55                  60

Leu Gln His Gly Ser Leu Phe Phe Ser Thr Ser Lys Ile Thr Ser Gly
65                  70                  75                  80

Lys Asp Tyr Ser Val Ser Ala Asn Ser Arg Ile Val Ile Val Thr Ala
                85                  90                  95

Gly Ala Arg Gln Gln Glu Gly Glu Thr Arg Leu Ala Leu Val Gln Arg
            100                 105                 110

Asn Val Ala Ile Met Lys Ser Ile Ile Pro Ala Ile Val His Tyr Ser
        115                 120                 125

Pro Asp Cys Lys Ile Leu Val Val Ser Asn Pro Val Asp Ile Leu Thr
    130                 135                 140

Tyr Ile Val Trp Lys Ile Ser Gly Leu Pro Val Thr Arg Val Ile Gly
145                 150                 155                 160

Ser Gly Cys Asn Leu Asp Ser Ala Arg Phe Arg Tyr Leu Ile Gly Glu
                165                 170                 175

Lys Leu Gly Val His Pro Thr Ser Cys His Gly Trp Ile Ile Gly Glu
            180                 185                 190

His Gly Asp Ser Ser Val Pro Leu Trp Ser Gly Val Asn Val Ala Gly
        195                 200                 205

Val Ala Leu Lys Thr Leu Asp Pro Lys Leu Gly Thr Asp Ser Asp Lys
    210                 215                 220

Glu His Trp Lys Asn Ile His Lys Gln Val Ile Gln Arg Asp Tyr Met
225                 230                 235                 240

Glu

<210> 14

<211> 23

<212> PRT

<213> Homo sapiens

<400> 14

Gly Ala Val Gly Met Ala Cys Ala Ile Ser Ile Leu Leu Lys Ile Thr
1               5                   10                  15

Val Tyr Leu Gln Thr Pro Glu
            20

<210> 15

<211> 17

<212> PRT

<213> Homo sapiens

<400>  15

Gly Ala Val Gly Met Ala Cys Ala Ile Ser Ile Leu Leu Lys Trp Ile
1               5                   10                  15

Phe


<210>  16

<211>  39

<212>  PRT

<213>  Homo sapiens


<400>  16

Gly Trp Ile Ile Gly Glu His Gly Asp Ser Ser Gly Ile Ile Trp Asn
1               5                   10                  15

Lys Arg Arg Thr Leu Ser Gln Tyr Pro Leu Cys Leu Gly Ala Glu Trp
            20                  25                  30

Cys Leu Arg Cys Cys Glu Asn
        35

<210>  17

<211>  23

<212>  PRT

<213>  Homo sapiens


<400>  17

Met Val Gly Leu Leu Glu Asn Met Val Ile Leu Val Gly Leu Tyr Gly
1               5                   10                  15

Ile Lys Glu Glu Leu Phe Leu
            20

<210>  18

<211>  17

<212>  PRT

<213>  Homo sapiens


<400>  18

Glu His Trp Lys Asn Ile His Lys Gln Val Ile Gln Arg Asp Tyr Met
1               5                   10                  15

Glu

<210>  19
<211>  2168
<212>  DNA
<213>  Homo sapiens

<400>  19
gaatccgcgg ggagggcaca acagctgcta cctgaacagt ttctgaccca acagttaccc      60
agcgccggac tcgctgcgcc ccggcggctc tagggacccc cggcgcctac acttagctcc     120
gcgcccgaga gaatgttgga ccgacgacac aagacctcag acttgtgtta ttctagcagc     180
tgaacacacc ccaggctctt ctgaccggca gtggctctgg aagcagtctg gtgtatagag     240
ttatggattc actaccagat tctactgtat gctcttgaca actatgacca caatggtcca     300
cccacaaatg aattatcagg agtgaaccca gaggcacgta tgaatgaaag tcctgatccg     360
actgacctgg cgggagtcat cattgagctc ggccccaatg acagtccaca gacaagtgaa     420
tttaaaggag caaccgagga ggcacctgcg aaagaaagcc cacacacaag tgaatttaaa     480
ggagcagccc gggtgtcacc tatcagtgaa agtgtgttag cacgactttc caagtttgaa     540
gttgaagatg ctgaaaatgt tgcttcatat gacagcaaga ttaagaaaat tgtgcattca     600
attgtatcat cctttgcatt tggactattt ggagttttcc tggtcttact ggatgtcact     660
ctcatccttg ccgacctaat tttcactgac agcaaacttt atattccttt ggagtatcgt     720
tctatttctc tagctattgc cttatttttt ctcatggatg ttcttcttcg agtatttgta     780
gaaaggagac agcagtattt ttctgactta tttaacattt tagatactgc cattattgtg     840
attcttctgc tggttgatgt cgtttacatt ttttttgaca ttaagttgct taggaatatt     900
cccagatgga cacatttact tcgacttcta cgacttatta ttctgttaag aatttttcat     960
ctgtttcatc aaaaaagaca acttgaaaag ctgataagaa ggcgggtttc agaaaacaaa    1020
aggcgataca caagggatgg atttgaccta gacctcactt acgttacaga acgtattatt    1080
gctatgtcat ttccatcttc tggaaggcag tctttctata gaaatccaat caaggaagtt    1140
gtgcggtttc tagataagaa acaccgaaac cactatcgag tctacaatct atgcagtgaa    1200
agagcttacg atcctaagca cttccataat agggtcgtta gaatcatgat tgatgatcat    1260
aatgtcccca ctctacatca gatggtggtt ttcaccaagg aagtaaatga gtggatggct    1320
caagatcttg aaaacatcgt agcgattcac tgtaaaggag gcacagatag aacaggaact    1380
atggtttgtg ccttccttat tgcctctgaa atatgttcaa ctgcaaagga aagcctgtat    1440

tattttggag aaaggcgaac agataaaacc cacagcgaaa aatttcaggg agtagaaact 1500
ccttctcaga agagatatgt tgcatatttt gcacaagtga aacatctcta caactggaat 1560
ctccctccaa gacggatact ctttataaaa cacttcatta tttattcgat tcctcgttat 1620
gtacgtgatc taaaaatcca aatagaaatg gagaaaaagg ttgtcttttc cactatttca 1680
ttaggaaaat gttcggtact tgataacatt acaacagaca aaatattaat tgatgtattc 1740
gacggtccac ctctgtatga tgatgtgaaa gtgcagtttt tctattcgaa tcttcctaca 1800
tactatgaca attgctcatt ttacttctgg ttgcacacat cttttattga aaataacagg 1860
ctttatctac caaaaaatga attggataat ctacataaac aaaaagcacg gagaatttat 1920
ccatcagatt ttgccgtgga gatacttttt ggcgagaaaa tgacttccag tgatgttgta 1980
gctggatccg attaagtata gctccccctt ccccttctgg gaaagaatta tgttctttcc 2040
aaccctgcca catgttcata tatcctaaat ctatcctaaa tgttcccttg aagtatttat 2100
ttatgtttat atatgtttat acatgttctt caataaatct attacatata tataaaaaaa 2160
aaaaaaaa 2168

<210> 20
<211> 2114
<212> DNA
<213> Homo sapiens

<400> 20
gaatccgcgg ggagggcaca acagctgcta cctgaacagt ttctgaccca acagttaccc 60
agcgccggac tcgctgcgcc ccggcggctc tagggacccc cggcgcctac acttagctcc 120
gcgcccgaga gaatgttgga ccgacgacac aagacctcag acttgtgtta ttctagcagc 180
tgaacacacc ccaggctctt ctgaccggca gtggctctgg aagcagtctg gtgtatagag 240
ttatggattc actaccagat tctactgtat gctcttgaca actatgacca caatggtcca 300
cccacaaatg aattatcagg agtgaaccca gaggcacgta tgaatgaaag tcctgatccg 360
actgacctgg cgggagtcat cattgagctc ggccccaatg acagtccaca gacaagtgaa 420
tttaaaggag caaccgagga ggcacctgcg aaagaaagtg tgttagcacg actttccaag 480
tttgaagttg aagatgctga aaatgttgct tcatatgaca gcaagattaa gaaaattgtg 540
cattcaattg tatcatcctt tgcatttgga ctatttggag ttttcctggt cttactggat 600
gtcactctca tccttgccga cctaattttc actgacagca aactttatat tcctttggag 660
tatcgttcta tttctctagc tattgcctta ttttttctca tggatgttct tcttcgagta 720
tttgtagaaa ggagacagca gtatttttct gacttattta acattttaga tactgccatt 780
attgtgattc ttctgctggt tgatgtcgtt tacatttttt ttgacattaa gttgcttagg 840
aatattccca gatggacaca tttacttcga cttctacgac ttattattct gttaagaatt 900

tttcatctgt ttcatcaaaa aagacaactt gaaaagctga taagaaggcg ggtttcagaa 960
aacaaaaggc gatacacaag ggatggattt gacctagacc tcacttacgt tacagaacgt 1020
attattgcta tgtcatttcc atcttctgga aggcagtctt tctatagaaa tccaatcaag 1080
gaagttgtgc ggtttctaga taagaaacac cgaaaccact atcgagtcta caatctatgc 1140
agtgaaagag cttacgatcc taagcacttc cataataggg tcgttagaat catgattgat 1200
gatcataatg tccccactct acatcagatg gtggttttca ccaaggaagt aaatgagtgg 1260
atggctcaag atcttgaaaa catcgtagcg attcactgta aaggaggcac agatagaaca 1320
ggaactatgg tttgtgcctt ccttattgcc tctgaaatat gttcaactgc aaaggaaagc 1380
ctgtattatt ttggagaaag gcgaacagat aaaacccaca gcgaaaaatt tcagggagta 1440
gaaactcctt ctcagaagag atatgttgca tattttgcac aagtgaaaca tctctacaac 1500
tggaatctcc ctccaagacg gatactcttt ataaaacact tcattattta ttcgattcct 1560
cgttatgtac gtgatctaaa aatccaaata gaaatggaga aaaaggttgt cttttccact 1620
atttcattag gaaaatgttc ggtacttgat aacattacaa cagacaaaat attaattgat 1680
gtattcgacg gtccacctct gtatgatgat gtgaaagtgc agtttttcta ttcgaatctt 1740
cctacatact atgacaattg ctcattttac ttctggttgc acacatcttt tattgaaaat 1800
aacaggcttt atctaccaaa aaatgaattg gataatctac ataaacaaaa agcacggaga 1860
atttatccat cagattttgc cgtggagata ctttttggcg agaaaatgac ttccagtgat 1920
gttgtagctg gatccgatta agtatagctc ccccttcccc ttctgggaaa gaattatgtt 1980
ctttccaacc ctgccacatg ttcatatatc ctaaatctat cctaaatgtt cccttgaagt 2040
atttatttat gtttatatat gtttatacat gttcttcaat aaatctatta catatatata 2100
aaaaaaaaaa aaaa 2114

<210> 21
<211> 2222
<212> DNA
<213> Homo sapiens

<400> 21
gaatccgcgg ggagggcaca acagctgcta cctgaacagt ttctgaccca acagttaccc 60
agcgccggac tcgctgcgcc ccggcggctc tagggacccc cggcgcctac acttagctcc 120
gcgcccgaga gaatgttgga ccgacgacac aagacctcag acttgtgtta ttctagcagc 180
tgaacacacc ccaggctctt ctgaccggca gtggctctgg aagcagtctg gtgtatagag 240
ttatggattc actaccagat tctactgtat gctcttgaca actatgacca caatggtcca 300
cccacaaatg aattatcagg agtgaaccca gaggcacgta tgaatgaaag tcctgatccg 360
actgacctgg cgggagtcat cattgagctc ggccccaatg acagtccaca gacaagtgaa 420

tttaaaggag caaccgagga ggcacctgcg aaagaaagcc cacacacaag tgaatttaaa 480
ggagcagccc gggtgtcacc tatcagtgaa agtgtgttag cacgactttc caagtttgaa 540
gttgaagatg ctgaaaatgt tgcttcatat gacagcaaga ttaagaaaat tgtgcattca 600
attgtatcat cctttgcatt tggactattt ggagttttcc tggtcttact ggatgtcact 660
ctcatccttg ccgacctaat tttcactgac agcaaacttt atattccttt ggagtatcgt 720
tctatttctc tagctattgc cttatttttt ctcatggatg ttcttcttcg agtatttgta 780
gaaaggagac agcagtattt ttctgactta tttaacattt tagatactgc cattattgtg 840
attcttctgc tggttgatgt cgtttacatt ttttttgaca ttaagttgct taggaatatt 900
cccagatgga cacatttact tcgacttcta cgacttatta ttctgttaag aatttttcat 960
ctgtttcatc aaaaaagaca acttgaaaag ctgataagaa ggcgggtttc agaaaacaaa 1020
aggcgataca caagggatgg atttgaccta gacctcactt acgttacaga acgtattatt 1080
gctatgtcat ttccatcttc tggaaggcag tctttctata gaaatccaat caaggaagtt 1140
gtgcggtttc tagataagaa acaccgaaac cactatcgag tctacaatct atgcagtatg 1200
tacattactc tatattgtgc tactgtagat agaaaacaga ttactgcacg tgaaagagct 1260
tacgatccta agcacttcca taatagggtc gttagaatca tgattgatga tcataatgtc 1320
cccactctac atcagatggt ggttttcacc aaggaagtaa atgagtggat ggctcaagat 1380
cttgaaaaca tcgtagcgat tcactgtaaa ggaggcacag atagaacagg aactatggtt 1440
tgtgccttcc ttattgcctc tgaaatatgt tcaactgcaa aggaaagcct gtattatttt 1500
ggagaaaggc gaacagataa aacccacagc gaaaaatttc agggagtaga aactccttct 1560
cagaagagat atgttgcata ttttgcacaa gtgaaacatc tctacaactg gaatctccct 1620
ccaagacgga tactctttat aaaacacttc attatttatt cgattcctcg ttatgtacgt 1680
gatctaaaaa tccaaataga aatggagaaa aaggttgtct tttccactat ttcattagga 1740
aaatgttcgg tacttgataa cattacaaca gacaaaatat taattgatgt attcgacggt 1800
ccacctctgt atgatgatgt gaaagtgcag tttttctatt cgaatcttcc tacatactat 1860
gacaattgct cattttactt ctggttgcac acatctttta ttgaaaataa caggctttat 1920
ctaccaaaaa atgaattgga taatctacat aaacaaaaag cacggagaat ttatccatca 1980
gattttgccg tggagatact ttttggcgag aaaatgactt ccagtgatgt tgtagctgga 2040
tccgattaag tatagctccc ccttcccctt ctgggaaaga attatgttct ttccaaccct 2100
gccacatgtt catatatcct aaatctatcc taaatgttcc cttgaagtat ttatttatgt 2160
ttatatatgt ttatacatgt tcttcaataa atctattaca tatatataaa aaaaaaaaaa 2220
aa 2222

<210> 22
<211> 551
<212> PRT

<213>  Homo sapiens

<400>  22

Met Asn Glu Ser Pro Asp Pro Thr Asp Leu Ala Gly Val Ile Ile Glu
1               5                   10                  15

Leu Gly Pro Asn Asp Ser Pro Gln Thr Ser Glu Phe Lys Gly Ala Thr
            20                  25                  30

Glu Glu Ala Pro Ala Lys Glu Ser Pro His Thr Ser Glu Phe Lys Gly
        35                  40                  45

Ala Ala Arg Val Ser Pro Ile Ser Glu Ser Val Leu Ala Arg Leu Ser
    50                  55                  60

Lys Phe Glu Val Glu Asp Ala Glu Asn Val Ala Ser Tyr Asp Ser Lys
65                  70                  75                  80

Ile Lys Lys Ile Val His Ser Ile Val Ser Ser Phe Ala Phe Gly Leu
                85                  90                  95

Phe Gly Val Phe Leu Val Leu Leu Asp Val Thr Leu Ile Leu Ala Asp
            100                 105                 110

Leu Ile Phe Thr Asp Ser Lys Leu Tyr Ile Pro Leu Glu Tyr Arg Ser
        115                 120                 125

Ile Ser Leu Ala Ile Ala Leu Phe Phe Leu Met Asp Val Leu Leu Arg
    130                 135                 140

Val Phe Val Glu Arg Arg Gln Gln Tyr Phe Ser Asp Leu Phe Asn Ile
145                 150                 155                 160

Leu Asp Thr Ala Ile Ile Val Ile Leu Leu Leu Val Asp Val Val Tyr
                165                 170                 175

Ile Phe Phe Asp Ile Lys Leu Leu Arg Asn Ile Pro Arg Trp Thr His
            180                 185                 190

Leu Leu Arg Leu Leu Arg Leu Ile Ile Leu Leu Arg Ile Phe His Leu
        195                 200                 205

Phe His Gln Lys Arg Gln Leu Glu Lys Leu Ile Arg Arg Arg Val Ser
    210                 215                 220

Glu Asn Lys Arg Arg Tyr Thr Arg Asp Gly Phe Asp Leu Asp Leu Thr
225                 230                 235                 240

Tyr Val Thr Glu Arg Ile Ile Ala Met Ser Phe Pro Ser Ser Gly Arg

                245                 250                 255

Gln Ser Phe Tyr Arg Asn Pro Ile Lys Glu Val Val Arg Phe Leu Asp
            260                 265                 270

Lys Lys His Arg Asn His Tyr Arg Val Tyr Asn Leu Cys Ser Glu Arg
        275                 280                 285

Ala Tyr Asp Pro Lys His Phe His Asn Arg Val Val Arg Ile Met Ile
    290                 295                 300

Asp Asp His Asn Val Pro Thr Leu His Gln Met Val Val Phe Thr Lys
305                 310                 315                 320

Glu Val Asn Glu Trp Met Ala Gln Asp Leu Glu Asn Ile Val Ala Ile
                325                 330                 335

His Cys Lys Gly Gly Thr Asp Arg Thr Gly Thr Met Val Cys Ala Phe
            340                 345                 350

Leu Ile Ala Ser Glu Ile Cys Ser Thr Ala Lys Glu Ser Leu Tyr Tyr
        355                 360                 365

Phe Gly Glu Arg Arg Thr Asp Lys Thr His Ser Glu Lys Phe Gln Gly
    370                 375                 380

Val Glu Thr Pro Ser Gln Lys Arg Tyr Val Ala Tyr Phe Ala Gln Val
385                 390                 395                 400

Lys His Leu Tyr Asn Trp Asn Leu Pro Pro Arg Arg Ile Leu Phe Ile
                405                 410                 415

Lys His Phe Ile Ile Tyr Ser Ile Pro Arg Tyr Val Arg Asp Leu Lys
            420                 425                 430

Ile Gln Ile Glu Met Glu Lys Lys Val Val Phe Ser Thr Ile Ser Leu
        435                 440                 445

Gly Lys Cys Ser Val Leu Asp Asn Ile Thr Thr Asp Lys Ile Leu Ile
    450                 455                 460

Asp Val Phe Asp Gly Pro Pro Leu Tyr Asp Asp Val Lys Val Gln Phe
465                 470                 475                 480

Phe Tyr Ser Asn Leu Pro Thr Tyr Tyr Asp Asn Cys Ser Phe Tyr Phe
                485                 490                 495

Trp Leu His Thr Ser Phe Ile Glu Asn Asn Arg Leu Tyr Leu Pro Lys
            500                 505                 510

Asn Glu Leu Asp Asn Leu His Lys Gln Lys Ala Arg Arg Ile Tyr Pro

515                    520                    525

Ser Asp Phe Ala Val Glu Ile Leu Phe Gly Glu Lys Met Thr Ser Ser
    530                    535                    540

Asp Val Val Ala Gly Ser Asp
545                    550

<210>  23

<211>  533

<212>  PRT

<213>  Homo sapiens

<400>  23

<210>  24

<211>  569

<212>  PRT

<213>  Homo sapiens

<400>  24

Met Asn Glu Ser Pro Asp Pro Thr Asp Leu Ala Gly Val Ile Ile Glu
1               5                   10                  15

Leu Gly Pro Asn Asp Ser Pro Gln Thr Ser Glu Phe Lys Gly Ala Thr
            20                  25                  30

Glu Glu Ala Pro Ala Lys Glu Ser Pro His Thr Ser Glu Phe Lys Gly
        35                  40                  45

Ala Ala Arg Val Ser Pro Ile Ser Glu Ser Val Leu Ala Arg Leu Ser
    50                  55                  60

Lys Phe Glu Val Glu Asp Ala Glu Asn Val Ala Ser Tyr Asp Ser Lys
65                  70                  75                  80

Ile Lys Lys Ile Val His Ser Ile Val Ser Ser Phe Ala Phe Gly Leu
                85                  90                  95

Phe Gly Val Phe Leu Val Leu Leu Asp Val Thr Leu Ile Leu Ala Asp
            100                 105                 110

Leu Ile Phe Thr Asp Ser Lys Leu Tyr Ile Pro Leu Glu Tyr Arg Ser
        115                 120                 125

Ile Ser Leu Ala Ile Ala Leu Phe Phe Leu Met Asp Val Leu Leu Arg

130                 135                 140

Val Phe Val Glu Arg Arg Gln Gln Tyr Phe Ser Asp Leu Phe Asn Ile
145                 150                 155                 160

Leu Asp Thr Ala Ile Ile Val Ile Leu Leu Leu Val Asp Val Val Tyr
                165                 170                 175

Ile Phe Phe Asp Ile Lys Leu Leu Arg Asn Ile Pro Arg Trp Thr His
            180                 185                 190

Leu Leu Arg Leu Leu Arg Leu Ile Ile Leu Leu Arg Ile Phe His Leu
        195                 200                 205

Phe His Gln Lys Arg Gln Leu Glu Lys Leu Ile Arg Arg Arg Val Ser
    210                 215                 220

Glu Asn Lys Arg Arg Tyr Thr Arg Asp Gly Phe Asp Leu Asp Leu Thr
225                 230                 235                 240

Tyr Val Thr Glu Arg Ile Ile Ala Met Ser Phe Pro Ser Ser Gly Arg
                245                 250                 255

Gln Ser Phe Tyr Arg Asn Pro Ile Lys Glu Val Val Arg Phe Leu Asp
            260                 265                 270

Lys Lys His Arg Asn His Tyr Arg Val Tyr Asn Leu Cys Ser Met Tyr
        275                 280                 285

Ile Thr Leu Tyr Cys Ala Thr Val Asp Arg Lys Gln Ile Thr Ala Arg
    290                 295                 300

Glu Arg Ala Tyr Asp Pro Lys His Phe His Asn Arg Val Val Arg Ile
305                 310                 315                 320

Met Ile Asp Asp His Asn Val Pro Thr Leu His Gln Met Val Val Phe
                325                 330                 335

Thr Lys Glu Val Asn Glu Trp Met Ala Gln Asp Leu Glu Asn Ile Val
            340                 345                 350

Ala Ile His Cys Lys Gly Gly Thr Asp Arg Thr Gly Thr Met Val Cys
        355                 360                 365

Ala Phe Leu Ile Ala Ser Glu Ile Cys Ser Thr Ala Lys Glu Ser Leu
    370                 375                 380

Tyr Tyr Phe Gly Glu Arg Arg Thr Asp Lys Thr His Ser Glu Lys Phe
385                 390                 395                 400

Gln Gly Val Glu Thr Pro Ser Gln Lys Arg Tyr Val Ala Tyr Phe Ala

405                 410                 415

Gln Val Lys His Leu Tyr Asn Trp Asn Leu Pro Pro Arg Arg Ile Leu
            420                 425                 430

Phe Ile Lys His Phe Ile Ile Tyr Ser Ile Pro Arg Tyr Val Arg Asp
        435                 440                 445

Leu Lys Ile Gln Ile Glu Met Glu Lys Lys Val Val Phe Ser Thr Ile
    450                 455                 460

Ser Leu Gly Lys Cys Ser Val Leu Asp Asn Ile Thr Thr Asp Lys Ile
465                 470                 475                 480

Leu Ile Asp Val Phe Asp Gly Pro Pro Leu Tyr Asp Asp Val Lys Val
                485                 490                 495

Gln Phe Phe Tyr Ser Asn Leu Pro Thr Tyr Tyr Asp Asn Cys Ser Phe
            500                 505                 510

Tyr Phe Trp Leu His Thr Ser Phe Ile Glu Asn Asn Arg Leu Tyr Leu
        515                 520                 525

Pro Lys Asn Glu Leu Asp Asn Leu His Lys Gln Lys Ala Arg Arg Ile
    530                 535                 540

Tyr Pro Ser Asp Phe Ala Val Glu Ile Leu Phe Gly Glu Lys Met Thr
545                 550                 555                 560

Ser Ser Asp Val Val Ala Gly Ser Asp
                565

<210>  25

<211>  21

<212>  DNA

<213>  Künstliche Sequenz

<220>
<223>  Beschreibung der künstlichen Sequenz: Oligonukleotid


<400>  25
tgccgtaggc atggcttgtg c                                                21

<210>  26

<211>  21

<212>  DNA

<213>  Künstliche Sequenz

<220>
<223>  Beschreibung der künstlichen Sequenz: Oligonukleotid

<400>  26
caacatctga gacaccattc c                                                21

<210>  27
<211>  21
<212>  DNA
<213>  Künstliche Sequenz

<220>
<223>  Beschreibung der künstlichen Sequenz: Oligonukleotid

<400>  27
tggatgtcac tctcatcctt g                                                21

<210>  28
<211>  21
<212>  DNA
<213>  Künstliche Sequenz

<220>
<223>  Beschreibung der künstlichen Sequenz: Oligonukleotid

<400>  28
ccatagttcc tgttctatct g                                                21

<210>  29
<211>  2192
<212>  DNA
<213>  Homo sapiens

<400>  29
agctcagctg ggagcgcaga ggctcacgcc tgtaatccca tcatttgctt aggtctgatc      60
aatctgctcc acacaatttc tcagtgatcc tctgcatctc tgcctacaag ggcctccctg     120
acacccaagt tcatattgct cagaaacagt gaacttgagt ttttcgtttt accttgatct     180
ctctctgaca aagaaatcca gatgatgcaa cacctgatga agacaataca tggaaaatga     240
cagtcttgga aataactttg gctgtcatcc tgactctact gggacttgcc atcctggcta     300
ttttgttaac aagatgggca cgacgtaagc aaagtgaaat gtatatctcc agatacagtt     360
cagaacaaag tgctagactt ctggactatg aggatggtag aggatcccga catgcatatc     420

aacacaaagt gacacttcat atgataaccg agagagatcc aaaaagagat tacacaccat 480
caaccaactc tctagcactg tctcgatcaa gtattgcttt acctcaagga tccatgagta 540
gtataaaatg tttacaaaca actgaagaac ctccttccag aactgcagga gccatgatgc 600
aattcacagc cctattcccg gagctacagg acctatcaag ctctctcaaa aaaccattgt 660
gcaaactcca ggacctattg tacaatatct ggatccaatg tcagatcgca tctcacacaa 720
tcactggtca ccttcagcac ccgcggtcac ccatggcacc cataataatt tcacagagaa 780
ccgcaagtca gctggcagca cctataagaa tacctcaagt tcacactatg gacagttctg 840
gaaaaatcac actgactcct gtggttatat taacaggtta catggacgaa gaacttcgaa 900
aaaaatcttg ttccaaaatc cagattctaa aatgtggagg cactgcaagg tctcagatag 960
ccgagaagaa aacaaggaag caactaaaga atgacatcat atttacgaat tctgtagaat 1020
ccttgaaatc agcacacata aaggagccag aaagagaagg aaaaggcact gatttagaga 1080
aagacaaaat aggaatggag gtcaaggtag acagtgacgc tggaatacca aaaagacagg 1140
aaacccaact aaaaatcagt gaagatgagt ataccacaag gacagggagc ccaaataaag 1200
aaaagtgtgt cagatgtacc aagaggacag gagtccaagt aaagaagagt gagtcaggtg 1260
tcccaaaagg acaagaagcc caagtaacga agagtgggtt ggttgtactg aaaggacagg 1320
aagcccaggt agagaagagt gagatgggtg tgccaagaag acaggaatcc caagtaaaga 1380
agagtcagtc tggtgtctca aagggacagg aagcccaggt aaagaagagg gagtcagttg 1440
tactgaaagg acaggaagcc caggtagaga agagtgagtt gaaggtacca aaaggacaag 1500
aaggccaagt agagaagact gaggcagatg tgccaaagga acaagaggtc caagaaaaga 1560
agagtgaggc aggtgtactg aaaggaccag aatcccaagt aaagaacact gaggtgagtg 1620
taccagaaac actggaatcc caagtaaaga agagtgagtc aggtgtacta aaaggacagg 1680
aagcccaaga aaagaaggag agttttgagg ataaaggaaa taatgataaa gaaaaggaga 1740
gagatgcaga gaaagatcca aataaaaaag aaaaaggtga caaaaacaca aaaggtgaca 1800
aaggaaagga caaagttaaa ggaaagagag aatcagaaat caatggtgaa aaatcaaaag 1860
gctcgaaaag gcgaaggcaa atacaggaag gaagtacaac aaaaaagtgg aagagtaagg 1920
ataaattttt taaaggccca taagacaagt gattattatg attcccatac tccagataca 1980
aaccatatcc cagccattgc ctaaacagat tacaattata aaatcccttt catcttcata 2040
tcacagtttc tgctcttcag aagtttcacc ctttttaatc tctcagccac aaacctcagt 2100
tccaatattg ttataagtta agacgtatat gattccgtca agaaagactg gatactttct 2160
gaagtaaaac attttaatta aagaaaaaaa aa 2192

<210> 30
<211> 568
<212> PRT
<213> Homo sapiens

<400> 30

Met Thr Val Leu Glu Ile Thr Leu Ala Val Ile Leu Thr Leu Leu Gly
1               5                   10                  15

Leu Ala Ile Leu Ala Ile Leu Leu Thr Arg Trp Ala Arg Arg Lys Gln
            20                  25                  30

Ser Glu Met Tyr Ile Ser Arg Tyr Ser Ser Glu Gln Ser Ala Arg Leu
        35                  40                  45

Leu Asp Tyr Glu Asp Gly Arg Gly Ser Arg His Ala Tyr Gln His Lys
    50                  55                  60

Val Thr Leu His Met Ile Thr Glu Arg Asp Pro Lys Arg Asp Tyr Thr
65                  70                  75                  80

Pro Ser Thr Asn Ser Leu Ala Leu Ser Arg Ser Ser Ile Ala Leu Pro
                85                  90                  95

Gln Gly Ser Met Ser Ser Ile Lys Cys Leu Gln Thr Thr Glu Glu Pro
            100                 105                 110

Pro Ser Arg Thr Ala Gly Ala Met Met Gln Phe Thr Ala Leu Phe Pro
        115                 120                 125

Glu Leu Gln Asp Leu Ser Ser Ser Leu Lys Lys Pro Leu Cys Lys Leu
    130                 135                 140

Gln Asp Leu Leu Tyr Asn Ile Trp Ile Gln Cys Gln Ile Ala Ser His
145                 150                 155                 160

Thr Ile Thr Gly His Leu Gln His Pro Arg Ser Pro Met Ala Pro Ile
                165                 170                 175

Ile Ile Ser Gln Arg Thr Ala Ser Gln Leu Ala Ala Pro Ile Arg Ile
            180                 185                 190

Pro Gln Val His Thr Met Asp Ser Ser Gly Lys Ile Thr Leu Thr Pro
        195                 200                 205

Val Val Ile Leu Thr Gly Tyr Met Asp Glu Glu Leu Arg Lys Lys Ser
    210                 215                 220

Cys Ser Lys Ile Gln Ile Leu Lys Cys Gly Gly Thr Ala Arg Ser Gln
225                 230                 235                 240

Ile Ala Glu Lys Lys Thr Arg Lys Gln Leu Lys Asn Asp Ile Ile Phe
                245                 250                 255

Thr Asn Ser Val Glu Ser Leu Lys Ser Ala His Ile Lys Glu Pro Glu
            260                 265                 270

Arg Glu Gly Lys Gly Thr Asp Leu Glu Lys Asp Lys Ile Gly Met Glu
        275                 280                 285

Val Lys Val Asp Ser Asp Ala Gly Ile Pro Lys Arg Gln Glu Thr Gln
    290                 295                 300

Leu Lys Ile Ser Glu Asp Glu Tyr Thr Thr Arg Thr Gly Ser Pro Asn
305                 310                 315                 320

Lys Glu Lys Cys Val Arg Cys Thr Lys Arg Thr Gly Val Gln Val Lys
                325                 330                 335

Lys Ser Glu Ser Gly Val Pro Lys Gly Gln Glu Ala Gln Val Thr Lys
            340                 345                 350

Ser Gly Leu Val Val Leu Lys Gly Gln Glu Ala Gln Val Glu Lys Ser
        355                 360                 365

Glu Met Gly Val Pro Arg Arg Gln Glu Ser Gln Val Lys Lys Ser Gln
    370                 375                 380

Ser Gly Val Ser Lys Gly Gln Glu Ala Gln Val Lys Lys Arg Glu Ser
385                 390                 395                 400

Val Val Leu Lys Gly Gln Glu Ala Gln Val Glu Lys Ser Glu Leu Lys
                405                 410                 415

Val Pro Lys Gly Gln Glu Gly Gln Val Glu Lys Thr Glu Ala Asp Val
            420                 425                 430

Pro Lys Glu Gln Glu Val Gln Glu Lys Lys Ser Glu Ala Gly Val Leu
        435                 440                 445

Lys Gly Pro Glu Ser Gln Val Lys Asn Thr Glu Val Ser Val Pro Glu
    450                 455                 460

Thr Leu Glu Ser Gln Val Lys Lys Ser Glu Ser Gly Val Leu Lys Gly
465                 470                 475                 480

Gln Glu Ala Gln Glu Lys Lys Glu Ser Phe Glu Asp Lys Gly Asn Asn
                485                 490                 495

Asp Lys Glu Lys Glu Arg Asp Ala Glu Lys Asp Pro Asn Lys Lys Glu
            500                 505                 510

Lys Gly Asp Lys Asn Thr Lys Gly Asp Lys Gly Lys Asp Lys Val Lys
        515                 520                 525

Gly Lys Arg Glu Ser Glu Ile Asn Gly Glu Lys Ser Lys Gly Ser Lys
    530                 535                 540

Arg Arg Arg Gln Ile Gln Glu Gly Ser Thr Thr Lys Lys Trp Lys Ser
545                 550                 555                 560

Lys Asp Lys Phe Phe Lys Gly Pro
                565

<210>  31

<211>  1686

<212>  DNA

<213>  Homo sapiens

<400>  31
atgacagtct tggaaataac tttggctgtc atcctgactc tactgggact tgccatcctg      60

gctattttgt taacaagatg ggcacgatgt aagcaaagtg aaatgtatat ctccagatac     120

agttcagaac aaagtgctag acttctggac tatgaggatg gtagaggatc ccgacatgca     180

tattcaacac aaagtgacac ttcatatgat aaccgagaga gatccaaaag agattacaca     240

ccatcaacca actctctagc actgtctcga tcaagtattg ctttacctca aggatccatg     300

agtagtataa aatgtttaca aacaactgaa gaacctcctt ccagaactgc aggagccatg     360

atgcaattca cagcccctat tcccggagct acaggaccta tcaagctctc tcaaaaaacc     420

attgtgcaaa ctccaggacc tattgtacaa tatcctggat ccaatgctgg tccaccttca     480

gcaccccgcg gtccacccat ggcacccata ataatttcac agagaaccgc aagtcagctg     540

gcagcaccta taataatttc gcagagaact gcaagaatac ctcaagttca cactatggac     600

agttctggaa aaatcacact gactcctgtg gttatattaa caggttacat ggatgaagaa     660

cttgcaaaaa aatcttgttc caaaatccag attctaaaat gtggaggcac tgcaaggtct     720

cagaatagcc gagaagaaaa caaggaagca ctaaagaatg acatcatatt tacgaattct     780

gtagaatcct tgaaatcagc acacataaag gagccagaaa gagaaggaaa aggcactgat     840

ttagagaaag acaaaatagg aatggaggtc aaggtagaca gtgacgctgg aataccaaaa     900

agacaggaaa cccaactaaa aatcagtgag atgagtatac cacaaggaca gggagcccaa     960

ataaagaaaa gtgtgtcaga tgtaccaaga ggacaggagt cccaagtaaa gaagagtgag    1020

tcaggtgtcc caaaaggaca agaagcccaa gtaacgaaga gtgggttggt tgtactgaaa    1080

ggacaggaag cccaggtaga gaagagtgag atgggtgtgc caagaagaca ggaatcccaa    1140

gtaaagaaga gtcagtctgg tgtctcaaag ggacaggaag cccaggtaaa gaagagggag    1200

tcagttgtac tgaaaggaca ggaagcccag gtagagaaga gtgagttgaa ggtaccaaaa    1260

ggacaagaag gccaagtaga gaagactgag gcagatgtgc caaaggaaca agaggtccaa    1320

gaaaagaaga gtgaggcagg tgtactgaaa ggaccagaat cccaagtaaa gaacactgag 1380
gtgagtgtac cagaaacact ggaatcccaa gtaaagaaga gtgagtcagg tgtactaaaa 1440
ggacaggaag cccaagaaaa gaaggagagt tttgaggata aaggaaataa tgataaagaa 1500
aaggagagag atgcagagaa agatccaaat aaaaaagaaa aaggtgacaa aaacacaaaa 1560
ggtgacaaag gaaaggacaa agttaaagga aagagagaat cagaaatcaa tggtgaaaaa 1620
tcaaaaggct cgaaaagggc gaaggcaaat acaggaagga agtacaacaa aaaagtggaa 1680
gagtaa 1686

<210> 32
<211> 1710
<212> DNA
<213> Homo sapiens

<400> 32
atgacagtct tggaaataac tttggctgtc atcctgactc tactgggact tgccatcctg 60
gctattttgt taacaagatg ggcacgacgt aagcaaagtg aaatgcatat ctccagatac 120
agttcagaac aaagtgctag acttctggac tatgaggatg gtagaggatc ccgacatgca 180
tattcaacac aaagtgacac ttcatgtgat aaccgagaga gatccaaaag agattacaca 240
ccatcaacca actctctagc actgtctcga tcaagtattg ctttacctca aggatccatg 300
agtagtataa aatgtttaca aacaactgaa gaacttcctt ccagaactgc aggagccatg 360
atgcaattca cagcccctat tcccggagct acaggaccta tcaagctctc tcaaaaaacc 420
attgtgcaaa ctccaggacc tattgtacaa tatcctggac ccaatgtcag atcgcatcct 480
cacacaatca ctggtccacc ttcagcaccc cgcggtccac ccatggcacc cataataatt 540
tcacagagaa ccgcaagtca gctggcagca cctataataa tttcgcagag aactgcaaga 600
atacctcaag ttcacactat ggacagttct ggaaaaacca cactgactcc tgtggttata 660
ttaacaggtt acatggatga agaacttgca aaaaaatctt gttccaaaat ccagattcta 720
aaatgtggag gcactgcaag gtctcagaat agccgagaag aaaacaagga agcactaaag 780
aatgacatca tatttacgaa ttctgtagaa tccttgaaat cagcacacat aaaggagcca 840
gaaagagaag gaaaaggcac tgatttagag aaagacaaaa taggaatgga ggtcaaggta 900
gacagtgacg ctggaatacc aaaaagacag gaaacccaac taaaaatcag tgagatgagt 960
ataccacaag gacagggagc ccaaataaag aaaagtgtgt cagatgtacc aagaggacag 1020
gagtcccaag taaagaagag tgagtcaggt gtcccaaaag gacaagaagc ccaagtaacg 1080
aagagtgggt tggttgtact gaaaggacag gaagcccagg tagagaagag tgagatgggt 1140
gtgccaagaa gacaggaatc ccaagtaaag aagagtcagt ctggtgtctc aaagggacag 1200
gaagcccagg taaagaagag ggagtcagtt gtactgaaag gacaggaagc ccaggtagag 1260

aagagtgagt tgaaggtacc aaaaggacaa gaaggccaag tagagaagac tgaggcagat 1320
gtgccaaagg aacaagaggt ccaagaaaag aagagtgagg caggtgtact gaaaggacca 1380
gaatcccaag taaagaacac tgaggtgagt gtaccagaaa cactggaatc ccaagtaaag 1440
aagagtgagt caggtgtact aaaaggacag gaagcccaag aaaagaagga gagttttgag 1500
gataaaggaa ataatgataa agaaaaggag agagatgcag agaaagatcc aaataaaaaa 1560
gaaaaaggtg acaaaaacac aaaaggtgac aaaggaaagg acaaagttaa aggaaagaga 1620
gaatcagaaa tcaatggtga aaaatcaaaa ggctcgaaaa gggcgaaggc aaatacagga 1680
aggaagtaca acaaaaaagt ggaagagtaa 1710

<210> 33
<211> 1665
<212> DNA
<213> Homo sapiens

<400> 33
atgacagtct tggaaataac tttggctgtc atcctgactc tactgggact tgccatcctg 60
gctattttgt taacaagatg ggcacgatgt aagcaaagtg aaatgtatat ctccagatac 120
agttcagaac aaagtgctag acttctggac tatgaggatg gtagaggatc ccgacatgca 180
tattcaacac aaagtgagag atccaaaaga gattacacac catcaaccaa ctctctagca 240
ctgtctcgat caagtattgc tttacctcaa ggatccatga gtagtataaa atgtttacaa 300
acaactgaag aacctccttc cagaactgca ggagccatga tgcaattcac agcccctatt 360
cccggagcta caggacctat caagctctct caaaaaacca ttgtgcaaac tccaggacct 420
attgtacaat atcctggatc caatgctggt ccaccttcag caccccgcgg tccacccatg 480
gcacccataa taatttcaca gagaaccgca agtcagctgg cagcacctat aataatttcg 540
cagagaactg caagaatacc tcaagttcac actatggaca gttctggaaa aatcacactg 600
actcctgtgg ttatattaac aggttacatg gatgaagaac ttgcaaaaaa atcttgttcc 660
aaaatccaga ttctaaaatg tggaggcact gcaaggtctc agaatagccg agaagaaaac 720
aaggaagcac taaagaatga catcatattt acgaattctg tagaatcctt gaaatcagca 780
cacataaagg agccagaaag agaaggaaaa ggcactgatt tagagaaaga caaaatagga 840
atggaggtca aggtagacag tgacgctgga ataccaaaaa gacaggaaac ccaactaaaa 900
atcagtgaga tgagtatacc acaaggacag ggagcccaaa taaagaaaag tgtgtcagat 960
gtaccaagag gacaggagtc ccaagtaaag aagagtgagt caggtgtccc aaaaggacaa 1020
gaagcccaag taacgaagag tgggttggtt gtactgaaag gacaggaagc ccaggtagag 1080
aagagtgaga tgggtgtgcc aagaagacag gaatcccaag taaagaagag tcagtctggt 1140
gtctcaaagg gacaggaagc ccaggtaaag aagagggagt cagttgtact gaaaggacag 1200

gaagcccagg tagagaagag tgagttgaag gtaccaaaag gacaagaagg ccaagtagag 1260
aagactgagg cagatgtgcc aaaggaacaa gaggtccaag aaaagaagag tgaggcaggt 1320
gtactgaaag gaccagaatc ccaagtaaag aacactgagg tgagtgtacc agaaacactg 1380
gaatcccaag taaagaagag tgagtcaggt gtactaaaag gacaggaagc ccaagaaaag 1440
aaggagagtt ttgaggataa aggaaataat gataaagaaa aggagagaga tgcagagaaa 1500
gatccaaata aaaaagaaaa aggtgacaaa aacacaaaag gtgacaaagg aaaggacaaa 1560
gttaaaggaa agagagaatc agaaatcaat ggtgaaaaat caaaaggctc gaaaagggcg 1620
aaggcaaata caggaaggaa gtacaacaaa aaagtggaag agtaa 1665

<210> 34
<211> 561
<212> PRT
<213> Homo sapiens

<400> 34

Met Thr Val Leu Glu Ile Thr Leu Ala Val Ile Leu Thr Leu Leu Gly
1               5                   10                  15

Leu Ala Ile Leu Ala Ile Leu Leu Thr Arg Trp Ala Arg Cys Lys Gln
            20                  25                  30

Ser Glu Met Tyr Ile Ser Arg Tyr Ser Ser Glu Gln Ser Ala Arg Leu
        35                  40                  45

Leu Asp Tyr Glu Asp Gly Arg Gly Ser Arg His Ala Tyr Ser Thr Gln
    50                  55                  60

Ser Asp Thr Ser Tyr Asp Asn Arg Glu Arg Ser Lys Arg Asp Tyr Thr
65                  70                  75                  80

Pro Ser Thr Asn Ser Leu Ala Leu Ser Arg Ser Ser Ile Ala Leu Pro
                85                  90                  95

Gln Gly Ser Met Ser Ser Ile Lys Cys Leu Gln Thr Thr Glu Glu Pro
            100                 105                 110

Pro Ser Arg Thr Ala Gly Ala Met Met Gln Phe Thr Ala Pro Ile Pro
        115                 120                 125

Gly Ala Thr Gly Pro Ile Lys Leu Ser Gln Lys Thr Ile Val Gln Thr
    130                 135                 140

Pro Gly Pro Ile Val Gln Tyr Pro Gly Ser Asn Ala Gly Pro Pro Ser
145                 150                 155                 160

Ala Pro Arg Gly Pro Pro Met Ala Pro Ile Ile Ile Ser Gln Arg Thr
                165                 170                 175

Ala Ser Gln Leu Ala Ala Pro Ile Ile Ile Ser Gln Arg Thr Ala Arg
            180                 185                 190

Ile Pro Gln Val His Thr Met Asp Ser Ser Gly Lys Ile Thr Leu Thr
        195                 200                 205

Pro Val Val Ile Leu Thr Gly Tyr Met Asp Glu Glu Leu Ala Lys Lys
    210                 215                 220

Ser Cys Ser Lys Ile Gln Ile Leu Lys Cys Gly Gly Thr Ala Arg Ser
225                 230                 235                 240

Gln Asn Ser Arg Glu Glu Asn Lys Glu Ala Leu Lys Asn Asp Ile Ile
                245                 250                 255

Phe Thr Asn Ser Val Glu Ser Leu Lys Ser Ala His Ile Lys Glu Pro
            260                 265                 270

Glu Arg Glu Gly Lys Gly Thr Asp Leu Glu Lys Asp Lys Ile Gly Met
        275                 280                 285

Glu Val Lys Val Asp Ser Asp Ala Gly Ile Pro Lys Arg Gln Glu Thr
    290                 295                 300

Gln Leu Lys Ile Ser Glu Met Ser Ile Pro Gln Gly Gln Gly Ala Gln
305                 310                 315                 320

Ile Lys Lys Ser Val Ser Asp Val Pro Arg Gly Gln Glu Ser Gln Val
                325                 330                 335

Lys Lys Ser Glu Ser Gly Val Pro Lys Gly Gln Glu Ala Gln Val Thr
            340                 345                 350

Lys Ser Gly Leu Val Val Leu Lys Gly Gln Glu Ala Gln Val Glu Lys
        355                 360                 365

Ser Glu Met Gly Val Pro Arg Arg Gln Glu Ser Gln Val Lys Lys Ser
    370                 375                 380

Gln Ser Gly Val Ser Lys Gly Gln Glu Ala Gln Val Lys Lys Arg Glu
385                 390                 395                 400

Ser Val Val Leu Lys Gly Gln Glu Ala Gln Val Glu Lys Ser Glu Leu
                405                 410                 415

Lys Val Pro Lys Gly Gln Glu Gly Gln Val Glu Lys Thr Glu Ala Asp
            420                 425                 430

Val Pro Lys Glu Gln Glu Val Gln Glu Lys Lys Ser Glu Ala Gly Val
        435                 440                 445

Leu Lys Gly Pro Glu Ser Gln Val Lys Asn Thr Glu Val Ser Val Pro
    450                 455                 460

Glu Thr Leu Glu Ser Gln Val Lys Lys Ser Glu Ser Gly Val Leu Lys
465                 470                 475                 480

Gly Gln Glu Ala Gln Glu Lys Lys Glu Ser Phe Glu Asp Lys Gly Asn
                485                 490                 495

Asn Asp Lys Glu Lys Glu Arg Asp Ala Glu Lys Asp Pro Asn Lys Lys
            500                 505                 510

Glu Lys Gly Asp Lys Asn Thr Lys Gly Asp Lys Gly Lys Asp Lys Val
        515                 520                 525

Lys Gly Lys Arg Glu Ser Glu Ile Asn Gly Glu Lys Ser Lys Gly Ser
    530                 535                 540

Lys Arg Ala Lys Ala Asn Thr Gly Arg Lys Tyr Asn Lys Lys Val Glu
545                 550                 555                 560

Glu

<210>  35
<211>  569
<212>  PRT
<213>  Homo sapiens

<400>  35

Met Thr Val Leu Glu Ile Thr Leu Ala Val Ile Leu Thr Leu Leu Gly
1               5                   10                  15

Leu Ala Ile Leu Ala Ile Leu Leu Thr Arg Trp Ala Arg Arg Lys Gln
            20                  25                  30

Ser Glu Met His Ile Ser Arg Tyr Ser Ser Glu Gln Ser Ala Arg Leu
        35                  40                  45

Leu Asp Tyr Glu Asp Gly Arg Gly Ser Arg His Ala Tyr Ser Thr Gln
    50                  55                  60

Ser Asp Thr Ser Cys Asp Asn Arg Glu Arg Ser Lys Arg Asp Tyr Thr
65                  70                  75                  80

Pro Ser Thr Asn Ser Leu Ala Leu Ser Arg Ser Ser Ile Ala Leu Pro
                85                  90                  95

Gln Gly Ser Met Ser Ser Ile Lys Cys Leu Gln Thr Thr Glu Glu Leu
            100                 105                 110

Pro Ser Arg Thr Ala Gly Ala Met Met Gln Phe Thr Ala Pro Ile Pro
        115                 120                 125

Gly Ala Thr Gly Pro Ile Lys Leu Ser Gln Lys Thr Ile Val Gln Thr
    130                 135                 140

Pro Gly Pro Ile Val Gln Tyr Pro Gly Pro Asn Val Arg Ser His Pro
145                 150                 155                 160

His Thr Ile Thr Gly Pro Pro Ser Ala Pro Arg Gly Pro Pro Met Ala
                165                 170                 175

Pro Ile Ile Ile Ser Gln Arg Thr Ala Ser Gln Leu Ala Ala Pro Ile
            180                 185                 190

Ile Ile Ser Gln Arg Thr Ala Arg Ile Pro Gln Val His Thr Met Asp
        195                 200                 205

Ser Ser Gly Lys Thr Thr Leu Thr Pro Val Val Ile Leu Thr Gly Tyr
    210                 215                 220

Met Asp Glu Glu Leu Ala Lys Lys Ser Cys Ser Lys Ile Gln Ile Leu
225                 230                 235                 240

Lys Cys Gly Gly Thr Ala Arg Ser Gln Asn Ser Arg Glu Glu Asn Lys
                245                 250                 255

Glu Ala Leu Lys Asn Asp Ile Ile Phe Thr Asn Ser Val Glu Ser Leu
            260                 265                 270

Lys Ser Ala His Ile Lys Glu Pro Glu Arg Glu Gly Lys Gly Thr Asp
        275                 280                 285

Leu Glu Lys Asp Lys Ile Gly Met Glu Val Lys Val Asp Ser Asp Ala
    290                 295                 300

Gly Ile Pro Lys Arg Gln Glu Thr Gln Leu Lys Ile Ser Glu Met Ser
305                 310                 315                 320

Ile Pro Gln Gly Gln Gly Ala Gln Ile Lys Lys Ser Val Ser Asp Val
                325                 330                 335

Pro Arg Gly Gln Glu Ser Gln Val Lys Lys Ser Glu Ser Gly Val Pro
            340                 345                 350

Lys Gly Gln Glu Ala Gln Val Thr Lys Ser Gly Leu Val Val Leu Lys
        355                 360                 365

Gly Gln Glu Ala Gln Val Glu Lys Ser Glu Met Gly Val Pro Arg Arg
    370                 375                 380

Gln Glu Ser Gln Val Lys Lys Ser Gln Ser Gly Val Ser Lys Gly Gln
385                 390                 395                 400

Glu Ala Gln Val Lys Lys Arg Glu Ser Val Val Leu Lys Gly Gln Glu
                405                 410                 415

Ala Gln Val Glu Lys Ser Glu Leu Lys Val Pro Lys Gly Gln Glu Gly
            420                 425                 430

Gln Val Glu Lys Thr Glu Ala Asp Val Pro Lys Glu Gln Glu Val Gln
        435                 440                 445

Glu Lys Lys Ser Glu Ala Gly Val Leu Lys Gly Pro Glu Ser Gln Val
    450                 455                 460

Lys Asn Thr Glu Val Ser Val Pro Glu Thr Leu Glu Ser Gln Val Lys
465                 470                 475                 480

Lys Ser Glu Ser Gly Val Leu Lys Gly Gln Glu Ala Gln Glu Lys Lys
                485                 490                 495

Glu Ser Phe Glu Asp Lys Gly Asn Asn Asp Lys Glu Lys Glu Arg Asp
            500                 505                 510

Ala Glu Lys Asp Pro Asn Lys Lys Glu Lys Gly Asp Lys Asn Thr Lys
        515                 520                 525

Gly Asp Lys Gly Lys Asp Lys Val Lys Gly Lys Arg Glu Ser Glu Ile
    530                 535                 540

Asn Gly Glu Lys Ser Lys Gly Ser Lys Arg Ala Lys Ala Asn Thr Gly
545                 550                 555                 560

Arg Lys Tyr Asn Lys Lys Val Glu Glu
                565

<210>  36

<211>  554

<212>  PRT

<213>  Homo sapiens


<400>  36

Met Thr Val Leu Glu Ile Thr Leu Ala Val Ile Leu Thr Leu Leu Gly
1               5                   10                  15

Leu Ala Ile Leu Ala Ile Leu Leu Thr Arg Trp Ala Arg Cys Lys Gln
            20                  25                  30

Ser Glu Met Tyr Ile Ser Arg Tyr Ser Ser Glu Gln Ser Ala Arg Leu
        35                  40                  45

Leu Asp Tyr Glu Asp Gly Arg Gly Ser Arg His Ala Tyr Ser Thr Gln
    50                  55                  60

Ser Glu Arg Ser Lys Arg Asp Tyr Thr Pro Ser Thr Asn Ser Leu Ala
65                  70                  75                  80

Leu Ser Arg Ser Ser Ile Ala Leu Pro Gln Gly Ser Met Ser Ser Ile
                85                  90                  95

Lys Cys Leu Gln Thr Thr Glu Glu Pro Pro Ser Arg Thr Ala Gly Ala
            100                 105                 110

Met Met Gln Phe Thr Ala Pro Ile Pro Gly Ala Thr Gly Pro Ile Lys
        115                 120                 125

Leu Ser Gln Lys Thr Ile Val Gln Thr Pro Gly Pro Ile Val Gln Tyr
    130                 135                 140

Pro Gly Ser Asn Ala Gly Pro Pro Ser Ala Pro Arg Gly Pro Pro Met
145                 150                 155                 160

Ala Pro Ile Ile Ile Ser Gln Arg Thr Ala Ser Gln Leu Ala Ala Pro
                165                 170                 175

Ile Ile Ile Ser Gln Arg Thr Ala Arg Ile Pro Gln Val His Thr Met
            180                 185                 190

Asp Ser Ser Gly Lys Ile Thr Leu Thr Pro Val Val Ile Leu Thr Gly
        195                 200                 205

Tyr Met Asp Glu Glu Leu Ala Lys Lys Ser Cys Ser Lys Ile Gln Ile
    210                 215                 220

Leu Lys Cys Gly Gly Thr Ala Arg Ser Gln Asn Ser Arg Glu Glu Asn
225                 230                 235                 240

Lys Glu Ala Leu Lys Asn Asp Ile Ile Phe Thr Asn Ser Val Glu Ser
                245                 250                 255

Leu Lys Ser Ala His Ile Lys Glu Pro Glu Arg Glu Gly Lys Gly Thr
            260                 265                 270

Asp Leu Glu Lys Asp Lys Ile Gly Met Glu Val Lys Val Asp Ser Asp
        275                 280                 285

Ala Gly Ile Pro Lys Arg Gln Glu Thr Gln Leu Lys Ile Ser Glu Met
    290                 295                 300

Ser Ile Pro Gln Gly Gln Gly Ala Gln Ile Lys Lys Ser Val Ser Asp
305                 310                 315                 320

Val Pro Arg Gly Gln Glu Ser Gln Val Lys Lys Ser Glu Ser Gly Val
                325                 330                 335

Pro Lys Gly Gln Glu Ala Gln Val Thr Lys Ser Gly Leu Val Val Leu
            340                 345                 350

Lys Gly Gln Glu Ala Gln Val Glu Lys Ser Glu Met Gly Val Pro Arg
        355                 360                 365

Arg Gln Glu Ser Gln Val Lys Lys Ser Gln Ser Gly Val Ser Lys Gly
    370                 375                 380

Gln Glu Ala Gln Val Lys Lys Arg Glu Ser Val Val Leu Lys Gly Gln
385                 390                 395                 400

Glu Ala Gln Val Glu Lys Ser Glu Leu Lys Val Pro Lys Gly Gln Glu
                405                 410                 415

Gly Gln Val Glu Lys Thr Glu Ala Asp Val Pro Lys Glu Gln Glu Val
            420                 425                 430

Gln Glu Lys Lys Ser Glu Ala Gly Val Leu Lys Gly Pro Glu Ser Gln
        435                 440                 445

Val Lys Asn Thr Glu Val Ser Val Pro Glu Thr Leu Glu Ser Gln Val
    450                 455                 460

Lys Lys Ser Glu Ser Gly Val Leu Lys Gly Gln Glu Ala Gln Glu Lys
465                 470                 475                 480

Lys Glu Ser Phe Glu Asp Lys Gly Asn Asn Asp Lys Glu Lys Glu Arg
                485                 490                 495

Asp Ala Glu Lys Asp Pro Asn Lys Lys Glu Lys Gly Asp Lys Asn Thr
            500                 505                 510

Lys Gly Asp Lys Gly Lys Asp Lys Val Lys Gly Lys Arg Glu Ser Glu
        515                 520                 525

Ile Asn Gly Glu Lys Ser Lys Gly Ser Lys Arg Ala Lys Ala Asn Thr
    530                 535                 540

Gly Arg Lys Tyr Asn Lys Lys Val Glu Glu
545                 550

<210>  37

<211>  1182

<212>  DNA

<213>  Homo sapiens


<400>  37
acacaggttg gagcagagaa agaggaaaca tagaggtgcc aaaggaacaa agacataatg      60

atgtcatcca agccaacaag ccatgctgaa gtaaatgaaa ccatacccaa cccttaccca     120

ccaggcagct ttatggctcc tggatttcaa cagcctctgg gttcaatcaa cttagaaaac     180

caagctcagg gtgctcagcg tgctcagccc tacggcatca catctccggg aatctttgct     240

agcagtcaac cgggtcaagg aaatatacaa atgataaatc caagtgtggg aacagcagta     300

atgaacttta aagaagaagc aaaggcacta ggggtgatcc agatcatggt tggattgatg     360

cacattggtt ttggaattgt tttgtgttta atatccttct cttttagaga agtattaggt     420

tttgcctcta ctgctgttat tggtggatac ccattctggg gtggcctttc ttttattatc     480

tctggctctc tctctgtgtc agcatccaag gagctttccc gttgtctggt gaaaggcagc     540

ctgggaatga acattgttag ttctatcttg gccttcattg gagtgattct gctgctggtg     600

gatatgtgca tcaatggggt agctggccaa gactactggg ccgtgctttc tggaaaaggc     660

atttcagcca cgctgatgat cttctccctc ttggagttct tcgtagcttg tgccacagcc     720

cattttgcca accaagcaaa caccacaacc aatatgtctg tcctggttat tccaaatatg     780

tatgaaagca accctgtgac accagcgtct tcttcagctc ctcccagatg caacaactac     840

tcagctaatg cccctaaata gtaaaagaaa aaggggtatc agtctaatct catggagaaa     900

aactacttgc aaaaacttct taagaagatg tcttttattg tctacaatga tttctagtct     960

ttaaaaactg tgtttgagat ttgtttttag gttggtcgct aatgatggct gtatctccct    1020

tcactgtctc ttcctacatt accactacta catgctggca aaggtgaagg atcagaggac    1080

tgaaaaatga ttctgcaact ctcttaaagt tagaaatgtt tctgttcata ttactttttc    1140

cttaataaaa tgtcattaga aacaaaaaaa aaaaaaaaaa aa                       1182

<210>  38

<211>  267

<212>  PRT

<213>  Homo sapiens


<400>  38

Met Met Ser Ser Lys Pro Thr Ser His Ala Glu Val Asn Glu Thr Ile
1               5                   10                  15

Pro Asn Pro Tyr Pro Pro Gly Ser Phe Met Ala Pro Gly Phe Gln Gln
            20                  25                  30

Pro Leu Gly Ser Ile Asn Leu Glu Asn Gln Ala Gln Gly Ala Gln Arg
        35                  40                  45

Ala Gln Pro Tyr Gly Ile Thr Ser Pro Gly Ile Phe Ala Ser Ser Gln
    50                  55                  60

Pro Gly Gln Gly Asn Ile Gln Met Ile Asn Pro Ser Val Gly Thr Ala
65                  70                  75                  80

Val Met Asn Phe Lys Glu Glu Ala Lys Ala Leu Gly Val Ile Gln Ile
                85                  90                  95

Met Val Gly Leu Met His Ile Gly Phe Gly Ile Val Leu Cys Leu Ile
            100                 105                 110

Ser Phe Ser Phe Arg Glu Val Leu Gly Phe Ala Ser Thr Ala Val Ile
        115                 120                 125

Gly Gly Tyr Pro Phe Trp Gly Gly Leu Ser Phe Ile Ile Ser Gly Ser
    130                 135                 140

Leu Ser Val Ser Ala Ser Lys Glu Leu Ser Arg Cys Leu Val Lys Gly
145                 150                 155                 160

Ser Leu Gly Met Asn Ile Val Ser Ser Ile Leu Ala Phe Ile Gly Val
                165                 170                 175

Ile Leu Leu Leu Val Asp Met Cys Ile Asn Gly Val Ala Gly Gln Asp
            180                 185                 190

Tyr Trp Ala Val Leu Ser Gly Lys Gly Ile Ser Ala Thr Leu Met Ile
        195                 200                 205

Phe Ser Leu Leu Glu Phe Phe Val Ala Cys Ala Thr Ala His Phe Ala
    210                 215                 220

Asn Gln Ala Asn Thr Thr Thr Asn Met Ser Val Leu Val Ile Pro Asn
225                 230                 235                 240

Met Tyr Glu Ser Asn Pro Val Thr Pro Ala Ser Ser Ser Ala Pro Pro
                245                 250                 255

Arg Cys Asn Asn Tyr Ser Ala Asn Ala Pro Lys
            260                 265

<210> 39
<211> 1948
<212> DNA
<213> Homo sapiens

<400> 39
gcacgaggtt ttgaggacca gcaacacagc aatacttcca gatctccata taacctctgt 60
tcatttggga ggggctttgt attttcaaca ggagagttca aagttcattt ttttttcagc 120
aactacagtt ctaagtgaaa tctattttta ttgatacatg gtattttaca tgtttatggg 180
atacatatga gtcataatct attttaaata ataccttagt gttgtaaaat caacagtgct 240
ttttaaaaga aatatacctt gttaattatc ccacatgtgt ctccagaagt acagcttgaa 300
caaatccacc ttctgtggac caagcaccac cctgggcatt tctagcatga gcaaaatcca 360
aggtcctggc tggactccag agatgctatt tacctcagaa gcatgacaat aggaggcaga 420
aggagcaggc aaatccaagt cctttcttgt agtttccttg tttggggagg aaaagttgag 480
ttttactatt atggaaaaga aacaggaaat agagacagac aaagagatat gacaatacag 540
tcctgccacc cagatactca tttccaccta ccattccatg catttgtttt gaatatataa 600
gtatgtacat aaaggtaggt actctcaagt ccatcagggc ttggctgtcc actgtttttg 660
aagttccaga atgtttttgc taagttgagg aaataccaaa tcaggactat gaaaattatg 720
gtatatattg atgtgtcaca gaacacagat gtgacataat aaagatgtgt aagattatat 780
atataacttg tgtgtacacc tacctcatct ggggataaca cctcaagttt aattttgagg 840
cttgggtcaa tcgtgcttcc cttccctttc ataggtcctc tatgagatat tgtcatagat 900
tccatgttat gcaatagcca tagaatatga catctctcta tgataattct atattacttt 960
aattgctgca cagaagttca ttgtatgtaa gtgccacagt atattataga tcttcttgtg 1020
ggacatctat ttctagttta tgtgatagta tagcactttc atgaatgttc ttgtacttga 1080
tctttacaca ttttcttttt tccttaggat gaattctgag agatgtaatt gatggggcaa 1140
aatgtactca ctgtttgagg tttgaaattt ttccatcaaa agctggtact cttggttttt 1200
taagacaaag agcaaatcct cccctgccag gattgacttt tggctctttt ttttcaaacc 1260
tcactgcttt ttggtttagt tgtcataaaa tgccaagcac catgaacagg gctccatgaa 1320
ggggctcaga ggtaggaggg ctgtgattag gagaaggctt ggactgatgg gcaatttgag 1380
tgctcagaat tagagtgagg gggtgggggt gctgcaggga cagatgctgg ggaaagacac 1440
cctgaagggc aaagggagca acaatggctg cagtacatgt ggcctttcag ctagcgcaga 1500
ggatggaaac cagagtgggc tgatgattgg atgccaggcc tgagccagca actgtgatcc 1560
tgagctgtgc acacttctgg ttgggattat ttctggtttc tacttcctgt ttgaagatgt 1620
ggcatggaga gtgctctgct ttgacctgaa gtattttatc tatcctcagt ctcaggacac 1680

tgttgatgga attaaggcca agcacatctg caaaaaagac attgctggag gaggtgcaaa 1740
gagctggaaa ccaagtctcc agtcctggga aaagcagtgg tatggaaaag caatggaaag 1800
agcattttga aaatgccatt ccactgtttt ctggccttta tgatttctgc tgagaaatcc 1860
actgttagtc tgatggggtc tccttcatag caccaatgac ctgaagagcc ttgttgaagg 1920
aagactccat ctgatgactc agagcaag 1948

<210> 40
<211> 1406
<212> DNA
<213> Homo sapiens

<400> 40
cggtgagagg ggcgcgcagc agcagctcct caacgccgca acgcgccggc ccaactgcag 60
gaaggtctgt gctctggagc cagggtaaat ggttataaaa ttatacacca tggccctcct 120
aaagacactc taggaaaacc atgtcatcct gatcttaaaa cacctgcaag aaagagcaca 180
gtacttcacc attaataaag tagatatttc atcctgctca gaaaaccaac atttccagca 240
atggctttac taccggtgtt gtttctggtt actgtgctgc ttccatcttt acctgcagaa 300
ggaaaggatc ccgcttttac tgctttgtta accacccagt tgcaagtgca aagggagatt 360
gtaaataaac acaatgaact aaggaaagca gtctctccac ctgccagtaa catgctaaag 420
atggaatgga gcagagaggt aacaacgaat gcccaaaggt gggcaaacaa gtgcacttta 480
caacatagtg atccagagga ccgcaaaacc agtacaagat gtggtgagaa tctctatatg 540
tcaagtgacc ctacttcctg gtcttctgca atccaaagct ggtatgacga gatcctagat 600
tttgtctatg gtgtaggacc aaagagtccc aatgcagttg ttggacatta tactcagctt 660
gtttggtact cgacttacca ggtaggctgt ggaattgcct actgtcccaa tcaagatagt 720
ctaaaatact actatgtttg ccaatattgt cctgctggta ataatatgaa tagaaagaat 780
accccgtacc aacaaggaac accttgtgcc ggttgccctg atgactgtga caaaggacta 840
tgcaccaata gttgccagta tcaagatctc ctaagtaact gtgattcctt gaagaataca 900
gctggctgtg aacatgagtt actcaaggaa aagtgcaagg ctacttgcct atgtgagaac 960
aaaatttact gatttaccta gtgagcattg tgcaagactg catggataag ggctgcatca 1020
tttaattgcg acataccagt ggaaattgta tgtatgttag tgacaaattt gatttcaaag 1080
agcaatgcat cttctccccc agatcatcac agaaatcact ttcaggcaat gatttacaaa 1140
agtagcatag tagatgatga caactgtgaa ctctgacata aatttagtgc tttataacga 1200
actgaatcag gttgaggatt ttgaaaactg tataaccata ggatttaggt cactaggact 1260
ttggatcaaa atggtgcatt acgtatttcc tgaaacatgc taaagaagaa gactgtaaca 1320
tcattgccat tcctactacc tgagttttta cttgcataaa caataaattc aaagctttac 1380

atctgcaaaa aaaaaaaaaa aaaaaa 1406

<210> 41

<211> 243

<212> PRT

<213> Homo sapiens

<400> 41

Met Ala Leu Leu Pro Val Leu Phe Leu Val Thr Val Leu Leu Pro Ser
1               5                   10                  15

Leu Pro Ala Glu Gly Lys Asp Pro Ala Phe Thr Ala Leu Leu Thr Thr
            20                  25                  30

Gln Leu Gln Val Gln Arg Glu Ile Val Asn Lys His Asn Glu Leu Arg
        35                  40                  45

Lys Ala Val Ser Pro Pro Ala Ser Asn Met Leu Lys Met Glu Trp Ser
    50                  55                  60

Arg Glu Val Thr Thr Asn Ala Gln Arg Trp Ala Asn Lys Cys Thr Leu
65                  70                  75                  80

Gln His Ser Asp Pro Glu Asp Arg Lys Thr Ser Thr Arg Cys Gly Glu
                85                  90                  95

Asn Leu Tyr Met Ser Ser Asp Pro Thr Ser Trp Ser Ser Ala Ile Gln
            100                 105                 110

Ser Trp Tyr Asp Glu Ile Leu Asp Phe Val Tyr Gly Val Gly Pro Lys
        115                 120                 125

Ser Pro Asn Ala Val Val Gly His Tyr Thr Gln Leu Val Trp Tyr Ser
    130                 135                 140

Thr Tyr Gln Val Gly Cys Gly Ile Ala Tyr Cys Pro Asn Gln Asp Ser
145                 150                 155                 160

Leu Lys Tyr Tyr Tyr Val Cys Gln Tyr Cys Pro Ala Gly Asn Asn Met
                165                 170                 175

Asn Arg Lys Asn Thr Pro Tyr Gln Gln Gly Thr Pro Cys Ala Gly Cys
            180                 185                 190

Pro Asp Asp Cys Asp Lys Gly Leu Cys Thr Asn Ser Cys Gln Tyr Gln
        195                 200                 205

Asp Leu Leu Ser Asn Cys Asp Ser Leu Lys Asn Thr Ala Gly Cys Glu

210                    215                    220

His Glu Leu Leu Lys Glu Lys Cys Lys Ala Thr Cys Leu Cys Glu Asn
225                 230                 235                 240

Lys Ile Tyr

<210>  42

<211>  21

<212>  DNA

<213>  Künstliche Sequenz

<220>
<223>  Beschreibung der künstlichen Sequenz: Oligonukleotid

<400>  42
tctagcactg tctcgatcaa g                                                21

<210>  43

<211>  21

<212>  DNA

<213>  Künstliche Sequenz

<220>
<223>  Beschreibung der künstlichen Sequenz: Oligonukleotid

<400>  43
tgtcctcttg gtacatctga c                                                21

<210>  44

<211>  21

<212>  DNA

<213>  Künstliche Sequenz

<220>
<223>  Beschreibung der künstlichen Sequenz: Oligonukleotid

<400>  44
ctgtgtcagc atccaaggag c                                                21

<210>  45

<211>  21

<212>  DNA

<213>  Künstliche Sequenz

<220>
<223>  Beschreibung der künstlichen Sequenz: Oligonukleotid

<400>  45
ttcacctttg ccagcatgta g                                                21

<210>  46

<211>  21

<212>  DNA

<213>  Künstliche Sequenz

<220>
<223>  Beschreibung der künstlichen Sequenz: Oligonukleotid

<400>  46
cttgctctga gtcatcagat g                                                21

<210>  47

<211>  21

<212>  DNA

<213>  Künstliche Sequenz

<220>
<223>  Beschreibung der künstlichen Sequenz: Oligonukleotid

<400>  47
cacagaatat gagccataca g                                                21

<210>  48

<211>  22

<212>  DNA

<213>  Künstliche Sequenz

<220>
<223>  Beschreibung der künstlichen Sequenz: Oligonukleotid

<400>  48
ggtgtcactt ctgtgccttc ct                                               22

<210>  49

<211>  21

<212>  DNA

<213>  Künstliche Sequenz

<220>
<223>  Beschreibung der künstlichen Sequenz: Oligonukleotid

<400>  49
cggcaccagt tccaacaata g                                                21

<210>  50

<211>  18

<212>  DNA

<213>  Künstliche Sequenz

<220>
<223>  Beschreibung der künstlichen Sequenz: Oligonukleotid

<400>  50
caaaggttct ccaaatgt                                                    18

<210>  51

<211>  21

<212>  DNA

<213>  Künstliche Sequenz

<220>
<223>  Beschreibung der künstlichen Sequenz: Oligonukleotid

<400>  51
tagcgcctca actgtcgttg g                                                21

<210>  52

<211>  23

<212>  DNA

<213>  Künstliche Sequenz

<220>
<223>  Beschreibung der künstlichen Sequenz: Oligonukleotid

<400>  52
cgtgagcgct tcgagatgtt ccg                                              23

<210>  53

<211>  23

<212>  DNA

<213>  Künstliche Sequenz

<220>
<223>  Beschreibung der künstlichen Sequenz: Oligonukleotid

<400>  53
cctaaccagc tgcccaactg tag                                              23

<210>  54

<211>  1550

<212>  DNA

<213>  Homo sapiens

<400>  54
atgaatgaaa gtcctgatcc gactgacctg gcgggagtca tcattgagct cggccccaat      60

gacagtccac agacaagtga atttaaagga gcaaccgagg aggcacctgc gaaagaaagc     120

ccacacacaa gtgaatttaa aggagcagcc cgggtgtcac ctatcagtga aagtgtgtta     180

gcacgacttt ccaagtttga agttgaagat gctgaaaatg ttgcttcata tgacagcaag     240

attaagaaaa ttgtgcattc aattgtatca tcctttgcat ttggactatt tggagttttc     300

ctggtcttac tggatgtcac tctcatcctt gccgacctaa ttttcactga cagcaaactt     360

tatattcctt tggagtatcg ttctatttct ctagctattg ccttattttt tctcatggat     420

gttcttcttc gagtatttgt agaaaggaga cagcagtatt tttctgactt atttaacatt     480

ttagatactg ccattattgt gattcttctg ctggttgatg tcgtttacat tttttttgac     540

attaagttgc ttaggaatat tcccagatgg acacatttac ttcgacttct acgacttatt     600

attctgttaa gaatttttca tctgtttcat caaaaaagac aacttgaaaa gctgataaga     660

aggcgggttt cagaaaacaa aaggcgatac acaagggatg gatttgacct agacctcact     720

tacgttacag aacgtattat tgctatgtca tttccatctt ctggaaggca gtctttctat     780

agaaatccaa tcaaggaagt tgtgcggttt ctagataaga aacaccgaaa ccactatcga     840

gtctacaatc tatgcagtga aagagcttac gatcctaagc acttccataa tagggtcgtt     900

agaatcatga ttgatgatca taatgtcccc actctacatc agatggtggt tttcaccaag     960

gaagtaaatg agtggatggc tcaagatctt gaaaacatcg tagcgattca ctgtaaagga    1020

ggcacagata gaacaggaac tatggtttgt gccttcctta ttgcctctga aatatgttca    1080

actgcaaagg aaagcctgta ttattttgga gaaaggcgaa cagataaaac ccacagcgaa    1140

aaatttcagg gagtagaaac tccttctcag gttatgtacg tgatctaaaa atccaaatag    1200

aaatggagaa aaaggttgtc ttttccacta tttcattagg aaaatgttcg gtacttgata    1260

acattacaac agacaaaata ttaattgatg tattcgacgg tccacctctg tatgatgatg    1320

tgaaagtgca gtttttctat tcgaatcttc ctacatacta tgacaattgc tcattttact    1380

tctggttgca cacatctttt attgaaaata acaggcttta tctaccaaaa aatgaattgg 1440
ataatctaca taaacaaaaa gcacggagaa tttatccatc agattttgcc gtggagatac 1500
tttttggcga gaaaatgact tccagtgatg ttgtagctgg atccgattaa 1550

<210> 55
<211> 1407
<212> DNA
<213> Homo sapiens

<400> 55
atgaatgaaa gtcctgatcc gactgacctg gcgggagtca tcattgagct cggccccaat 60
gacagtccac agacaagtga atttaaagga gcaaccgagg aggcacctgc gaaagaaagc 120
ccacacacaa gtgaatttaa aggagcagcc cgggtgtcac ctatcagtga aagtgtgtta 180
gcacgacttt ccaagtttga agttgaagat gctgaaaatg ttgcttcata tgacagcaag 240
attaagaaaa ttgtgcattc aattgtatca tcctttgcat ttggactatt tggagttttc 300
ctggtcttac tggatgtcac tctcatcctt gccgacctaa ttttcactga cagcaaactt 360
tatattcctt tggagtatcg ttctatttct ctagctattg ccttattttt tctcatggat 420
gttcttcttc gagtatttgt agaaaggaga cagcagtatt tttctgactt atttaacatt 480
ttagatactg ccattattgt gattcttctg ctggttgatg tcgtttacat tttttttgac 540
attaagttgc ttaggaatat tcccagatgg acacatttac ttcgacttct acgacttatt 600
attctgttaa gaatttttca tctgtttcat caaaaaagac aacttgaaaa gctgataaga 660
aggcgggttt cagaaaacaa aaggcgatac acaagggatg gatttgacct agacctcact 720
tacgttacag aacgtattat tgctatgtca tttccatctt ctggaaggca gtctttctat 780
agaaatccaa tcaaggaagt tgtgcggttt ctagataaga aacaccgaaa ccactatcga 840
gtctacaatc tatgcagtga aagagcttac gatcctaagc acttccataa tagggtcgtt 900
agaatcatga ttgatgatca taatgtcccc actctacatc agatggtggt tttcaccaag 960
gaagtaaatg agtggatggc tcaagatctt gaaaacatcg tagcgattca ctgtaaagga 1020
ggcacaggtt atgtacgtga tctaaaaatc caaatagaaa tggagaaaaa ggttgtcttt 1080
tccactattt cattaggaaa atgttcggta cttgataaca ttacaacaga caaaatatta 1140
attgatgtat tcgacggtcc acctctgtat gatgatgtga aagtgcagtt tttctattcg 1200
aatcttccta catactatga caattgctca ttttacttct ggttgcacac atcttttatt 1260
gaaaataaca ggctttatct accaaaaaat gaattggata atctacataa acaaaaagca 1320
cggagaattt atccatcaga ttttgccgtg gagatacttt ttggcgagaa aatgacttcc 1380
agtgatgttg tagctggatc cgattaa 1407

<210> 56

<211> 1413
<212> DNA
<213> Homo sapiens

<400> 56
atgaatgaaa gtcctgatcc gactgacctg gcgggagtca tcattgagct cggccccaat 60
gacagtccac agacaagtga atttaaagga gcaaccgagg aggcacctgc gaaagaaagt 120
gtgttagcac gactttccaa gtttgaagtt gaagatgctg aaaatgttgc ttcatatgac 180
agcaagatta agaaaattgt gcattcaatt gtatcatcct ttgcatttgg actatttgga 240
gttttcctgg tcttactgga tgtcactctc atccttgccg acctaatttt cactgacagc 300
aaactttata ttcctttgga gtatcgttct atttctctag ctattgcctt attttttctc 360
atggatgttc ttcttcgagt atttgtagaa aggagacagc agtatttttc tgacttattt 420
aacattttag atactgccat tattgtgatt cttctgctgg ttgatgtcgt ttacattttt 480
tttgacatta agttgcttag gaatattccc agatggacac atttacttcg acttctacga 540
cttattattc tgttaagaat ttttcatctg tttcatcaaa aaagacaact tgaaaagctg 600
ataagaaggc gggtttcaga aaacaaaagg cgatacacaa gggatggatt tgacctagac 660
ctcacttacg ttacagaacg tattattgct atgtcatttc catcttctgg aaggcagtct 720
ttctatagaa atccaatcaa ggaagttgtg cggtttctag ataagaaaca ccgaaaccac 780
tatcgagtct acaatctatg cagtgaaaga gcttacgatc ctaagcactt ccataatagg 840
gtcgttagaa tcatgattga tgatcataat gtccccactc tacatcagat ggtggttttc 900
accaaggaag taaatgagtg gatggctcaa gatcttgaaa acatcgtagc gattcactgt 960
aaaggaggca cagatagaac aggaactatg gtttgtgcct tccttattgc ctctgaaata 1020
tgttcaactg caaaggaaag cctgtattat tttggagaaa ggcgaacaga taaaacccac 1080
agcgaaaaat ttcagggagt agaaactcct tctgtacttg ataacattac aacagacaaa 1140
atattaattg atgtattcga cggtccacct ctgtatgatg atgtgaaagt gcagtttttc 1200
tattcgaatc ttcctacata ctatgacaat tgctcatttt acttctggtt gcacacatct 1260
tttattgaaa ataacaggct ttatctacca aaaaatgaat tggataatct acataaacaa 1320
aaagcacgga gaatttatcc atcagatttt gccgtggaga tactttttgg cgagaaaatg 1380
acttccagtg atgttgtagc tggatccgat taa 1413

<210> 57
<211> 1353
<212> DNA
<213> Homo sapiens

<400> 57
atgaatgaaa gtcctgatcc gactgacctg gcgggagtca tcattgagct cggccccaat 60
gacagtccac agacaagtga atttaaagga gcaaccgagg aggcacctgc gaaagaaagt 120
gtgttagcac gactttccaa gtttgaagtt gaagatgctg aaaatgttgc ttcatatgac 180
agcaagatta agaaaattgt gcattcaatt gtatcatcct ttgcatttgg actatttgga 240
gttttcctgg tcttactgga tgtcactctc atccttgccg acctaatttt cactgacagc 300
aaactttata ttcctttgga gtatcgttct atttctctag ctattgcctt attttttctc 360
atggatgttc ttcttcgagt atttgtagaa aggagacagc agtatttttc tgacttattt 420
aacattttag atactgccat tattgtgatt cttctgctgg ttgatgtcgt ttacattttt 480
tttgacatta agttgcttag gaatattccc agatggacac atttacttcg acttctacga 540
cttattattc tgttaagaat ttttcatctg tttcatcaaa aaagacaact tgaaaagctg 600
ataagaaggc gggtttcaga aaacaaaagg cgatacacaa gggatggatt tgacctagac 660
ctcacttacg ttacagaacg tattattgct atgtcatttc catcttctgg aaggcagtct 720
ttctatagaa atccaatcaa ggaagttgtg cggtttctag ataagaaaca ccgaaaccac 780
tatcgagtct acaatctatg cagtgaaaga gcttacgatc ctaagcactt ccataatagg 840
gtcgttagaa tcatgattga tgatcataat gtccccactc tacatcagat ggtggttttc 900
accaaggaag taaatgagtg gatggctcaa gatcttgaaa acatcgtagc gattcactgt 960
aaaggaggca caggttatgt acgtgatcta aaaatccaaa tagaaatgga gaaaaaggtt 1020
gtcttttcca ctatttcatt aggaaaatgt tcggtacttg ataacattac aacagacaaa 1080
atattaattg atgtattcga cggtccacct ctgtatgatg atgtgaaagt gcagtttttc 1140
tattcgaatc ttcctacata ctatgacaat tgctcatttt acttctggtt gcacacatct 1200
tttattgaaa ataacaggct ttatctacca aaaaatgaat tggataatct acataaacaa 1260
aaagcacgga gaatttatcc atcagatttt gccgtggaga tactttttgg cgagaaaatg 1320
acttccagtg atgttgtagc tggatccgat taa 1353

<210> 58
<211> 395
<212> PRT
<213> Homo sapiens

<400> 58

Met Asn Glu Ser Pro Asp Pro Thr Asp Leu Ala Gly Val Ile Ile Glu
1               5                   10                  15

Leu Gly Pro Asn Asp Ser Pro Gln Thr Ser Glu Phe Lys Gly Ala Thr
            20                  25                  30

Glu Glu Ala Pro Ala Lys Glu Ser Pro His Thr Ser Glu Phe Lys Gly
        35                  40                  45

Ala Ala Arg Val Ser Pro Ile Ser Glu Ser Val Leu Ala Arg Leu Ser
    50                  55                  60

Lys Phe Glu Val Glu Asp Ala Glu Asn Val Ala Ser Tyr Asp Ser Lys
65                  70                  75                  80

Ile Lys Lys Ile Val His Ser Ile Val Ser Ser Phe Ala Phe Gly Leu
                85                  90                  95

Phe Gly Val Phe Leu Val Leu Leu Asp Val Thr Leu Ile Leu Ala Asp
            100                 105                 110

Leu Ile Phe Thr Asp Ser Lys Leu Tyr Ile Pro Leu Glu Tyr Arg Ser
        115                 120                 125

Ile Ser Leu Ala Ile Ala Leu Phe Phe Leu Met Asp Val Leu Leu Arg
    130                 135                 140

Val Phe Val Glu Arg Arg Gln Gln Tyr Phe Ser Asp Leu Phe Asn Ile
145                 150                 155                 160

Leu Asp Thr Ala Ile Ile Val Ile Leu Leu Leu Val Asp Val Val Tyr
                165                 170                 175

Ile Phe Phe Asp Ile Lys Leu Leu Arg Asn Ile Pro Arg Trp Thr His
            180                 185                 190

Leu Leu Arg Leu Leu Arg Leu Ile Ile Leu Leu Arg Ile Phe His Leu
        195                 200                 205

Phe His Gln Lys Arg Gln Leu Glu Lys Leu Ile Arg Arg Arg Val Ser
    210                 215                 220

Glu Asn Lys Arg Arg Tyr Thr Arg Asp Gly Phe Asp Leu Asp Leu Thr
225                 230                 235                 240

Tyr Val Thr Glu Arg Ile Ile Ala Met Ser Phe Pro Ser Ser Gly Arg
                245                 250                 255

Gln Ser Phe Tyr Arg Asn Pro Ile Lys Glu Val Val Arg Phe Leu Asp
            260                 265                 270

Lys Lys His Arg Asn His Tyr Arg Val Tyr Asn Leu Cys Ser Glu Arg
        275                 280                 285

Ala Tyr Asp Pro Lys His Phe His Asn Arg Val Val Arg Ile Met Ile
    290                 295                 300

Asp Asp His Asn Val Pro Thr Leu His Gln Met Val Val Phe Thr Lys
305                 310                 315                 320

Glu Val Asn Glu Trp Met Ala Gln Asp Leu Glu Asn Ile Val Ala Ile
                325                 330                 335

His Cys Lys Gly Gly Thr Asp Arg Thr Gly Thr Met Val Cys Ala Phe
            340                 345                 350

Leu Ile Ala Ser Glu Ile Cys Ser Thr Ala Lys Glu Ser Leu Tyr Tyr
        355                 360                 365

Phe Gly Glu Arg Arg Thr Asp Lys Thr His Ser Glu Lys Phe Gln Gly
    370                 375                 380

Val Glu Thr Pro Ser Gln Val Met Tyr Val Ile
385                 390                 395

<210>  59

<211>  468

<212>  PRT

<213>  Homo sapiens

<400>  59

Met Asn Glu Ser Pro Asp Pro Thr Asp Leu Ala Gly Val Ile Ile Glu
1               5                   10                  15

Leu Gly Pro Asn Asp Ser Pro Gln Thr Ser Glu Phe Lys Gly Ala Thr
            20                  25                  30

Glu Glu Ala Pro Ala Lys Glu Ser Pro His Thr Ser Glu Phe Lys Gly
        35                  40                  45

Ala Ala Arg Val Ser Pro Ile Ser Glu Ser Val Leu Ala Arg Leu Ser
    50                  55                  60

Lys Phe Glu Val Glu Asp Ala Glu Asn Val Ala Ser Tyr Asp Ser Lys
65                  70                  75                  80

Ile Lys Lys Ile Val His Ser Ile Val Ser Ser Phe Ala Phe Gly Leu
                85                  90                  95

Phe Gly Val Phe Leu Val Leu Leu Asp Val Thr Leu Ile Leu Ala Asp
            100                 105                 110

Leu Ile Phe Thr Asp Ser Lys Leu Tyr Ile Pro Leu Glu Tyr Arg Ser
        115                 120                 125

Ile Ser Leu Ala Ile Ala Leu Phe Phe Leu Met Asp Val Leu Leu Arg
    130                 135                 140

Val Phe Val Glu Arg Arg Gln Gln Tyr Phe Ser Asp Leu Phe Asn Ile
145                 150                 155                 160

Leu Asp Thr Ala Ile Ile Val Ile Leu Leu Leu Val Asp Val Val Tyr
                165                 170                 175

Ile Phe Phe Asp Ile Lys Leu Leu Arg Asn Ile Pro Arg Trp Thr His
            180                 185                 190

Leu Leu Arg Leu Leu Arg Leu Ile Ile Leu Leu Arg Ile Phe His Leu
        195                 200                 205

Phe His Gln Lys Arg Gln Leu Glu Lys Leu Ile Arg Arg Arg Val Ser
    210                 215                 220

Glu Asn Lys Arg Arg Tyr Thr Arg Asp Gly Phe Asp Leu Asp Leu Thr
225                 230                 235                 240

Tyr Val Thr Glu Arg Ile Ile Ala Met Ser Phe Pro Ser Ser Gly Arg
                245                 250                 255

Gln Ser Phe Tyr Arg Asn Pro Ile Lys Glu Val Val Arg Phe Leu Asp
            260                 265                 270

Lys Lys His Arg Asn His Tyr Arg Val Tyr Asn Leu Cys Ser Glu Arg
        275                 280                 285

Ala Tyr Asp Pro Lys His Phe His Asn Arg Val Val Arg Ile Met Ile
    290                 295                 300

Asp Asp His Asn Val Pro Thr Leu His Gln Met Val Val Phe Thr Lys
305                 310                 315                 320

Glu Val Asn Glu Trp Met Ala Gln Asp Leu Glu Asn Ile Val Ala Ile
                325                 330                 335

His Cys Lys Gly Gly Thr Gly Tyr Val Arg Asp Leu Lys Ile Gln Ile
            340                 345                 350

Glu Met Glu Lys Lys Val Val Phe Ser Thr Ile Ser Leu Gly Lys Cys
        355                 360                 365

Ser Val Leu Asp Asn Ile Thr Thr Asp Lys Ile Leu Ile Asp Val Phe
    370                 375                 380

Asp Gly Pro Pro Leu Tyr Asp Asp Val Lys Val Gln Phe Phe Tyr Ser
385                 390                 395                 400

Asn Leu Pro Thr Tyr Tyr Asp Asn Cys Ser Phe Tyr Phe Trp Leu His
                405                 410                 415

Thr Ser Phe Ile Glu Asn Asn Arg Leu Tyr Leu Pro Lys Asn Glu Leu
            420                 425                 430

Asp Asn Leu His Lys Gln Lys Ala Arg Arg Ile Tyr Pro Ser Asp Phe
        435                 440                 445

Ala Val Glu Ile Leu Phe Gly Glu Lys Met Thr Ser Ser Asp Val Val
    450                 455                 460

Ala Gly Ser Asp
465

<210> 60

<211> 470

<212> PRT

<213> Homo sapiens

<400> 60

Met Asn Glu Ser Pro Asp Pro Thr Asp Leu Ala Gly Val Ile Ile Glu
1               5                   10                  15

Leu Gly Pro Asn Asp Ser Pro Gln Thr Ser Glu Phe Lys Gly Ala Thr
            20                  25                  30

Glu Glu Ala Pro Ala Lys Glu Ser Val Leu Ala Arg Leu Ser Lys Phe
        35                  40                  45

Glu Val Glu Asp Ala Glu Asn Val Ala Ser Tyr Asp Ser Lys Ile Lys
    50                  55                  60

Lys Ile Val His Ser Ile Val Ser Ser Phe Ala Phe Gly Leu Phe Gly
65                  70                  75                  80

Val Phe Leu Val Leu Leu Asp Val Thr Leu Ile Leu Ala Asp Leu Ile
                85                  90                  95

Phe Thr Asp Ser Lys Leu Tyr Ile Pro Leu Glu Tyr Arg Ser Ile Ser
            100                 105                 110

Leu Ala Ile Ala Leu Phe Phe Leu Met Asp Val Leu Leu Arg Val Phe
        115                 120                 125

Val Glu Arg Arg Gln Gln Tyr Phe Ser Asp Leu Phe Asn Ile Leu Asp
    130                 135                 140

Thr Ala Ile Ile Val Ile Leu Leu Leu Val Asp Val Val Tyr Ile Phe
145                 150                 155                 160

Phe Asp Ile Lys Leu Leu Arg Asn Ile Pro Arg Trp Thr His Leu Leu
                165                 170                 175

Arg Leu Leu Arg Leu Ile Ile Leu Leu Arg Ile Phe His Leu Phe His
            180                 185                 190

Gln Lys Arg Gln Leu Glu Lys Leu Ile Arg Arg Arg Val Ser Glu Asn
        195                 200                 205

Lys Arg Arg Tyr Thr Arg Asp Gly Phe Asp Leu Asp Leu Thr Tyr Val
    210                 215                 220

Thr Glu Arg Ile Ile Ala Met Ser Phe Pro Ser Ser Gly Arg Gln Ser
225                 230                 235                 240

Phe Tyr Arg Asn Pro Ile Lys Glu Val Val Arg Phe Leu Asp Lys Lys
                245                 250                 255

His Arg Asn His Tyr Arg Val Tyr Asn Leu Cys Ser Glu Arg Ala Tyr
            260                 265                 270

Asp Pro Lys His Phe His Asn Arg Val Val Arg Ile Met Ile Asp Asp
        275                 280                 285

His Asn Val Pro Thr Leu His Gln Met Val Val Phe Thr Lys Glu Val
    290                 295                 300

Asn Glu Trp Met Ala Gln Asp Leu Glu Asn Ile Val Ala Ile His Cys
305                 310                 315                 320

Lys Gly Gly Thr Asp Arg Thr Gly Thr Met Val Cys Ala Phe Leu Ile
                325                 330                 335

Ala Ser Glu Ile Cys Ser Thr Ala Lys Glu Ser Leu Tyr Tyr Phe Gly
            340                 345                 350

Glu Arg Arg Thr Asp Lys Thr His Ser Glu Lys Phe Gln Gly Val Glu
        355                 360                 365

Thr Pro Ser Val Leu Asp Asn Ile Thr Thr Asp Lys Ile Leu Ile Asp
    370                 375                 380

Val Phe Asp Gly Pro Pro Leu Tyr Asp Asp Val Lys Val Gln Phe Phe
385                 390                 395                 400

Tyr Ser Asn Leu Pro Thr Tyr Tyr Asp Asn Cys Ser Phe Tyr Phe Trp
                405                 410                 415

Leu His Thr Ser Phe Ile Glu Asn Asn Arg Leu Tyr Leu Pro Lys Asn
            420                 425                 430

Glu Leu Asp Asn Leu His Lys Gln Lys Ala Arg Arg Ile Tyr Pro Ser
        435                 440                 445

Asp Phe Ala Val Glu Ile Leu Phe Gly Glu Lys Met Thr Ser Ser Asp
    450                 455                 460

Val Val Ala Gly Ser Asp
465                 470

<210> 61

<211> 450

<212> PRT

<213> Homo sapiens

<400> 61

Met Asn Glu Ser Pro Asp Pro Thr Asp Leu Ala Gly Val Ile Ile Glu
1               5                   10                  15

Leu Gly Pro Asn Asp Ser Pro Gln Thr Ser Glu Phe Lys Gly Ala Thr
            20                  25                  30

Glu Glu Ala Pro Ala Lys Glu Ser Val Leu Ala Arg Leu Ser Lys Phe
        35                  40                  45

Glu Val Glu Asp Ala Glu Asn Val Ala Ser Tyr Asp Ser Lys Ile Lys
    50                  55                  60

Lys Ile Val His Ser Ile Val Ser Ser Phe Ala Phe Gly Leu Phe Gly
65                  70                  75                  80

Val Phe Leu Val Leu Leu Asp Val Thr Leu Ile Leu Ala Asp Leu Ile
                85                  90                  95

Phe Thr Asp Ser Lys Leu Tyr Ile Pro Leu Glu Tyr Arg Ser Ile Ser
            100                 105                 110

Leu Ala Ile Ala Leu Phe Phe Leu Met Asp Val Leu Leu Arg Val Phe
        115                 120                 125

Val Glu Arg Arg Gln Gln Tyr Phe Ser Asp Leu Phe Asn Ile Leu Asp
    130                 135                 140

Thr Ala Ile Ile Val Ile Leu Leu Leu Val Asp Val Val Tyr Ile Phe
145                 150                 155                 160

Phe Asp Ile Lys Leu Leu Arg Asn Ile Pro Arg Trp Thr His Leu Leu
                165                 170                 175

Arg Leu Leu Arg Leu Ile Ile Leu Leu Arg Ile Phe His Leu Phe His
            180                 185                 190

Gln Lys Arg Gln Leu Glu Lys Leu Ile Arg Arg Arg Val Ser Glu Asn
        195                 200                 205

Lys Arg Arg Tyr Thr Arg Asp Gly Phe Asp Leu Asp Leu Thr Tyr Val
    210                 215                 220

Thr Glu Arg Ile Ile Ala Met Ser Phe Pro Ser Ser Gly Arg Gln Ser
225                 230                 235                 240

Phe Tyr Arg Asn Pro Ile Lys Glu Val Val Arg Phe Leu Asp Lys Lys
                245                 250                 255

His Arg Asn His Tyr Arg Val Tyr Asn Leu Cys Ser Glu Arg Ala Tyr
            260                 265                 270

Asp Pro Lys His Phe His Asn Arg Val Val Arg Ile Met Ile Asp Asp
        275                 280                 285

His Asn Val Pro Thr Leu His Gln Met Val Val Phe Thr Lys Glu Val
    290                 295                 300

Asn Glu Trp Met Ala Gln Asp Leu Glu Asn Ile Val Ala Ile His Cys
305                 310                 315                 320

Lys Gly Gly Thr Gly Tyr Val Arg Asp Leu Lys Ile Gln Ile Glu Met
                325                 330                 335

Glu Lys Lys Val Val Phe Ser Thr Ile Ser Leu Gly Lys Cys Ser Val
            340                 345                 350

Leu Asp Asn Ile Thr Thr Asp Lys Ile Leu Ile Asp Val Phe Asp Gly
        355                 360                 365

Pro Pro Leu Tyr Asp Asp Val Lys Val Gln Phe Phe Tyr Ser Asn Leu
    370                 375                 380

Pro Thr Tyr Tyr Asp Asn Cys Ser Phe Tyr Phe Trp Leu His Thr Ser
385                 390                 395                 400

Phe Ile Glu Asn Asn Arg Leu Tyr Leu Pro Lys Asn Glu Leu Asp Asn
                405                 410                 415

Leu His Lys Gln Lys Ala Arg Arg Ile Tyr Pro Ser Asp Phe Ala Val
            420                 425                 430

Glu Ile Leu Phe Gly Glu Lys Met Thr Ser Ser Asp Val Val Ala Gly
        435                 440                 445

Ser Asp
    450

<210>  62
<211>  1299
<212>  DNA
<213>  Homo sapiens

<400>  62
cgcccttaga catggctcag atgtgcagcc acagtgagct tctgaacatt tcttctcaga      60
ctaagctctt acacacagtt gcagttgaaa gaaagaattg cttgacatgg ccacaggagc     120
aggcagcttc ctgcagacat gacagtcaac gcaaactcat gtcactgtgg gcagacacat     180
gtttgcaaag agactcagag ccaaacaagc acactcaatg tgctttgccc aaatttaccc     240
attaggtaaa tcttccctcc tcccaagaag aaagtggaga gagcatgagt cctcacatgg     300
gaacttgaag tcagggaaat gaaggctcac caattatttg tgcatgggtt taagttttcc     360
ttgaaattaa gttcaggttt gtctttgtgt gtaccaatta atgacaagag gttagataga     420
agtatgctag atggcaaaga gaaatatgtt ttgtgtcttc aattttgcta aaaataaccc     480
agaacatgga taattcattt attaattgat tttggtaagc caagtcctat ttggagaaaa     540
ttaatagttt ttctaaaaaa gaattttctc aatatcacct ggcttgataa catttttctc     600
cttcgagttc ctttttctgg agtttaacaa acttgttctt tacaaataga ttatattgac     660
tacctctcac tgatgttatg atattagttt ctattgctta ctttgtattt ctaattttag     720
gattcacaat ttagctggag aactattttt taacctgttg cacctaaaca tgattgagct     780
agaagacagt tttaccatat gcatgcattt tctctgagtt atattttaaa atctatacat     840
ttctcctaaa tatggaggaa atcactggca tcaaatgcca gtctcagacg gaagacctaa     900
agcccatttc tggcctggag ctacttggct ttgtgaccta tggtgaggca taagtgctct     960
gagtttgtgt tgcctctttt gtaaaatgag ggtttgactt aatcagtgat tttcatagct    1020
taaaattttt ttgaagaaca gaactttttt taaaaacagt tagatgcaac catattatat    1080
aaaacagaac agatacaagt agagctaact tgctaaagaa aggatggagg ctctgaagct    1140
gtgacttcat tatcccttaa tactgctatg tcctctgtag taccttagat ttctatggga    1200
catcgtttaa aaactattgt ttatgcgaga gccttgctaa tttcctaaaa attgtggata    1260
cattttttct cccatgtata attttctcac cttctattt                            1299

<210>  63
<211>  405

<212>  DNA

<213>  Homo sapiens

<400>  63
gcacaaggcc tgctcttact ccaaaaagat ggacccaggt ccgaaggggc actgccactg      60

tggggggcat ggccatcctc caggtcactg cgggccaccc cctggccatg gcccagggcc      120

ctgcgggcca ccccccacca tggtccaggg ccctgcgggc caccccctgg ccatggccca      180

gggccctgcg ggccaccccc ccaccatggt ccagggccct gcgggcctcc ccctggccat      240

ggcccaggtc acccaccccc tggtccacat cactgaggaa gtagaagaaa acaggacaca      300

agatggcaag cctgagagaa ttgcccagct gacctggaat gaggcctaaa ccacaatctt      360

ctcttcctaa taaacagcct cctagaggcc acattctatt ctgta                      405

<210>  64

<211>  106

<212>  PRT

<213>  Homo sapiens

<400>  64

Met Asp Pro Gly Pro Lys Gly His Cys His Cys Gly Gly His Gly His
1               5                   10                  15

Pro Pro Gly His Cys Gly Pro Pro Pro Gly His Gly Pro Gly Pro Cys
            20                  25                  30

Gly Pro Pro Pro Thr Met Val Gln Gly Pro Ala Gly His Pro Leu Ala
        35                  40                  45

Met Ala Gln Gly Pro Ala Gly His Pro Pro Thr Met Val Gln Gly Pro
    50                  55                  60

Ala Gly Leu Pro Leu Ala Met Ala Gln Val Thr His Pro Leu Val His
65                  70                  75                  80

Ile Thr Glu Glu Val Glu Glu Asn Arg Thr Gln Asp Gly Lys Pro Glu
                85                  90                  95

Arg Ile Ala Gln Leu Thr Trp Asn Glu Ala
            100                 105

<210>  65

<211>  71

<212>  PRT

<213>  Homo sapiens

<400>  65

Met Ala Ile Leu Gln Val Thr Ala Gly His Pro Leu Ala Met Ala Gln
1               5                   10                  15

Gly Pro Ala Gly His Pro Pro Pro Trp Ser Arg Ala Leu Arg Ala Thr
            20                  25                  30

Pro Trp Pro Trp Pro Arg Ala Leu Arg Ala Thr Pro Pro Pro Trp Ser
        35                  40                  45

Arg Ala Leu Arg Ala Ser Pro Trp Pro Trp Pro Arg Ser Pro Thr Pro
    50                  55                  60

Trp Ser Thr Ser Leu Arg Lys
65                  70

<210>  66

<211>  21

<212>  DNA

<213>  Künstliche Sequenz

<220>
<223>  Beschreibung der künstlichen Sequenz: Oligonukleotid

<400>  66
agacatggct cagatgtgca g                                              21

<210>  67

<211>  21

<212>  DNA

<213>  Künstliche Sequenz

<220>
<223>  Beschreibung der künstlichen Sequenz: Oligonukleotid

<400>  67
ggaaattagc aaggctctcg c                                              21

<210>  68

<211>  21

<212>  DNA

<213>  Künstliche Sequenz

<220>
<223>  Beschreibung der künstlichen Sequenz: Oligonukleotid

<400>  68
tcaggtattc cctgctctta c                                                21

<210>  69
<211>  21
<212>  DNA
<213>  Künstliche Sequenz

<220>
<223>  Beschreibung der künstlichen Sequenz: Oligonukleotid

<400>  69
tgggcaattc tctcaggctt g                                                21

<210>  70
<211>  908
<212>  DNA
<213>  Homo sapiens

<400>  70
aaaattcggc acgaggccgg gctgtggtct agcataaagg cggagcccag aagaaggggc      60
ggggtatggg agaagcctcc ccacctgccc ccgcaaggcg gcatctgctg gtcctgctgc     120
tgctcctctc taccctggtg atcccctccg ctgcagctcc tatccatgat gctgacgccc     180
aagagagctc cttgggtctc acaggcctcc agagcctact ccaaggcttc agccgacttt     240
tcctgaaagg taacctgctt cggggcatag acagcttatt ctctgccccc atggacttcc     300
ggggcctccc tgggaactac cacaaagagg agaaccagga gcaccagctg gggaacaaca     360
ccctctccag ccacctccag atcgacaaga tgaccgacaa caagacagga gaggtgctga     420
tctccgagaa tgtggtggca tccattcaac cagcggaggg gagcttcgag ggtgatttga     480
aggtacccag gatggaggag aaggaggccc tggtacccat ccagaaggcc acggacagct     540
tccacacaga actccatccc cgggtggcct tctggatcat taagctgcca cggcggaggt     600
cccaccagga tgccctggag ggcggccact ggctcagcga gaagcgacac cgcctgcagg     660
ccatccggga tggactccgc aaggggaccc acaaggacgt cctagaagag gggaccgaga     720
gctcctccca ctccaggctg tccccccgaa agacccactt actgtacatc ctcaggccct     780
ctcggcagct gtaggggtgg ggaccgggga gcacctgcct gtagccccca tcagaccctg     840
ccccaagcac catatggaaa taaagttctt tcttacatct aaaaaaaaaa aaaaaaaaaa     900

aaaaaaaa 908

<210> 71

<211> 242

<212> PRT

<213> Homo sapiens

<400> 71

Met Gly Glu Ala Ser Pro Pro Ala Pro Ala Arg Arg His Leu Leu Val
1               5                   10                  15

Leu Leu Leu Leu Leu Ser Thr Leu Val Ile Pro Ser Ala Ala Ala Pro
            20                  25                  30

Ile His Asp Ala Asp Ala Gln Glu Ser Ser Leu Gly Leu Thr Gly Leu
        35                  40                  45

Gln Ser Leu Leu Gln Gly Phe Ser Arg Leu Phe Leu Lys Gly Asn Leu
    50                  55                  60

Leu Arg Gly Ile Asp Ser Leu Phe Ser Ala Pro Met Asp Phe Arg Gly
65                  70                  75                  80

Leu Pro Gly Asn Tyr His Lys Glu Glu Asn Gln Glu His Gln Leu Gly
                85                  90                  95

Asn Asn Thr Leu Ser Ser His Leu Gln Ile Asp Lys Met Thr Asp Asn
            100                 105                 110

Lys Thr Gly Glu Val Leu Ile Ser Glu Asn Val Val Ala Ser Ile Gln
        115                 120                 125

Pro Ala Glu Gly Ser Phe Glu Gly Asp Leu Lys Val Pro Arg Met Glu
    130                 135                 140

Glu Lys Glu Ala Leu Val Pro Ile Gln Lys Ala Thr Asp Ser Phe His
145                 150                 155                 160

Thr Glu Leu His Pro Arg Val Ala Phe Trp Ile Ile Lys Leu Pro Arg
                165                 170                 175

Arg Arg Ser His Gln Asp Ala Leu Glu Gly Gly His Trp Leu Ser Glu
            180                 185                 190

Lys Arg His Arg Leu Gln Ala Ile Arg Asp Gly Leu Arg Lys Gly Thr
        195                 200                 205

His Lys Asp Val Leu Glu Glu Gly Thr Glu Ser Ser Ser His Ser Arg

210                 215                 220

Leu Ser Pro Arg Lys Thr His Leu Leu Tyr Ile Leu Arg Pro Ser Arg
225                 230                 235                 240

Gln Leu

<210>  72

<211>  21

<212>  DNA

<213>  Künstliche Sequenz

<220>
<223>  Beschreibung der künstlichen Sequenz: Oligonukleotid

<400>  72
ctcctatcca tgatgctgac g                                              21

<210>  73

<211>  21

<212>  DNA

<213>  Künstliche Sequenz

<220>
<223>  Beschreibung der künstlichen Sequenz: Oligonukleotid

<400>  73
cctgaggatg tacagtaagt g                                              21

<210>  74

<211>  2987

<212>  DNA

<213>  Homo sapiens

<400>  74
tttcccagcg aggtggtcat tcagagccta cacatctgtt ctgtatttta acccatggat      60

gagaatattc attcaagcca agagagttaa aactaaacat ctttgctatt gcctctacag     120

acccagaaag tatctttatg tcacatcttc ttttaaagga gcatttaaag atgaagttaa     180

aaaggcagaa gaagcagtaa agattgctga atccatattg aaagaagcac aaatcaaagt     240

aaaccagtgt gacagaacct ctttatcttc tgccaaggat gtattacaga gagctttgga     300

agatgtagaa gcaaagcaaa agaatcttaa agagaaacaa agagaattaa aaacagcaag     360

aacgctctcc ctgttctatg gagtgaacgt agaaaaccga agccaagctg gaatgttcat 420
ttacagtaat aaccgtttga tcaaaatgca tgaaaaagtg ggctcacagt tgaaactgaa 480
gtccttactt ggcgcaggcg tggttggaat tgttaatata cccttggagg tcatggaacc 540
atcccataat aaacaggaat ttctcaatgt ccaagagtat aatcatctac taaaagtcat 600
gggacagtac ttggtccagt actgtaagga caccggcatc aataatagaa atttaacatt 660
gttttgcaat gaatttggat accagaatga catcgatgtg gagaaacctt taaattcttt 720
tcaatatcaa agaagacaag ccatgggtat cccattcatc atacaatgtg atctttgtct 780
taaatggaga gtcttgcctt cctctactaa ttatcaggaa aaagaatttt ttgacatttg 840
gatttgtgct aataatccca accgcttgga aaacagttgt catcaggtag aatgtctacc 900
ttccatccca ctgggcacca tgagcacaat atcaccatca aaaaatgaga aagagaagca 960
acttagagag tcggtcataa agtatcaaaa tagactggca gaacagcagc cacagcctca 1020
atttatacca gtggacgaaa tcactgtcac ttccacctgc ctaacttcag cacataagga 1080
aaataccaaa acccagaaaa tcaggctttt gggcgatgac ttgaagcatg aatctctttc 1140
atcctttgag ctttcagcga gccgtagagg acagaaaaga aacatagaag agacagactc 1200
tgatgtagag tatatttcag aaacaaaaat tatgaaaaag tctatggagg agaaaatgaa 1260
ctctcaacag cagagaattc cagtagctct gccagaaaat gtcaaactag ctgagagatc 1320
ccagagaagt cagattgcta atattaccac tgtctggaga gctcaaccaa ctgaagggtg 1380
cctgaagaat gcccaggccg cttcttggga aatgaaaagg aagcagagtc tgaactttgt 1440
agaggaatgt aaggtattga ctgaagatga gaacacgagt gattcagata taatcctggt 1500
ttcagataaa agcaacactg atgtttcatt gaaacaagaa aaaaaggaaa ttcctctttt 1560
aaaccaagaa aaacaggagc tgtgcaatga tgttctagca atgaaaagaa gctcttcatt 1620
acctagctgg aaaagcttgc tcaatgtgcc gatggaagat gtgaatctaa gttctggaca 1680
catagccaga gtttctgtga gtggcagttg taaagttgct tcttcgccag cgtcttctca 1740
aagcacacct gtcaaggaaa cagtgagaaa actgaagtct aagttaaggg agattcttct 1800
gtattttttt cctgagcatc agctaccatc agaattggaa gaacctgcat taagttgtga 1860
gctggagcag tgcccagagc agatgaacaa aaagctgaaa atgtgtttca accagataca 1920
gaatacttac atggtccaat atgaaaaaaa aataaagagg aaattgcagt ccattatcta 1980
tgattcaaat acaagaggaa tacataatga aatctctctg gggcaatgtg aaaataaaag 2040
aaaaatctct gaggataagc tgaagaatct tcgtataaaa ctggcactat tgttgcagaa 2100
actccaactg ggtggtccag aaggtgacct ggagcagact gacacttatt tagaagcttt 2160
gcttaaagaa gataatcttc tcttccagaa caatttaaat aaagtaacta tagatgcaag 2220
acatagactc cctttagaaa aaaatgaaaa gacttcggaa aattaagtca gagatggtat 2280
taccttttaa aaaatgctaa taagaaaatt ggaagattct tttaaaaatt tttctttttt 2340
gttgttgtta ctgtaaagtc tattctgttt aacaataaga aataagaaat aatttttttc 2400

aaataagaaa attgtgtact ctagaaatgg agaccgattt acaatttatg tattccctaa 2460

tccaattatc taaatcttcc ttttctttca gaaatattaa taatatctag agttctctaa 2520

ttttcatgtg agctactgaa aaaaatgaaa atgtcactca agcttaactt ttgttattcc 2580

ttaaaagatt gttattgtaa ttttgttatt ccttaaaaac atttaaaagc agattttttc 2640

aaaatcgata tgtgaaggac tacagaatca cctcctcttg aagatattga aaaagaaaga 2700

cattatgccc tttctccact atagccaaca ctcagtcaag cagaaaatac aaatcccccc 2760

aaaactttga gacatagctt atataatttt attatttagt catagtaaaa gaataaatct 2820

cctaagcata atatgtatac atattacaca tatgtaaaaa ttgttgtttt acatttacat 2880

atacgtaaag aagtatgttt ttacactttt cttgataagt gttttttttt tgtttagaaa 2940

tgtctgaaac tttagacaaa aacagtaaaa catttaatat tcatttg 2987

<210> 75

<211> 735

<212> PRT

<213> Homo sapiens

<400> 75

Met Arg Ile Phe Ile Gln Ala Lys Arg Val Lys Thr Lys His Leu Cys
1               5                   10                  15

Tyr Cys Leu Tyr Arg Pro Arg Lys Tyr Leu Tyr Val Thr Ser Ser Phe
            20                  25                  30

Lys Gly Ala Phe Lys Asp Glu Val Lys Lys Ala Glu Glu Ala Val Lys
        35                  40                  45

Ile Ala Glu Ser Ile Leu Lys Glu Ala Gln Ile Lys Val Asn Gln Cys
    50                  55                  60

Asp Arg Thr Ser Leu Ser Ser Ala Lys Asp Val Leu Gln Arg Ala Leu
65                  70                  75                  80

Glu Asp Val Glu Ala Lys Gln Lys Asn Leu Lys Glu Lys Gln Arg Glu
                85                  90                  95

Leu Lys Thr Ala Arg Thr Leu Ser Leu Phe Tyr Gly Val Asn Val Glu
            100                 105                 110

Asn Arg Ser Gln Ala Gly Met Phe Ile Tyr Ser Asn Asn Arg Leu Ile
        115                 120                 125

Lys Met His Glu Lys Val Gly Ser Gln Leu Lys Leu Lys Ser Leu Leu
    130                 135                 140

Gly Ala Gly Val Val Gly Ile Val Asn Ile Pro Leu Glu Val Met Glu
145                 150                 155                 160

Pro Ser His Asn Lys Gln Glu Phe Leu Asn Val Gln Glu Tyr Asn His
                165                 170                 175

Leu Leu Lys Val Met Gly Gln Tyr Leu Val Gln Tyr Cys Lys Asp Thr
            180                 185                 190

Gly Ile Asn Asn Arg Asn Leu Thr Leu Phe Cys Asn Glu Phe Gly Tyr
        195                 200                 205

Gln Asn Asp Ile Asp Val Glu Lys Pro Leu Asn Ser Phe Gln Tyr Gln
    210                 215                 220

Arg Arg Gln Ala Met Gly Ile Pro Phe Ile Ile Gln Cys Asp Leu Cys
225                 230                 235                 240

Leu Lys Trp Arg Val Leu Pro Ser Ser Thr Asn Tyr Gln Glu Lys Glu
                245                 250                 255

Phe Phe Asp Ile Trp Ile Cys Ala Asn Asn Pro Asn Arg Leu Glu Asn
            260                 265                 270

Ser Cys His Gln Val Glu Cys Leu Pro Ser Ile Pro Leu Gly Thr Met
        275                 280                 285

Ser Thr Ile Ser Pro Ser Lys Asn Glu Lys Glu Lys Gln Leu Arg Glu
    290                 295                 300

Ser Val Ile Lys Tyr Gln Asn Arg Leu Ala Glu Gln Gln Pro Gln Pro
305                 310                 315                 320

Gln Phe Ile Pro Val Asp Glu Ile Thr Val Thr Ser Thr Cys Leu Thr
                325                 330                 335

Ser Ala His Lys Glu Asn Thr Lys Thr Gln Lys Ile Arg Leu Leu Gly
            340                 345                 350

Asp Asp Leu Lys His Glu Ser Leu Ser Ser Phe Glu Leu Ser Ala Ser
        355                 360                 365

Arg Arg Gly Gln Lys Arg Asn Ile Glu Glu Thr Asp Ser Asp Val Glu
    370                 375                 380

Tyr Ile Ser Glu Thr Lys Ile Met Lys Lys Ser Met Glu Glu Lys Met
385                 390                 395                 400

Asn Ser Gln Gln Gln Arg Ile Pro Val Ala Leu Pro Glu Asn Val Lys
                405                 410                 415

Leu Ala Glu Arg Ser Gln Arg Ser Gln Ile Ala Asn Ile Thr Thr Val
            420                 425                 430

Trp Arg Ala Gln Pro Thr Glu Gly Cys Leu Lys Asn Ala Gln Ala Ala
        435                 440                 445

Ser Trp Glu Met Lys Arg Lys Gln Ser Leu Asn Phe Val Glu Glu Cys
    450                 455                 460

Lys Val Leu Thr Glu Asp Glu Asn Thr Ser Asp Ser Asp Ile Ile Leu
465                 470                 475                 480

Val Ser Asp Lys Ser Asn Thr Asp Val Ser Leu Lys Gln Glu Lys Lys
                485                 490                 495

Glu Ile Pro Leu Leu Asn Gln Glu Lys Gln Glu Leu Cys Asn Asp Val
            500                 505                 510

Leu Ala Met Lys Arg Ser Ser Ser Leu Pro Ser Trp Lys Ser Leu Leu
        515                 520                 525

Asn Val Pro Met Glu Asp Val Asn Leu Ser Ser Gly His Ile Ala Arg
    530                 535                 540

Val Ser Val Ser Gly Ser Cys Lys Val Ala Ser Ser Pro Ala Ser Ser
545                 550                 555                 560

Gln Ser Thr Pro Val Lys Glu Thr Val Arg Lys Leu Lys Ser Lys Leu
                565                 570                 575

Arg Glu Ile Leu Leu Tyr Phe Phe Pro Glu His Gln Leu Pro Ser Glu
            580                 585                 590

Leu Glu Glu Pro Ala Leu Ser Cys Glu Leu Glu Gln Cys Pro Glu Gln
        595                 600                 605

Met Asn Lys Lys Leu Lys Met Cys Phe Asn Gln Ile Gln Asn Thr Tyr
    610                 615                 620

Met Val Gln Tyr Glu Lys Lys Ile Lys Arg Lys Leu Gln Ser Ile Ile
625                 630                 635                 640

Tyr Asp Ser Asn Thr Arg Gly Ile His Asn Glu Ile Ser Leu Gly Gln
                645                 650                 655

Cys Glu Asn Lys Arg Lys Ile Ser Glu Asp Lys Leu Lys Asn Leu Arg
            660                 665                 670

Ile Lys Leu Ala Leu Leu Leu Gln Lys Leu Gln Leu Gly Gly Pro Glu
        675                 680                 685

Gly Asp Leu Glu Gln Thr Asp Thr Tyr Leu Glu Ala Leu Leu Lys Glu
    690                 695                 700

Asp Asn Leu Leu Phe Gln Asn Asn Leu Asn Lys Val Thr Ile Asp Ala
705                 710                 715                 720

Arg His Arg Leu Pro Leu Glu Lys Asn Glu Lys Thr Ser Glu Asn
                725                 730                 735

<210>  76

<211>  21

<212>  DNA

<213>  Künstliche Sequenz

<220>
<223>  Beschreibung der künstlichen Sequenz: Oligonukleotid

<400>  76
ctgagtatca gctaccatca g                                                21

<210>  77

<211>  21

<212>  DNA

<213>  Künstliche Sequenz

<220>
<223>  Beschreibung der künstlichen Sequenz: Oligonukleotid

<400>  77
tctgtagtcc ttcacatatc g                                                21

<210>  78

<211>  21

<212>  DNA

<213>  Künstliche Sequenz

<220>
<223>  Beschreibung der künstlichen Sequenz: Oligonukleotid

<400>  78
ttttgtctat ggtgtaggac c                                                21

<210>  79

<211>  21

<212>  DNA

<213>  Künstliche Sequenz

<220>
<223>  Beschreibung der künstlichen Sequenz: Oligonukleotid

<400>  79
ggaatggcaa tgatgttaca g                                                21

<210>  80

<211>  20

<212>  PRT

<213>  Homo sapiens

<400>  80

Met Ser Thr Val Lys Glu Gln Leu Ile Glu Lys Leu Ile Glu Asp Asp
1               5                   10                  15

Glu Asn Ser Gln
            20

<210>  81

<211>  14

<212>  PRT

<213>  Homo sapiens

<400>  81

Phe Thr Asp Ser Lys Leu Tyr Ile Pro Leu Glu Tyr Arg Ser
1               5                   10

<210>  82

<211>  13

<212>  PRT

<213>  Homo sapiens

<400>  82

Phe Asp Ile Lys Leu Leu Arg Asn Ile Pro Arg Trp Thr
1               5                   10

<210>  83

<211>  15

<212>  PRT
<213>  Homo sapiens

<400>  83
Gly Val Ala Gly Gln Asp Tyr Trp Ala Val Leu Ser Gly Lys Gly
1               5                   10                  15

<210>  84
<211>  10
<212>  PRT
<213>  Homo sapiens

<400>  84
Ser Arg Glu Val Thr Thr Asn Ala Gln Arg
1               5                   10

<210>  85
<211>  216
<212>  DNA
<213>  Homo sapiens

<400>  85
tgctcttact ccaaaaagat ggacccaggg ccctgcgggc ctccccctgg ccatggccca      60
ggtcacccac cccctggtcc acatcactga ggaagtagaa gaaaacagga cacaagatgg     120
caagcctgag agaattgccc agctgacctg gaaggaggcc taaaccgcaa tattctcttc     180
ctaataaaca gcctcctaga ggccacattc tattct                               216

<210>  86
<211>  227
<212>  DNA
<213>  Homo sapiens

<400>  86
tgctcttact ccaaaaagat ggacccaggt ccgaaggggc actgccactg tgggggcat      60
ggccatcctc caggtcaccc accccctggt ccacatcact gaggaagtag aagaaaacag     120
gacacaagat ggcaagcctg agagaattgc ccagctgacc tggaatgagg cctaaaccac     180
aatcttctct tcctaataaa cagcctccta gaggccacat tctattc                   227

<210> 87

<211> 261

<212> DNA

<213> Homo sapiens

<400> 87
tgctcttact ccaaaaagat ggacccaggt ccgaaggggc actgccactg tggggggcat 60

ggccatcctc caggtcactg cgggcctccc cctggccatg gcccaggtca cccaccccct 120

ggtccacatc actgaggaag tagaagaaaa caggacacaa gatggcaagc ctgagagaat 180

tgcccagctg acctggaatg aggcctaaac cacaatcttc tcttcctaat aaacagcctc 240

ctagaggcca cattctattc t 261

<210> 88

<211> 327

<212> DNA

<213> Homo sapiens

<400> 88
tgctcttact ccaaaaagat ggacccaggt ccgaaggggc actgccactg tgggggcat 60

ggccatcctc caggtcactg cgggccaccc ccccaccatg gtccagggcc ctgcgggcca 120

cccccccacc atggtccagg gccctgcggg cctccccctg gccatggccc aggtcaccca 180

ccccctggtc cacatcactg aggaagtaga agaaaacagg acacaagatg gcaagcctga 240

gagaattgcc cagctgacct ggaatgaggc ctaaaccaca atcttctctt cctaataaac 300

agcctcctag aggccacatt ctattct 327

<210> 89

<211> 31

<212> PRT

<213> Homo sapiens

<400> 89

Leu Leu Leu Gln Lys Asp Gly Pro Arg Ala Leu Arg Ala Ser Pro Trp
1               5                   10                  15

Pro Trp Pro Arg Ser Pro Thr Pro Trp Ser Thr Ser Leu Arg Lys
            20                  25                  30

<210> 90

<211> 23

<212> PRT

<213> Homo sapiens

<400> 90

Met Asp Pro Gly Pro Cys Gly Pro Pro Pro Gly His Gly Pro Gly His
1               5                   10                  15

Pro Pro Pro Gly Pro His His
            20

<210> 91

<211> 36

<212> PRT

<213> Homo sapiens

<400> 91

Met Ala Gln Val Thr His Pro Leu Val His Ile Thr Glu Glu Val Glu
1               5                   10                  15

Glu Asn Arg Thr Gln Asp Gly Lys Pro Glu Arg Ile Ala Gln Leu Thr
            20                  25                  30

Trp Lys Glu Ala
        35

<210> 92

<211> 34

<212> PRT

<213> Homo sapiens

<400> 92

Leu Leu Gln Lys Asp Gly Pro Arg Ser Glu Gly Ala Leu Pro Leu Trp
1               5                   10                  15

Gly Ala Trp Pro Ser Ser Arg Ser Pro Thr Pro Trp Ser Thr Ser Leu
            20                  25                  30

Arg Lys

<210> 93

<211>  27

<212>  PRT

<213>  Homo sapiens


<400>  93

Met Asp Pro Gly Pro Lys Gly His Cys His Cys Gly Gly His Gly His
1               5                   10                  15

Pro Pro Gly His Pro Pro Pro Gly Pro His His
            20                  25

<210>  94

<211>  38

<212>  PRT

<213>  Homo sapiens


<400>  94

Met Ala Ile Leu Gln Val Thr His Pro Leu Val His Ile Thr Glu Glu
1               5                   10                  15

Val Glu Glu Asn Arg Thr Gln Asp Gly Lys Pro Glu Arg Ile Ala Gln
            20                  25                  30

Leu Thr Trp Asn Glu Ala
        35

<210>  95

<211>  46

<212>  PRT

<213>  Homo sapiens


<400>  95

Leu Leu Leu Gln Lys Asp Gly Pro Arg Ser Glu Gly Ala Leu Pro Leu
1               5                   10                  15

Trp Gly Ala Trp Pro Ser Ser Arg Ser Leu Arg Ala Ser Pro Trp Pro
            20                  25                  30

Trp Pro Arg Ser Pro Thr Pro Trp Ser Thr Ser Leu Arg Lys
        35                  40                  45

<210>  96

<211>  38

<212>  PRT

<213>  Homo sapiens


<400>  96

Met Asp Pro Gly Pro Lys Gly His Cys His Cys Gly Gly His Gly His
1               5                   10                  15

Pro Pro Gly His Cys Gly Pro Pro Pro Gly His Gly Pro Gly His Pro
            20                  25                  30

Pro Pro Gly Pro His His
        35

<210>  97

<211>  49

<212>  PRT

<213>  Homo sapiens


<400>  97

Met Ala Ile Leu Gln Val Thr Ala Gly Leu Pro Leu Ala Met Ala Gln
1               5                   10                  15

Val Thr His Pro Leu Val His Ile Thr Glu Glu Val Glu Glu Asn Arg
            20                  25                  30

Thr Gln Asp Gly Lys Pro Glu Arg Ile Ala Gln Leu Thr Trp Asn Glu
        35                  40                  45

Ala


<210>  98

<211>  68

<212>  PRT

<213>  Homo sapiens


<400>  98

<210>  99

<211>  60

<212>  PRT

<213>  Homo sapiens

<400>  99

Met Asp Pro Gly Pro Lys Gly His Cys His Cys Gly Gly His Gly His
1               5                   10                  15

Pro Pro Gly His Cys Gly Pro Pro Pro His His Gly Pro Gly Pro Cys
            20                  25                  30

Gly Pro Pro Pro His His Gly Pro Gly Pro Cys Gly Pro Pro Pro Gly
        35                  40                  45

His Gly Pro Gly His Pro Pro Pro Gly Pro His His
    50                  55                  60

<210>  100

<211>  71

<212>  PRT

<213>  Homo sapiens

<400>  100

Met Ala Ile Leu Gln Val Thr Ala Gly His Pro Pro Thr Met Val Gln
1               5                   10                  15

Gly Pro Ala Gly His Pro Pro Thr Met Val Gln Gly Pro Ala Gly Leu
            20                  25                  30

Pro Leu Ala Met Ala Gln Val Thr His Pro Leu Val His Ile Thr Glu
        35                  40                  45

Glu Val Glu Glu Asn Arg Thr Gln Asp Gly Lys Pro Glu Arg Ile Ala

50                    55                    60

Gln Leu Thr Trp Asn Glu Ala
65                  70

<210>  101
<211>  15
<212>  PRT
<213>  Homo sapiens

<400>  101

Arg Asn Gly Val Ser Asp Val Val Lys Ile Asn Leu Asn Ser Glu
1               5                   10                  15

<210>  102
<211>  15
<212>  PRT
<213>  Homo sapiens

<400>  102

Gly Ile Ile Trp Asn Lys Arg Arg Thr Leu Ser Gln Tyr Pro Leu
1               5                   10                  15

<210>  103
<211>  15
<212>  PRT
<213>  Homo sapiens

<400>  103

Phe Thr Asp Ser Lys Leu Tyr Ile Pro Leu Glu Tyr Arg Ser Cys
1               5                   10                  15

<210>  104
<211>  14
<212>  PRT
<213>  Homo sapiens

<400>  104

Cys Phe Asp Ile Lys Leu Leu Arg Asn Ile Pro Arg Trp Thr
1               5                   10

<210>  105
<211>  54
<212>  PRT
<213>  Homo sapiens

<400>  105

Met Asn Glu Ser Pro Asp Pro Thr Asp Leu Ala Gly Val Ile Ile Glu
1               5                   10                  15

Leu Gly Pro Asn Asp Ser Pro Gln Thr Ser Glu Phe Lys Gly Ala Thr
            20                  25                  30

Glu Glu Ala Pro Ala Lys Glu Ser Pro His Thr Ser Glu Phe Lys Gly
        35                  40                  45

Ala Ala Arg Val Ser Pro
    50

<210>  106
<211>  16
<212>  PRT
<213>  Homo sapiens

<400>  106

Cys Tyr Gln His Lys Val Thr Leu His Met Ile Thr Glu Arg Asp Pro
1               5                   10                  15

<210>  107
<211>  15
<212>  PRT
<213>  Homo sapiens

<400>  107

Cys Arg Ile Pro Gln Val His Thr Met Asp Ser Ser Gly Lys Ile
1               5                   10                  15

<210>  108
<211>  541
<212>  PRT
<213>  Homo sapiens

<400>  108

Arg Arg Lys Gln Ser Glu Met His Ile Ser Arg Tyr Ser Ser Glu Gln
1               5                   10                  15

Ser Ala Arg Leu Leu Asp Tyr Glu Asp Gly Arg Gly Ser Arg His Ala
            20                  25                  30

Tyr Ser Thr Gln Ser Asp Thr Ser Cys Asp Asn Arg Glu Arg Ser Lys
        35                  40                  45

Arg Asp Tyr Thr Pro Ser Thr Asn Ser Leu Ala Leu Ser Arg Ser Ser
    50                  55                  60

Ile Ala Leu Pro Gln Gly Ser Met Ser Ser Ile Lys Cys Leu Gln Thr
65                  70                  75                  80

Thr Glu Glu Leu Pro Ser Arg Thr Ala Gly Ala Met Met Gln Phe Thr
                85                  90                  95

Ala Pro Ile Pro Gly Ala Thr Gly Pro Ile Lys Leu Ser Gln Lys Thr
            100                 105                 110

Ile Val Gln Thr Pro Gly Pro Ile Val Gln Tyr Pro Gly Pro Asn Val
        115                 120                 125

Arg Ser His Pro His Thr Ile Thr Gly Pro Pro Ser Ala Pro Arg Gly

130                 135                 140

Pro Pro Met Ala Pro Ile Ile Ile Ser Gln Arg Thr Ala Ser Gln Leu
145                 150                 155                 160

Ala Ala Pro Ile Ile Ile Ser Gln Arg Thr Ala Arg Ile Pro Gln Val
                165                 170                 175

His Thr Met Asp Ser Ser Gly Lys Thr Thr Leu Thr Pro Val Val Ile
            180                 185                 190

Leu Thr Gly Tyr Met Asp Glu Glu Leu Ala Lys Lys Ser Cys Ser Lys
        195                 200                 205

Ile Gln Ile Leu Lys Cys Gly Gly Thr Ala Arg Ser Gln Asn Ser Arg
    210                 215                 220

Glu Glu Asn Lys Glu Ala Leu Lys Asn Asp Ile Ile Phe Thr Asn Ser
225                 230                 235                 240

Val Glu Ser Leu Lys Ser Ala His Ile Lys Glu Pro Glu Arg Glu Gly
                245                 250                 255

Lys Gly Thr Asp Leu Glu Lys Asp Lys Ile Gly Met Glu Val Lys Val
            260                 265                 270

Asp Ser Asp Ala Gly Ile Pro Lys Arg Gln Glu Thr Gln Leu Lys Ile
        275                 280                 285

Ser Glu Met Ser Ile Pro Gln Gly Gln Gly Ala Gln Ile Lys Lys Ser
    290                 295                 300

Val Ser Asp Val Pro Arg Gly Gln Glu Ser Gln Val Lys Lys Ser Glu
305                 310                 315                 320

Ser Gly Val Pro Lys Gly Gln Glu Ala Gln Val Thr Lys Ser Gly Leu
                325                 330                 335

Val Val Leu Lys Gly Gln Glu Ala Gln Val Glu Lys Ser Glu Met Gly
            340                 345                 350

Val Pro Arg Arg Gln Glu Ser Gln Val Lys Lys Ser Gln Ser Gly Val
        355                 360                 365

Ser Lys Gly Gln Glu Ala Gln Val Lys Lys Arg Glu Ser Val Val Leu
    370                 375                 380

Lys Gly Gln Glu Ala Gln Val Glu Lys Ser Glu Leu Lys Val Pro Lys
385                 390                 395                 400

Gly Gln Glu Gly Gln Val Glu Lys Thr Glu Ala Asp Val Pro Lys Glu

405                 410                 415

Gln Glu Val Gln Glu Lys Lys Ser Glu Ala Gly Val Leu Lys Gly Pro
            420                 425                 430

Glu Ser Gln Val Lys Asn Thr Glu Val Ser Val Pro Glu Thr Leu Glu
        435                 440                 445

Ser Gln Val Lys Lys Ser Glu Ser Gly Val Leu Lys Gly Gln Glu Ala
    450                 455                 460

Gln Glu Lys Lys Glu Ser Phe Glu Asp Lys Gly Asn Asn Asp Lys Glu
465                 470                 475                 480

Lys Glu Arg Asp Ala Glu Lys Asp Pro Asn Lys Lys Glu Lys Gly Asp
                485                 490                 495

Lys Asn Thr Lys Gly Asp Lys Gly Lys Asp Lys Val Lys Gly Lys Arg
            500                 505                 510

Glu Ser Glu Ile Asn Gly Glu Lys Ser Lys Gly Ser Lys Arg Ala Lys
        515                 520                 525

Ala Asn Thr Gly Arg Lys Tyr Asn Lys Lys Val Glu Glu
    530                 535                 540

<210>  109
<211>  16
<212>  PRT
<213>  Homo sapiens

<400>  109

Met Met Ser Ser Lys Pro Thr Ser His Ala Glu Val Asn Glu Thr Cys
1               5                   10                  15

<210>  110
<211>  16
<212>  PRT
<213>  Homo sapiens

<400>  110

Cys Gly Val Ala Gly Gln Asp Tyr Trp Ala Val Leu Ser Gly Lys Gly
1               5                   10                  15

<210>  111
<211>  63
<212>  PRT
<213>  Homo sapiens

<400>  111

Met Met Ser Ser Lys Pro Thr Ser His Ala Glu Val Asn Glu Thr Ile
1               5                   10                  15

Pro Asn Pro Tyr Pro Pro Gly Ser Phe Met Ala Pro Gly Phe Gln Gln
            20                  25                  30

Pro Leu Gly Ser Ile Asn Leu Glu Asn Gln Ala Gln Gly Ala Gln Arg
        35                  40                  45

Ala Gln Pro Tyr Gly Ile Thr Ser Pro Gly Ile Phe Ala Ser Ser
    50                  55                  60

<210>  112
<211>  13
<212>  PRT
<213>  Homo sapiens

<400>  112

Gly His Gly Pro Gly His Pro Pro Pro Gly Pro His His
1               5                   10

<210>  113
<211>  13
<212>  PRT
<213>  Homo sapiens

<400>  113

Lys Pro Glu Arg Ile Ala Gln Leu Thr Trp Asn Glu Ala
1               5                   10

<210>  114
<211>  13
<212>  PRT
<213>  Homo sapiens

<400>  114

Pro Arg Ser Pro Thr Pro Trp Ser Thr Ser Leu Arg Lys
1               5                   10

<210>  115
<211>  13
<212>  PRT
<213>  Homo sapiens

<400>  115

Ile Ala Pro Asn Thr Arg Gly Gln Gln Thr Ile Val Leu
1               5                   10

<210>  116
<211>  14
<212>  PRT
<213>  Homo sapiens

<400>  116

Val Trp Lys Ser Asn Gly Lys Ser Ile Leu Lys Met Pro Phe
1               5                   10

<210>  117

<211>  91
<212>  PRT
<213>  Homo sapiens

<400>  117

Ala Gln Gly Leu Leu Leu Leu Gln Lys Asp Gly Pro Arg Ser Glu Gly
1               5                   10                  15

Ala Leu Pro Leu Trp Gly Ala Trp Pro Ser Ser Arg Ser Leu Arg Ala
            20                  25                  30

Thr Pro Trp Pro Trp Pro Arg Ala Leu Arg Ala Thr Pro His His Gly
        35                  40                  45

Pro Gly Pro Cys Gly Pro Pro Pro Gly His Gly Pro Gly Pro Cys Gly
    50                  55                  60

Pro Pro Pro His His Gly Pro Gly Pro Cys Gly Pro Pro Pro Gly His
65                  70                  75                  80

Gly Pro Gly His Pro Pro Pro Gly Pro His His
                85                  90

<210>  118
<211>  8
<212>  PRT
<213>  Homo sapiens

<400>  118

Ile Thr Ile Val Gly Thr Gly Ala
1               5

<210>  119
<211>  8
<212>  PRT
<213>  Homo sapiens

<400>  119

Gly Thr Gly Ala Val Gly Met Ala
1               5

<210>  120
<211>  8
<212>  PRT
<213>  Homo sapiens

<400>  120

Gly Ala Val Gly Met Ala Cys Ala
1               5

<210>  121
<211>  8

<212>  PRT
<213>  Homo sapiens

<400>  121

Ser Ile Leu Leu Lys Asp Leu Ala
1               5


<210>  122
<211>  8
<212>  PRT
<213>  Homo sapiens

<400>  122

Lys Asp Leu Ala Asp Glu Leu Ala
1               5


<210>  123
<211>  8
<212>  PRT
<213>  Homo sapiens

<400>  123

Glu Leu Ala Leu Val Asp Val Ala
1               5


<210>  124
<211>  8
<212>  PRT
<213>  Homo sapiens

<400>  124

Gly Lys Asp Tyr Ser Val Ser Ala
1               5


<210>  125
<211>  8
<212>  PRT
<213>  Homo sapiens

<400>  125

Ser Arg Ile Val Ile Val Thr Ala
1               5


<210>  126
<211>  8
<212>  PRT
<213>  Homo sapiens

<400>  126

Ile Val Ile Val Thr Ala Gly Ala
1               5


<210>  127
<211>  8
<212>  PRT
<213>  Homo sapiens

<400>  127

Gln Glu Gly Glu Thr Arg Leu Ala
1               5


<210>  128
<211>  8
<212>  PRT
<213>  Homo sapiens

<400>  128

Ala Leu Val Gln Arg Asn Val Ala
1               5


<210>  129
<211>  8
<212>  PRT
<213>  Homo sapiens

<400>  129

Ile Met Lys Ser Ile Ile Pro Ala
1               5


<210>  130
<211>  8
<212>  PRT
<213>  Homo sapiens

<400>  130

Ser Gly Cys Asn Leu Asp Ser Ala
1               5


<210>  131
<211>  8
<212>  PRT
<213>  Homo sapiens

<400>  131

Leu Trp Ser Gly Val Asn Val Ala
1               5


<210>  132
<211>  8
<212>  PRT
<213>  Homo sapiens

<400>  132

Gly Val Asn Val Ala Gly Val Ala
1               5


<210>  133
<211>  8
<212>  PRT
<213>  Homo sapiens

<400>  133

His Lys Gln Val Ile Gln Ser Ala
1               5

<210>  134
<211>  8
<212>  PRT
<213>  Homo sapiens

<400>  134

Leu Lys Gly Tyr Thr Ser Trp Ala
1               5

<210>  135
<211>  8
<212>  PRT
<213>  Homo sapiens

<400>  135

Asn Leu Asn Ser Glu Glu Glu Ala
1               5

<210>  136
<211>  8
<212>  PRT
<213>  Homo sapiens

<400>  136

Glu Ala Leu Phe Lys Lys Ser Ala
1               5

<210>  137
<211>  9
<212>  PRT
<213>  Homo sapiens

<400>  137

Asp Leu Ala Asp Glu Leu Ala Leu Val
1               5

<210>  138
<211>  9
<212>  PRT
<213>  Homo sapiens

<400>  138

Tyr Leu Ile Gly Glu Lys Leu Gly Val
1               5

<210>  139
<211>  9
<212>  PRT
<213>  Homo sapiens

<400>  139

Tyr Ile Val Trp Lys Ile Ser Gly Leu

1               5


<210>  140
<211>  9
<212>  PRT
<213>  Homo sapiens

<400>  140

Ser Ile Leu Lys Asn Leu Arg Arg Val
1               5


<210>  141
<211>  9
<212>  PRT
<213>  Homo sapiens

<400>  141

Gly Leu Tyr Gly Ile Lys Glu Glu Leu
1               5


<210>  142
<211>  9
<212>  PRT
<213>  Homo sapiens

<400>  142

Gly Met Ala Cys Ala Ile Ser Ile Leu
1               5


<210>  143
<211>  9
<212>  PRT
<213>  Homo sapiens

<400>  143

Leu Leu Lys Asp Leu Ala Asp Glu Leu
1               5


<210>  144
<211>  9
<212>  PRT
<213>  Homo sapiens

<400>  144

Ala Leu Lys Thr Leu Asp Pro Lys Leu
1               5


<210>  145
<211>  9
<212>  PRT
<213>  Homo sapiens

<400>  145

Ala Ile Ser Ile Leu Leu Lys Asp Leu
1               5

<210>  146
<211>  9
<212>  PRT
<213>  Homo sapiens

<400>  146

Ser Leu Phe Phe Ser Thr Ser Lys Ile
1               5


<210>  147
<211>  9
<212>  PRT
<213>  Homo sapiens

<400>  147

Ile Leu Thr Tyr Ile Val Trp Lys Ile
1               5


<210>  148
<211>  9
<212>  PRT
<213>  Homo sapiens

<400>  148

Ala Ile Gly Leu Ser Val Met Asp Leu
1               5


<210>  149
<211>  9
<212>  PRT
<213>  Homo sapiens

<400>  149

Thr Met Val Lys Gly Leu Tyr Gly Ile
1               5


<210>  150
<211>  9
<212>  PRT
<213>  Homo sapiens

<400>  150

Ile Thr Ile Val Gly Thr Gly Ala Val
1               5


<210>  151
<211>  9
<212>  PRT
<213>  Homo sapiens

<400>  151

Ser Ala Asn Ser Arg Ile Val Ile Val
1               5


<210>  152

<211>  9
<212>  PRT
<213>  Homo sapiens

<400>  152

Ala Leu Val Gln Arg Asn Val Ala Ile
1               5


<210>  153
<211>  9
<212>  PRT
<213>  Homo sapiens

<400>  153

Ile Met Lys Ser Ile Ile Pro Ala Ile
1               5


<210>  154
<211>  9
<212>  PRT
<213>  Homo sapiens

<400>  154

Ser Val Met Asp Leu Val Gly Ser Ile
1               5


<210>  155
<211>  9
<212>  PRT
<213>  Homo sapiens

<400>  155

Glu Leu Phe Leu Ser Ile Pro Cys Val
1               5


<210>  156
<211>  9
<212>  PRT
<213>  Homo sapiens

<400>  156

Glu Leu Ala Leu Val Asp Val Ala Leu
1               5


<210>  157
<211>  9
<212>  PRT
<213>  Homo sapiens

<400>  157

Leu Val Asp Val Ala Leu Asp Lys Leu
1               5


<210>  158
<211>  9
<212>  PRT

<213>  Homo sapiens

<400>  158

Lys Leu Lys Gly Glu Met Met Asp Leu
1               5


<210>  159
<211>  9
<212>  PRT
<213>  Homo sapiens

<400>  159

Ile Thr Ser Gly Lys Asp Tyr Ser Val
1               5


<210>  160
<211>  9
<212>  PRT
<213>  Homo sapiens

<400>  160

Arg Leu Ala Leu Val Gln Arg Asn Val
1               5


<210>  161
<211>  9
<212>  PRT
<213>  Homo sapiens

<400>  161

Lys Ile Leu Val Val Ser Asn Pro Val
1               5


<210>  162
<211>  9
<212>  PRT
<213>  Homo sapiens

<400>  162

Asn Leu Asn Ser Glu Glu Glu Ala Leu
1               5


<210>  163
<211>  9
<212>  PRT
<213>  Homo sapiens

<400>  163

Ala Leu Phe Lys Lys Ser Ala Glu Thr
1               5


<210>  164
<211>  9
<212>  PRT
<213>  Homo sapiens

<400>  164

Gln Leu Ile Glu Lys Leu Ile Glu Asp
1               5


<210>  165
<211>  9
<212>  PRT
<213>  Homo sapiens

<400>  165

Ile Leu Leu Lys Asp Leu Ala Asp Glu
1               5


<210>  166
<211>  9
<212>  PRT
<213>  Homo sapiens

<400>  166

Met Met Asp Leu Gln His Gly Ser Leu
1               5


<210>  167
<211>  9
<212>  PRT
<213>  Homo sapiens

<400>  167

Ser Ile Ile Pro Ala Ile Val His Tyr
1               5


<210>  168
<211>  9
<212>  PRT
<213>  Homo sapiens

<400>  168

Val Val Ser Asn Pro Val Asp Ile Leu
1               5


<210>  169
<211>  9
<212>  PRT
<213>  Homo sapiens

<400>  169

Val Ala Gly Val Ala Leu Lys Thr Leu
1               5


<210>  170
<211>  9
<212>  PRT
<213>  Homo sapiens

<400>  170

Thr Leu Trp Asn Ile Gln Lys Asp Leu
1               5

<210>  171
<211>  9
<212>  PRT
<213>  Homo sapiens

<400>  171

Ala Leu Asp Lys Leu Lys Gly Glu Met
1               5

<210>  172
<211>  9
<212>  PRT
<213>  Homo sapiens

<400>  172

Ala Ile Met Lys Ser Ile Ile Pro Ala
1               5

<210>  173
<211>  9
<212>  PRT
<213>  Homo sapiens

<400>  173

Pro Leu Trp Ser Gly Val Asn Val Ala
1               5

<210>  174
<211>  9
<212>  PRT
<213>  Homo sapiens

<400>  174

Gly Val Asn Val Ala Gly Val Ala Leu
1               5

<210>  175
<211>  9
<212>  PRT
<213>  Homo sapiens

<400>  175

Thr Leu Asp Pro Lys Leu Gly Thr Asp
1               5

<210>  176
<211>  9
<212>  PRT
<213>  Homo sapiens

<400>  176

Lys Leu Lys Gly Tyr Thr Ser Trp Ala
1               5

<210> 177
<211> 9
<212> PRT
<213> Homo sapiens

<400> 177

Val Met Asp Leu Val Gly Ser Ile Leu
1               5


<210> 178
<211> 9
<212> PRT
<213> Homo sapiens

<400> 178

Leu Val Gly Ser Ile Leu Lys Asn Leu
1               5


<210> 179
<211> 9
<212> PRT
<213> Homo sapiens

<400> 179

Ser Pro Asp Cys Lys Ile Leu Val Val
1               5


<210> 180
<211> 9
<212> PRT
<213> Homo sapiens

<400> 180

Arg Val Ile Gly Ser Gly Cys Asn Leu
1               5


<210> 181
<211> 9
<212> PRT
<213> Homo sapiens

<400> 181

Asp Leu Val Gly Ser Ile Leu Lys Asn
1               5


<210> 182
<211> 9
<212> PRT
<213> Homo sapiens

<400> 182

Lys Asn Leu Arg Arg Val His Pro Val
1               5

<210>  183
<211>  9
<212>  PRT
<213>  Homo sapiens

<400>  183

Leu Gly Arg Asn Gly Val Ser Asp Val
1               5


<210>  184
<211>  10
<212>  PRT
<213>  Homo sapiens

<400>  184

Ala Leu Val Asp Val Ala Leu Asp Lys Leu
1               5                   10


<210>  185
<211>  10
<212>  PRT
<213>  Homo sapiens

<400>  185

Ile Leu Leu Lys Asp Leu Ala Asp Glu Leu
1               5                   10


<210>  186
<211>  10
<212>  PRT
<213>  Homo sapiens

<400>  186

Ala Ile Met Lys Ser Ile Ile Pro Ala Ile
1               5                   10


<210>  187
<211>  10
<212>  PRT
<213>  Homo sapiens

<400>  187

Lys Ile Ser Gly Leu Pro Val Thr Arg Val
1               5                   10


<210>  188
<211>  10
<212>  PRT
<213>  Homo sapiens

<400>  188

Val Leu Gly Arg Asn Gly Val Ser Asp Val
1               5                   10


<210>  189
<211>  10

<212>  PRT
<213>  Homo sapiens

<400>  189

Ala Leu Phe Lys Lys Ser Ala Glu Thr Leu
1               5                   10


<210>  190
<211>  10
<212>  PRT
<213>  Homo sapiens

<400>  190

Leu Ala Asp Glu Leu Ala Leu Val Asp Val
1               5                   10


<210>  191
<211>  10
<212>  PRT
<213>  Homo sapiens

<400>  191

Ile Ile Gly Glu His Gly Asp Ser Ser Val
1               5                   10


<210>  192
<211>  10
<212>  PRT
<213>  Homo sapiens

<400>  192

Asp Leu Val Gly Ser Ile Leu Lys Asn Leu
1               5                   10


<210>  193
<211>  10
<212>  PRT
<213>  Homo sapiens

<400>  193

Asp Ile Leu Thr Tyr Ile Val Trp Lys Ile
1               5                   10


<210>  194
<211>  10
<212>  PRT
<213>  Homo sapiens

<400>  194

Asn Val Ala Gly Val Ala Leu Lys Thr Leu
1               5                   10


<210>  195
<211>  10
<212>  PRT
<213>  Homo sapiens

<400>  195

Ala Leu Val Gln Arg Asn Val Ala Ile Met
1               5                   10


<210>  196
<211>  10
<212>  PRT
<213>  Homo sapiens

<400>  196

Ile Leu Val Val Ser Asn Pro Val Asp Ile
1               5                   10


<210>  197
<211>  10
<212>  PRT
<213>  Homo sapiens

<400>  197

Trp Ala Ile Gly Leu Ser Val Met Asp Leu
1               5                   10


<210>  198
<211>  10
<212>  PRT
<213>  Homo sapiens

<400>  198

Thr Val Lys Glu Gln Leu Ile Glu Lys Leu
1               5                   10


<210>  199
<211>  10
<212>  PRT
<213>  Homo sapiens

<400>  199

Gly Met Ala Cys Ala Ile Ser Ile Leu Leu
1               5                   10


<210>  200
<211>  10
<212>  PRT
<213>  Homo sapiens

<400>  200

Lys Ile Thr Ser Gly Lys Asp Tyr Ser Val
1               5                   10


<210>  201
<211>  10
<212>  PRT
<213>  Homo sapiens

<400>  201

Ile Met Lys Ser Ile Ile Pro Ala Ile Val
1               5                   10


<210>  202
<211>  10
<212>  PRT
<213>  Homo sapiens

<400>  202

Leu Trp Ser Gly Val Asn Val Ala Gly Val
1               5                   10


<210>  203
<211>  10
<212>  PRT
<213>  Homo sapiens

<400>  203

Lys Leu Lys Gly Tyr Thr Ser Trp Ala Ile
1               5                   10


<210>  204
<211>  10
<212>  PRT
<213>  Homo sapiens

<400>  204

Ser Thr Met Val Lys Gly Leu Tyr Gly Ile
1               5                   10


<210>  205
<211>  10
<212>  PRT
<213>  Homo sapiens

<400>  205

Gly Ile Lys Glu Glu Leu Phe Leu Ser Ile
1               5                   10


<210>  206
<211>  10
<212>  PRT
<213>  Homo sapiens

<400>  206

Leu Val Val Ser Asn Pro Val Asp Ile Leu
1               5                   10


<210>  207
<211>  10
<212>  PRT
<213>  Homo sapiens

<400>  207

Asn Leu Asp Ser Ala Arg Phe Arg Tyr Leu

1                   5                   10

<210>  208
<211>  10
<212>  PRT
<213>  Homo sapiens

<400>  208

Tyr Leu Ile Gly Glu Lys Leu Gly Val His
1               5                   10

<210>  209
<211>  10
<212>  PRT
<213>  Homo sapiens

<400>  209

Val Ala Leu Lys Thr Leu Asp Pro Lys Leu
1               5                   10

<210>  210
<211>  10
<212>  PRT
<213>  Homo sapiens

<400>  210

Ala Ile Gly Leu Ser Val Met Asp Leu Val
1               5                   10

<210>  211
<211>  10
<212>  PRT
<213>  Homo sapiens

<400>  211

Lys Ile Thr Ile Val Gly Thr Gly Ala Val
1               5                   10

<210>  212
<211>  10
<212>  PRT
<213>  Homo sapiens

<400>  212

Cys Ala Ile Ser Ile Leu Leu Lys Asp Leu
1               5                   10

<210>  213
<211>  10
<212>  PRT
<213>  Homo sapiens

<400>  213

Lys Asp Leu Ala Asp Glu Leu Ala Leu Val
1               5                   10

<210> 214
<211> 10
<212> PRT
<213> Homo sapiens

<400> 214

Glu Met Met Asp Leu Gln His Gly Ser Leu
1               5                   10


<210> 215
<211> 10
<212> PRT
<213> Homo sapiens

<400> 215

Val Ser Ala Asn Ser Arg Ile Val Ile Val
1               5                   10


<210> 216
<211> 10
<212> PRT
<213> Homo sapiens

<400> 216

Leu Ala Leu Val Gln Arg Asn Val Ala Ile
1               5                   10


<210> 217
<211> 10
<212> PRT
<213> Homo sapiens

<400> 217

Ser Ile Ile Pro Ala Ile Val His Tyr Ser
1               5                   10


<210> 218
<211> 10
<212> PRT
<213> Homo sapiens

<400> 218

Tyr Thr Ser Trp Ala Ile Gly Leu Ser Val
1               5                   10


<210> 219
<211> 10
<212> PRT
<213> Homo sapiens

<400> 219

Thr Leu Trp Asn Ile Gln Lys Asp Leu Ile
1               5                   10


<210> 220

<211>  10
<212>  PRT
<213>  Homo sapiens

<400>  220

Gln Leu Ile Glu Lys Leu Ile Glu Asp Asp
1               5                   10


<210>  221
<211>  10
<212>  PRT
<213>  Homo sapiens

<400>  221

Lys Leu Ile Glu Asp Asp Glu Asn Ser Gln
1               5                   10


<210>  222
<211>  10
<212>  PRT
<213>  Homo sapiens

<400>  222

Ser Ile Leu Leu Lys Asp Leu Ala Asp Glu
1               5                   10


<210>  223
<211>  10
<212>  PRT
<213>  Homo sapiens

<400>  223

Gln Glu Gly Glu Thr Arg Leu Ala Leu Val
1               5                   10


<210>  224
<211>  10
<212>  PRT
<213>  Homo sapiens

<400>  224

Tyr Ser Pro Asp Cys Lys Ile Leu Val Val
1               5                   10


<210>  225
<211>  10
<212>  PRT
<213>  Homo sapiens

<400>  225

Ile Val Trp Lys Ile Ser Gly Leu Pro Val
1               5                   10


<210>  226
<211>  10
<212>  PRT

<213>  Homo sapiens

<400>  226

Ser Val Pro Leu Trp Ser Gly Val Asn Val
1               5                   10


<210>  227
<211>  10
<212>  PRT
<213>  Homo sapiens

<400>  227

Asn Ile His Lys Gln Val Ile Gln Ser Ala
1               5                   10


<210>  228
<211>  10
<212>  PRT
<213>  Homo sapiens

<400>  228

Ile Gln Ser Ala Tyr Glu Ile Ile Lys Leu
1               5                   10


<210>  229
<211>  10
<212>  PRT
<213>  Homo sapiens

<400>  229

Gly Leu Ser Val Met Asp Leu Val Gly Ser
1               5                   10


<210>  230
<211>  10
<212>  PRT
<213>  Homo sapiens

<400>  230

Ser Val Met Asp Leu Val Gly Ser Ile Leu
1               5                   10


<210>  231
<211>  10
<212>  PRT
<213>  Homo sapiens

<400>  231

Leu Lys Asn Leu Arg Arg Val His Pro Val
1               5                   10


<210>  232
<211>  10
<212>  PRT
<213>  Homo sapiens

<400>  232

Asn Leu Arg Arg Val His Pro Val Ser Thr
1               5                   10


<210>  233
<211>  10
<212>  PRT
<213>  Homo sapiens

<400>  233

Glu Leu Phe Leu Ser Ile Pro Cys Val Leu
1               5                   10


<210>  234
<211>  9
<212>  PRT
<213>  Homo sapiens

<400>  234

Leu Tyr Gly Ile Lys Glu Glu Leu Phe
1               5


<210>  235
<211>  9
<212>  PRT
<213>  Homo sapiens

<400>  235

His Tyr Ser Pro Asp Cys Lys Ile Leu
1               5


<210>  236
<211>  9
<212>  PRT
<213>  Homo sapiens

<400>  236

Gly Tyr Thr Ser Trp Ala Ile Gly Leu
1               5


<210>  237
<211>  9
<212>  PRT
<213>  Homo sapiens

<400>  237

Leu Phe Leu Ser Ile Pro Cys Val Leu
1               5


<210>  238
<211>  9
<212>  PRT
<213>  Homo sapiens

<400>  238

Leu Phe Lys Lys Ser Ala Glu Thr Leu
1               5


<210>  239
<211>  9
<212>  PRT
<213>  Homo sapiens

<400>  239

Val Lys Glu Gln Leu Ile Glu Lys Leu
1               5


<210>  240
<211>  9
<212>  PRT
<213>  Homo sapiens

<400>  240

Met Asp Leu Gln His Gly Ser Leu Phe
1               5


<210>  241
<211>  9
<212>  PRT
<213>  Homo sapiens

<400>  241

Lys Glu Gln Leu Ile Glu Lys Leu Ile
1               5


<210>  242
<211>  9
<212>  PRT
<213>  Homo sapiens

<400>  242

Val His Tyr Ser Pro Asp Cys Lys Ile
1               5


<210>  243
<211>  9
<212>  PRT
<213>  Homo sapiens

<400>  243

Ile Leu Thr Tyr Ile Val Trp Lys Ile
1               5


<210>  244
<211>  10
<212>  PRT
<213>  Homo sapiens

<400>  244

Thr Tyr Ile Val Trp Lys Ile Ser Gly Leu
1               5                   10

<210>  245
<211>  10
<212>  PRT
<213>  Homo sapiens

<400>  245

Asp Tyr Ser Val Ser Ala Asn Ser Arg Ile
1               5                   10


<210>  246
<211>  10
<212>  PRT
<213>  Homo sapiens

<400>  246

Leu Tyr Gly Ile Lys Glu Glu Leu Phe Leu
1               5                   10


<210>  247
<211>  10
<212>  PRT
<213>  Homo sapiens

<400>  247

Arg Phe Arg Tyr Leu Ile Gly Glu Lys Leu
1               5                   10


<210>  248
<211>  10
<212>  PRT
<213>  Homo sapiens

<400>  248

Val Ala Leu Lys Thr Leu Asp Pro Lys Leu
1               5                   10


<210>  249
<211>  10
<212>  PRT
<213>  Homo sapiens

<400>  249

Thr Val Lys Glu Gln Leu Ile Glu Lys Leu
1               5                   10


<210>  250
<211>  10
<212>  PRT
<213>  Homo sapiens

<400>  250

Asp Ile Leu Thr Tyr Ile Val Trp Lys Ile
1               5                   10

<210>  251
<211>  10
<212>  PRT
<213>  Homo sapiens

<400>  251

Val His Pro Thr Ser Cys His Gly Trp Ile
1               5                   10


<210>  252
<211>  10
<212>  PRT
<213>  Homo sapiens

<400>  252

Val Lys Glu Gln Leu Ile Glu Lys Leu Ile
1               5                   10


<210>  253
<211>  10
<212>  PRT
<213>  Homo sapiens

<400>  253

Glu Asp Asp Glu Asn Ser Gln Cys Lys Ile
1               5                   10


<210>  254
<211>  9
<212>  PRT
<213>  Homo sapiens

<400>  254

Asn Leu Asp Ser Ala Arg Phe Arg Tyr
1               5


<210>  255
<211>  9
<212>  PRT
<213>  Homo sapiens

<400>  255

Val Ser Thr Met Val Lys Gly Leu Tyr
1               5


<210>  256
<211>  9
<212>  PRT
<213>  Homo sapiens

<400>  256

Ser Asn Pro Val Asp Ile Leu Thr Tyr
1               5


<210>  257
<211>  9

<212>  PRT
<213>  Homo sapiens

<400>  257

Ser Ile Ile Pro Ala Ile Val His Tyr
1               5


<210>  258
<211>  9
<212>  PRT
<213>  Homo sapiens

<400>  258

Val Ser Asp Val Val Lys Ile Asn Leu
1               5


<210>  259
<211>  9
<212>  PRT
<213>  Homo sapiens

<400>  259

Asn Ser Glu Glu Glu Ala Leu Phe Lys
1               5


<210>  260
<211>  9
<212>  PRT
<213>  Homo sapiens

<400>  260

Ser Pro Asp Cys Lys Ile Leu Val Val
1               5


<210>  261
<211>  9
<212>  PRT
<213>  Homo sapiens

<400>  261

Ala Tyr Glu Ile Ile Lys Leu Lys Gly
1               5


<210>  262
<211>  9
<212>  PRT
<213>  Homo sapiens

<400>  262

Ile Lys Glu Glu Leu Phe Leu Ser Ile
1               5


<210>  263
<211>  9
<212>  PRT
<213>  Homo sapiens

<400>  263

Ser Lys Ile Thr Ser Gly Lys Asp Tyr
1               5


<210>  264
<211>  9
<212>  PRT
<213>  Homo sapiens

<400>  264

Leu Ala Asp Glu Leu Ala Leu Val Asp
1               5


<210>  265
<211>  9
<212>  PRT
<213>  Homo sapiens

<400>  265

Val Ser Asn Pro Val Asp Ile Leu Thr
1               5


<210>  266
<211>  9
<212>  PRT
<213>  Homo sapiens

<400>  266

Tyr Glu Ile Ile Lys Leu Lys Gly Tyr
1               5


<210>  267
<211>  9
<212>  PRT
<213>  Homo sapiens

<400>  267

Lys Gly Glu Met Met Asp Leu Gln His
1               5


<210>  268
<211>  9
<212>  PRT
<213>  Homo sapiens

<400>  268

Gly Thr Asp Ser Asp Lys Glu His Trp
1               5


<210>  269
<211>  9
<212>  PRT
<213>  Homo sapiens

<400>  269

Asp Ser Asp Lys Glu His Trp Lys Asn
1               5

<210>  270
<211>  9
<212>  PRT
<213>  Homo sapiens

<400>  270

Thr Leu Asp Pro Lys Leu Gly Thr Asp
1               5

<210>  271
<211>  9
<212>  PRT
<213>  Homo sapiens

<400>  271

His Lys Gln Val Ile Gln Ser Ala Tyr
1               5

<210>  272
<211>  9
<212>  PRT
<213>  Homo sapiens

<400>  272

Ser Thr Met Val Lys Gly Leu Tyr Gly
1               5

<210>  273
<211>  9
<212>  PRT
<213>  Homo sapiens

<400>  273

Leu Ser Ile Pro Cys Val Leu Gly Arg
1               5

<210>  274
<211>  9
<212>  PRT
<213>  Homo sapiens

<400>  274

Glu Glu Glu Ala Leu Phe Lys Lys Ser
1               5

<210>  275
<211>  10
<212>  PRT
<213>  Homo sapiens

<400>  275

Val Ser Asn Pro Val Asp Ile Leu Thr Tyr

1                 5                   10


<210>  276
<211>  10
<212>  PRT
<213>  Homo sapiens

<400>  276

Ala Tyr Glu Ile Ile Lys Leu Lys Gly Tyr
1               5                   10


<210>  277
<211>  10
<212>  PRT
<213>  Homo sapiens

<400>  277

Thr Ser Lys Ile Thr Ser Gly Lys Asp Tyr
1               5                   10


<210>  278
<211>  10
<212>  PRT
<213>  Homo sapiens

<400>  278

Lys Ser Ile Ile Pro Ala Ile Val His Tyr
1               5                   10


<210>  279
<211>  10
<212>  PRT
<213>  Homo sapiens

<400>  279

Gln Gln Glu Gly Glu Thr Arg Leu Ala Leu
1               5                   10


<210>  280
<211>  10
<212>  PRT
<213>  Homo sapiens

<400>  280

Glu Gly Glu Thr Arg Leu Ala Leu Val Gln
1               5                   10


<210>  281
<211>  10
<212>  PRT
<213>  Homo sapiens

<400>  281

Val Met Asp Leu Val Gly Ser Ile Leu Lys
1               5                   10

<210> 282
<211> 10
<212> PRT
<213> Homo sapiens

<400> 282

Pro Val Ser Thr Met Val Lys Gly Leu Tyr
1               5                   10


<210> 283
<211> 10
<212> PRT
<213> Homo sapiens

<400> 283

Val Ser Asp Val Val Lys Ile Asn Leu Asn
1               5                   10


<210> 284
<211> 10
<212> PRT
<213> Homo sapiens

<400> 284

Ser Glu Glu Glu Ala Leu Phe Lys Lys Ser
1               5                   10


<210> 285
<211> 10
<212> PRT
<213> Homo sapiens

<400> 285

Ser Ala Glu Thr Leu Trp Asn Ile Gln Lys
1               5                   10


<210> 286
<211> 10
<212> PRT
<213> Homo sapiens

<400> 286

Leu Lys Asp Leu Ala Asp Glu Leu Ala Leu
1               5                   10


<210> 287
<211> 10
<212> PRT
<213> Homo sapiens

<400> 287

Cys Asn Leu Asp Ser Ala Arg Phe Arg Tyr
1               5                   10


<210> 288

<211>  10
<212>  PRT
<213>  Homo sapiens

<400>  288

His Gly Asp Ser Ser Val Pro Leu Trp Ser
1               5                   10


<210>  289
<211>  10
<212>  PRT
<213>  Homo sapiens

<400>  289

Thr Leu Asp Pro Lys Leu Gly Thr Asp Ser
1               5                   10


<210>  290
<211>  10
<212>  PRT
<213>  Homo sapiens

<400>  290

Gly Thr Asp Ser Asp Lys Glu His Trp Lys
1               5                   10


<210>  291
<211>  10
<212>  PRT
<213>  Homo sapiens

<400>  291

Asn Ser Glu Glu Glu Ala Leu Phe Lys Lys
1               5                   10


<210>  292
<211>  10
<212>  PRT
<213>  Homo sapiens

<400>  292

Pro Val Asp Ile Leu Thr Tyr Ile Val Trp
1               5                   10


<210>  293
<211>  10
<212>  PRT
<213>  Homo sapiens

<400>  293

Asp Lys Glu His Trp Lys Asn Ile His Lys
1               5                   10


<210>  294
<211>  10
<212>  PRT

<213>  Homo sapiens

<400>  294

Ile His Lys Gln Val Ile Gln Ser Ala Tyr
1               5                   10


<210>  295
<211>  10
<212>  PRT
<213>  Homo sapiens

<400>  295

Tyr Thr Ser Trp Ala Ile Gly Leu Ser Val
1               5                   10


<210>  296
<211>  9
<212>  PRT
<213>  Homo sapiens

<400>  296

Ala Leu Val Asp Val Ala Leu Asp Lys
1               5


<210>  297
<211>  9
<212>  PRT
<213>  Homo sapiens

<400>  297

Lys Ile Ser Gly Leu Pro Val Thr Arg
1               5


<210>  298
<211>  9
<212>  PRT
<213>  Homo sapiens

<400>  298

Ser Ile Ile Pro Ala Ile Val His Tyr
1               5


<210>  299
<211>  9
<212>  PRT
<213>  Homo sapiens

<400>  299

Asp Ile Leu Thr Tyr Ile Val Trp Lys
1               5


<210>  300
<211>  9
<212>  PRT
<213>  Homo sapiens

<400>  300

Ile Val Ile Val Thr Ala Gly Ala Arg
1               5


<210>  301
<211>  9
<212>  PRT
<213>  Homo sapiens

<400>  301

Met Val Lys Gly Leu Tyr Gly Ile Lys
1               5


<210>  302
<211>  9
<212>  PRT
<213>  Homo sapiens

<400>  302

Ile Val His Tyr Ser Pro Asp Cys Lys
1               5


<210>  303
<211>  9
<212>  PRT
<213>  Homo sapiens

<400>  303

Ile Leu Lys Asn Leu Arg Arg Val His
1               5


<210>  304
<211>  9
<212>  PRT
<213>  Homo sapiens

<400>  304

Thr Val Lys Glu Gln Leu Ile Glu Lys
1               5


<210>  305
<211>  9
<212>  PRT
<213>  Homo sapiens

<400>  305

Ile Val Thr Ala Gly Ala Arg Gln Gln
1               5


<210>  306
<211>  9
<212>  PRT
<213>  Homo sapiens

<400>  306

Arg Val Ile Gly Ser Gly Cys Asn Leu
1               5

<210>  307
<211>  9
<212>  PRT
<213>  Homo sapiens

<400>  307

Val Leu Gly Arg Asn Gly Val Ser Asp
1               5

<210>  308
<211>  9
<212>  PRT
<213>  Homo sapiens

<400>  308

Arg Asn Gly Val Ser Asp Val Val Lys
1               5

<210>  309
<211>  9
<212>  PRT
<213>  Homo sapiens

<400>  309

Ile Val Gly Thr Gly Ala Val Gly Met
1               5

<210>  310
<211>  9
<212>  PRT
<213>  Homo sapiens

<400>  310

Asn Leu Arg Arg Val His Pro Val Ser
1               5

<210>  311
<211>  9
<212>  PRT
<213>  Homo sapiens

<400>  311

Cys Val Leu Gly Arg Asn Gly Val Ser
1               5

<210>  312
<211>  9
<212>  PRT
<213>  Homo sapiens

<400>  312

Thr Ile Val Gly Thr Gly Ala Val Gly
1               5

<210>  313
<211>  9
<212>  PRT
<213>  Homo sapiens

<400>  313

Ala Leu Val Gln Arg Asn Val Ala Ile
1               5


<210>  314
<211>  9
<212>  PRT
<213>  Homo sapiens

<400>  314

Asn Val Ala Gly Val Ala Leu Lys Thr
1               5


<210>  315
<211>  9
<212>  PRT
<213>  Homo sapiens

<400>  315

Ile Ile Lys Leu Lys Gly Tyr Thr Ser
1               5


<210>  316
<211>  9
<212>  PRT
<213>  Homo sapiens

<400>  316

Lys Leu Lys Gly Tyr Thr Ser Trp Ala
1               5


<210>  317
<211>  9
<212>  PRT
<213>  Homo sapiens

<400>  317

Arg Val His Pro Val Ser Thr Met Val
1               5


<210>  318
<211>  9
<212>  PRT
<213>  Homo sapiens

<400>  318

Asp Leu Gln His Gly Ser Leu Phe Phe
1               5

<210> 319
<211> 9
<212> PRT
<213> Homo sapiens

<400> 319

Val Gln Arg Asn Val Ala Ile Met Lys
1               5

<210> 320
<211> 9
<212> PRT
<213> Homo sapiens

<400> 320

Leu Ile Gly Glu Lys Leu Gly Val His
1               5

<210> 321
<211> 9
<212> PRT
<213> Homo sapiens

<400> 321

Thr Leu Asp Pro Lys Leu Gly Thr Asp
1               5

<210> 322
<211> 9
<212> PRT
<213> Homo sapiens

<400> 322

Ala Cys Ala Ile Ser Ile Leu Leu Lys
1               5

<210> 323
<211> 9
<212> PRT
<213> Homo sapiens

<400> 323

Glu Leu Ala Leu Val Asp Val Ala Leu
1               5

<210> 324
<211> 9
<212> PRT
<213> Homo sapiens

<400> 324

Lys Ser Ile Ile Pro Ala Ile Val His
1               5

<210> 325
<211> 9

<212>  PRT
<213>  Homo sapiens

<400>  325

Arg Phe Arg Tyr Leu Ile Gly Glu Lys
1               5


<210>  326
<211>  9
<212>  PRT
<213>  Homo sapiens

<400>  326

Tyr Leu Ile Gly Glu Lys Leu Gly Val
1               5


<210>  327
<211>  9
<212>  PRT
<213>  Homo sapiens

<400>  327

Gly Val Asn Val Ala Gly Val Ala Leu
1               5


<210>  328
<211>  9
<212>  PRT
<213>  Homo sapiens

<400>  328

Gln Val Ile Gln Ser Ala Tyr Glu Ile
1               5


<210>  329
<211>  10
<212>  PRT
<213>  Homo sapiens

<400>  329

Arg Val His Pro Val Ser Thr Met Val Lys
1               5                   10


<210>  330
<211>  10
<212>  PRT
<213>  Homo sapiens

<400>  330

Leu Val Gln Arg Asn Val Ala Ile Met Lys
1               5                   10


<210>  331
<211>  10
<212>  PRT
<213>  Homo sapiens

<400>  331

Tyr Leu Ile Gly Glu Lys Leu Gly Val His
1               5                   10


<210>  332
<211>  10
<212>  PRT
<213>  Homo sapiens

<400>  332

Gly Val Asn Val Ala Gly Val Ala Leu Lys
1               5                   10


<210>  333
<211>  10
<212>  PRT
<213>  Homo sapiens

<400>  333

Asn Leu Arg Arg Val His Pro Val Ser Thr
1               5                   10


<210>  334
<211>  10
<212>  PRT
<213>  Homo sapiens

<400>  334

Cys Val Leu Gly Arg Asn Gly Val Ser Asp
1               5                   10


<210>  335
<211>  10
<212>  PRT
<213>  Homo sapiens

<400>  335

Asp Leu Ala Asp Glu Leu Ala Leu Val Asp
1               5                   10


<210>  336
<211>  10
<212>  PRT
<213>  Homo sapiens

<400>  336

Arg Ile Val Ile Val Thr Ala Gly Ala Arg
1               5                   10


<210>  337
<211>  10
<212>  PRT
<213>  Homo sapiens

<400>  337

Ala Ile Val His Tyr Ser Pro Asp Cys Lys
1               5                   10

<210>  338
<211>  10
<212>  PRT
<213>  Homo sapiens

<400>  338

Gly Val Ala Leu Lys Thr Leu Asp Pro Lys
1               5                   10

<210>  339
<211>  10
<212>  PRT
<213>  Homo sapiens

<400>  339

Ile Val Ile Val Thr Ala Gly Ala Arg Gln
1               5                   10

<210>  340
<211>  10
<212>  PRT
<213>  Homo sapiens

<400>  340

Arg Leu Ala Leu Val Gln Arg Asn Val Ala
1               5                   10

<210>  341
<211>  10
<212>  PRT
<213>  Homo sapiens

<400>  341

Leu Val Asp Val Ala Leu Asp Lys Leu Lys
1               5                   10

<210>  342
<211>  10
<212>  PRT
<213>  Homo sapiens

<400>  342

Lys Leu Gly Val His Pro Thr Ser Cys His
1               5                   10

<210>  343
<211>  10
<212>  PRT
<213>  Homo sapiens

<400>  343

Ser Ile Leu Lys Asn Leu Arg Arg Val His

1                 5                   10


<210>  344
<211>  10
<212>  PRT
<213>  Homo sapiens

<400>  344

Gly Leu Tyr Gly Ile Lys Glu Glu Leu Phe
1               5                   10


<210>  345
<211>  10
<212>  PRT
<213>  Homo sapiens

<400>  345

Gly Arg Asn Gly Val Ser Asp Val Val Lys
1               5                   10


<210>  346
<211>  10
<212>  PRT
<213>  Homo sapiens

<400>  346

Ala Leu Phe Lys Lys Ser Ala Glu Thr Leu
1               5                   10


<210>  347
<211>  10
<212>  PRT
<213>  Homo sapiens

<400>  347

Lys Leu Ile Glu Asp Asp Glu Asn Ser Gln
1               5                   10


<210>  348
<211>  10
<212>  PRT
<213>  Homo sapiens

<400>  348

Val Asp Ile Leu Thr Tyr Ile Val Trp Lys
1               5                   10


<210>  349
<211>  10
<212>  PRT
<213>  Homo sapiens

<400>  349

Pro Val Thr Arg Val Ile Gly Ser Gly Cys
1               5                   10

<210> 350
<211> 10
<212> PRT
<213> Homo sapiens

<400> 350

Arg Val Ile Gly Ser Gly Cys Asn Leu Asp
1               5                   10


<210> 351
<211> 10
<212> PRT
<213> Homo sapiens

<400> 351

Ser Val Pro Leu Trp Ser Gly Val Asn Val
1               5                   10


<210> 352
<211> 10
<212> PRT
<213> Homo sapiens

<400> 352

Ile Leu Lys Asn Leu Arg Arg Val His Pro
1               5                   10


<210> 353
<211> 10
<212> PRT
<213> Homo sapiens

<400> 353

Ala Val Gly Met Ala Cys Ala Ile Ser Ile
1 . 5 10


<210> 354
<211> 10
<212> PRT
<213> Homo sapiens

<400> 354

Leu Ala Leu Val Asp Val Ala Leu Asp Lys
1               5                   10


<210> 355
<211> 10
<212> PRT
<213> Homo sapiens

<400> 355

Ser Val Ser Ala Asn Ser Arg Ile Val Ile
1               5                   10


<210> 356

<211>  10
<212>  PRT
<213>  Homo sapiens

<400>  356

Ile Val Thr Ala Gly Ala Arg Gln Gln Glu
1               5                   10


<210>  357
<211>  10
<212>  PRT
<213>  Homo sapiens

<400>  357

Ile Val Trp Lys Ile Ser Gly Leu Pro Val
1               5                   10


<210>  358
<211>  10
<212>  PRT
<213>  Homo sapiens

<400>  358

Trp Ile Ile Gly Glu His Gly Asp Ser Ser
1               5                   10


<210>  359
<211>  10
<212>  PRT
<213>  Homo sapiens

<400>  359

Val Ile Gln Ser Ala Tyr Glu Ile Ile Lys
1               5                   10


<210>  360
<211>  10
<212>  PRT
<213>  Homo sapiens

<400>  360

Glu Ile Ile Lys Leu Lys Gly Tyr Thr Ser
1               5                   10


<210>  361
<211>  10
<212>  PRT
<213>  Homo sapiens

<400>  361

Ser Val Met Asp Leu Val Gly Ser Ile Leu
1               5                   10


<210>  362
<211>  10
<212>  PRT

<213>  Homo sapiens

<400>  362

Phe Leu Ser Ile Pro Cys Val Leu Gly Arg
1               5                   10


<210>  363
<211>  9
<212>  PRT
<213>  Homo sapiens

<400>  363

Ser Pro Asp Cys Lys Ile Leu Val Val
1               5


<210>  364
<211>  9
<212>  PRT
<213>  Homo sapiens

<400>  364

Val Pro Leu Trp Ser Gly Val Asn Val
1               5


<210>  365
<211>  9
<212>  PRT
<213>  Homo sapiens

<400>  365

Asn Pro Val Asp Ile Leu Thr Tyr Ile
1               5


<210>  366
<211>  9
<212>  PRT
<213>  Homo sapiens

<400>  366

Val Val Ser Asn Pro Val Asp Ile Leu
1               5


<210>  367
<211>  9
<212>  PRT
<213>  Homo sapiens

<400>  367

His Pro Thr Ser Cys His Gly Trp Ile
1               5


<210>  368
<211>  9
<212>  PRT
<213>  Homo sapiens

<400>  368

Lys Asp Leu Ala Asp Glu Leu Ala Leu
1               5


<210>  369
<211>  9
<212>  PRT
<213>  Homo sapiens

<400>  369

Glu Leu Ala Leu Val Asp Val Ala Leu
1               5


<210>  370
<211>  9
<212>  PRT
<213>  Homo sapiens

<400>  370

Gln Glu Gly Glu Thr Arg Leu Ala Leu
1               5


<210>  371
<211>  9
<212>  PRT
<213>  Homo sapiens

<400>  371

Glu His Gly Asp Ser Ser Val Pro Leu
1               5


<210>  372
<211>  9
<212>  PRT
<213>  Homo sapiens

<400>  372

Ala Ile Ser Ile Leu Leu Lys Asp Leu
1               5


<210>  373
<211>  10
<212>  PRT
<213>  Homo sapiens

<400>  373

His Pro Val Ser Thr Met Val Lys Gly Leu
1               5                   10


<210>  374
<211>  10
<212>  PRT
<213>  Homo sapiens

<400>  374

Val Pro Leu Trp Ser Gly Val Asn Val Ala
1               5                   10

<210>  375
<211>  10
<212>  PRT
<213>  Homo sapiens

<400>  375

Ile Pro Cys Val Leu Gly Arg Asn Gly Val
1               5                   10

<210>  376
<211>  10
<212>  PRT
<213>  Homo sapiens

<400>  376

Asn Pro Val Asp Ile Leu Thr Tyr Ile Val
1               5                   10

<210>  377
<211>  10
<212>  PRT
<213>  Homo sapiens

<400>  377

Gln Gln Glu Gly Glu Thr Arg Leu Ala Leu
1               5                   10

<210>  378
<211>  10
<212>  PRT
<213>  Homo sapiens

<400>  378

His Pro Thr Ser Cys His Gly Trp Ile Ile
1               5                   10

<210>  379
<211>  10
<212>  PRT
<213>  Homo sapiens

<400>  379

Gly Glu His Gly Asp Ser Ser Val Pro Leu
1               5                   10

<210>  380
<211>  10
<212>  PRT
<213>  Homo sapiens

<400>  380

Leu Lys Asp Leu Ala Asp Glu Leu Ala Leu
1               5                   10

<210>  381
<211>  10
<212>  PRT
<213>  Homo sapiens

<400>  381

Asp Glu Leu Ala Leu Val Asp Val Ala Leu
1               5                   10


<210>  382
<211>  10
<212>  PRT
<213>  Homo sapiens

<400>  382

Ser Pro Asp Cys Lys Ile Leu Val Val Ser
1               5                   10


<210>  383
<211>  8
<212>  PRT
<213>  Homo sapiens

<400>  383

Ser Pro Asp Pro Thr Asp Leu Ala
1               5


<210>  384
<211>  8
<212>  PRT
<213>  Homo sapiens

<400>  384

Gln Thr Ser Glu Phe Lys Gly Ala
1               5


<210>  385
<211>  8
<212>  PRT
<213>  Homo sapiens

<400>  385

Phe Lys Gly Ala Thr Glu Glu Ala
1               5


<210>  386
<211>  8
<212>  PRT
<213>  Homo sapiens

<400>  386

Gly Ala Thr Glu Glu Ala Pro Ala
1               5

<210> 387
<211> 8
<212> PRT
<213> Homo sapiens

<400> 387

His Thr Ser Glu Phe Lys Gly Ala
1               5


<210> 388
<211> 8
<212> PRT
<213> Homo sapiens

<400> 388

Thr Ser Glu Phe Lys Gly Ala Ala
1               5


<210> 389
<211> 8
<212> PRT
<213> Homo sapiens

<400> 389

Pro Ile Ser Glu Ser Val Leu Ala
1               5


<210> 390
<211> 8
<212> PRT
<213> Homo sapiens

<400> 390

Ser Lys Phe Glu Val Glu Asp Ala
1               5


<210> 391
<211> 8
<212> PRT
<213> Homo sapiens

<400> 391

Val Glu Asp Ala Glu Asn Val Ala
1               5


<210> 392
<211> 8
<212> PRT
<213> Homo sapiens

<400> 392

His Ser Ile Val Ser Ser Phe Ala
1               5


<210> 393
<211> 8

<212>  PRT
<213>  Homo sapiens

<400>  393

Leu Asp Val Thr Leu Ile Leu Ala
1               5


<210>  394
<211>  8
<212>  PRT
<213>  Homo sapiens

<400>  394

Glu Tyr Arg Ser Ile Ser Leu Ala
1               5


<210>  395
<211>  8
<212>  PRT
<213>  Homo sapiens

<400>  395

Arg Ser Ile Ser Leu Ala Ile Ala
1               5


<210>  396
<211>  8
<212>  PRT
<213>  Homo sapiens

<400>  396

Leu Phe Asn Ile Leu Asp Thr Ala
1               5


<210>  397
<211>  8
<212>  PRT
<213>  Homo sapiens

<400>  397

Tyr Val Thr Glu Arg Ile Ile Ala
1               5


<210>  398
<211>  8
<212>  PRT
<213>  Homo sapiens

<400>  398

Tyr Asn Leu Cys Ser Glu Arg Ala
1               5


<210>  399
<211>  8
<212>  PRT
<213>  Homo sapiens

<400>  399

Lys Glu Val Asn Glu Trp Met Ala
1               5


<210>  400
<211>  8
<212>  PRT
<213>  Homo sapiens

<400>  400

Gln Asp Leu Glu Asn Ile Val Ala
1               5


<210>  401
<211>  8
<212>  PRT
<213>  Homo sapiens

<400>  401

Arg Thr Gly Thr Met Val Cys Ala
1               5


<210>  402
<211>  8
<212>  PRT
<213>  Homo sapiens

<400>  402

Met Val Cys Ala Phe Leu Ile Ala
1               5


<210>  403
<211>  8
<212>  PRT
<213>  Homo sapiens

<400>  403

Ala Ser Glu Ile Cys Ser Thr Ala
1               5


<210>  404
<211>  8
<212>  PRT
<213>  Homo sapiens

<400>  404

Pro Ser Gln Lys Arg Tyr Val Ala
1               5


<210>  405
<211>  8
<212>  PRT
<213>  Homo sapiens

<400>  405

Lys Arg Tyr Val Ala Tyr Phe Ala
1               5


<210>  406
<211>  8
<212>  PRT
<213>  Homo sapiens

<400>  406

Asp Asn Leu His Lys Gln Lys Ala
1               5


<210>  407
<211>  8
<212>  PRT
<213>  Homo sapiens

<400>  407

Arg Ile Tyr Pro Ser Asp Phe Ala
1               5


<210>  408
<211>  8
<212>  PRT
<213>  Homo sapiens

<400>  408

Met Thr Ser Ser Asp Val Val Ala
1               5


<210>  409
<211>  9
<212>  PRT
<213>  Homo sapiens

<400>  409

Asp Leu Ala Gly Val Ile Ile Glu Leu
1               5


<210>  410
<211>  9
<212>  PRT
<213>  Homo sapiens

<400>  410

Ser Leu Ala Ile Ala Leu Phe Phe Leu
1               5


<210>  411
<211>  9
<212>  PRT
<213>  Homo sapiens

<400>  411

Phe Leu Met Asp Val Leu Leu Arg Val

1                 5


<210>  412
<211>  9
<212>  PRT
<213>  Homo sapiens

<400>  412

Leu Leu Leu Val Asp Val Val Tyr Ile
1               5


<210>  413
<211>  9
<212>  PRT
<213>  Homo sapiens

<400>  413

Val Leu Leu Asp Val Thr Leu Ile Leu
1               5


<210>  414
<211>  9
<212>  PRT
<213>  Homo sapiens

<400>  414

Ala Ile Ile Val Ile Leu Leu Leu Val
1               5


<210>  415
<211>  9
<212>  PRT
<213>  Homo sapiens

<400>  415

Arg Leu Leu Arg Leu Ile Ile Leu Leu
1               5


<210>  416
<211>  9
<212>  PRT
<213>  Homo sapiens

<400>  416

Phe Leu Val Leu Leu Asp Val Thr Leu
1               5


<210>  417
<211>  9
<212>  PRT
<213>  Homo sapiens

<400>  417

Val Ile Leu Leu Leu Val Asp Val Val
1               5

<210> 418
<211> 9
<212> PRT
<213> Homo sapiens

<400> 418

Ile Ile Leu Leu Arg Ile Phe His Leu
1               5


<210> 419
<211> 9
<212> PRT
<213> Homo sapiens

<400> 419

Gly Leu Phe Gly Val Phe Leu Val Leu
1               5


<210> 420
<211> 9
<212> PRT
<213> Homo sapiens

<400> 420

Ile Val Ile Leu Leu Leu Val Asp Val
1               5


<210> 421
<211> 9
<212> PRT
<213> Homo sapiens

<400> 421

His Leu Leu Arg Leu Leu Arg Leu Ile
1               5


<210> 422
<211> 9
<212> PRT
<213> Homo sapiens

<400> 422

Ser Ile Pro Arg Tyr Val Arg Asp Leu
1               5


<210> 423
<211> 9
<212> PRT
<213> Homo sapiens

<400> 423

Arg Leu Tyr Leu Pro Lys Asn Glu Leu
1               5


<210> 424

<211>   9
<212>   PRT
<213>   Homo sapiens

<400>   424

Ile Leu Asp Thr Ala Ile Ile Val Ile
1               5


<210>   425
<211>   9
<212>   PRT
<213>   Homo sapiens

<400>   425

Lys Ile Lys Lys Ile Val His Ser Ile
1               5


<210>   426
<211>   9
<212>   PRT
<213>   Homo sapiens

<400>   426

Ala Leu Phe Phe Leu Met Asp Val Leu
1               5


<210>   427
<211>   9
<212>   PRT
<213>   Homo sapiens

<400>   427

Tyr Ile Phe Phe Asp Ile Lys Leu Leu
1               5


<210>   428
<211>   9
<212>   PRT
<213>   Homo sapiens

<400>   428

Ile Leu Ala Asp Leu Ile Phe Thr Asp
1               5


<210>   429
<211>   9
<212>   PRT
<213>   Homo sapiens

<400>   429

Asp Leu Ile Phe Thr Asp Ser Lys Leu
1               5


<210>   430
<211>   9
<212>   PRT

<213>  Homo sapiens

<400>  430

Ile Ala Leu Phe Phe Leu Met Asp Val
1               5


<210>  431
<211>  9
<212>  PRT
<213>  Homo sapiens

<400>  431

Leu Leu Arg Leu Leu Arg Leu Ile Ile
1               5


<210>  432
<211>  9
<212>  PRT
<213>  Homo sapiens

<400>  432

Gln Met Val Val Phe Thr Lys Glu Val
1               5


<210>  433
<211>  9
<212>  PRT
<213>  Homo sapiens

<400>  433

Trp Met Ala Gln Asp Leu Glu Asn Ile
1               5


<210>  434
<211>  9
<212>  PRT
<213>  Homo sapiens

<400>  434

Leu Ile Ala Ser Glu Ile Cys Ser Thr
1               5


<210>  435
<211>  9
<212>  PRT
<213>  Homo sapiens

<400>  435

Ile Leu Phe Ile Lys His Phe Ile Ile
1               5


<210>  436
<211>  9
<212>  PRT
<213>  Homo sapiens

<400>  436

Thr Ile Ser Leu Gly Lys Cys Ser Val
1               5


<210>  437
<211>  9
<212>  PRT
<213>  Homo sapiens

<400>  437

Arg Ile Tyr Pro Ser Asp Phe Ala Val
1               5


<210>  438
<211>  9
<212>  PRT
<213>  Homo sapiens

<400>  438

Gly Val Phe Leu Val Leu Leu Asp Val
1               5


<210>  439
<211>  9
<212>  PRT
<213>  Homo sapiens

<400>  439

Asn Ile Leu Asp Thr Ala Ile Ile Val
1               5


<210>  440
<211>  9
<212>  PRT
<213>  Homo sapiens

<400>  440

Thr Ala Ile Ile Val Ile Leu Leu Leu
1               5


<210>  441
<211>  9
<212>  PRT
<213>  Homo sapiens

<400>  441

Asn Ile Pro Arg Trp Thr His Leu Leu
1               5


<210>  442
<211>  9
<212>  PRT
<213>  Homo sapiens

<400>  442

His Leu Phe His Gln Lys Arg Gln Leu
1               5

<210>  443
<211>  9
<212>  PRT
<213>  Homo sapiens

<400>  443

Ile Ile Tyr Ser Ile Pro Arg Tyr Val
1               5

<210>  444
<211>  9
<212>  PRT
<213>  Homo sapiens

<400>  444

Ser Phe Ala Phe Gly Leu Phe Gly Val
1               5

<210>  445
<211>  9
<212>  PRT
<213>  Homo sapiens

<400>  445

Leu Val Leu Leu Asp Val Thr Leu Ile
1               5

<210>  446
<211>  9
<212>  PRT
<213>  Homo sapiens

<400>  446

Leu Leu Asp Val Thr Leu Ile Leu Ala
1               5

<210>  447
<211>  9
<212>  PRT
<213>  Homo sapiens

<400>  447

Leu Ile Leu Ala Asp Leu Ile Phe Thr
1               5

<210>  448
<211>  9
<212>  PRT
<213>  Homo sapiens

<400>  448

Tyr Ile Pro Leu Glu Tyr Arg Ser Ile
1               5

<210> 449
<211> 9
<212> PRT
<213> Homo sapiens

<400> 449

Asp Leu Phe Asn Ile Leu Asp Thr Ala
1               5


<210> 450
<211> 9
<212> PRT
<213> Homo sapiens

<400> 450

Pro Ile Lys Glu Val Val Arg Phe Leu
1               5


<210> 451
<211> 9
<212> PRT
<213> Homo sapiens

<400> 451

Arg Ile Met Ile Asp Asp His Asn Val
1               5


<210> 452
<211> 9
<212> PRT
<213> Homo sapiens

<400> 452

Thr Leu His Gln Met Val Val Phe Thr
1               5


<210> 453
<211> 9
<212> PRT
<213> Homo sapiens

<400> 453

Leu Phe Gly Val Phe Leu Val Leu Leu
1               5


<210> 454
<211> 9
<212> PRT
<213> Homo sapiens

<400> 454

Asp Thr Ala Ile Ile Val Ile Leu Leu
1               5

<210>  455
<211>  9
<212>  PRT
<213>  Homo sapiens

<400>  455

Ile Asp Asp His Asn Val Pro Thr Leu
1               5


<210>  456
<211>  9
<212>  PRT
<213>  Homo sapiens

<400>  456

Gln Asp Leu Glu Asn Ile Val Ala Ile
1               5


<210>  457
<211>  9
<212>  PRT
<213>  Homo sapiens

<400>  457

Phe Ile Glu Asn Asn Arg Leu Tyr Leu
1               5


<210>  458
<211>  9
<212>  PRT
<213>  Homo sapiens

<400>  458

Arg Val Ser Pro Ile Ser Glu Ser Val
1               5


<210>  459
<211>  9
<212>  PRT
<213>  Homo sapiens

<400>  459

Leu Ala Arg Leu Ser Lys Phe Glu Val
1               5


<210>  460
<211>  9
<212>  PRT
<213>  Homo sapiens

<400>  460

Ile Val Ser Ser Phe Ala Phe Gly Leu
1               5


<210>  461
<211>  9

<212>   PRT
<213>   Homo sapiens

<400>   461

Phe Gly Leu Phe Gly Val Phe Leu Val
1               5


<210>   462
<211>   9
<212>   PRT
<213>   Homo sapiens

<400>   462

Asp Val Thr Leu Ile Leu Ala Asp Leu
1               5


<210>   463
<211>   9
<212>   PRT
<213>   Homo sapiens

<400>   463

Leu Leu Val Asp Val Val Tyr Ile Phe
1               5


<210>   464
<211>   9
<212>   PRT
<213>   Homo sapiens

<400>   464

Lys Leu Leu Arg Asn Ile Pro Arg Trp
1               5


<210>   465
<211>   9
<212>   PRT
<213>   Homo sapiens

<400>   465

Leu Arg Leu Leu Arg Leu Ile Ile Leu
1               5


<210>   466
<211>   9
<212>   PRT
<213>   Homo sapiens

<400>   466

Arg Ile Leu Phe Ile Lys His Phe Ile
1               5


<210>   467
<211>   9
<212>   PRT
<213>   Homo sapiens

<400>  467

Lys Val Val Phe Ser Thr Ile Ser Leu
1               5


<210>  468
<211>  9
<212>  PRT
<213>  Homo sapiens

<400>  468

Thr Thr Asp Lys Ile Leu Ile Asp Val
1               5


<210>  469
<211>  10
<212>  PRT
<213>  Homo sapiens

<400>  469

Gly Leu Phe Gly Val Phe Leu Val Leu Leu
1               5                   10


<210>  470
<211>  10
<212>  PRT
<213>  Homo sapiens

<400>  470

Ile Leu Asp Thr Ala Ile Ile Val Ile Leu
1               5                   10


<210>  471
<211>  10
<212>  PRT
<213>  Homo sapiens

<400>  471

Ile Leu Leu Leu Val Asp Val Val Tyr Ile
1               5                   10


<210>  472
<211>  10
<212>  PRT
<213>  Homo sapiens

<400>  472

Ala Ile Ala Leu Phe Phe Leu Met Asp Val
1               5                   10


<210>  473
<211>  10
<212>  PRT
<213>  Homo sapiens

<400>  473

Ile Ile Val Ile Leu Leu Leu Val Asp Val
1               5                   10

<210>  474
<211>  10
<212>  PRT
<213>  Homo sapiens

<400>  474

Leu Leu Arg Leu Leu Arg Leu Ile Ile Leu
1               5                   10

<210>  475
<211>  10
<212>  PRT
<213>  Homo sapiens

<400>  475

Leu Ile Ile Leu Leu Arg Ile Phe His Leu
1               5                   10

<210>  476
<211>  10
<212>  PRT
<213>  Homo sapiens

<400>  476

Met Ile Asp Asp His Asn Val Pro Thr Leu
1               5                   10

<210>  477
<211>  10
<212>  PRT
<213>  Homo sapiens

<400>  477

Tyr Leu Pro Lys Asn Glu Leu Asp Asn Leu
1               5                   10

<210>  478
<211>  10
<212>  PRT
<213>  Homo sapiens

<400>  478

Val Leu Ala Arg Leu Ser Lys Phe Glu Val
1               5                   10

<210>  479
<211>  10
<212>  PRT
<213>  Homo sapiens

<400>  479

Leu Leu Arg Leu Ile Ile Leu Leu Arg Ile

1                   5                        10

<210>  480
<211>  10
<212>  PRT
<213>  Homo sapiens

<400>  480

Asn Leu Pro Pro Arg Arg Ile Leu Phe Ile
1               5                   10

<210>  481
<211>  10
<212>  PRT
<213>  Homo sapiens

<400>  481

Ala Leu Phe Phe Leu Met Asp Val Leu Leu
1               5                   10

<210>  482
<211>  10
<212>  PRT
<213>  Homo sapiens

<400>  482

Pro Ile Ser Glu Ser Val Leu Ala Arg Leu
1               5                   10

<210>  483
<211>  10
<212>  PRT
<213>  Homo sapiens

<400>  483

Phe Leu Val Leu Leu Asp Val Thr Leu Ile
1               5                   10

<210>  484
<211>  10
<212>  PRT
<213>  Homo sapiens

<400>  484

Asn Ile Leu Asp Thr Ala Ile Ile Val Ile
1               5                   10

<210>  485
<211>  10
<212>  PRT
<213>  Homo sapiens

<400>  485

Phe Leu Ile Ala Ser Glu Ile Cys Ser Thr
1               5                   10

<210> 486
<211> 10
<212> PRT
<213> Homo sapiens

<400> 486

Phe Ile Lys His Phe Ile Ile Tyr Ser Ile
1               5                   10


<210> 487
<211> 10
<212> PRT
<213> Homo sapiens

<400> 487

Val Leu Leu Asp Val Thr Leu Ile Leu Ala
1               5                   10


<210> 488
<211> 10
<212> PRT
<213> Homo sapiens

<400> 488

Leu Met Asp Val Leu Leu Arg Val Phe Val
1               5                   10


<210> 489
<211> 10
<212> PRT
<213> Homo sapiens

<400> 489

Ile Val Ile Leu Leu Leu Val Asp Val Val
1               5                   10


<210> 490
<211> 10
<212> PRT
<213> Homo sapiens

<400> 490

Ser Pro Asp Pro Thr Asp Leu Ala Gly Val
1               5                   10


<210> 491
<211> 10
<212> PRT
<213> Homo sapiens

<400> 491

Ser Ile Val Ser Ser Phe Ala Phe Gly Leu
1               5                   10


<210> 492

<211>  10
<212>  PRT
<213>  Homo sapiens

<400>  492

Thr Ala Ile Ile Val Ile Leu Leu Leu Val
1               5                   10


<210>  493
<211>  10
<212>  PRT
<213>  Homo sapiens

<400>  493

His Leu Leu Arg Leu Leu Arg Leu Ile Ile
1               5                   10


<210>  494
<211>  10
<212>  PRT
<213>  Homo sapiens

<400>  494

Trp Met Ala Gln Asp Leu Glu Asn Ile Val
1               5                   10


<210>  495
<211>  10
<212>  PRT
<213>  Homo sapiens

<400>  495

Val Leu Asp Asn Ile Thr Thr Asp Lys Ile
1               5                   10


<210>  496
<211>  10
<212>  PRT
<213>  Homo sapiens

<400>  496

Leu Ile Asp Val Phe Asp Gly Pro Pro Leu
1               5                   10


<210>  497
<211>  10
<212>  PRT
<213>  Homo sapiens

<400>  497

Phe Ala Phe Gly Leu Phe Gly Val Phe Leu
1               5                   10


<210>  498
<211>  10
<212>  PRT

<213>  Homo sapiens

<400>  498

Thr Leu Ile Leu Ala Asp Leu Ile Phe Thr
1               5                   10


<210>  499
<211>  10
<212>  PRT
<213>  Homo sapiens

<400>  499

Leu Ile Phe Thr Asp Ser Lys Leu Tyr Ile
1               5                   10


<210>  500
<211>  10
<212>  PRT
<213>  Homo sapiens

<400>  500

Asp Thr Ala Ile Ile Val Ile Leu Leu Leu
1               5                   10


<210>  501
<211>  10
<212>  PRT
<213>  Homo sapiens

<400>  501

Phe Ile Ile Tyr Ser Ile Pro Arg Tyr Val
1               5                   10


<210>  502
<211>  10
<212>  PRT
<213>  Homo sapiens

<400>  502

Tyr Ser Ile Pro Arg Tyr Val Arg Asp Leu
1               5                   10


<210>  503
<211>  10
<212>  PRT
<213>  Homo sapiens

<400>  503

Lys Ile Gln Ile Glu Met Glu Lys Lys Val
1               5                   10


<210>  504
<211>  10
<212>  PRT
<213>  Homo sapiens

<400>  504

Ser Thr Ile Ser Leu Gly Lys Cys Ser Val
1               5                   10


<210>  505
<211>  10
<212>  PRT
<213>  Homo sapiens

<400>  505

Ile Thr Thr Asp Lys Ile Leu Ile Asp Val
1               5                   10


<210>  506
<211>  10
<212>  PRT
<213>  Homo sapiens

<400>  506

Ser Lys Ile Lys Lys Ile Val His Ser Ile
1               5                   10


<210>  507
<211>  10
<212>  PRT
<213>  Homo sapiens

<400>  507

Leu Val Leu Leu Asp Val Thr Leu Ile Leu
1               5                   10


<210>  508
<211>  10
<212>  PRT
<213>  Homo sapiens

<400>  508

Asp Leu Phe Asn Ile Leu Asp Thr Ala Ile
1               5                   10


<210>  509
<211>  10
<212>  PRT
<213>  Homo sapiens

<400>  509

Leu Leu Leu Val Asp Val Val Tyr Ile Phe
1               5                   10


<210>  510
<211>  10
<212>  PRT
<213>  Homo sapiens

<400>  510

Trp Thr His Leu Leu Arg Leu Leu Arg Leu
1               5                   10

<210>  511
<211>  10
<212>  PRT
<213>  Homo sapiens

<400>  511

Gln Leu Glu Lys Leu Ile Arg Arg Arg Val
1               5                   10

<210>  512
<211>  10
<212>  PRT
<213>  Homo sapiens

<400>  512

Lys Leu Ile Arg Arg Arg Val Ser Glu Asn
1               5                   10

<210>  513
<211>  10
<212>  PRT
<213>  Homo sapiens

<400>  513

Ile Met Ile Asp Asp His Asn Val Pro Thr
1               5                   10

<210>  514
<211>  10
<212>  PRT
<213>  Homo sapiens

<400>  514

Thr Asp Leu Ala Gly Val Ile Ile Glu Leu
1               5                   10

<210>  515
<211>  10
<212>  PRT
<213>  Homo sapiens

<400>  515

Arg Leu Ser Lys Phe Glu Val Glu Asp Ala
1               5                   10

<210>  516
<211>  10
<212>  PRT
<213>  Homo sapiens

<400>  516

Lys Ile Lys Lys Ile Val His Ser Ile Val
1               5                   10

<210>  517
<211>  10
<212>  PRT
<213>  Homo sapiens

<400>  517

Ser Ser Phe Ala Phe Gly Leu Phe Gly Val
1               5                   10


<210>  518
<211>  10
<212>  PRT
<213>  Homo sapiens

<400>  518

Leu Leu Asp Val Thr Leu Ile Leu Ala Asp
1               5                   10


<210>  519
<211>  10
<212>  PRT
<213>  Homo sapiens

<400>  519

Leu Asp Val Thr Leu Ile Leu Ala Asp Leu
1               5                   10


<210>  520
<211>  10
<212>  PRT
<213>  Homo sapiens

<400>  520

Leu Ile Leu Ala Asp Leu Ile Phe Thr Asp
1               5                   10


<210>  521
<211>  10
<212>  PRT
<213>  Homo sapiens

<400>  521

Ile Pro Leu Glu Tyr Arg Ser Ile Ser Leu
1               5                   10


<210>  522
<211>  10
<212>  PRT
<213>  Homo sapiens

<400>  522

Ile Ser Leu Ala Ile Ala Leu Phe Phe Leu
1               5                   10

<210>  523
<211>  10
<212>  PRT
<213>  Homo sapiens

<400>  523

Ser Leu Ala Ile Ala Leu Phe Phe Leu Met
1               5                   10


<210>  524
<211>  10
<212>  PRT
<213>  Homo sapiens

<400>  524

Phe Phe Leu Met Asp Val Leu Leu Arg Val
1               5                   10


<210>  525
<211>  10
<212>  PRT
<213>  Homo sapiens

<400>  525

Val Val Tyr Ile Phe Phe Asp Ile Lys Leu
1               5                   10


<210>  526
<211>  10
<212>  PRT
<213>  Homo sapiens

<400>  526

Leu Arg Leu Leu Arg Leu Ile Ile Leu Leu
1               5                   10


<210>  527
<211>  10
<212>  PRT
<213>  Homo sapiens

<400>  527

Tyr Thr Arg Asp Gly Phe Asp Leu Asp Leu
1               5                   10


<210>  528
<211>  10
<212>  PRT
<213>  Homo sapiens

<400>  528

Ser Phe Tyr Arg Asn Pro Ile Lys Glu Val
1               5                   10


<210>  529
<211>  10

<212>  PRT
<213>  Homo sapiens

<400>  529

Leu Ile Ala Ser Glu Ile Cys Ser Thr Ala
1               5                   10


<210>  530
<211>  10
<212>  PRT
<213>  Homo sapiens

<400>  530

Val Ala Tyr Phe Ala Gln Val Lys His Leu
1               5                   10


<210>  531
<211>  10
<212>  PRT
<213>  Homo sapiens

<400>  531

Thr Ile Ser Leu Gly Lys Cys Ser Val Leu
1               5                   10


<210>  532
<211>  10
<212>  PRT
<213>  Homo sapiens

<400>  532

Asn Leu His Lys Gln Lys Ala Arg Arg Ile
1               5                   10


<210>  533
<211>  9
<212>  PRT
<213>  Homo sapiens

<400>  533

Val Tyr Ile Phe Phe Asp Ile Lys Leu
1               5


<210>  534
<211>  9
<212>  PRT
<213>  Homo sapiens

<400>  534

Ser Tyr Asp Ser Lys Ile Lys Lys Ile
1               5


<210>  535
<211>  9
<212>  PRT
<213>  Homo sapiens

<400>  535

Ala Tyr Phe Ala Gln Val Lys His Leu
1               5


<210>  536
<211>  9
<212>  PRT
<213>  Homo sapiens

<400>  536

Leu Tyr Asp Asp Val Lys Val Gln Phe
1               5


<210>  537
<211>  9
<212>  PRT
<213>  Homo sapiens

<400>  537

Gln Tyr Phe Ser Asp Leu Phe Asn Ile
1               5


<210>  538
<211>  9
<212>  PRT
<213>  Homo sapiens

<400>  538

Arg Tyr Thr Arg Asp Gly Phe Asp Leu
1               5


<210>  539
<211>  9
<212>  PRT
<213>  Homo sapiens

<400>  539

Tyr Tyr Asp Asn Cys Ser Phe Tyr Phe
1               5


<210>  540
<211>  9
<212>  PRT
<213>  Homo sapiens

<400>  540

Phe Tyr Phe Trp Leu His Thr Ser Phe
1               5


<210>  541
<211>  9
<212>  PRT
<213>  Homo sapiens

<400>  541

Glu Tyr Arg Ser Ile Ser Leu Ala Ile
1               5

<210> 542
<211> 9
<212> PRT
<213> Homo sapiens

<400> 542

Leu Phe Gly Val Phe Leu Val Leu Leu
1               5

<210> 543
<211> 9
<212> PRT
<213> Homo sapiens

<400> 543

Leu Phe Phe Leu Met Asp Val Leu Leu
1               5

<210> 544
<211> 9
<212> PRT
<213> Homo sapiens

<400> 544

Tyr Phe Ser Asp Leu Phe Asn Ile Leu
1               5

<210> 545
<211> 9
<212> PRT
<213> Homo sapiens

<400> 545

Ile Phe Thr Asp Ser Lys Leu Tyr Ile
1               5

<210> 546
<211> 9
<212> PRT
<213> Homo sapiens

<400> 546

Thr Ala Ile Ile Val Ile Leu Leu Leu
1               5

<210> 547
<211> 9
<212> PRT
<213> Homo sapiens

<400> 547

His Phe His Asn Arg Val Val Arg Ile

1                   5


<210>  548
<211>  9
<212>  PRT
<213>  Homo sapiens

<400>  548

Tyr Phe Trp Leu His Thr Ser Phe Ile
1               5


<210>  549
<211>  9
<212>  PRT
<213>  Homo sapiens

<400>  549

Leu Tyr Leu Pro Lys Asn Glu Leu Asp
1               5


<210>  550
<211>  9
<212>  PRT
<213>  Homo sapiens

<400>  550

Ala Phe Gly Leu Phe Gly Val Phe Leu
1               5


<210>  551
<211>  9
<212>  PRT
<213>  Homo sapiens

<400>  551

Val Leu Leu Asp Val Thr Leu Ile Leu
1               5


<210>  552
<211>  9
<212>  PRT
<213>  Homo sapiens

<400>  552

Val Thr Leu Ile Leu Ala Asp Leu Ile
1               5


<210>  553
<211>  9
<212>  PRT
<213>  Homo sapiens

<400>  553

Leu Phe Asn Ile Leu Asp Thr Ala Ile
1               5

<210>  554
<211>  9
<212>  PRT
<213>  Homo sapiens

<400>  554

Ile Ile Leu Leu Arg Ile Phe His Leu
1               5


<210>  555
<211>  9
<212>  PRT
<213>  Homo sapiens

<400>  555

Ile Leu Leu Arg Ile Phe His Leu Phe
1               5


<210>  556
<211>  9
<212>  PRT
<213>  Homo sapiens

<400>  556

Asn Leu Pro Pro Arg Arg Ile Leu Phe
1               5


<210>  557
<211>  9
<212>  PRT
<213>  Homo sapiens

<400>  557

Ser Val Leu Ala Arg Leu Ser Lys Phe
1               5


<210>  558
<211>  9
<212>  PRT
<213>  Homo sapiens

<400>  558

Val Ser Ser Phe Ala Phe Gly Leu Phe
1               5


<210>  559
<211>  9
<212>  PRT
<213>  Homo sapiens

<400>  559

Leu Val Leu Leu Asp Val Thr Leu Ile
1               5


<210>  560

<211>  9
<212>  PRT
<213>  Homo sapiens

<400>  560

Arg Asn Ile Pro Arg Trp Thr His Leu
1               5


<210>  561
<211>  9
<212>  PRT
<213>  Homo sapiens

<400>  561

Arg Leu Leu Arg Leu Ile Ile Leu Leu
1               5


<210>  562
<211>  9
<212>  PRT
<213>  Homo sapiens

<400>  562

Ala Tyr Asp Pro Lys His Phe His Asn
1               5


<210>  563
<211>  9
<212>  PRT
<213>  Homo sapiens

<400>  563

Gln Asp Leu Glu Asn Ile Val Ala Ile
1               5


<210>  564
<211>  10
<212>  PRT
<213>  Homo sapiens

<400>  564

Val Tyr Ile Phe Phe Asp Ile Lys Leu Leu
1               5                   10


<210>  565
<211>  10
<212>  PRT
<213>  Homo sapiens

<400>  565

Leu Tyr Ile Pro Leu Glu Tyr Arg Ser Ile
1               5                   10


<210>  566
<211>  10
<212>  PRT

<213>  Homo sapiens

<400>  566

Thr Tyr Tyr Asp Asn Cys Ser Phe Tyr Phe
1               5                   10


<210>  567
<211>  10
<212>  PRT
<213>  Homo sapiens

<400>  567

Leu Tyr Asp Asp Val Lys Val Gln Phe Phe
1               5                   10


<210>  568
<211>  10
<212>  PRT
<213>  Homo sapiens

<400>  568

Ile Tyr Pro Ser Asp Phe Ala Val Glu Ile
1               5                   10


<210>  569
<211>  10
<212>  PRT
<213>  Homo sapiens

<400>  569

Gln Tyr Phe Ser Asp Leu Phe Asn Ile Leu
1               5                   10


<210>  570
<211>  10
<212>  PRT
<213>  Homo sapiens

<400>  570

Val Phe Leu Val Leu Leu Asp Val Thr Leu
1               5                   10


<210>  571
<211>  10
<212>  PRT
<213>  Homo sapiens

<400>  571

Val Phe Val Glu Arg Arg Gln Gln Tyr Phe
1               5                   10


<210>  572
<211>  10
<212>  PRT
<213>  Homo sapiens

<400>  572

Arg Tyr Val Arg Asp Leu Lys Ile Gln Ile
1               5                   10


<210>  573
<211>  10
<212>  PRT
<213>  Homo sapiens

<400>  573

Phe Tyr Phe Trp Leu His Thr Ser Phe Ile
1               5                   10


<210>  574
<211>  10
<212>  PRT
<213>  Homo sapiens

<400>  574

Ser Phe Ile Glu Asn Asn Arg Leu Tyr Leu
1               5                   10


<210>  575
<211>  10
<212>  PRT
<213>  Homo sapiens

<400>  575

Ser Phe Pro Ser Ser Gly Arg Gln Ser Phe
1               5                   10


<210>  576
<211>  10
<212>  PRT
<213>  Homo sapiens

<400>  576

Ser Phe Ala Phe Gly Leu Phe Gly Val Phe
1               5                   10


<210>  577
<211>  10
<212>  PRT
<213>  Homo sapiens

<400>  577

Ser Phe Tyr Phe Trp Leu His Thr Ser Phe
1               5                   10


<210>  578
<211>  10
<212>  PRT
<213>  Homo sapiens

<400>  578

Leu Phe Asn Ile Leu Asp Thr Ala Ile Ile
1               5                   10

<210>  579
<211>  10
<212>  PRT
<213>  Homo sapiens

<400>  579

Phe Phe Asp Ile Lys Leu Leu Arg Asn Ile
1               5                   10

<210>  580
<211>  10
<212>  PRT
<213>  Homo sapiens

<400>  580

Arg Ser Ile Ser Leu Ala Ile Ala Leu Phe
1               5                   10

<210>  581
<211>  10
<212>  PRT
<213>  Homo sapiens

<400>  581

Arg Asn Ile Pro Arg Trp Thr His Leu Leu
1               5                   10

<210>  582
<211>  10
<212>  PRT
<213>  Homo sapiens

<400>  582

Thr His Leu Leu Arg Leu Leu Arg Leu Ile
1               5                   10

<210>  583
<211>  10
<212>  PRT
<213>  Homo sapiens

<400>  583

Leu Arg Leu Leu Arg Leu Ile Ile Leu Leu
1               5                   10

<210>  584
<211>  10
<212>  PRT
<213>  Homo sapiens

<400>  584

Leu Ile Ile Leu Leu Arg Ile Phe His Leu
1               5                   10

<210> 585
<211> 10
<212> PRT
<213> Homo sapiens

<400> 585

Ile Ile Leu Leu Arg Ile Phe His Leu Phe
1               5                   10


<210> 586
<211> 10
<212> PRT
<213> Homo sapiens

<400> 586

Trp Asn Leu Pro Pro Arg Arg Ile Leu Phe
1               5                   10


<210> 587
<211> 9
<212> PRT
<213> Homo sapiens

<400> 587

Ser Thr Ala Lys Glu Ser Leu Tyr Tyr
1               5


<210> 588
<211> 9
<212> PRT
<213> Homo sapiens

<400> 588

Ser Lys Leu Tyr Ile Pro Leu Glu Tyr
1               5


<210> 589
<211> 9
<212> PRT
<213> Homo sapiens

<400> 589

Ser Pro Asp Pro Thr Asp Leu Ala Gly
1               5


<210> 590
<211> 9
<212> PRT
<213> Homo sapiens

<400> 590

Phe Ser Asp Leu Phe Asn Ile Leu Asp
1               5

<210>  591
<211>  9
<212>  PRT
<213>  Homo sapiens

<400>  591

Leu Phe Ile Lys His Phe Ile Ile Tyr
1               5


<210>  592
<211>  9
<212>  PRT
<213>  Homo sapiens

<400>  592

Asp Gly Phe Asp Leu Asp Leu Thr Tyr
1               5


<210>  593
<211>  9
<212>  PRT
<213>  Homo sapiens

<400>  593

Cys Ser Thr Ala Lys Glu Ser Leu Tyr
1               5


<210>  594
<211>  9
<212>  PRT
<213>  Homo sapiens

<400>  594

Tyr Phe Ala Gln Val Lys His Leu Tyr
1               5


<210>  595
<211>  9
<212>  PRT
<213>  Homo sapiens

<400>  595

Pro Ser Ser Gly Arg Gln Ser Phe Tyr
1               5


<210>  596
<211>  9
<212>  PRT
<213>  Homo sapiens

<400>  596

Val Glu Thr Pro Ser Gln Lys Arg Tyr
1               5


<210>  597
<211>  9

<212>   PRT
<213>   Homo sapiens

<400>   597

Thr Thr Asp Lys Ile Leu Ile Asp Val
1               5


<210>   598
<211>   9
<212>   PRT
<213>   Homo sapiens

<400>   598

Ser Phe Ile Glu Asn Asn Arg Leu Tyr
1               5


<210>   599
<211>   9
<212>   PRT
<213>   Homo sapiens

<400>   599

Ala Thr Glu Glu Ala Pro Ala Lys Glu
1               5


<210>   600
<211>   9
<212>   PRT
<213>   Homo sapiens

<400>   600

Ile Leu Leu Leu Val Asp Val Val Tyr
1               5


<210>   601
<211>   9
<212>   PRT
<213>   Homo sapiens

<400>   601

Pro Ser Gln Lys Arg Tyr Val Ala Tyr
1               5


<210>   602
<211>   9
<212>   PRT
<213>   Homo sapiens

<400>   602

Asp Val Phe Asp Gly Pro Pro Leu Tyr
1               5


<210>   603
<211>   9
<212>   PRT
<213>   Homo sapiens

<400>   603

Val Phe Asp Gly Pro Pro Leu Tyr Asp
1               5


<210>   604
<211>   9
<212>   PRT
<213>   Homo sapiens

<400>   604

Val Thr Glu Arg Ile Ile Ala Met Ser
1               5


<210>   605
<211>   9
<212>   PRT
<213>   Homo sapiens

<400>   605

Gly Thr Asp Arg Thr Gly Thr Met Val
1               5


<210>   606
<211>   9
<212>   PRT
<213>   Homo sapiens

<400>   606

Phe Ile Glu Asn Asn Arg Leu Tyr Leu
1               5


<210>   607
<211>   9
<212>   PRT
<213>   Homo sapiens

<400>   607

Ser Ser Asp Val Val Ala Gly Ser Asp
1               5


<210>   608
<211>   9
<212>   PRT
<213>   Homo sapiens

<400>   608

Pro Thr Asp Leu Ala Gly Val Ile Ile
1               5


<210>   609
<211>   9
<212>   PRT
<213>   Homo sapiens

<400>   609

Leu Leu Asp Val Thr Leu Ile Leu Ala
1               5

<210>  610
<211>  9
<212>  PRT
<213>  Homo sapiens

<400>  610

Leu Ile Phe Thr Asp Ser Lys Leu Tyr
1               5

<210>  611
<211>  9
<212>  PRT
<213>  Homo sapiens

<400>  611

Phe Thr Asp Ser Lys Leu Tyr Ile Pro
1               5

<210>  612
<211>  9
<212>  PRT
<213>  Homo sapiens

<400>  612

Val Phe Val Glu Arg Arg Gln Gln Tyr
1               5

<210>  613
<211>  9
<212>  PRT
<213>  Homo sapiens

<400>  613

Phe Phe Asp Ile Lys Leu Leu Arg Asn
1               5

<210>  614
<211>  9
<212>  PRT
<213>  Homo sapiens

<400>  614

Thr Arg Asp Gly Phe Asp Leu Asp Leu
1               5

<210>  615
<211>  9
<212>  PRT
<213>  Homo sapiens

<400>  615

Tyr Asn Leu Cys Ser Glu Arg Ala Tyr

1                   5


<210>  616
<211>  9
<212>  PRT
<213>  Homo sapiens

<400>  616

Ala Gln Asp Leu Glu Asn Ile Val Ala
1               5


<210>  617
<211>  9
<212>  PRT
<213>  Homo sapiens

<400>  617

Asp Asp Val Lys Val Gln Phe Phe Tyr
1               5


<210>  618
<211>  9
<212>  PRT
<213>  Homo sapiens

<400>  618

Lys Asn Glu Leu Asp Asn Leu His Lys
1               5


<210>  619
<211>  9
<212>  PRT
<213>  Homo sapiens

<400>  619

Ile Ser Glu Ser Val Leu Ala Arg Leu
1               5


<210>  620
<211>  9
<212>  PRT
<213>  Homo sapiens

<400>  620

Glu Asp Ala Glu Asn Val Ala Ser Tyr
1               5


<210>  621
<211>  9
<212>  PRT
<213>  Homo sapiens

<400>  621

Ile Leu Asp Thr Ala Ile Ile Val Ile
1               5

<210> 622
<211> 9
<212> PRT
<213> Homo sapiens

<400> 622

Leu Val Asp Val Val Tyr Ile Phe Phe
1               5


<210> 623
<211> 9
<212> PRT
<213> Homo sapiens

<400> 623

Val Ser Glu Asn Lys Arg Arg Tyr Thr
1               5


<210> 624
<211> 9
<212> PRT
<213> Homo sapiens

<400> 624

Leu Asp Lys Lys His Arg Asn His Tyr
1               5


<210> 625
<211> 9
<212> PRT
<213> Homo sapiens

<400> 625

Lys His Arg Asn His Tyr Arg Val Tyr
1               5


<210> 626
<211> 9
<212> PRT
<213> Homo sapiens

<400> 626

Arg Thr Asp Lys Thr His Ser Glu Lys
1               5


<210> 627
<211> 9
<212> PRT
<213> Homo sapiens

<400> 627

Phe Ile Ile Tyr Ser Ile Pro Arg Tyr
1               5


<210> 628

<211>  9
<212>  PRT
<213>  Homo sapiens

<400>  628

Phe Phe Tyr Ser Asn Leu Pro Thr Tyr
1               5


<210>  629
<211>  9
<212>  PRT
<213>  Homo sapiens

<400>  629

Phe Tyr Ser Asn Leu Pro Thr Tyr Tyr
1               5


<210>  630
<211>  9
<212>  PRT
<213>  Homo sapiens

<400>  630

His Lys Gln Lys Ala Arg Arg Ile Tyr
1               5


<210>  631
<211>  9
<212>  PRT
<213>  Homo sapiens

<400>  631

Thr Ser Glu Phe Lys Gly Ala Thr Glu
1               5


<210>  632
<211>  9
<212>  PRT
<213>  Homo sapiens

<400>  632

Thr Ser Glu Phe Lys Gly Ala Ala Arg
1               5


<210>  633
<211>  9
<212>  PRT
<213>  Homo sapiens

<400>  633

Pro Leu Glu Tyr Arg Ser Ile Ser Leu
1               5


<210>  634
<211>  9
<212>  PRT

<213>  Homo sapiens

<400>  634

Trp Thr His Leu Leu Arg Leu Leu Arg
1               5


<210>  635
<211>  9
<212>  PRT
<213>  Homo sapiens

<400>  635

Arg Val Ser Glu Asn Lys Arg Arg Tyr
1               5


<210>  636
<211>  9
<212>  PRT
<213>  Homo sapiens

<400>  636

Tyr Thr Arg Asp Gly Phe Asp Leu Asp
1               5


<210>  637
<211>  9
<212>  PRT
<213>  Homo sapiens

<400>  637

Ile Lys Glu Val Val Arg Phe Leu Asp
1               5


<210>  638
<211>  9
<212>  PRT
<213>  Homo sapiens

<400>  638

Ala Tyr Asp Pro Lys His Phe His Asn
1               5


<210>  639
<211>  9
<212>  PRT
<213>  Homo sapiens

<400>  639

Ala Ser Glu Ile Cys Ser Thr Ala Lys
1               5


<210>  640
<211>  9
<212>  PRT
<213>  Homo sapiens

<400>  640

His Ser Glu Lys Phe Gln Gly Val Glu
1               5

<210>  641
<211>  9
<212>  PRT
<213>  Homo sapiens

<400>  641

Thr Tyr Tyr Asp Asn Cys Ser Phe Tyr
1               5

<210>  642
<211>  9
<212>  PRT
<213>  Homo sapiens

<400>  642

Pro Ser Asp Phe Ala Val Glu Ile Leu
1               5

<210>  643
<211>  10
<212>  PRT
<213>  Homo sapiens

<400>  643

Phe Leu Asp Lys Lys His Arg Asn His Tyr
1               5                   10

<210>  644
<211>  10
<212>  PRT
<213>  Homo sapiens

<400>  644

Val Glu Asp Ala Glu Asn Val Ala Ser Tyr
1               5                   10

<210>  645
<211>  10
<212>  PRT
<213>  Homo sapiens

<400>  645

Tyr Asp Asp Val Lys Val Gln Phe Phe Tyr
1               5                   10

<210>  646
<211>  10
<212>  PRT
<213>  Homo sapiens

<400>  646

Asp Ser Lys Leu Tyr Ile Pro Leu Glu Tyr
1               5                   10

<210>  647
<211>  10
<212>  PRT
<213>  Homo sapiens

<400>  647

Cys Ser Thr Ala Lys Glu Ser Leu Tyr Tyr
1               5                   10

<210>  648
<211>  10
<212>  PRT
<213>  Homo sapiens

<400>  648

Gly Val Glu Thr Pro Ser Gln Lys Arg Tyr
1               5                   10

<210>  649
<211>  10
<212>  PRT
<213>  Homo sapiens

<400>  649

Ile Leu Phe Ile Lys His Phe Ile Ile Tyr
1               5                   10

<210>  650
<211>  10
<212>  PRT
<213>  Homo sapiens

<400>  650

Thr Ser Phe Ile Glu Asn Asn Arg Leu Tyr
1               5                   10

<210>  651
<211>  10
<212>  PRT
<213>  Homo sapiens

<400>  651

Pro Thr Asp Leu Ala Gly Val Ile Ile Glu
1               5                   10

<210>  652
<211>  10
<212>  PRT
<213>  Homo sapiens

<400>  652

Phe Thr Asp Ser Lys Leu Tyr Ile Pro Leu
1               5                   10

<210> 653
<211> 10
<212> PRT
<213> Homo sapiens

<400> 653

Phe Ser Asp Leu Phe Asn Ile Leu Asp Thr
1               5                   10


<210> 654
<211> 10
<212> PRT
<213> Homo sapiens

<400> 654

Arg Asp Gly Phe Asp Leu Asp Leu Thr Tyr
1               5                   10


<210> 655
<211> 10
<212> PRT
<213> Homo sapiens

<400> 655

Pro Thr Tyr Tyr Asp Asn Cys Ser Phe Tyr
1               5                   10


<210> 656
<211> 10
<212> PRT
<213> Homo sapiens

<400> 656

Pro Ser Asp Phe Ala Val Glu Ile Leu Phe
1               5                   10


<210> 657
<211> 10
<212> PRT
<213> Homo sapiens

<400> 657

Ile Lys Glu Val Val Arg Phe Leu Asp Lys
1               5                   10


<210> 658
<211> 10
<212> PRT
<213> Homo sapiens

<400> 658

Asp Leu Ile Phe Thr Asp Ser Lys Leu Tyr
1               5                   10

<210> 659
<211> 10
<212> PRT
<213> Homo sapiens

<400> 659

Ala Tyr Asp Pro Lys His Phe His Asn Arg
1               5                   10


<210> 660
<211> 10
<212> PRT
<213> Homo sapiens

<400> 660

Arg Thr Asp Lys Thr His Ser Glu Lys Phe
1               5                   10


<210> 661
<211> 10
<212> PRT
<213> Homo sapiens

<400> 661

Ala Tyr Phe Ala Gln Val Lys His Leu Tyr
1               5                   10


<210> 662
<211> 10
<212> PRT
<213> Homo sapiens

<400> 662

Ala Thr Glu Glu Ala Pro Ala Lys Glu Ser
1               5                   10


<210> 663
<211> 10
<212> PRT
<213> Homo sapiens

<400> 663

Leu Leu Asp Val Thr Leu Ile Leu Ala Asp
1               5                   10


<210> 664
<211> 10
<212> PRT
<213> Homo sapiens

<400> 664

Val Ile Leu Leu Leu Val Asp Val Val Tyr
1               5                   10


<210> 665
<211> 10

<212>  PRT
<213>  Homo sapiens

<400>  665

Val Thr Glu Arg Ile Ile Ala Met Ser Phe
1               5                   10


<210>  666
<211>  10
<212>  PRT
<213>  Homo sapiens

<400>  666

Gly Thr Asp Arg Thr Gly Thr Met Val Cys
1               5                   10


<210>  667
<211>  10
<212>  PRT
<213>  Homo sapiens

<400>  667

Ala Ser Glu Ile Cys Ser Thr Ala Lys Glu
1               5                   10


<210>  668
<211>  10
<212>  PRT
<213>  Homo sapiens

<400>  668

Ile Cys Ser Thr Ala Lys Glu Ser Leu Tyr
1               5                   10


<210>  669
<211>  10
<212>  PRT
<213>  Homo sapiens

<400>  669

Thr Thr Asp Lys Ile Leu Ile Asp Val Phe
1               5                   10


<210>  670
<211>  10
<212>  PRT
<213>  Homo sapiens

<400>  670

Ile Asp Val Phe Asp Gly Pro Pro Leu Tyr
1               5                   10


<210>  671
<211>  10
<212>  PRT
<213>  Homo sapiens

<400>  671

Ser Pro Asp Pro Thr Asp Leu Ala Gly Val
1               5                   10


<210>  672
<211>  10
<212>  PRT
<213>  Homo sapiens

<400>  672

Ile Ser Glu Ser Val Leu Ala Arg Leu Ser
1               5                   10


<210>  673
<211>  10
<212>  PRT
<213>  Homo sapiens

<400>  673

Arg Val Phe Val Glu Arg Arg Gln Gln Tyr
1               5                   10


<210>  674
<211>  10
<212>  PRT
<213>  Homo sapiens

<400>  674

Val Ser Glu Asn Lys Arg Arg Tyr Thr Arg
1               5                   10


<210>  675
<211>  10
<212>  PRT
<213>  Homo sapiens

<400>  675

Phe Pro Ser Ser Gly Arg Gln Ser Phe Tyr
1               5                   10


<210>  676
<211>  10
<212>  PRT
<213>  Homo sapiens

<400>  676

Lys Lys His Arg Asn His Tyr Arg Val Tyr
1               5                   10


<210>  677
<211>  10
<212>  PRT
<213>  Homo sapiens

<400>  677

Val Tyr Asn Leu Cys Ser Glu Arg Ala Tyr
1               5                   10


<210>  678
<211>  10
<212>  PRT
<213>  Homo sapiens

<400>  678

Cys Ser Glu Arg Ala Tyr Asp Pro Lys His
1               5                   10


<210>  679
<211>  10
<212>  PRT
<213>  Homo sapiens

<400>  679

Phe Phe Tyr Ser Asn Leu Pro Thr Tyr Tyr
1               5                   10


<210>  680
<211>  10
<212>  PRT
<213>  Homo sapiens

<400>  680

Leu His Lys Gln Lys Ala Arg Arg Ile Tyr
1               5                   10


<210>  681
<211>  10
<212>  PRT
<213>  Homo sapiens

<400>  681

Arg Arg Val Ser Glu Asn Lys Arg Arg Tyr
1               5                   10


<210>  682
<211>  10
<212>  PRT
<213>  Homo sapiens

<400>  682

Asp Leu Glu Asn Ile Val Ala Ile His Cys
1               5                   10


<210>  683
<211>  10
<212>  PRT
<213>  Homo sapiens

<400>  683

Gly Thr Met Val Cys Ala Phe Leu Ile Ala

1                 5                    10


<210>  684
<211>  10
<212>  PRT
<213>  Homo sapiens

<400>  684

His Ser Glu Lys Phe Gln Gly Val Glu Thr
1               5                   10


<210>  685
<211>  10
<212>  PRT
<213>  Homo sapiens

<400>  685

Thr Pro Ser Gln Lys Arg Tyr Val Ala Tyr
1               5                   10


<210>  686
<211>  10
<212>  PRT
<213>  Homo sapiens

<400>  686

His Phe Ile Ile Tyr Ser Ile Pro Arg Tyr
1               5                   10


<210>  687
<211>  10
<212>  PRT
<213>  Homo sapiens

<400>  687

Val Arg Asp Leu Lys Ile Gln Ile Glu Met
1               5                   10


<210>  688
<211>  10
<212>  PRT
<213>  Homo sapiens

<400>  688

Gln Phe Phe Tyr Ser Asn Leu Pro Thr Tyr
1               5                   10


<210>  689
<211>  10
<212>  PRT
<213>  Homo sapiens

<400>  689

Tyr Tyr Asp Asn Cys Ser Phe Tyr Phe Trp
1               5                   10

<210> 690
<211> 9
<212> PRT
<213> Homo sapiens

<400> 690

Ile Leu Leu Leu Val Asp Val Val Tyr
1               5


<210> 691
<211> 9
<212> PRT
<213> Homo sapiens

<400> 691

Lys Leu Ile Arg Arg Arg Val Ser Glu
1               5


<210> 692
<211> 9
<212> PRT
<213> Homo sapiens

<400> 692

Tyr Val Ala Tyr Phe Ala Gln Val Lys
1               5


<210> 693
<211> 9
<212> PRT
<213> Homo sapiens

<400> 693

Arg Val Tyr Asn Leu Cys Ser Glu Arg
1               5


<210> 694
<211> 9
<212> PRT
<213> Homo sapiens

<400> 694

Val Val Tyr Ile Phe Phe Asp Ile Lys
1               5


<210> 695
<211> 9
<212> PRT
<213> Homo sapiens

<400> 695

Arg Ile Phe His Leu Phe His Gln Lys
1               5


<210> 696

<211>  9
<212>  PRT
<213>  Homo sapiens

<400>  696

Ala Val Glu Ile Leu Phe Gly Glu Lys
1               5


<210>  697
<211>  9
<212>  PRT
<213>  Homo sapiens

<400>  697

Ser Val Leu Ala Arg Leu Ser Lys Phe
1               5


<210>  698
<211>  9
<212>  PRT
<213>  Homo sapiens

<400>  698

Val Leu Leu Arg Val Phe Val Glu Arg
1               5


<210>  699
<211>  9
<212>  PRT
<213>  Homo sapiens

<400>  699

Glu Leu Asp Asn Leu His Lys Gln Lys
1               5


<210>  700
<211>  9
<212>  PRT
<213>  Homo sapiens

<400>  700

Glu Val Val Arg Phe Leu Asp Lys Lys
1               5


<210>  701
<211>  9
<212>  PRT
<213>  Homo sapiens

<400>  701

Lys Leu Tyr Ile Pro Leu Glu Tyr Arg
1               5


<210>  702
<211>  9
<212>  PRT

<213>  Homo sapiens

<400>  702

Arg Leu Ile Ile Leu Leu Arg Ile Phe
1               5


<210>  703
<211>  9
<212>  PRT
<213>  Homo sapiens

<400>  703

Ser Leu Tyr Tyr Phe Gly Glu Arg Arg
1               5


<210>  704
<211>  9
<212>  PRT
<213>  Homo sapiens

<400>  704

Lys Ile Gln Ile Glu Met Glu Lys Lys
1               5


<210>  705
<211>  9
<212>  PRT
<213>  Homo sapiens

<400>  705

Val Leu Asp Asn Ile Thr Thr Asp Lys
1               5


<210>  706
<211>  9
<212>  PRT
<213>  Homo sapiens

<400>  706

Ile Val His Ser Ile Val Ser Ser Phe
1               5


<210>  707
<211>  9
<212>  PRT
<213>  Homo sapiens

<400>  707

Thr Leu Ile Leu Ala Asp Leu Ile Phe
1               5


<210>  708
<211>  9
<212>  PRT
<213>  Homo sapiens

<400> 708

Leu Leu Arg Leu Ile Ile Leu Leu Arg
1               5


<210> 709
<211> 9
<212> PRT
<213> Homo sapiens

<400> 709

Ile Leu Leu Arg Ile Phe His Leu Phe
1               5


<210> 710
<211> 9
<212> PRT
<213> Homo sapiens

<400> 710

Leu Leu Arg Ile Phe His Leu Phe His
1               5


<210> 711
<211> 9
<212> PRT
<213> Homo sapiens

<400> 711

Arg Val Ser Glu Asn Lys Arg Arg Tyr
1               5


<210> 712
<211> 9
<212> PRT
<213> Homo sapiens

<400> 712

Gly Leu Phe Gly Val Phe Leu Val Leu
1               5


<210> 713
<211> 9
<212> PRT
<213> Homo sapiens

<400> 713

Ile Val Ile Leu Leu Leu Val Asp Val
1               5


<210> 714
<211> 9
<212> PRT
<213> Homo sapiens

<400> 714

Ala Ile His Cys Lys Gly Gly Thr Asp
1               5

<210>  715
<211>  9
<212>  PRT
<213>  Homo sapiens

<400>  715

Gln Gly Val Glu Thr Pro Ser Gln Lys
1               5

<210>  716
<211>  9
<212>  PRT
<213>  Homo sapiens

<400>  716

Asn Leu Pro Pro Arg Arg Ile Leu Phe
1               5

<210>  717
<211>  9
<212>  PRT
<213>  Homo sapiens

<400>  717

Asp Val Phe Asp Gly Pro Pro Leu Tyr
1               5

<210>  718
<211>  9
<212>  PRT
<213>  Homo sapiens

<400>  718

Arg Ile Tyr Pro Ser Asp Phe Ala Val
1               5

<210>  719
<211>  9
<212>  PRT
<213>  Homo sapiens

<400>  719

Arg Val Ser Pro Ile Ser Glu Ser Val
1               5

<210>  720
<211>  9
<212>  PRT
<213>  Homo sapiens

<400>  720

Phe Leu Val Leu Leu Asp Val Thr Leu
1               5

<210>  721
<211>  9
<212>  PRT
<213>  Homo sapiens

<400>  721

Asp Val Leu Leu Arg Val Phe Val Glu
1               5


<210>  722
<211>  9
<212>  PRT
<213>  Homo sapiens

<400>  722

Phe Val Glu Arg Arg Gln Gln Tyr Phe
1               5


<210>  723
<211>  9
<212>  PRT
<213>  Homo sapiens

<400>  723

Arg Leu Leu Arg Leu Ile Ile Leu Leu
1               5


<210>  724
<211>  9
<212>  PRT
<213>  Homo sapiens

<400>  724

Leu Ile Arg Arg Arg Val Ser Glu Asn
1               5


<210>  725
<211>  9
<212>  PRT
<213>  Homo sapiens

<400>  725

Ile Arg Arg Arg Val Ser Glu Asn Lys
1               5


<210>  726
<211>  9
<212>  PRT
<213>  Homo sapiens

<400>  726

Asp Leu Asp Leu Thr Tyr Val Thr Glu
1               5

<210> 727
<211> 9
<212> PRT
<213> Homo sapiens

<400> 727

Ser Val Leu Asp Asn Ile Thr Thr Asp
1               5


<210> 728
<211> 9
<212> PRT
<213> Homo sapiens

<400> 728

Pro Leu Tyr Asp Asp Val Lys Val Gln
1               5


<210> 729
<211> 9
<212> PRT
<213> Homo sapiens

<400> 729

Arg Leu Tyr Leu Pro Lys Asn Glu Leu
1               5


<210> 730
<211> 9
<212> PRT
<213> Homo sapiens

<400> 730

Ile Leu Phe Gly Glu Lys Met Thr Ser
1               5


<210> 731
<211> 9
<212> PRT
<213> Homo sapiens

<400> 731

Arg Leu Ser Lys Phe Glu Val Glu Asp
1               5


<210> 732
<211> 9
<212> PRT
<213> Homo sapiens

<400> 732

Ala Ser Tyr Asp Ser Lys Ile Lys Lys
1               5


<210> 733
<211> 9

<212>  PRT
<213>  Homo sapiens

<400>  733

Ile Leu Ala Asp Leu Ile Phe Thr Asp
1               5


<210>  734
<211>  9
<212>  PRT
<213>  Homo sapiens

<400>  734

Ala Asp Leu Ile Phe Thr Asp Ser Lys
1               5


<210>  735
<211>  9
<212>  PRT
<213>  Homo sapiens

<400>  735

Arg Val Phe Val Glu Arg Arg Gln Gln
1               5


<210>  736
<211>  9
<212>  PRT
<213>  Homo sapiens

<400>  736

Ile Ile Val Ile Leu Leu Leu Val Asp
1               5


<210>  737
<211>  9
<212>  PRT
<213>  Homo sapiens

<400>  737

Lys Leu Leu Arg Asn Ile Pro Arg Trp
1               5


<210>  738
<211>  9
<212>  PRT
<213>  Homo sapiens

<400>  738

Leu Leu Arg Leu Leu Arg Leu Ile Ile
1               5


<210>  739
<211>  9
<212>  PRT
<213>  Homo sapiens

<400>  739

Arg Asn Pro Ile Lys Glu Val Val Arg
1               5


<210>  740
<211>  9
<212>  PRT
<213>  Homo sapiens

<400>  740

Lys Glu Val Val Arg Phe Leu Asp Lys
1               5


<210>  741
<211>  9
<212>  PRT
<213>  Homo sapiens

<400>  741

Val Val Arg Ile Met Ile Asp Asp His
1               5


<210>  742
<211>  9
<212>  PRT
<213>  Homo sapiens

<400>  742

Asp Leu Glu Asn Ile Val Ala Ile His
1               5


<210>  743
<211>  9
<212>  PRT
<213>  Homo sapiens

<400>  743

His Leu Tyr Asn Trp Asn Leu Pro Pro
1               5


<210>  744
<211>  10
<212>  PRT
<213>  Homo sapiens

<400>  744

Arg Leu Leu Arg Leu Ile Ile Leu Leu Arg
1               5                   10


<210>  745
<211>  10
<212>  PRT
<213>  Homo sapiens

<400>  745

Pro Leu Tyr Asp Asp Val Lys Val Gln Phe
1               5                   10

<210>  746
<211>  10
<212>  PRT
<213>  Homo sapiens

<400>  746

Arg Ile Tyr Pro Ser Asp Phe Ala Val Glu
1               5                   10

<210>  747
<211>  10
<212>  PRT
<213>  Homo sapiens

<400>  747

Val Ile Leu Leu Leu Val Asp Val Val Tyr
1               5                   10

<210>  748
<211>  10
<212>  PRT
<213>  Homo sapiens

<400>  748

Ser Val Leu Asp Asn Ile Thr Thr Asp Lys
1               5                   10

<210>  749
<211>  10
<212>  PRT
<213>  Homo sapiens

<400>  749

Arg Val Ser Pro Ile Ser Glu Ser Val Leu
1               5                   10

<210>  750
<211>  10
<212>  PRT
<213>  Homo sapiens

<400>  750

Arg Val Phe Val Glu Arg Arg Gln Gln Tyr
1               5                   10

<210>  751
<211>  10
<212>  PRT
<213>  Homo sapiens

<400>  751

Ile Leu Leu Arg Ile Phe His Leu Phe His

1                   5                   10

<210>  752
<211>  10
<212>  PRT
<213>  Homo sapiens

<400>  752

Leu Ile Arg Arg Arg Val Ser Glu Asn Lys
1               5                   10

<210>  753
<211>  10
<212>  PRT
<213>  Homo sapiens

<400>  753

Thr Leu His Gln Met Val Val Phe Thr Lys
1               5                   10

<210>  754
<211>  10
<212>  PRT
<213>  Homo sapiens

<400>  754

Leu Leu Arg Asn Ile Pro Arg Trp Thr His
1               5                   10

<210>  755
<211>  10
<212>  PRT
<213>  Homo sapiens

<400>  755

Arg Leu Ile Ile Leu Leu Arg Ile Phe His
1               5                   10

<210>  756
<211>  10
<212>  PRT
<213>  Homo sapiens

<400>  756

Arg Tyr Val Ala Tyr Phe Ala Gln Val Lys
1               5                   10

<210>  757
<211>  10
<212>  PRT
<213>  Homo sapiens

<400>  757

Ile Ile Tyr Ser Ile Pro Arg Tyr Val Arg
1               5                   10

<210>  758
<211>  10
<212>  PRT
<213>  Homo sapiens

<400>  758

Ser Ile Pro Arg Tyr Val Arg Asp Leu Lys
1               5                   10


<210>  759
<211>  10
<212>  PRT
<213>  Homo sapiens

<400>  759

Asp Leu Lys Ile Gln Ile Glu Met Glu Lys
1               5                   10


<210>  760
<211>  10
<212>  PRT
<213>  Homo sapiens

<400>  760

Val Val Phe Ser Thr Ile Ser Leu Gly Lys
1               5                   10


<210>  761
<211>  10
<212>  PRT
<213>  Homo sapiens

<400>  761

Lys Leu Ile Arg Arg Arg Val Ser Glu Asn
1               5                   10


<210>  762
<211>  10
<212>  PRT
<213>  Homo sapiens

<400>  762

Gln Ile Glu Met Glu Lys Lys Val Val Phe
1               5                   10


<210>  763
<211>  10
<212>  PRT
<213>  Homo sapiens

<400>  763

Asn Val Ala Ser Tyr Asp Ser Lys Ile Lys
1               5                   10


<210>  764

<211>  10
<212>  PRT
<213>  Homo sapiens

<400>  764

Asp Val Leu Leu Arg Val Phe Val Glu Arg
1               5                   10


<210>  765
<211>  10
<212>  PRT
<213>  Homo sapiens

<400>  765

Ala Ile Ile Val Ile Leu Leu Leu Val Asp
1               5                   10


<210>  766
<211>  10
<212>  PRT
<213>  Homo sapiens

<400>  766

Arg Val Val Arg Ile Met Ile Asp Asp His
1               5                   10


<210>  767
<211>  10
<212>  PRT
<213>  Homo sapiens

<400>  767

Lys Ile Val His Ser Ile Val Ser Ser Phe
1               5                   10


<210>  768
<211>  10
<212>  PRT
<213>  Homo sapiens

<400>  768

Ser Ile Ser Leu Ala Ile Ala Leu Phe Phe
1               5                   10


<210>  769
<211>  10
<212>  PRT
<213>  Homo sapiens

<400>  769

Ile Val Ile Leu Leu Leu Val Asp Val Val
1               5                   10


<210>  770
<211>  10
<212>  PRT

<213>  Homo sapiens

<400>  770

Asp Val Val Tyr Ile Phe Phe Asp Ile Lys
1               5                   10


<210>  771
<211>  10
<212>  PRT
<213>  Homo sapiens

<400>  771

Lys Leu Leu Arg Asn Ile Pro Arg Trp Thr
1               5                   10


<210>  772
<211>  10
<212>  PRT
<213>  Homo sapiens

<400>  772

Asp Leu Ile Phe Thr Asp Ser Lys Leu Tyr
1               5                   10


<210>  773
<211>  10
<212>  PRT
<213>  Homo sapiens

<400>  773

Phe Leu Met Asp Val Leu Leu Arg Val Phe
1               5                   10


<210>  774
<211>  10
<212>  PRT
<213>  Homo sapiens

<400>  774

Val Leu Leu Arg Val Phe Val Glu Arg Arg
1               5                   10


<210>  775
<211>  10
<212>  PRT
<213>  Homo sapiens

<400>  775

Ile Leu Leu Leu Val Asp Val Val Tyr Ile
1               5                   10


<210>  776
<211>  10
<212>  PRT
<213>  Homo sapiens

<400> 776

His Leu Leu Arg Leu Leu Arg Leu Ile Ile
1               5                   10


<210> 777
<211> 10
<212> PRT
<213> Homo sapiens

<400> 777

Arg Ile Ile Ala Met Ser Phe Pro Ser Ser
1               5                   10


<210> 778
<211> 10
<212> PRT
<213> Homo sapiens

<400> 778

Arg Val Tyr Asn Leu Cys Ser Glu Arg Ala
1               5                   10


<210> 779
<211> 10
<212> PRT
<213> Homo sapiens

<400> 779

Ala Ile His Cys Lys Gly Gly Thr Asp Arg
1               5                   10


<210> 780
<211> 10
<212> PRT
<213> Homo sapiens

<400> 780

Tyr Val Ala Tyr Phe Ala Gln Val Lys His
1               5                   10


<210> 781
<211> 10
<212> PRT
<213> Homo sapiens

<400> 781

His Leu Tyr Asn Trp Asn Leu Pro Pro Arg
1               5                   10


<210> 782
<211> 10
<212> PRT
<213> Homo sapiens

<400> 782

Ile Leu Phe Ile Lys His Phe Ile Ile Tyr
1               5                   10

<210>  783
<211>  10
<212>  PRT
<213>  Homo sapiens

<400>  783

Arg Leu Tyr Leu Pro Lys Asn Glu Leu Asp
1               5                   10

<210>  784
<211>  10
<212>  PRT
<213>  Homo sapiens

<400>  784

Lys Gly Ala Thr Glu Glu Ala Pro Ala Lys
1               5                   10

<210>  785
<211>  10
<212>  PRT
<213>  Homo sapiens

<400>  785

Lys Leu Tyr Ile Pro Leu Glu Tyr Arg Ser
1               5                   10

<210>  786
<211>  10
<212>  PRT
<213>  Homo sapiens

<400>  786

Asn Ile Pro Arg Trp Thr His Leu Leu Arg
1               5                   10

<210>  787
<211>  10
<212>  PRT
<213>  Homo sapiens

<400>  787

Arg Arg Thr Asp Lys Thr His Ser Glu Lys
1               5                   10

<210>  788
<211>  9
<212>  PRT
<213>  Homo sapiens

<400>  788

Ala Pro Ala Lys Glu Ser Pro His Thr
1               5

<210> 789
<211> 9
<212> PRT
<213> Homo sapiens

<400> 789

Asn Pro Ile Lys Glu Val Val Arg Phe
1               5


<210> 790
<211> 9
<212> PRT
<213> Homo sapiens

<400> 790

Thr Pro Ser Gln Lys Arg Tyr Val Ala
1               5


<210> 791
<211> 9
<212> PRT
<213> Homo sapiens

<400> 791

Leu Pro Lys Asn Glu Leu Asp Asn Leu
1               5


<210> 792
<211> 9
<212> PRT
<213> Homo sapiens

<400> 792

Tyr Pro Ser Asp Phe Ala Val Glu Ile
1               5


<210> 793
<211> 9
<212> PRT
<213> Homo sapiens

<400> 793

Phe Pro Ser Ser Gly Arg Gln Ser Phe
1               5


<210> 794
<211> 9
<212> PRT
<213> Homo sapiens

<400> 794

Leu Pro Pro Arg Arg Ile Leu Phe Ile
1               5

<210>  795
<211>  9
<212>  PRT
<213>  Homo sapiens

<400>  795

Ser Pro Ile Ser Glu Ser Val Leu Ala
1               5


<210>  796
<211>  9
<212>  PRT
<213>  Homo sapiens

<400>  796

Pro Pro Leu Tyr Asp Asp Val Lys Val
1               5


<210>  797
<211>  9
<212>  PRT
<213>  Homo sapiens

<400>  797

Asp Pro Thr Asp Leu Ala Gly Val Ile
1               5


<210>  798
<211>  9
<212>  PRT
<213>  Homo sapiens

<400>  798

Ala Phe Gly Leu Phe Gly Val Phe Leu
1               5


<210>  799
<211>  9
<212>  PRT
<213>  Homo sapiens

<400>  799

Val Pro Thr Leu His Gln Met Val Val
1               5


<210>  800
<211>  9
<212>  PRT
<213>  Homo sapiens

<400>  800

Gly Leu Phe Gly Val Phe Leu Val Leu
1               5


<210>  801
<211>  9

<212>  PRT
<213>  Homo sapiens

<400>  801

Asp Pro Lys His Phe His Asn Arg Val
1               5


<210>  802
<211>  9
<212>  PRT
<213>  Homo sapiens

<400>  802

Ser Pro Asp Pro Thr Asp Leu Ala Gly
1               5


<210>  803
<211>  9
<212>  PRT
<213>  Homo sapiens

<400>  803

Ile Pro Arg Trp Thr His Leu Leu Arg
1               5


<210>  804
<211>  9
<212>  PRT
<213>  Homo sapiens

<400>  804

Thr Arg Asp Gly Phe Asp Leu Asp Leu
1               5


<210>  805
<211>  9
<212>  PRT
<213>  Homo sapiens

<400>  805

Trp Asn Leu Pro Pro Arg Arg Ile Leu
1               5


<210>  806
<211>  10
<212>  PRT
<213>  Homo sapiens

<400>  806

Ile Pro Arg Trp Thr His Leu Leu Arg Leu
1               5                   10


<210>  807
<211>  10
<212>  PRT
<213>  Homo sapiens

<400>  807

Asn Pro Ile Lys Glu Val Val Arg Phe Leu
1               5                   10


<210>  808
<211>  10
<212>  PRT
<213>  Homo sapiens

<400>  808

Tyr Pro Ser Asp Phe Ala Val Glu Ile Leu
1               5                   10


<210>  809
<211>  10
<212>  PRT
<213>  Homo sapiens

<400>  809

Ile Pro Leu Glu Tyr Arg Ser Ile Ser Leu
1               5                   10


<210>  810
<211>  10
<212>  PRT
<213>  Homo sapiens

<400>  810

Ile Pro Arg Tyr Val Arg Asp Leu Lys Ile
1               5                   10


<210>  811
<211>  10
<212>  PRT
<213>  Homo sapiens

<400>  811

Ser Pro Asp Pro Thr Asp Leu Ala Gly Val
1               5                   10


<210>  812
<211>  10
<212>  PRT
<213>  Homo sapiens

<400>  812

Val Pro Thr Leu His Gln Met Val Val Phe
1               5                   10


<210>  813
<211>  10
<212>  PRT
<213>  Homo sapiens

<400>  813

Asp Pro Thr Asp Leu Ala Gly Val Ile Ile
1               5                   10


<210>  814
<211>  10
<212>  PRT
<213>  Homo sapiens

<400>  814

Gly Pro Pro Leu Tyr Asp Asp Val Lys Val
1               5                   10


<210>  815
<211>  10
<212>  PRT
<213>  Homo sapiens

<400>  815

Ser Pro Gln Thr Ser Glu Phe Lys Gly Ala
1               5                   10


<210>  816
<211>  10
<212>  PRT
<213>  Homo sapiens

<400>  816

Ser Pro His Thr Ser Glu Phe Lys Gly Ala
1               5                   10


<210>  817
<211>  10
<212>  PRT
<213>  Homo sapiens

<400>  817

Asp Pro Lys His Phe His Asn Arg Val Val
1               5                   10


<210>  818
<211>  10
<212>  PRT
<213>  Homo sapiens

<400>  818

Leu Pro Thr Tyr Tyr Asp Asn Cys Ser Phe
1               5                   10


<210>  819
<211>  10
<212>  PRT
<213>  Homo sapiens

<400>  819

Arg Val Ser Pro Ile Ser Glu Ser Val Leu

1                   5                   10

<210>  820
<211>  10
<212>  PRT
<213>  Homo sapiens

<400>  820

Phe Ala Phe Gly Leu Phe Gly Val Phe Leu
1               5                   10

<210>  821
<211>  10
<212>  PRT
<213>  Homo sapiens

<400>  821

Thr Pro Ser Gln Lys Arg Tyr Val Ala Tyr
1               5                   10

<210>  822
<211>  1320
<212>  DNA
<213>  Homo sapiens

<400>  822
accttgcaga ggagggggcg gcggggacag gacccgggtc tgtgcctgcg cacaccctcg      60

cgggccaacc ccctcccccc ccgccccgcc gggtacctgt cacggctact gccgcggcgc     120

gcgcccgcca cgttaagccg gatggcggga ggaggggtgc ggggagggtg gcacggcgcg     180

cgcgtgcgcg ggagtcgccg agcaagaact ggtagtgcgc gcgctcgccg ctcgctcgcg     240

tcccggaggc ggagtctgcg gcggcgggcg gaacgggggc gcgcctatgc tagtcacgtg     300

ggcgctgggg cggggccggg cggccgttca aggcaggggg cggggcgtct ccgagcggcg     360

gggccaaggg agggcacaac agctgctacc tgaacagttt ctgacccaac agttacccag     420

cgccggactc gctgcgcccc ggcggctcta gggacccccg gcgcctacac ttagctccgc     480

gcccgaggtg agcccaggcc ctaagtcctc cgggcggggg taggggtggg ggacgctcct     540

ttttgttggg ggggggtctt ggaggcgcga aggcactagg cgcctcggcg gatggctgaa     600

cccctcgccc gcggctcccc gtgtcttttg gggggccggg tgcgggcgcg gaatccggga     660

ggtgtccgca caaaaggccg agaaaaactc cgcgacgcct ccctccctcc ctccgccctc     720

cccgtcccct cctctccgcg cccgctcctc ctcattcaaa cccggccggc ctgagtggtg     780

ttagctcagt cccggccgcc gccgcgtgag gaaatggcct aggagccgga gccgcaggta     840

aagggggcgc gccccccgcc cgcgccagcc ggggcgcccg cccggtcctg cggaggctcc     900

cgcgccgccc ccgaggcgcc cggctctcgt tgtcttgtcc ccccccagga tgacctgagt     960

tccccgtgtc tgctttctag ccgctcctct agggcgcccc ctccccagct ggctcctgcg    1020

cttccgccca cgggaaggtg cgggcgatcc cgggctgcat ccgctcttgg ccgtcacact    1080

cacgctgcac gatttagaag ggcttggggt gcggggttcc ctgagtctcc gccgggagcg    1140

ggggtccggg ggtctgcggg ggtgggcggt ggggtgctgt tccgaaaagt tgggtcgccc 1200
cgcggggcgg gtctcccaat ttgcccagtc ggcccttggg gcgcgggcgg aggggggacg 1260
tcggcgtagg gtccccagcg atgccccggg aagggctggg acaagttgga aacggtcaaa 1320

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von tumor-assoziierten Antigenen. An der spezifischen Erkennung von tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC-(Major Histocompatibility complex = HistokompatibilitätsAntigene) Klassen II bzw. I präsentiert werden, während B-Lymphozyten zirkulierende Antikörpermoleküle produzieren, die direkt an unprozessierte Antigene binden. Die potentielle klinisch-therapeutische Bedeutung von tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionbanken von Tumoren systematisch auf tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumorspezifischem Lysemuster, oder zirkulierende Autoantikörper wurden genutzt, um die respektiven Antigene zu klonieren.

Durch diese Ansätze sind in den letzten Jahren eine Vielzahl von Antigenen in verschiedenen Neoplasien definiert worden. Von großem Interesse ist dabei die Klasse der Cancer/Testis-Antigene (CTA). CTA und sie kodierende Gene (Cancer/Testis-Gene oder CTG) sind durch ihr charakteristisches Expressionsmuster definiert [Tureci et al, Mol Med Today. 3:342-9, 1997]. Sie finden sich nicht in Normalgeweben bis auf Testis bzw. Keimzellen, werden jedoch in einer Reihe von humanen Malignomen exprimiert und zwar nicht tumortypspezifisch, sondern mit unterschiedlicher Häufigkeit in Tumorentitäten ganz unterschiedlicher Herkunft (Chen & Old, Cancer J. Sci. Am. 5:16-7, 1999). Auch Serumreaktivitäten gegen CTA finden sich nicht in gesunden Kontrollen, sondern lediglich in Tumorpatienten. Insbesondere aufgrund ihrer Gewebeverteilung ist diese Antigenklasse von besonderem Wert für immuntherapeutische Vorhaben und wird in derzeit laufenden klinischen Patientenstudien getestet (Marchand et al., Int. J. Cancer 80:219-30, 1999; Knuth et al., Cancer Chemother. Pharmacol. 46: S46-51, 2000).

Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumoren z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Erfindungsgemäß wurde eine Strategie für eine Identifizierung und Bereitstellung Tumor-assoziiert exprimierter Antigene und der dafür kodierenden Nukleinsäuren verfolgt. Diese Strategie beruht auf der Tatsache, dass eigentlich Testis- und damit Keimzell-spezifische Gene, die normalerweise in adulten Geweben silent sind, in Tumorzellen ektop und unerlaubt reaktiviert werden. Durch Datamining wird zunächst eine möglichst komplette Liste aller bekannten Testis-spezifischen Gene aufgestellt und diese sodann durch Expressionsanalysen mittels spezifischer RT-PCR auf ihre aberrante Aktivierung in Tumoren evaluiert. Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumor-spezifisch sind (Schmitt et al., Nucleic Acids Res. 27:4251-60, 1999; Vasmatzis et al., Proc. Natl. Acad. Sci. U S A. 95:300-4, 1998. Scheurle et al., Cancer Res. 60:4037-43, 2000).

Das erfindungsgemäße Konzept, das sich als viel erfolgreicher erwiesen hat, beruht jedoch darauf, Datamining zur elektronischen Extraktion aller Testis-spezifischer Gene zu nutzen und diese sodann auf ektope Expression in Tumoren zu evaluieren.

Somit betrifft die Erfindung in einem Aspekt eine Strategie zur Identifizierung von differentiell in Tumoren exprimierten Genen. Diese kombiniert Datamining von öffentlichen Sequenzbanken (*"in silico"*) mit darauffolgenden evaluierenden labor-experimentellen ("wet bench") Untersuchungen.

Eine kombinierte Strategie basierend auf zwei unterschiedlichen bioinformatischen Skripten ermöglichte erfindungsgemäß die Identifizierung neuer Mitglieder der Cancer/Testis (CT) Genklasse. Diese sind bisher als rein Testis-, Keimzell-; oder Spermien-spezifisch eingestuft worden. Die Erkenntnis, dass diese Gene aberrant in Tumorzellen aktiviert werden, erlaubt, ihnen eine substantiell neue Qualität mit funktionellen Implikationen zuzuordnen. Die Identifizierung und Bereitstellung dieser tumor-assoziierten Gene und der dadurch kodierten Genprodukte erfolgte erfindungsgemäß unabhängig von einer immunogenen Wirkung.

Die erfindungsgemäß identifizierten Tumor-assoziierten Antigene weisen eine Aminosäuresequenz auf, die von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NOs: 1-5, 19-21, 29, 31-33, 37, 39, 40, 54-57, 62, 63, 70, 74, 85-88 einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. In einer bevorzugten Ausführungsform weist ein erfindungsgemäß identifiziertes Tumor-assoziiertes Antigen eine Aminosäuresequenz auf, die von einer Nukleinsäure kodiert wird, die aus der Gruppe bestehend aus SEQ ID NOs: 1-5, 19-21, 29, 31-33, 37, 39, 40, 54-57, 62, 63, 70, 74, 85-88 ausgewählt ist. In einer weiteren bevorzugten Ausführungsform umfasst ein erfindungsgemäß identifiziertes tumor-assoziiertes Antigen eine Aminosäuresequenz, die aus der Gruppe bestehend aus SEQ ID NOs: 6-13, 14-18, 22-24, 30; 34-36, 38, 41, 58-61, 64, 65, 71, 75, 80-84, 89-100, 101-117 einem Teil oder Derivat davon ausgewählt ist.

Die vorliegende Erfindung betrifft allgemein die Verwendung von erfindungsgemäß identifizierten Tumor-assoziierten Antigenen oder von Teilen davon, von dafür kodierenden Nukleinsäuren oder von Nukleinsäuren, die gegen die kodierenden Nukleinsäuren gerichtet sind oder von Antikörpern, die gegen die erfindungsgemäß identifizierten Tumor-assoziierten Antigene oder Teile davon gerichtet sind, für die Therapie und Diagnose. Diese Nutzung kann einzelne, aber auch Kombinationen von mehreren dieser Antigene, funktionalen Fragmente, Nukleinsäuren, Antikörper etc betreffen, in einer Ausführungsform auch in Kombination mit anderen tumor-assoziierten Genen und Antigenen für eine Diagnose, Therapie und Verlaufskontrolle.

Bevorzugte Erkrankungen für eine Therapie und/oder Diagnose sind solche, bei denen eine selektive Expression oder abnormale Expression von einem oder mehreren der erfindungsgemäß identifizierten Tumor-assoziierten Antigenen vorliegt.

Die ektope Aktivierung von Genen in Tumoren kann auch zurückführbar sein auf ein verändertes Genmethylierungsmuster ihrer Nukleotidsequenz. So ist z.B. beschrieben worden, dass Alterationen der Methylierung am Cytosin hierzu beitragen (De Smet et al, 1996 und 1999).

Die Erfindung betrifft auch Nukleinsäuren und Genprodukte, die tumorzellassoziiert exprimiert werden und die durch verändertes Spleißen (Spleißvarianten) bekannter Gene bzw. durch veränderte Translation unter Nutzung alternativer offener Leserahmen entstehen. Diese Nukleinsäuren umfassen die Sequenzen gemäß (SEQ ID NO:2-5, 20, 21, 31-33, 54-57, 85-88) des Sequenzprotokolls. Ferner umfassen die Genprodukte Sequenzen gemäß (SEQ ID NO:7-13, 23, 24, 34-36, 58-61, 89-100) des Sequenzprotokolls. Die erfindungsgemäßen Spleißvarianten sind erfindungsgemäß als Targets für die Diagnostik und Therapie von Tumorerkrankungen verwendbar.

Für die Entstehung von Spleißvarianten können verschiedenste Mechanismen ursächlich sein, beispielsweise
- die Nutzung variabler Transkriptionsinitiationsstellen
- die Nutzung zusätzlicher Exons
- vollständiges oder unvollständiges Aus Spleißen von einzelnen oder mehreren Exons,
- über Mutation veränderte Spleißregulatorsequenzen (Deletion bzw. Schaffung neuer Donor/Acceptorsequenzen),
- die unvollständige Elimination von Intronsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei tumorassoziierten Spleißvarianten können tumorassoziierte Transkripte und tumorassoziierte Proteine/Antigene entstehen. Diese können als molekulare Marker sowohl zum Nachweis von Tumorzellen als auch zum therapeutischen Targeting von Tumoren genutzt werden. Die Detektion von Tumorzellen z.B. im Blut, Serum, Knochenmark, Sputum, Bronchial-Lavage, Körpersekreten und Gewebsbiopsien kann erfindungsgemäß z.B. nach Extraktion von Nukleinsäuren durch PCR-Amplifikation mit Spleißvariantenspezifischen Oligonukleotiden erfolgen. Zum Nachweis eignen sich erfindungsgemäß alle Sequenz-abhängigen Detektionssysteme. Neben der PCR sind diese z.B. Genchip-/Microarraysysteme, Northern-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch erfindungsgemäß durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. Für die Immunisierung eignen sich besonders die Aminosäuren, die deutliche Epitopunterschiede zu der Variante(n) des Genprodukts aufweisen, welche(s) bevorzugt in gesunden Zellen gebildet wird. Der Nachweis der Tumorzellen mit Antikörper kann dabei an einer vom Patienten isolierten Probe oder als Imaging mit intravenös applizierten Antikörpern erfolgen. Neben der diagnostischen Nutzbarkeit stellen Spleißvarianten, die neue oder veränderte Epitope aufweisen, attraktive Targets für die Immuntherapie dar. Die erfindungsgemäßen Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferriert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo- oder Polypeptide kodieren, die diese Epitope beinhalten. Verschiedene Techniken zur in vitro oder in vivo Generierung von epitopspezifischen T-Lymphozyten sind bekannt und ausführlich beschrieben z.B. (Kessler JH, et al. 2001, Sahin et al., 1997) und basieren ebenfalls auf der Nutzung von Oligo-oder Polypeptide, die die Spleißvarianten-spezifischen Epitope beinhalten oder Nukleinsäuren, die für diese kodieren. Oligo-oder Polypeptiden, die die Spleißvariantenspezifischen Epitope beinhalten oder Nukleinsäuren, die für diese Polypeptide kodieren sind auch für die Nutzung als pharmazeutisch wirksame Substanzen bei der aktiven Immuntherapie (Vakzinierung, Vakzintherapie) verwendbar.

In einem Aspekt betrifft die Erfindung eine pharmazeutische Zusammensetzung umfassend ein Mittel, das das erfindungsgemäß identifizierte Tumor-assoziierte Antigen erkennt und vorzugsweise selektiv für Zellen ist, die eine Expression oder abnormale Expression eines erfindungsgemäß identifizierten Tumor-assoziierten Antigens aufweisen. Das Mittel kann in bestimmten Ausführungsformen die Induktion des Zelltods, die Reduktion des Zellwachstums, die Schädigung der Zellmembran oder die Sekretion von Zytokinen bewirken und weist vorzugsweise eine tumorhemmende Aktivität auf. In einer Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet, insbesondere ein komplementaktivierter Antikörper, der selektiv an das Tumor-assoziierte Antigen bindet. In einer weiteren Ausführungsform umfasst das Mittel mehrere Mittel, die jeweils selektiv verschiedene Tumor-assoziierte Antigene erkennen, wobei mindestens eines der Tumorassoziierten Antigene ein erfindungsgemäß identifiziertes Tumor-assoziiertes Antigen ist. Die Erkennung muss nicht direkt mit einer Hemmung von Aktivität oder Expression des Antigens einhergehen. In diesem Aspekt der Erfindung dient das selektiv auf Tumoren beschränkte Antigen vorzugsweise als Markierung zur Rekrutierung von Effektormechanismen an diesen spezifischen Ort. In einer bevorzugten Ausführungsform ist das Mittel ein cytotoxischer T-Lymphozyt, der das Antigen auf einem HLA-Molekül erkennt und die derartig markierte Zelle lysiert. In einer weiteren Ausführungsform ist das Mittel ein Antikörper, der selektiv an das Tumor-assoziierte Antigen bindet und somit natürliche oder artifizielle Effektormechanismen zu dieser Zelle rekrutiert. In einer weiteren Ausführungsform ist das Mittel ein T-Helfer-Lymphozyt, der Effektorfunktionen von anderen Zellen, die spezifisch dieses Antigen erkennen, stärkt.

In einem Aspekt betrifft die Erfindung eine pharmazeutische Zusammensetzung umfassend ein Mittel, das die Expression oder Aktivität eines erfindungsgemäß identifizierten Tumorassoziierten Antigens hemmt. In einer bevorzugten Ausführungsform ist das Mittel eine Antisense-Nukleinsäure, die selektiv mit der Nukleinsäure hybridisiert, die für das Tumorassoziierte Antigen kodiert. In einer weiteren Ausführungsform ist das Mittel ein Antikörper, der selektiv an das Tumor-assoziierte Antigen bindet. In einer weiteren Ausführungsform umfasst das Mittel mehrere Mittel, die jeweils selektiv die Expression oder Aktivität verschiedener Tumor-assoziierter Antigene hemmen, wobei mindestens eines der Tumorassoziierten Antigene ein erfindungsgemäß identifiziertes Tumor-assoziiertes Antigen ist.

Die Aktivität eines erfindungsgemäß identifizierten Tumor-assoziierten Antigens kann eine jegliche Aktivität eines Proteins oder Peptids sein. In einer Ausführungsform ist die Aktivität eines enzymatische Aktivität wie die Laktatdehydrogenaseaktivität im Fall von Sequenzen, die LDHC betreffen (vgl. Beispiel 1). In einer weiteren Ausführungsform betrifft diese Aktivität eine Beteiligung bei einer zellulären Migration und/oder Metastatisierung wie im Fall der TPTE betreffenden Sequenzen (vgl. Beispiel 2). Somit können die erfindungsgemäßen Therapie- und Diagnosverfahren auch auf Hemmung oder Reduktion dieser Aktivität oder auf ein Testen dieser Aktivität abzielen.

Des weiteren betrifft die Erfindung eine pharmazeutische Zusammensetzung, die ein Mittel umfasst, das bei einer Verabreichung selektiv die Menge an Komplexen zwischen einem HLA-Molekül und einem Peptidepitop aus dem erfindungsgemäß identifizierten Tumorassoziierten Antigen erhöht. Das Mittel umfasst in einer Ausführungsform einen oder mehrere Bestandteile, die aus der Gruppe ausgewählt sind, bestehend aus (i) dem Tumor-assoziierten Antigen oder einem Teil davon, (ii) einer Nukleinsäure, die für das Tumor-assoziierte Antigen oder einen Teil davon kodiert, (iii) einer Wirtszelle, die das Tumor-assoziierte Antigen oder einen Teil davon exprimiert, und (iv) isolierten Komplexen zwischen Peptidepitopen aus dem Tumor-assoziierten Antigen und einem MHC-Molekül. In einer Ausführungsform umfasst das Mittel mehrere Mittel, die jeweils selektiv die Menge an Komplexen zwischen MHC-Molekülen und Peptidepitopen verschiedener Tumor-assoziierter Antigene erhöhen, wobei mindestens eines der Tumor-assoziierten Antigene ein erfindungsgemäß identifiziertes Tumor-assoziiertes Antigen ist.

Des weiteren betrifft die Erfindung eine pharmazeutische Zusammensetzung, die einen oder mehrer Bestandteile umfasst, die aus der Gruppe ausgewählt sind, bestehend aus (i) einem erfindungsgemäß identifizierten Tumor-assoziierten Antigen oder einem Teil davon, (ii) einer Nukleinsäure, die für ein erfindungsgemäß identifiziertes Tumor-assoziiertes Antigen oder einen Teil davon kodiert, (iii) einem Antikörper, der an ein erfindungsgemäß identifiziertes Tumor-assoziiertes Antigen oder einen Teil davon bindet, (iv) einer Antisense-Nukleinsäure, die spezifisch mit einer Nukleinsäure, die für ein erfindungsgemäß identifiziertes Tumor-assoziiertes Antigen kodiert, hybridisiert, (v) einer Wirtszelle, die ein erfindungsgemäß identifiziertes Tumor-assoziiertes Antigen oder einen Teil davon exprimiert, und (vi) isolierten Komplexen zwischen einem erfindungsgemäß identifizierten Tumor-assoziierten Antigen oder einem Teil davon und einem HLA-Molekül.

Eine Nukleinsäure, die für ein erfindungsgemäß identifiziertes Tumor-assoziiertes Antigen oder einen Teil davon kodiert, kann in der pharmazeutische Zusammensetzung in einem Expressionsvektor vorliegen und funktionell mit einem Promotor verbunden sein.

Eine in einer erfindungsgemäßen pharmazeutischen Zusammensetzung enthaltene Wirtszelle kann das Tumor-assoziierte Antigen oder den Teil davon sekretieren, auf der Oberfläche exprimieren oder kann zusätzlich ein HLA-Molekül exprimieren, das an das Tumorassoziierte Antigen oder den Teil davon bindet. In einer Ausführungsform exprimiert die Wirtszelle das HLA-Molekül endogen. In einer weiteren Ausführungsform exprimiert die Wirtszelle das HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

Ein in einer erfindungsgemäßen pharmazeutischen Zusammensetzung enthaltener Antikörper kann ein monoklonaler Antikörper sein. In weiteren Ausführungsformen ist der Antikörper ein chimärer oder humanisierter Antikörper, ein Fragment eines natürlichen Antikörpers, oder ein synthetischer Antikörper, die durch kombinatorische Techniken hergestellt werden können. Der Antikörper kann mif einem therapeutisch oder diagnostisch nützlichen Mittel gekoppelt sein.

Eine in einer erfindungsgemäßen pharmazeutischen Zusammensetzung enthaltene Antisense-Nukleinsäure kann eine Sequenz von 6-50, insbesondere 10-30, 15-30 und 20-30 zusammenhängenden Nukleotiden aus der Nukleinsäure, die für das erfindungsgemäß identifizierte Tumor-assoziierte Antigen kodiert, umfassen.

In weiteren Ausführungsformen bindet ein durch eine erfindungsgemäße pharmazeutische Zusammensetzung entweder direkt oder durch die Expression einer Nukleinsäure bereitgestelltes Tumor-assoziiertes Antigen oder ein Teil davon an MHC-Moleküle auf der Oberfläche von Zellen, wobei die Bindung vorzugsweise eine cytolytische Reaktion hervorruft und/oder eine Cytokinausschüttung induziert.

Eine erfindungsgemäße pharmazeutische Zusammensetzung kann einen pharmazeutisch verträglichen Träger und/oder ein Adjuvans umfassen. Das Adjuvans kann aus Saponin, GM-CSF, CpG-Nukleotiden, RNA, einem Zytokin oder einem Chemokin ausgewählt sein. Eine erfindungsgemäße pharmazeutische Zusammensetzung wird vorzugsweise zur Behandlung einer Erkrankung eingesetzt, die sich durch die selektive Expression oder abnormale Expression eines Tumor-assoziierten Antigens auszeichnet. In einer bevorzugten Ausführungsform ist die Erkrankung Krebs.

Des weiteren betrifft die Erfindung Verfahren zur Behandlung oder Diagnose einer Erkrankung, die sich durch die Expression oder abnormale Expression eines oder mehrerer Tumor-assoziierter Antigene auszeichnet. In einer Ausführungsform umfasst die Behandlung die Verabreichung einer erfindungsgemäßen pharmazeutischen Zusammensetzung.

In einem Aspekt betrifft die Erfindung ein Verfahren zur Diagnose einer Erkrankung, die sich durch die Expression oder abnormale Expression eines erfindungsgemäß identifizierten Tumor-assoziierten Antigens auszeichnet. Das Verfahren umfasst den Nachweis (i) einer Nukleinsäure, die für das Tumor-assoziierte Antigen kodiert, oder eines Teils davon und/oder (ii) den Nachweis des Tumor-assoziierten Antigens oder eines Teils davon, und/oder (iii) den Nachweis eines Antikörpers gegen das Tumor-assoziierte Antigen oder einen Teil davon und/oder (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe. In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an cytotoxische oder Helfer-T-Lymphozyten, die für das Tumor-assoziierte Antigen oder Teile davon spezifisch sind, bindet und (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten. In einer Ausführungsform zeichnet sich die Erkrankung durch die Expression oder abnormale Expression mehrerer verschiedener Tumor-assoziierter Antigene aus und der Nachweis umfasst einen Nachweis mehrerer Nukleinsäuren, die für die mehreren verschiedenen Tumorassoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden oder den Nachweis mehrerer cytotoxischer oder Helfer-T-Lymphozyten, die für die mehreren verschiedenen Tumor-assoziierten Antigene spezifisch sind. In einer weiteren Ausführungsform wird die isolierte biologische Probe aus dem Patienten mit einer vergleichbaren normalen biologischen Probe verglichen.

Die erfindungsgemäßen Diagnoseverfahren können auch eine Nutzung der erfindungsgemäß identifizierten Tumor-assoziierten Antigene als prognostische Marker betreffen, um eine Metastatisierung z.B. durch Testen des Migrationsverhalten von Zellen und daher einen verschlechterten Krankheitsverlauf zu prädizieren, wodurch unter anderem die Planung einer aggressiveren Therapie ermöglicht wird. Insebsondere sind die TPTE betreffenden Sequenzen (vgl. Beispiel 2) für diesen Zweck geeignet. Sie sind auch dafür geeignet, noch gutartige Veränderungen z.B. Hyperplasien von bereits ungünstig zu wertenden Tumor-Vorstufen abzugrenzen und somit eine Krebsneigung vorherzusehen noch bevor ein invasiver Tumor sich gebildet hat.

In dieser Ausführungsform betrifft die Erfindung ein Verfahren zur Diagnose einer Erkrankung, die sich durch die Expression oder abnormale Expression eines erfindungsgemäß identifizierten Tumor-assoziierten Antigens auszeichnet, wobei das Verfahren eine Bewertung des Migrationsverhaltens von Zellen aus einem Patienten umfasst. In besonders bevorzugten Ausführungsformen dient das Verfahren einer Bestimmung des Ausmaßes oder einer Prädiktion einer Metastatisierung und/oder von Lymphknoten- und/oder Fernmetastasen, wobei ein erhöhtes Migrationsverhalten von Zellen in bestimmten Ausführungsformen darauf hinweist, dass der getestete Patient an der Erkrankung, die sich durch die Expression oder abnormale Expression eines erfindungsgemäß identifizierten Tumor-assoziierten Antigens auszeichnet, erkrankt ist, daran erkranken wird und/oder ein Potential für eine derartige Erkrankung aufweist. In besonders bevorzugten Ausführungsformen weist ein erhöhtes Migrationsverhalten auf eine Metastatisierung und/oder Bildung von Lymphknoten- und/oder Fernmetastasen oder ein Potential dafür hin. In dieser Ausführungsform weist das erfindungsgemäß identifizierte Tumor-assoziierte Antigen vorzugsweise eine Sequenz auf, die von einer Nukleinsäure kodiert wird, die aus der Gruppe ausgewählt ist, bestehend aus: (a) einer Nukleinsäure, die eine Nukleinsäuresequenz umfasst, die aus der Gruppe bestehend aus SEQ ID NOs: 19-21, und 54-57, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. Das Tumor-assoziierte Antigen umfaßt vorzugsweise eine Aminosäuresequenz, die aus der Gruppe bestehend aus SEQ ID NOs: 22-24, 58-61, 81, 82, und 103-105, einem Teil oder Derivat davon ausgewählt ist.

Erfindungsgemäße Verfahren zur Behandlung zielen in diesem Aspekt vorzugsweise auf die Verabreichung von pharmazeutischen Zusammensetzungen ab, die das Migrationsverhalten normalisieren, vorzugsweise hemmen.

In einer weiteren Ausführungsform betrifft die Erfindung ein Verfahren zur Diagnose einer Erkrankung, die sich durch die Expression oder abnormale Expression eines erfindungsgemäß identifizierten Tumor-assoziierten Antigens auszeichnet, wobei das Verfahren eine Bestimmung des Methylierungsmusters und/oder des Grades an Methylierung innerhalb einer Nukleinsäure, die eine Nukleinsäuresequenz umfasst, die für das Tumor-assoziierte Antigen kodiert, insbesondere innerhalb der nicht-kodierenden Bereiche davon, bevorzugt innerhalb des Promotorbereiches davon umfaßt. Vorzugsweise weist das erfindungsgemäß identifizierte Tumor-assoziierte Antigen eine Sequenz auf, die von einer Nukleinsäure kodiert wird, die aus der Gruppe ausgewählt ist, bestehend aus: (a) einer Nukleinsäure, die eine Nukleinsäuresequenz umfasst, die aus der Gruppe bestehend aus SEQ ID NOs: 19-21, und 54-57, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist, und die Bestimmung erfolgt bezüglich dieser Nukleinsäure, vorzugsweise innerhalb des Promotorbereiches davon, und insbesondere innerhalb der in SEQ ID NO:822 gezeigten Sequenz, insbesondere innerhalb der Nukleotide von Position 121 bis 540 der in SEQ ID NO:822 gezeigten Sequenz (TPTE Promotorsequenz auf Chromosom 21; Position -368/+952 bezogen auf den Transkriptionsstart). Das Tumor-assoziierte Antigen umfaßt vorzugsweise eine Aminosäuresequenz, die aus der Gruppe bestehend aus SEQ ID NOs: 22-24, 58-61, 81, 82, und 103-105, einem Teil oder Derivat davon ausgewählt ist. Eine im Vergleich zu einer Kontrolle geringere Methylierung oder eine fehlende Methylierung weist vorzugsweise darauf hin, dass der getestete Patient an der Erkrankung, die sich durch die Expression oder abnormale Expression eines erfindungsgemäß identifizierten Tumor-assoziierten Antigens auszeichnet, erkrankt ist, daran erkranken wird und/oder ein erhöhtes Potential für eine derartige Erkrankung aufweist.

Erfindungsgemäße Verfahren zur Behandlung zielen in diesem Aspekt vorzugsweise auf die Verabreichung von pharmazeutischen Zusammensetzungen ab, die das Methylierungsmuster und/oder den Grad der Methylierung normalisieren, vorzugsweise erhöhen.

In einem weiteren Aspekt betrifft die Erfindung ein Verfahren zur Bestimmung der Regression, des Verlaufs oder des Ausbruchs einer Erkrankung, die sich durch die Expression oder abnormale Expression eines erfindungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, die aus der Gruppe ausgewählt sind, bestehend aus (i) der Menge der Nukleinsäure, die für das Tumor-assoziierte Antigen kodiert, oder eines Teil davon, (ii) der Menge des Tumor-assoziierten Antigens oder eines Teils davon, (iii) der Menge an Antikörpern, die an das Tumor-assoziierte Antigen oder einen Teil davon binden, und (iv) der Menge an cytolytischen T-Zellen oder Helfer-T-Zellen, die für einen Komplex zwischen dem Tumor-assoziierten Antigen oder einem Teil davon und einem MHC-Molekül spezifisch sind. Vorzugsweise umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben der Verlauf der Erkrankung ermittelt wird. In bestimmten Ausführungsformen zeichnet sich die Erkrankung durch die Expression oder abnormale Expression mehrerer verschiedener Tumor-assoziierter Antigene aus und die Überwachung umfasst eine Überwachung (i) der Menge mehrerer Nukleinsäuren, die für die mehreren verschiedenen Tumor-assoziierten Antigene kodieren, oder von Teilen davon und/oder (ii) der Menge der mehreren verschiedenen Tumor-assoziierten Antigene oder von Teilen davon und/oder (iii) der Menge mehrerer Antikörper, die an die mehreren verschiedenen Tumor-assoziierten Antigene oder an Teile davon binden, und/oder (iv) der Menge mehrerer cytolytischer T-Zellen oder Helfer-T-Zellen, die für Komplexe zwischen den mehreren verschiedenen Tumor-assoziierten Antigenen oder von Teilen davon und MHC-Molekülen spezifisch sind.

Ein Nachweis einer Nukleinsäure oder eines Teils davon oder eine Überwachung der Menge einer Nukleinsäure oder eines Teils davon kann erfindungsgemäß mit einer Polynukleotid-Sonde erfolgen, die spezifisch mit der Nukleinsäure oder dem Teil davon hybridisiert oder kann durch selektive Amplifikation der Nukleinsäure oder des Teils davon erfolgen. In einer Ausführungsform umfasst die Polynukleotid-Sonde eine Sequenz von 6-50, insbesondere 10-30, 15-30 und 20-30 zusammenhängenden Nukleotiden aus der Nukleinsäure.

In bestimmten Ausführungsformen liegt das nachzuweisende Tumor-assoziierte Antigen oder der Teil davon intrazellulär oder auf der Zelloberfläche vor. Ein Nachweis eines Tumorassoziierten Antigens oder eines Teils davon oder eine Überwachung der Menge eines Tumor-assoziierten Antigens oder eines Teils davon kann erfindungsgemäß mit einem Antikörper erfolgen, der spezifisch an das Tumor-assoziierte Antigen oder den Teil davon bindet.

In weiteren Ausführungsformen liegt das nachzuweisende Tumor-assoziierte Antigen oder der Teil davon in einem Komplex mit einem MHC-Molekül, insbesondere einem HLA-Molekül vor.

Ein Nachweis eines Antikörpers oder die Überwachung der Menge an Antikörpern kann erfindungsgemäß mit einem Protein oder Peptid erfolgen, das spezifisch an den Antikörper bindet.

Ein Nachweis von cytolytischen T-Zellen oder Helfer-T-Zellen oder die Überwachung der Menge an cytolytischen T-Zellen oder Helfer-T-Zellen, die für Komplexe zwischen einem Antigen oder einem Teil davon und MHC-Molekülen spezifisch sind, kann erfindungsgemäß mit einer Zelle erfolgen, die den Komplex zwischen dem Antigen oder dem Teil davon und einem MHC-Molekül präsentiert.

Die für einen Nachweis oder für eine Überwachung verwendete Polynukleotid-Sonde, der Antikörper, das Protein oder Peptid oder die Zelle sind vorzugsweise nachweisbar markiert. In bestimmten Ausführungsformen ist der nachweisbare Marker ein radioaktiver Marker oder ein Enzymmarker. Der Nachweis von T-Lymphozyten kann zusätzlich erfolgen durch Nachweis ihrer Proliferation, ihrer Zytokinproduktion, sowie ihret cytotoxischen Aktivität, die ausgelöst wird durch die spezifische Stimulation mit dem Komplex aus MHC und tumorassoziiertem Antigen oder Teilen davon. Der Nachweis von T-Lymphozyten kann ferner erfolgen durch ein rekombinantes MHC-Molekül oder auch ein Komplex aus mehreren MHC-Molekülen, die beladen sind mit dem jeweiligen immunogenen Fragment aus einem oder mehreren der tumorassoziierten Antigene und durch Kontaktierung des spezifischen T-Zell-Rezeptors die spezifischen T-Lymphozyten identifizieren können.

In einem weiteren Aspekt betrifft die Erfindung ein Verfahren zur Behandlung, Diagnose oder Überwachung einer Erkrankung, die sich durch die Expression oder abnormale Expression eines erfindungsgemäß identifizierten Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel gekoppelt ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

Die Erfindung betrifft auch ein Verfahren zur Behandlung eines Patienten mit einer Erkrankung, die sich durch die Expression oder abnormale Expression eines erfindungsgemäß identifizierten Tumor-assoziierten Antigens auszeichnet, umfassend (i) die Entfernung einer Probe mit immunreaktiver Zellen aus dem Patienten, (ii) die Kontaktierung der Probe mit einer Wirtszelle, die das Tumor-assoziierte Antigen oder einen Teil davon exprimiert, unter Bedingungen, die eine Produktion cytolytischer T-Zellen gegen das Tumor-assoziierte Antigen oder einen Teil davon begünstigen, und (iii) das Einbringen der cytolytischen T-Zellen in den Patienten in einer Menge, die geeignet ist, Zellen zu lysieren, die das Tumorassoziierte Antigen oder einen Teil davon exprimieren. Die Erfindung betrifft ebenfalls die Klonierung des T-Zell-Rezeptors von cytolytischen T-Zellen gegen das Tumor-assoziierte Antigen. Dieser kann in andere T-Zellen transferiert werden, die damit die erwünschte Spezifität erhalten und wie unter (iii) in den Patienten eingebracht werden können.

In einer Ausführungsform exprimiert die Wirtszelle ein HLA-Molekül endogen. In einer weiteren Ausführungsform exprimiert die Wirtszelle ein HLA-Molekül und/oder das Tumorassoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einem weiteren Aspekt betrifft die Erfindung ein Verfahren zur Behandlung eines Patienten mit einer Erkrankung, die sich durch die Expression oder abnormale Expression eines Tumorassoziierten Antigens auszeichnet, umfassend (i) die Identifizierung einer für ein erfindungsgemäß identifiziertes Tumor-assoziiertes Antigen kodierenden Nukleinsäure, die von Zellen exprimiert wird, die mit der Erkrankung assoziiert sind, (ii) die Transfektion einer Wirtszelle mit der Nukleinsäure oder einem Teil davon, (iii) die Kultivierung der transfizierten Wirtszelle für eine Expression der Nukleinsäure (dies ist bei Erreichen einer hohen Transfektionsrate nicht obligat), und (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen. Das Verfahren kann ferner die Identifizierung eines MHC-Moleküls, das das Tumor-assoziierte Antigen oder einen Teil davon präsentiert, umfassen, wobei die Wirtszelle das identifizierte MHC-Molekül exprimiert und das Tumor-assoziierte Antigen oder einen Teil davon präsentiert. Die Immunreaktion kann eine B-Zellen-Reaktion oder eine T-Zellen-Reaktion umfassen. Des weiteren kann eine T-Zellen-Reaktion die Produktion von cytolytischen T-Zellen und/oder Helfer-T-Zellen umfassen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.

Die Erfindung betrifft auch ein Verfahren zur Behandlung einer Erkrankung, die sich durch die Expression oder abnormale Expression eines erfindungsgemäß identifizierten Tumorassoziierten Antigens auszeichnet, umfassend (i) die Identifikation von Zellen aus dem Patienten, die abnormale Mengen des Tumor-assoziierten Antigens exprimieren, (ii) die Isolierung einer Probe der Zellen, (iii) die Kultivierung der Zellen und (iv) das Einbringen der Zellen in den Patienten in einer Menge, die geeignet ist, eine Immunreaktion gegen die Zellen auszulösen.

Vorzugsweise sind die erfindungsgemäß verwendeten Wirtszellen nicht-proliferativ oder werden nicht-proliferativ gemacht Eine Erkrankung, die sich durch die Expression oder abnormale Expression eines Tumor-assoziierten Antigens auszeichnet, ist insbesondere Krebs.

Des weiteren betrifft die vorliegende Erfindung eine Nukleinsäure, die aus der Gruppe ausgewählt ist, bestehend aus (a) einer Nukleinsäure, die eine Nukleinsäuresequenz umfasst, die aus der Gruppe bestehend aus SEQ ID NOs: 2-5, 20-21, 31-33, 39, 54-57, 62, 63, 85-88 einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. Des weiteren betrifft die Erfindung eine Nukleinsäure die für ein Protein oder Polypeptid kodiert, das eine Aminosäuresequenz umfasst, ausgewählt aus der Gruppe bestehend aus SEQ ID NOs: 7-13, 14-18, 23-24, 34-36, 58-61, 64, 65, 89-100, 101-107 einem Teil oder Derivat davon.

In einem weiteren Aspekt betrifft die Erfindung Promotorsequenzen von erfindungsgemäßen Nukleinsäuren. Diese können funktionell mit einem anderen Gen vorzugsweise in einem Expressionsvektor verbunden werden, und somit die selektive Expression dieses Gens in entsprechenden Zellen gewährleisten.

In einem weiteren Aspekt betrifft die Erfindung ein rekombinantes Nukleinsäuremolekül, insbesondere DNA- oder RNA-Molekül, das eine erfindungsgemäße Nukleinsäure umfasst.

Die Erfindung betrifft auch Wirtszellen, die eine erfindungsgemäße Nukleinsäure oder ein rekombinantes Nukleinsäuremolekül, das eine erfindungsgemäße Nukleinsäure umfasst, enthalten.

Die Wirtszelle kann ferner eine Nukleinsäure umfassen, die für ein HLA-Molekül kodiert. In einer Ausführungsform exprimiert die Wirtszelle das HLA-Molekül endogen. In einer weiteren Ausführungsform exprimiert die Wirtszelle das HLA-Molekül und/oder die erfindungsgemäße Nukleinsäure oder einen Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einer weiteren Ausführungsform betrifft die Erfindung Oligonukleotide, die mit einer erfindungsgemäß identifizierten Nukleinsäure hybridisieren und als genetische Sonden oder als "Antisense"-Moleküle verwendet werden können. Nukleinsäuremoleküle in der Form von Oligonukleotid-Primern oder kompetenten Proben, die mit einer erfindungsgemäß identifizierten Nukleinsäure oder Teilen davon hybridisieren, können zum Auffinden von Nukleinsäuren verwendet werden, die zu der erfindungsgemäß identifizierten Nukleinsäure homolog sind. PCR-Amplifikation, Southem- und Northern-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

In einem weiteren Aspekt betrifft die Erfindung ein Protein oder Polypeptid, das von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NOs: 2-5, 20-21, 31-33, 39, 54-57, 62, 63, 85-88 einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. In einer bevorzugten Ausführungsform betrifft die Erfindung ein Protein oder Polypeptid, das eine Aminosäuresequenz umfasst, ausgewählt aus der Gruppe bestehend aus SEQ ID NOs: 7-13, 14-18, 23-24, 34-36, 58-61, 64, 65, 89-100, 101-107 einem Teil oder Derivat davon.

In einem weiteren Aspekt betrifft die Erfindung ein immunogenes Fragment eines erfindungsgemäß identifizierten Tumor-assoziierten Antigens. Das Fragment bindet vorzugsweise an einen menschlichen HLA-Rezeptor oder menschlichen Antikörper. Vorzugsweise umfasst ein erfindungsgemäßes Fragment eine Sequenz von mindestens 6, insbesondere mindestens 8, mindestens 10, mindestens 12, mindestens 15, mindestens 20, mindestens 30 oder mindestens 50 Aminosäuren.

In einem weiteren Aspekt betrifft die Erfindung ein Mittel, das an ein erfindungsgemäß identifiziertes Tumor-assoziiertes Antigen oder an einen Teil davon bindet. In einer bevorzugten Ausführungsform ist das Mittel ein Antikörper. In weiteren Ausführungsformen ist der Antikörper ein chimärer, ein humanisierter oder mit kombinatorischen Techniken hergestellte Antikörper oder ein Fragment eines Antikörpers. Des weiteren betrifft die Erfindung einen Antikörper, der selektiv an einen Komplex aus (i) einem erfindungsgemäß identifizierten Tumor-assoziierten Antigen oder einem Teil davon und (ii) einem MHC-Molekül bindet, an das das erfindungsgemäß identifizierte Tumor-assoziierte Antigen oder der Teil davon bindet, wobei der Antiköper nicht alleine an (i) oder (ii) bindet. Ein erfindungsgemäßer Antikörper kann ein monoklonaler Antikörper sein. In weiteren Ausführungsformen ist der Antikörper ein chimärer oder humanisierter Antikörper oder ein Fragment eines natürlichen Antikörpers.

Des weiteren betrifft die Erfindung ein Konjugat zwischen einem erfindungsgemäßen Mittel, das an ein erfindungsgemäß identifiziertes Tumor-assoziiertes Antigen oder an einen Teil davon bindet, oder einem erfindungsgemäßen Antikörper und einem therapeutischen oder diagnostischen Mittel. In einer Ausführungsform ist das therapeutische oder diagnostische Mittel ein Toxin.

In einem weiteren Aspekt betrifft die Erfindung einen Kit zum Nachweis der Expression oder abnormalen Expression eines erfindungsgemäß identifizierten Tumor-assoziierten Antigens, umfassend Mittel zum Nachweis (i) der Nukleinsäure, die für das Tumor-assoziierte Antigen kodiert, oder eines Teils davon, (ii) des Tumor-assoziierten Antigens oder eines Teils davon, (iii) von Antikörpern, die an das Tumor-assoziierte Antigen oder einen Teil davon binden, und/oder (iv) von T-Zellen, die für einen Komplex zwischen dem Tumor-assoziierten Antigen oder einem Teil davon und einem MHC-Molekül spezifisch sind. In einer Ausführungsform sind die Mittel zum Nachweis der Nukleinsäure oder des Teils davon Nukleinsäuremoleküle für die selektive Amplifikation der Nukleinsäure, die insbesondere eine Sequenz von 6-50, insbesondere 10-30, 15-30 und 20-30 zusammenhängenden Nukleotiden aus der Nukleinsäure umfassen.

Die Erfindung betrifft insbesondere folgendes:
1. Pharmazeutische Zusammensetzung, umfassend ein Mittel, das die Expression oder Aktivität eines Tumor-assoziierten Antigens hemmt, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88 einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
2. Pharmazeutische Zusammensetzung, umfassend ein Mittel mit tumorhemmender Aktivität, das selektiv ist für Zellen, die eine Expression oder abnormale Expression eines tumorassoziierten Antigens aufweisen, wobei das Tumor-assoziierte Antigen eine Sequenz aufweist, die von einer Nukleinsäure kodiert wird, die aus der Gruppe ausgewählt ist, bestehend aus
   (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist
3. Pharmazeutische Zusammensetzung nach Ziffer 2, wobei das Mittel die Induktion des Zelltods, die Reduktion des Zellwachstums, eine Schädigung der Zellmembran oder eine Sekretion von Zytokinen bewirkt.
4. Pharmazeutische Zusammensetzung nach Ziffer 1 oder 2, wobei das Mittel eine Antisense-Nukleinsäure ist, die selektiv mit der Nukleinsäure hybridisiert, die für das Tumorassoziierte Antigen kodiert.
5. Pharmazeutische Zusammensetzung nach Ziffer 1 oder 2, wobei das Mittel ein Antikörper ist, der selektiv an das Tumor-assoziierte Antigen bindet.
6. Pharmazeutische Zusammensetzung nach Ziffer 2, wobei das Mittel ein komplementaktivierender Antikörper ist, der selektiv an das Tumor-assoziierter Antigen bindet.
7. Pharmazeutische Zusammensetzung, umfassend ein Mittel, das bei einer Verabreichung selektiv die Menge an Komplexen zwischen einem HLA-Molekül und einem Tumor-assoziierten Antigen oder einem Teil davon erhöht, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
8. Pharmazeutische Zusammensetzung nach Ziffer 7, wobei das Mittel einen oder mehrere Bestandteile umfasst, die aus der Gruppe ausgewählt sind, bestehend aus:
   (i) dem Tumor-assoziierten Antigen oder einem Teil davon,
   (ii) einer Nukleinsäure, die für das Tumor-assoziierte Antigen oder einen Teil davon kodiert,
   (iii) einer Wirtszelle, die das Tumor-assoziierte Antigen oder einen Teil davon exprimiert, und
   (iv) isolierten Komplexen zwischen dem Tumor-assoziierten Antigen oder einem Teil davon und einem HLA-Molekül.
9. Pharmazeutische Zusammensetzung nach Ziffer 1, 2 oder 7, wobei das Mittel mehrere Mittel umfasst, die jeweils selektiv die Expression oder Aktivität verschiedener Tumor-assoziierter Antigene hemmen, jeweils selektiv für Zellen sind, die verschiedene Tumor-assoziierte Antigene exprimieren oder die Menge an Komplexen zwischen HLA-Molekülen und verschiedenen Tumor-assoziierten Antigenen oder Teilen davon erhöhen, wobei mindestens eines der Tumor-assoziierten Antigene eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
10. Pharmazeutische Zusammensetzung umfassend einen oder mehrer Bestandteile, die aus der Gruppe ausgewählt sind, bestehend aus:
   (i) einem Tumor-assoziierten Antigen oder einem Teil davon,
   (ii) einer Nukleinsäure, die für ein Tumor-assoziiertes Antigen oder einen Teil davon kodiert,
   (iii) einem Antikörper, der an ein Tumor-assoziiertes Antigen oder einen Teil davon bindet,
   (iv) einer Antisense-Nukleinsäure, die spezifisch mit einer Nukleinsäure, die für ein Tumor-assoziiertes Antigen kodiert, hybridisiert,
   (v) einer Wirtszelle, die ein Tumor-assoziiertes Antigen oder einen Teil davon exprimiert, und
   (vi) isolierten Komplexen zwischen einem Tumor-assoziierten Antigen oder einem Teil davon und einem HLA-Molekül,
   wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
11. Pharmazeutische Zusammensetzung nach Ziffer 8 oder 10, wobei die Nukleinsäure unter (ii) in einem Expressionsvektor vorliegt.
12. Pharmazeutische Zusammensetzung nach Ziffer 8 oder 10, wobei die Nukleinsäure unter (ii) funktionell mit einem Promotor verbunden ist.
13. Pharmazeutische Zusammensetzung nach Ziffer 8 oder 10, wobei die Wirtszelle das Tumor-assoziierte Antigen oder den Teil davon sekretiert.
14. Pharmazeutische Zusammensetzung nach Ziffer 8 oder 10, wobei die Wirtszelle zusätzlich ein HLA-Molekül exprimiert, das an das Tumor-assoziierte Antigen oder den Teil davon bindet.
15. Pharmazeutische Zusammensetzung nach Ziffer 14, wobei die Wirtszelle das HLA-Molekül und/oder das Tumor-assoziierte Antigen oder den Teil davon rekombinant exprimiert.
16. Pharmazeutische Zusammensetzung nach Ziffer 14, wobei die Wirtszelle das HLA-Molekül endogen exprimiert.
17. Pharmazeutische Zusammensetzung nach Ziffer 8, 10, 14 oder 16, wobei die Wirtszelle eine Antigen-präsentierende Zelle ist.
18. Pharmazeutische Zusammensetzung nach Ziffer 17, wobei die Antigen-präsentierende Zelle eine dendritische Zelle oder ein Makrophage ist.
19. Pharmazeutische Zusammensetzung nach einer der Ziffern 8, 10 und 13-18, wobei die Wirtszelle nicht-proliferativ ist.
20. Pharmazeutische Zusammensetzung nach Ziffer 5 oder 10, wobei der Antikörper ein monoklonaler Antikörper ist.
21. Pharmazeutische Zusammensetzung nach Ziffer 5 oder 10, wobei der Antikörper ein chimärer oder humanisierter Antikörper ist.
22. Pharmazeutische Zusammensetzung nach Ziffer 5 oder 10, wobei der Antikörper ein Fragment eines natürlichen Antikörpers ist
23. Pharmazeutische Zusammensetzung nach Ziffer 5 oder 10, wobei der Antikörper mit einem therapeutischen oder diagnostischen Mittel gekoppelt ist.
24. Pharmazeutische Zusammensetzung nach Ziffer 4 oder 10, wobei die Antisense-Nukleinsäure eine Sequenz von 6-50 zusammenhängenden Nukleotiden aus der Nukleinsäure, die für das Tumor-assoziierte Antigen kodiert, umfasst.
25. Pharmazeutische Zusammensetzung nach einer der Ziffern 8 und 10-13, wobei das durch die pharmazeutische Zusammensetzung bereitgestellte Tumor-assoziierte Antigen oder der Teil davon an MHC-Moleküle auf der Oberfläche von Zellen bindet, die eine abnormale Menge des Tumor-assoziierten Antigens oder eines Teils davon exprimieren.
26. Pharmazeutische Zusammensetzung nach Ziffer 25, wobei die Bindung eine cytolytische Reaktion hervorruft und/ oder eine Cytokinausschüttung induziert
27. Pharmazeutische Zusammensetzung nach einer der Ziffern 1-26, ferner umfassend einen pharmazeutisch verträglichen Träger und/oder ein Adjuvans.
28. Pharmazeutische Zusammensetzung nach Ziffer 27, wobei das Adjuvans Saponin, GM-CSF, CpG, Zytokin oder ein Chemokin ist.
29. Pharmazeutische Zusammensetzung nach einer der Ziffern 1-28, die zur Behandlung einer Erkrankung eingesetzt werden kann, die sich durch die Expression oder abnormale Expression eines Tumor-assoziierten Antigens auszeichnet.
30. Pharmazeutische Zusammensetzung nach Ziffer 29, wobei die Erkrankung Krebs ist.
31. Pharmazeutische Zusammensetzung nach Ziffer 29, wobei die Erkrankung ein Lungentumor, ein Brusttumor, ein Prostatatumor, ein Melanom, ein Colontumor, eine Metastase eines Colontumors, ein Nierenzellkarzinom oder ein Zervixkarzinom, ein Coloncarcinom oder ein Mammacarcinom ist.
32. Pharmazeutische Zusammensetzung nach einer der Ziffern 1-31, wobei das Tumor-assoziierte Antigen eine Aminosäuresequenz umfasst, die aus der Gruppe bestehend aus SEQ ID NOs: 22-24, 58-61, 81, 82, 103-105, 6-13, 14-18, 30, 34-36, 38, 41, 64, 65, 71, 75, 80, 83, 84, 89-100, 101, 102, 106-117 einem Teil oder Derivat davon ausgewählt ist.
33. Verfahren zur Diagnose einer Erkrankung, die sich durch die Expression oder abnormale Expression eines Tumor-assoziierten Antigens auszeichnet, umfassend
   (i) den Nachweis einer Nukleinsäure, die für das Tumor-assoziierte Antigen kodiert, oder eines Teils davon, und/oder
   (ii) den Nachweis des Tumor-assoziierten Antigens oder eines Teils davon, und/oder
   (iii) den Nachweis eines Antikörpers gegen das Tumor-assoziierte Antigen oder eines Teils davon und/oder
   (iv) den Nachweis von cytotoxischen oder Helfer-T-Lymphozyten, die für das Tumorassoziierte Antigen oder einen Teil davon spezifisch sind in einer aus einem Patienten isolierten biologischen Probe, wobei
   das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
34. Verfahren nach Ziffer 33, wobei der Nachweis
   (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an die cytotoxischen oder Helfer-T-Lymphozyten bindet, und
   (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten umfasst.
35. Verfahren nach Ziffer 33 oder 34, wobei der Nachweis mit dem Nachweis in einer vergleichbaren normalen biologischen Probe verglichen wird.
36. Verfahren nach einer der Ziffern 33-35, wobei sich die Erkrankung durch die Expression oder abnormale Expression mehrerer verschiedener Tumor-assoziierter Antigene auszeichnet und der Nachweis einen Nachweis mehrerer Nukleinsäuren, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden, oder den Nachweis mehrerer cytotoxischer oder Helfer-T-Lymphozyten, die für die mehreren verschiedenen Tumor-assoziierten Antigene spezifisch sind, umfasst.
37. Verfahren nach einer der Ziffern 33-36, wobei der Nachweis der Nukleinsäure oder des Teils davon mit einer Polynukleotid-Sonde erfolgt, die spezifisch mit der Nukleinsäure oder dem Teil davon hybridisiert.
38. Verfahren nach Ziffer 37, wobei die Polynukleotid-Sonde eine Sequenz von 6-50 zusammenhängenden Nukleotiden aus der Nukleinsäure, die für das Tumor-assoziierte Antigen kodiert, umfasst.
39. Verfahren nach einer der Ziffern 33-36, wobei der Nachweis der Nukleinsäure oder des Teils davon durch selektive Amplifikation der Nukleinsäure oder des Teils davon erfolgt.
40. Verfahren nach einer der Ziffern 33-36, wobei das nachzuweisende Tumorassoziierte Antigen oder der Teil davon in einem Komplex mit einem MHC-Molekül vorliegt.
41. Verfahren nach Ziffer 40, wobei das MHC-Molekül ein HLA-Molekül ist.
42. Verfahren nach einer der Ziffern 33-36 und 40-41, wobei der Nachweis des Tumor-assoziierten Antigens oder des Teils davon mit einem Antikörper erfolgt, der spezifisch an das Tumor-assoziierte Antigen oder den Teil davon bindet.
43. Verfahren nach einer der Ziffern 33-36, wobei der Nachweis des Antikörpers mit einem Protein oder Peptid erfolgt, das spezifisch an den Antikörper bindet.
44. Verfahren zur Bestimmung der Regression, des Verlaufs oder des Ausbruchs einer Erkrankung, die sich durch die Expression oder abnormale Expression eines Tumorassoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, ausgewählt aus der Gruppe, bestehend aus:
   (i) der Menge der Nukleinsäure, die für das Tumor-assoziierte Antigen kodiert, oder eines Teil davon,
   (ii) der Menge des Tumor-assoziierten Antigens oder eines Teils davon,
   (iii) der Menge an Antikörpern, die an das Tumor-assoziierte Antigen oder einen Teil davon binden, und
   (iv) der Menge an cytolytischen oder Cytokin-ausschüttenden T-Zellen, die für einen Komplex zwischen dem Tumor-assoziierten Antigen oder einem Teil davon und einem MHC-Molekül spezifisch sind, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist
45. Verfahren nach Ziffer 44, wobei das Verfahren die Bestimmung des oder der Parameter zu einem ersten Zeitpunkt in einer ersten Probe und zu einem zweiten Zeitpunkt in einer weiteren Probe umfasst und durch einen Vergleich der beiden Proben der Verlauf der Erkrankung ermittelt wird.
46. Verfahren nach Ziffer 44 oder 45, wobei die Erkrankung sich durch die Expression oder abnormale Expression mehrerer verschiedener Tumor-assoziierter Antigene auszeichnet und die Überwachung eine Überwachung
   (i) der Menge mehrerer Nukleinsäuren, die für die mehreren verschiedenen Tumorassoziierten Antigene kodieren, oder von Teilen davon,
   (ii) der Menge der mehreren verschiedenen Tumor-assoziierten Antigene oder von Teilen davon,
   (iii) der Menge mehrerer Antikörper, die an die mehreren verschiedenen Tumor-assoziierten Antigene oder an Teile davon binden, und/oder
   (iv) der Menge mehrerer cytolytischer oder Cytokine-ausschüttender T-Zellen, die für Komplexe zwischen den mehreren verschiedenen Tumor-assoziierten Antigenen oder von Teilen davon und MHC-Molekülen spezifisch sind, umfasst.
47. Verfahren nach einer der Ziffern 44-46, wobei die Überwachung der Menge der Nukleinsäure oder des Teils davon mit einer Polynukleotid-Sonde erfolgt, die spezifisch mit der Nukleinsäure oder dem Teil davon hybridisiert.
48. Verfahren nach Ziffer 47, wobei die Polynukleotid-Sonde eine Sequenz von 6-50 zusammenhängenden Nukleotiden aus der Nukleinsäure, die für das Tumor-assoziierte Antigen kodiert, umfasst.
49. Verfahren nach einer der Ziffern 44-46, wobei die Überwachung der Menge der Nukleinsäure oder des Teils davon durch selektive Amplifikation der Nukleinsäure oder des Teils davon erfolgt.
50. Verfahren nach einer der Ziffern 44-46, wobei die Überwachung der Menge des Tumor-assoziierten Antigens oder des Teils davon mit einem Antikörper erfolgt, der spezifisch an das Tumor-assoziierte Antigen oder den Teil davon bindet.
51. Verfahren nach einer der Ziffern 44-46, wobei die Überwachung der Menge an Antikörpern mit einem Protein oder Peptid erfolgt, das spezifisch an den Antikörper bindet.
52. Verfahren nach einer der Ziffern 44-46, wobei die Überwachung der Menge an cytolytischen oder Cytokin-aussschüttenden T-Zellen mit einer Zelle erfolgt, die den Komplex zwischen dem Tumor-assoziierten Antigen oder dem Teil davon und einem MHC-Molekül präsentiert.
53. Verfahren nach einer der Ziffern 37-38, 42-43, 47-48 und 50-52, wobei die Polynukleotid-Sonde, der Antikörper, das Protein oder Peptid oder die Zelle nachweisbar markiert sind.
54. Verfahren nach Ziffer 53, wobei der nachweisbare Marker ein radioaktiver Marker oder ein Enzymmarker ist.
55. Verfahren nach einer der Ziffern 33-54, wobei die Probe Körperflüssigkeit und/oder Körpergewebe umfasst.
56. Verfahren zur Behandlung einer Erkrankung, die sich durch die Expression oder abnormale Expression eines Tumor-assoziierten Antigens auszeichnet, umfassend die Verabreichung einer pharmazeutischen Zusammensetzung nach einer der Ziffern 1-32, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
57. Verfahren zur Behandlung, Diagnose oder Überwachung einer Erkrankung, die sich durch die Expression oder abnormale Expression eines Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel gekoppelt ist, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
58. Verfahren nach Ziffer 42, 50 oder 57, wobei der Antikörper ein monoklonaler Antikörper ist.
59. Verfahren nach Ziffer 42, 50 oder 57, wobei der Antikörper ein chimärer oder humanisierter Antikörper ist.
60. Verfahren nach Ziffer 42, 50 oder 57, wobei der Antikörper ein Fragment eines natürlichen Antikörpers ist.
61. Verfahren zur Behandlung eines Patienten mit einer Erkrankung, die sich durch die Expression oder abnormale Expression eines Tumor-assoziierten Antigens auszeichnet, umfassend:
   (i) die Entfernung einer Probe mit immunreaktiver Zellen aus dem Patienten,
   (ii) die Kontaktierung der Probe mit einer Wirtszelle, die das Tumor-assoziierte Antigen oder einen Teil davon exprimiert, unter Bedingungen, die eine Produktion cytolytischer oder Cytokine-ausschüttender T-Zellen gegen das Tumor-assoziierte Antigen oder einen Teil davon begünstigen, und
   (iii) das Einbringen der cytolytischen oder Cytokine-ausschüttenden T-Zellen in den Patienten in einer Menge, die geeignet ist, Zellen zu lysieren, die das Tumor-assoziierte Antigen oder einen Teil davon exprimieren, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
62. Verfahren nach Ziffer 61, wobei die Wirtszelle ein HLA-Molekül rekombinant exprimiert, das an das Tumor-assoziierte Antigen oder einen Teil davon bindet.
63. Verfahren nach Ziffer 62, wobei die Wirtszelle ein HLA-Molekül endogen exprimiert, das an das Tumor-assoziierte Antigen oder einen Teil davon bindet.
64. Verfahren zur Behandlung eines Patienten mit einer Erkrankung, die sich durch die Expression oder abnormale Expression eines Tumor-assoziierten Antigens auszeichnet, umfassend:
   (i) die Identifizierung einer Nukleinsäure, die von Zellen exprimiert wird, die mit der Erkrankung assoziiert sind, wobei die Nukleinsäure aus der Gruppe ausgewählt ist, bestehend aus:
      (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist,
   (ii) die Transfektion einer Wirtszelle mit der Nukleinsäure oder einem Teil davon,
   (iii) die Kultivierung der transfizierten Wirtszelle für eine Expression der Nukleinsäure, und
   (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen.
65. Verfahren nach Ziffer 64, ferner umfassend die Identifizierung eines MHC-Moleküls, das das Tumor-assoziierte Antigen oder einen Teil davon präsentiert, wobei die Wirtszelle das identifizierte MHC-Molekül exprimiert und das Tumor-assoziierte Antigen oder einen Teil davon präsentiert.
66. Verfahren nach Ziffer 64 oder 65, wobei die Immunreaktion eine B-Zellen-Reaktion oder eine T-Zellen-Reaktion umfasst.
67. Verfahren nach Ziffer 66, wobei die Immunreaktion eine T-Zellen-Reaktion ist, umfassend die Produktion cytolytischer oder Cytokine-ausschüttenden T-Zellen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.
68. Verfahren nach einer der Ziffern 61-67, wobei die Wirtszellen nicht-proliferativ sind.
69. Verfahren zur Behandlung einer Erkrankung, die sich durch die Expression oder abnormale Expression eines Tumor-assoziierten Antigens auszeichnet, umfassend:
   (i) die Identifikation von Zellen aus dem Patienten, die abnormale Mengen des Tumorassoziierten Antigens exprimieren,
   (ii) die Isolierung einer Probe der Zellen,
   (iii) die Kultivierung der Zellen, und
   (iv) das Einbringen der Zellen in den Patienten in einer Menge, die geeignet ist, eine Immunreaktion gegen die Zellen auszulösen, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
70. Verfahren nach einer der Ziffern 33-69, wobei die Erkrankung Krebs ist.
71. Verfahren zur Hemmung der Entwicklung von Krebs bei einem Patienten, umfassend die Verabreichung einer wirksamen Menge einer pharmazeutischen Zusammensetzung nach einer der Ziffern 1-32.
72. Verfahren nach einer der Ziffern 33-71, wobei das Tumor-assoziierte Antigen eine Aminosäuresequenz umfasst, die aus der Gruppe bestehend aus SEQ ID NOs: 22-24, 58-61, 81, 82, 103-105, 6-13, 14-18, 30, 34-36, 38, 41, 64, 65, 71, 75, 80, 83, 84, 89-100, 101, 102, 106-117 einem Teil oder Derivat davon ausgewählt ist.
73. Nukleinsäure, die aus der Gruppe ausgewählt ist, bestehend aus:
   (a) einer Nukleinsäure, die eine Nukleinsäuresequenz umfasst, die aus der Gruppe bestehend aus SEQ ID NOs: 20-21, 54-57, 2-5, 31-33, 37, 39, 62, 63, 85-88 einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
74. Nukleinsäure, die für ein Protein oder Polypeptid kodiert, das eine Aminosäuresequenz umfasst, ausgewählt aus der Gruppe bestehend aus SEQ ID NOs: 23-24, 58-61, 81, 82, 103-105, 7-13, 14-18, 34-36, 64, 65, 80, 83, 84, 89-100, 101, 102, 106-117 einem Teil oder Derivat davon.
75. Rekombinantes DNA- oder RNA-Molekül, das eine Nukleinsäure nach Ziffer 73 oder 74 umfasst.
76. Rekombinantes DNA-Molekül nach Ziffer 75, wobei das rekombinante DNA-Molekül ein Vektor ist.
77. Rekombinantes DNA-Molekül nach Ziffer 76, wobei der Vektor ein viraler Vektor oder ein Bakteriophage ist.
78. Rekombinantes DNA-Molekül nach einer der Ziffern 75-77, das ferner Expressionskontrollsequenzen umfasst, die die Expression der Nukleinsäure steuern.
79. Rekombinantes DNA-Molekül nach Ziffer 78, wobei die Expressionskontrollsequenzen homo- oder heterolog zu der Nukleinsäure sind.
80. Wirtszelle, die eine Nukleinsäure nach Ziffer 73 oder 74 oder ein rekombinantes DNA-Molekül nach einer der Ziffern 75-79 umfasst.
81. Wirtszelle nach Ziffer 80, die ferner eine Nukleinsäure umfasst, die für ein HLA-Molekül kodiert.
82. Protein oder Polypeptid, das von einer Nukleinsäure nach Ziffer 73 kodiert wird.
83. Protein oder Polypeptid, das eine Aminosäuresequenz umfasst, ausgewählt aus der Gruppe bestehend aus SEQ ID NOs: 23-24, 58-61, 81, 82, 103-105, 7-13, 14-18, 34-36, 64, 65, 80, 83, 84, 89-100, 101, 102, 106-117 einem Teil oder Derivat davon.
84. Immunogenes Fragment des Proteins oder Polypeptids nach Ziffer 82 oder 83.
85. Fragment des Proteins oder Polypeptids nach Ziffer 82 oder 83, das an menschlichen HLA-Rezeptor oder menschlichen Antikörper bindet.
86. Mittel, das spezifisch an ein Protein oder Polypeptid oder an einen Teil davon bindet, wobei das Protein oder Polypeptid von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
87. Mittel nach Ziffer 86, wobei das Protein oder Polypeptid eine Aminosäuresequenz umfasst, die aus der Gruppe ausgewählt ist, bestehend aus SEQ ID NOs: 22-24, 58-61, 81, 82, 103-105, 6-13,14-18,30, 34-36, 38,41,64,65, 71, 75, 80, 83, 84, 89-100, 101, 102, 106-117 einem Teil oder Derivat davon.
88. Mittel nach Ziffer 86 oder 87, wobei das Mittel ein Antikörper ist.
89. Mittel nach Ziffer 88, wobei der Antikörper ein monoklonaler, chimärer oder humanisierter Antikörper oder ein Fragment eines Antikörpers ist.
90. Antikörper, der selektiv an einen Komplex aus:
   (i) einem Protein oder Polypeptid oder einem Teil davon und
   (ii) einem MHC-Molekül bindet, an das das Protein oder Polypeptid oder der Teil davon bindet, wobei der Antiköper nicht alleine an (i) oder (ii) bindet und das Protein oder Polypeptid von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
91. Antikörper nach Ziffer 90, wobei das Protein oder Polypeptid eine Aminosäuresequenz umfasst, die aus der Gruppe ausgewählt ist, bestehend aus SEQ ID NOs: 22-24, 58-61, 81, 82, 103-105, 6-13, 14-18, 30, 34-36, 38, 41, 64, 65, 71, 75, 80, 83, 84, 89-100, 101, 102, 106-117 einem Teil oder Derivat davon.
92. Antikörper nach Ziffer 90 oder 91, wobei der Antikörper ein monoklonaler, chimärer oder humanisierter Antikörper oder ein Fragment eines Antikörpers ist.
93. Konjugat zwischen einem Mittel nach einer der Ziffern 86-89 oder einem Antikörper nach einer der Ziffern 90-92 und einem therapeutischen oder diagnostischen Mittel.
94. Konjugat nach Ziffer 93, wobei das therapeutische oder diagnostische Mittel ein Toxin ist.
95. Kit zum Nachweis der Expression oder abnormalen Expression eines Tumorassoziierten Antigens, umfassend Mittel zum Nachweis
   (i) der Nukleinsäure, die für das Tumor-assoziierte Antigen kodiert, oder eines Teils davon,
   (ii) des Tumor-assoziierten Antigens oder eines Teils davon,
   (iii) von Antikörpern, die an das Tumor-assoziierte Antigen oder einen Teil davon binden, und/oder
   (iv) von T-Zellen, die für einen Komplex zwischen dem Tumor-assoziierten Antigen oder einem Teil davon und einem MHC-Molekül spezifisch sind, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88 einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
96. Kit nach Ziffer 95, wobei die Mittel zum Nachweis der Nukleinsäure, die für das Tumor-assoziierte Antigen kodiert, oder eines Teils davon Nukleinsäuremoleküle für die selektive Amplifikation der Nukleinsäure sind.
97. Kit nach Ziffer 96, wobei die Nukleinsäuremoleküle für die selektive Amplifikation der Nukleinsäure eine Sequenz von 6-50 zusammenhängenden Nukleotiden aus der Nukleinsäure, die für das Tumor-assoziierte Antigen kodiert, umfassen.
98. Rekombinantes DNA-Molekül, umfassend eine Promotorregion, die von einer Nukleinsäuresequenz abgeleitet ist, die aus der Gruppe bestehend aus SEQ ID NOs: 19-21, 54-57, 1-5, 29, 31-33, 37, 39, 40, 62, 63, 70, 74, 85-88 ausgewählt ist.
99. Pharmazeutische Zusammensetzung, umfassend ein Mittel, das die Expression oder Aktivität des Tumorantigens TPTE SEQ ID NOs: 19, 22 hemmt.
100. Pharmazeutische Zusammensetzung, umfassend ein Mittel, das die Migrations- und Metastasierungsaktivität von TPTE hemmt.
101. Mittel nach Ziffer 99 oder 100, wobei das Mittel ein Antikörper ist.
102. Mittel nach Ziffer 101, wobei der Antikörper ein monoklonaler, chimärer oder humanisierter Antikörper oder ein Fragment eines Antikörpers ist.
103. Antikörper, der an die extrazellulären Proteinbereiche umfassend die Sequenzen SEQ ID NOs:81-82 bindet.
104. Mittel nach Ziffer 99 oder 100, wobei das Mittel eine Antisense-Nukleinsäure ist, die selektiv mit der Nukleinsäure hybridisiert, die für TPTE kodiert.
105. Mittel nach Ziffer 104, wobei die Antisense-Nukleinsäure eine Sequenz von 6-50 zusammenhängenden Nukleotiden aus der Nukleinsäure, die für TPTE kodiert, umfasst.
106. Mittel nach Ziffer 99 oder 100, wobei das Mittel RNA Interferenz (RNAi) ist.
107. Mittel nach Ziffer 106, wobei RNAi eine sog. short hairpin Struktur (shRNA) enthält.
108. Mittel nach Ziffer 107, wobei shRNA durch Transkription nach Transfektion mit Expressionsvektoren entstehen.
109. Mittel nach Ziffer 107, wobei shRNA durch Transkription von Retroviren entsteht
110. Mittel nach Ziffer 107, wobei shRNA durch lentivirale Systeme vermittelt wird.
111. Mittel nach Ziffer 99 oder 100, wobei das Mittel ein kleines chemisches Molekül ist.
112. Mittel nach Ziffer 111, wobei die kleinen chemischen Moleküle an TPTE binden
113. Mittel nach Ziffer 112, wobei die kleinen chemischen Moleküle an die extrazellulären Bereiche umfassend SEQ ID NO:81-82 binden.
114. Verfahren zur Behandlung Diagnose oder Überwachung eines metastasierenden Tumors, der sich durch die Expression oder abnormaler Expression von TPTE auszeichnet, umfassend die Verabreichung eines Antikörpers, der an TPTE oder einen Teil davon bindet und mit einem therapeutischen oder diagnostischen Mittel gekoppelt ist, wobei das TPTE eine Sequenz aufweist, die von einer Nukleinsäure kodiert wird bestehend aus:
   (a) einer Nukleinsäure, die eine Nukleinsäuresequenz umfassend die SEQ ID NO:19 einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
115. Verfahren nach Ziffer 114, wobei der Antikörper ein monoklonaler Antikörper ist.
116. Verfahren nach Ziffer 114, wobei der Antikörper ein chimärer oder humanisierter Antikörper ist.
117. Verfahren nach Ziffer 114, wobei der Antikörper ein Fragment eines natürlichen Antikörpers ist.

### Detaillierte Beschreibung der Erfindung

Erfindungsgemäß werden Gene beschrieben, die in Tumorzellen selektiv exprimiert oder aberrant exprimiert werden und Tumor-assoziierte Antigene darstellen.

Erfindungsgemäß sind diese Gene oder ihre Derivate bevorzugte Zielstrukturen für therapeutische Ansätze. Konzeptionell können die therapeutischen Ansätze auf eine Hemmung der Aktivität des selektiv exprimierten tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten tumorassoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist erfindungsgemäß gemeint, dass einzelne oder multiple Nukleotidsubstitution, -deletion und/oder -addition in der Nukleinsäure vorliegen. Weiterhin umfasst der Begriff "Derivat" auch eine chemische Derivatisierung einer Nukleinsäure an einer Nukleotidbase, am Zucker oder am Phosphat. Der Begriff "Derivat" umfasst auch Nukleinsäuren, die nicht in der Natur vorkommende Nukleotide und Nukleotidanaloga enthalten.

Eine Nukleinsäure ist erfindungsgemäß vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann erfindungsgemäß als einzelsträngiges oder doppelsträngiges und lineares oder kovalent kreisförmig geschlossenes Molekül vorliegen.

Die erfindungsgemäß beschriebenen Nukleinsäuren sind vorzugsweise isoliert. Der Begriff "isolierte Nukleinsäure" bedeutet erfindungsgemäß, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH7), 0,5% SDS, 2mM EDTA). SSC ist 0,15 M Natriumchlorid/ 0,15 M Natriumcitrat, pH 7. Nach der Hybridisierung wird die Membran, auf die die DNA übertragen wurde beispielsweise in 2 x SSC bei Raumtemperatur und sodann in 0,1 - 0,5 x SSC/ 0,1 x SDS bei Temperaturen bis 68°C gewaschen.

Komplementäre Nukleinsäuren weisen erfindungsgemäß mindestens 40%, insbesondere mindestens 50%, mindestens 60%, mindestens 70%, mindestens 80%, mindestens 90% und vorzugsweise mindestens 95%, mindestens 98 oder mindestens 99% Identität der Nukleotide auf.

Nukleinsäuren, die für Tumor-assoziierte Antigene kodieren, können erfindungsgemäß alleine oder in Kombination mit anderen Nukleinsäuren, insbesondere heterologen Nukleinsäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst erfindungsgemäß Promotoren, Enhancer und andere Kontrollelemente, die die Expression eines Gens steuern. In bestimmten erfindungsgemäßen Ausführungsformen sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch EnhancerSequenzen oder stromaufwärts gelegene Aktivatorsequenzen umfassen.

Zum einen können also die hier dargestellten tumorassoziierten Antigene mit beliebigen Expressionskontrollsequenzen und Promotoren kombiniert werden. Zum anderen aber können erfindungsgemäß die Promotoren der hier dargestellten tumor-assoziierten Genprodukte mit beliebigen anderen Genen kombiniert werden. Dies erlaubt, die selektive Aktivität dieser Promotoren zu nutzen.

Des weiteren kann eine Nukleinsäure erfindungsgemäß in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure erfindungsgemäß in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt.

In einer bevorzugten Ausführungsform ist ein rekombinantes DNA-Molekül erfindungsgemäß ein Vektor, gegebenenfalls mit einem Promotor, der die Expression einer Nukleinsäure, z.B. einer Nukleinsäure, die für eine erfindungsgemäßes Tumor-assoziiertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen die Nukleinsäure in prokaryotische und/oder in eukaryotische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide oder Virusgenome.

Die Nukleinsäuren, die für ein erfindungsgemäß identifiziertes Tumor-assoziiertes Antigen kodieren, können für eine Transfektion von Wirtszellen eingesetzt werden. Mit Nukleinsäuren ist dabei sowohl rekombinante DNA wie auch RNA gemeint. Rekombinante RNA kann durch in-vitro-Transkription von einer DNA-Matritze hergestellt werden. Sie kann des weiteren vor Applikation durch stabilisierende Sequenzen, Capping und Poly-Adenylierung modifiziert werden. Der Begriff "Wirtszelle" betrifft erfindungsgemäß jede Zelle, die mit einer exogenen Nukleinsäure transformierbar oder transfizierbar ist. Der Begriff "Wirtszellen" umfasst erfindungsgemäß prokaryontische (z.B. *E. coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege, Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, Monozyt oder ein Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht) und die Enhancer-Promotor-Sequenzen von Cytomegalovirus (CMV).

In den Fällen der Erfindung, in denen ein HLA-Molekül ein Tumor-assoziiertes Antigen oder einen Teil davon präsentiert, kann ein Expressionsvektor auch eine Nukleinsäuresequenz umfassen, das für das HLA-Molekül kodiert. Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Erfindungsgemäß umfasst sind Kits zur Amplifikation einer Nukleinsäure, die für ein Tumor-assoziiertes Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 und 20-30 zusammenhängenden Nukleotiden aus der Nukleinsäure und sind nichtüberlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure" betrifft erfindungsgemäß ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül" umfasst erfindungsgemäß auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann eine Duplex mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. Bevorzugte erfindungsgemäße Antisense-Oligonukleotide weisen eine Sequenz von 6-50, insbesondere 10-30, 15-30 und 20-30 zusammenhängenden Nukleotiden aus der Ziel-Nukleinsäure auf und sind vorzugsweise vollständig zu der Ziel-Nukleinsäure oder einem Teil davon komplementär.

In bevorzugten Ausführungsformen hybridisiert das Antisense-Oligonukleotid mit einer N-terminalen oder 5'-stromaufwärts gelegenen Stelle wie einer Translationsinitiations-, Transkriptionsinitiations- oder Promotorstelle. In weiteren Ausführungsformen hybridisiert das Antisense-Oligonukleotid mit einer 3'-nicht-translatierten Region oder mRNA-Splicing-Stelle.

In einer Ausführungsform besteht ein erfindungsgemäßes Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

In bevorzugten Ausführungsformen ist ein erfindungsgemäßes Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet erfindungsgemäß ein Oligonukleotid bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die erfindungsgemäß beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im wesentlichen aufgereinigten Zustand vorliegen. Der Begriff "im wesentlichen aufgereinigt" bedeutet, dass das Protein oder Polypeptid im wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Erfindungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften ersetzt, wie Hydrophobizität, Hydrophilizität, Elektronegativität, Volumen der Seitenkette und ähnliches (konservative Substitution). Konservative Substitutionen betreffen beispielsweise den Austausch einer Aminosäure durch eine andere, nachstehend in derselben Gruppe wie die substituierte Aminosäure aufgeführte Aminosäure:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen oder Polypeptiden umfassen erfindungsgemäß auch einzelne oder multiple Substitutionen, Deletionen und/oder Additionen jeglicher Moleküle, die mit dem Enzym assoziiert sind, wie Kohlenhydrate, Lipide und/oder Proteine oder Polypeptide. Ferner erstreckt sich der Begriff "Derivat" auch auf alle funktionellen chemischen Äquivalente der Proteine oder Polypeptide.

Ein Teil oder Fragment eines Tumor-assoziierten Antigens weist erfindungsgemäß eine funktionelle Eigenschaft des Polypeptids auf, aus dem es abgeleitet sind. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer T-Zellen beruhen. Vorzugsweise umfasst ein erfindungsgemäßer Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen.

Bevorzugte Teile oder Fragmente eines Tumor-assoziierten Antigens weisen erfindungsgemäß vorzugsweise eine der nachstehend aufgeführten Sequenzen auf, die von den erfindungsgemäß identifizierten Tumor-assoziierten Antigenen abgeleitet sind, und sind vorzugsweise Peptidepitope, die erfindungsgemäß insbesondere zur Stimulierung von cytotoxischen T-Lymphozyten in vivo aber auch zur Herstellung expandierter und stimulierter T-Lymphozyten zum therapeutischen adoptiven Transfer ex vivo geeignet sind. Diese Sequenzen sind insbesondere Peptidepitope für die nachstehend angegebenen MHC Klasse I Allele:
HLA-A* 0201
HLA-A*2402
HLA-A*01
HLA-A* 03
HLA-B* 0702

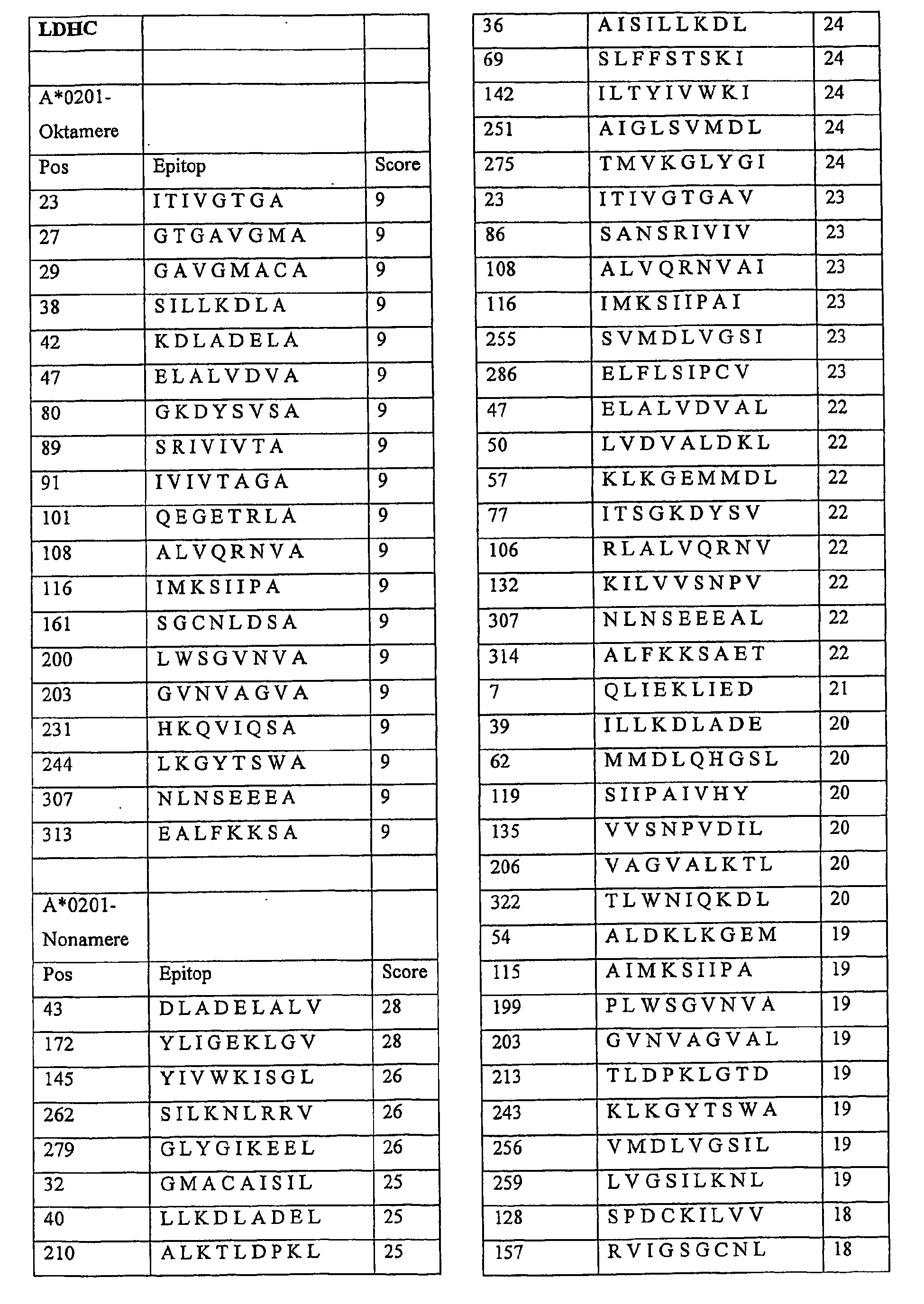

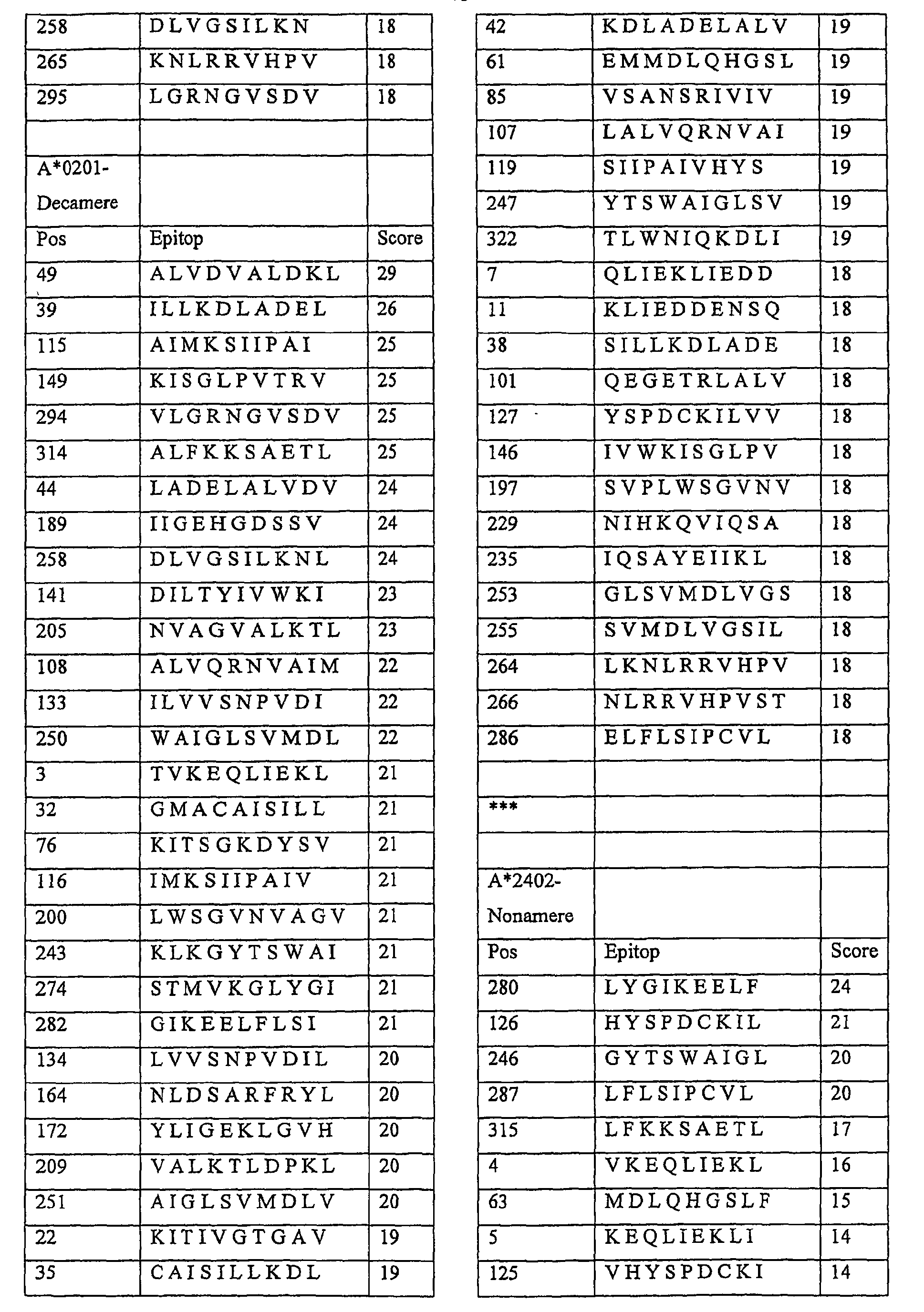

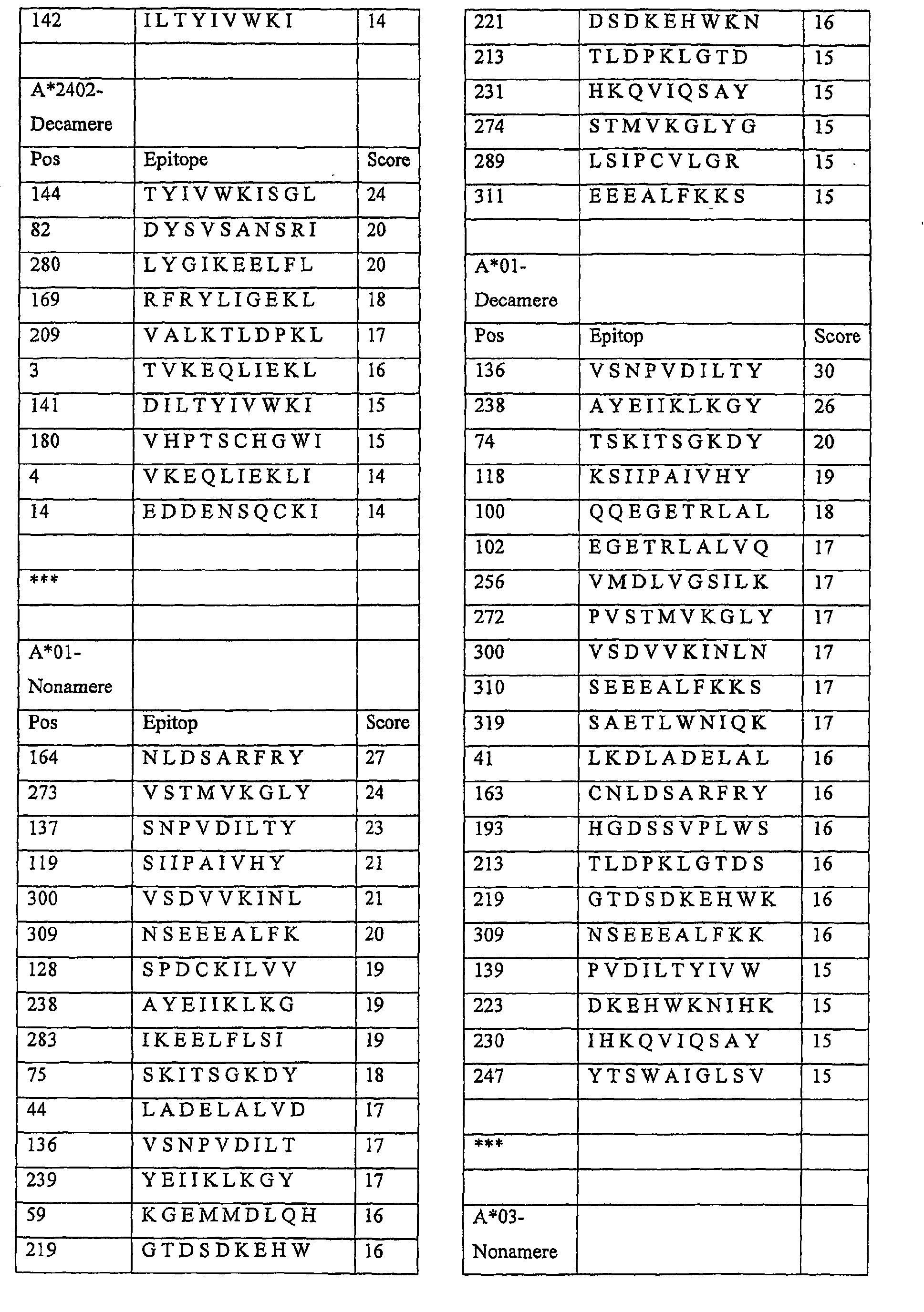

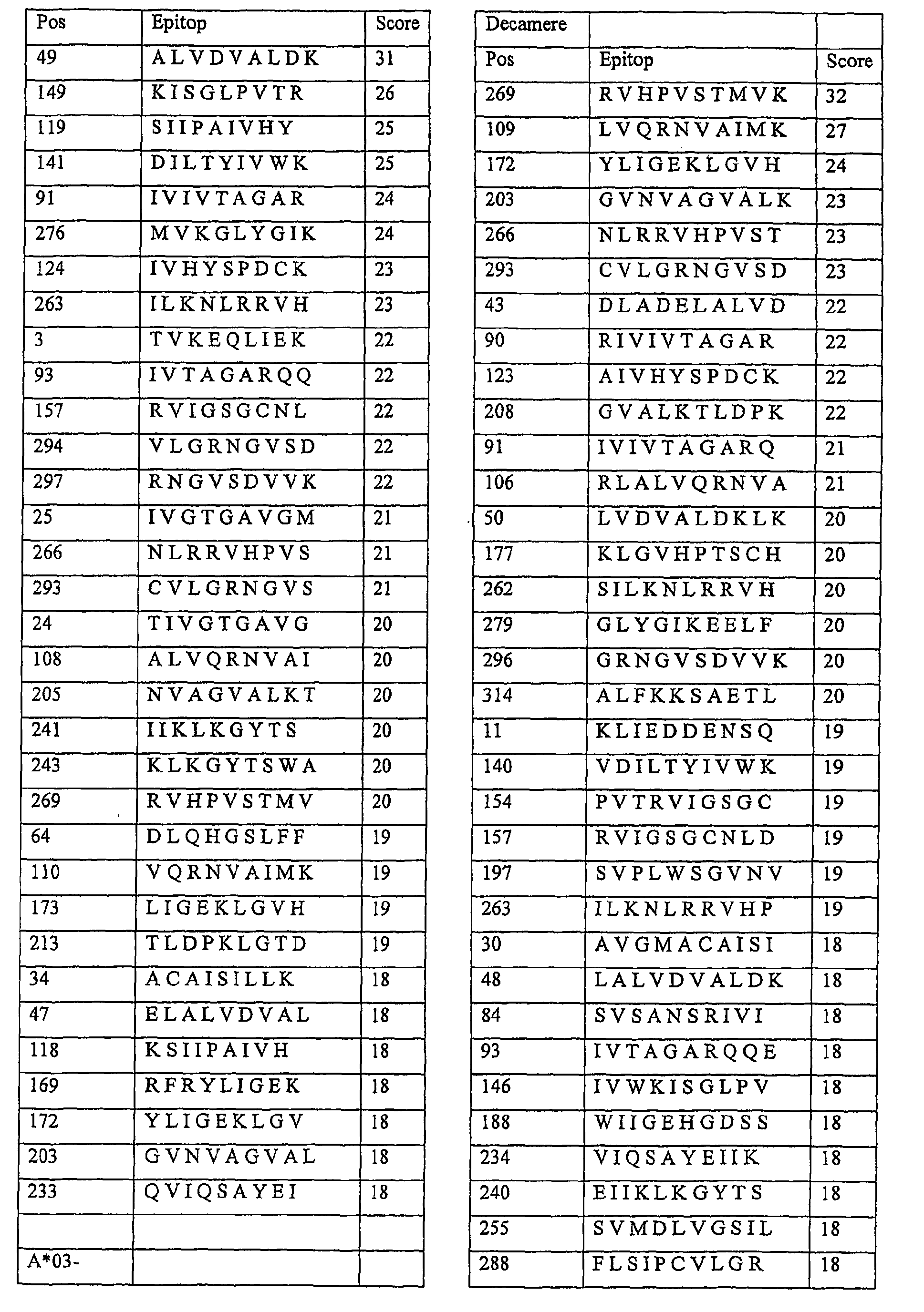

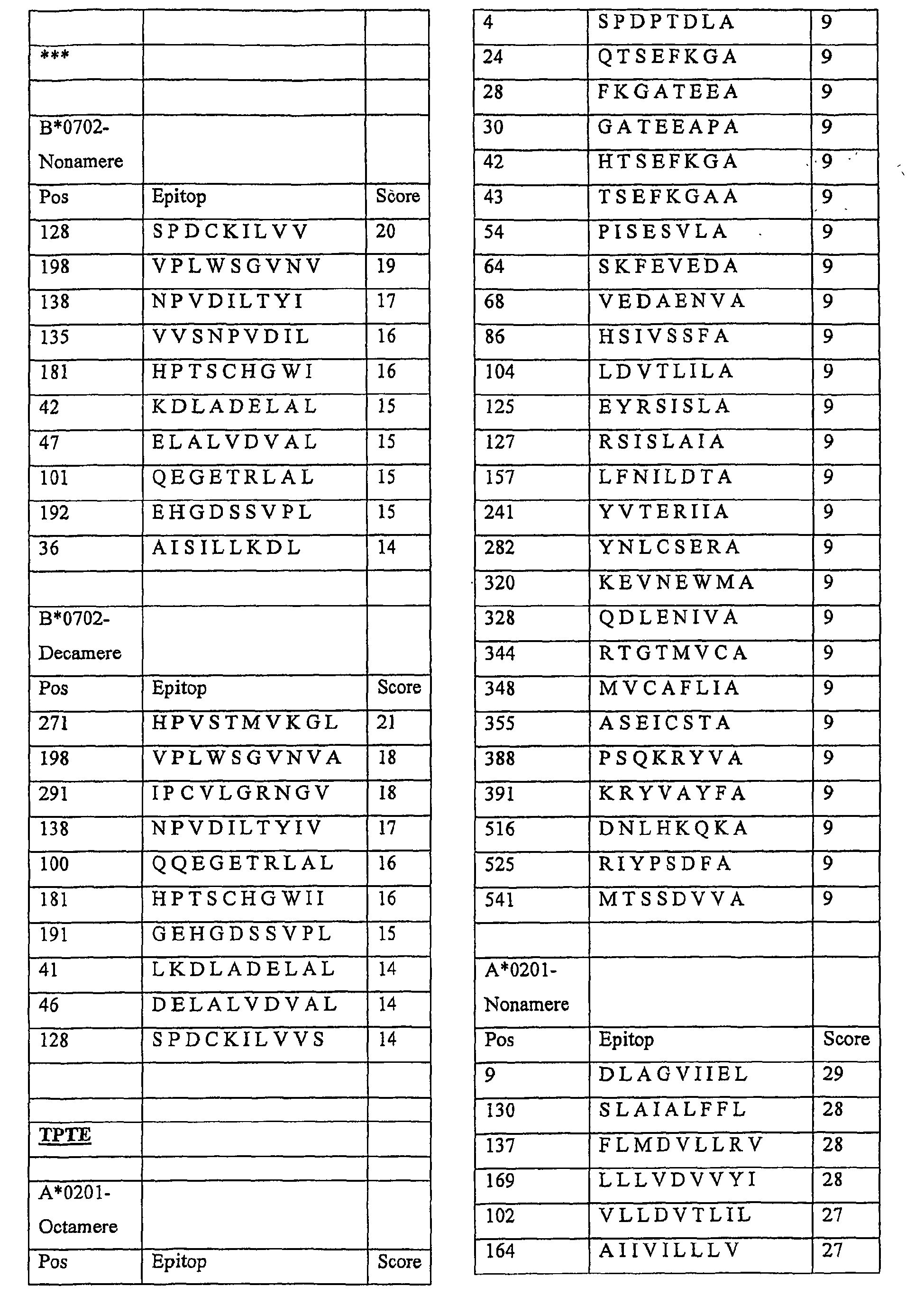

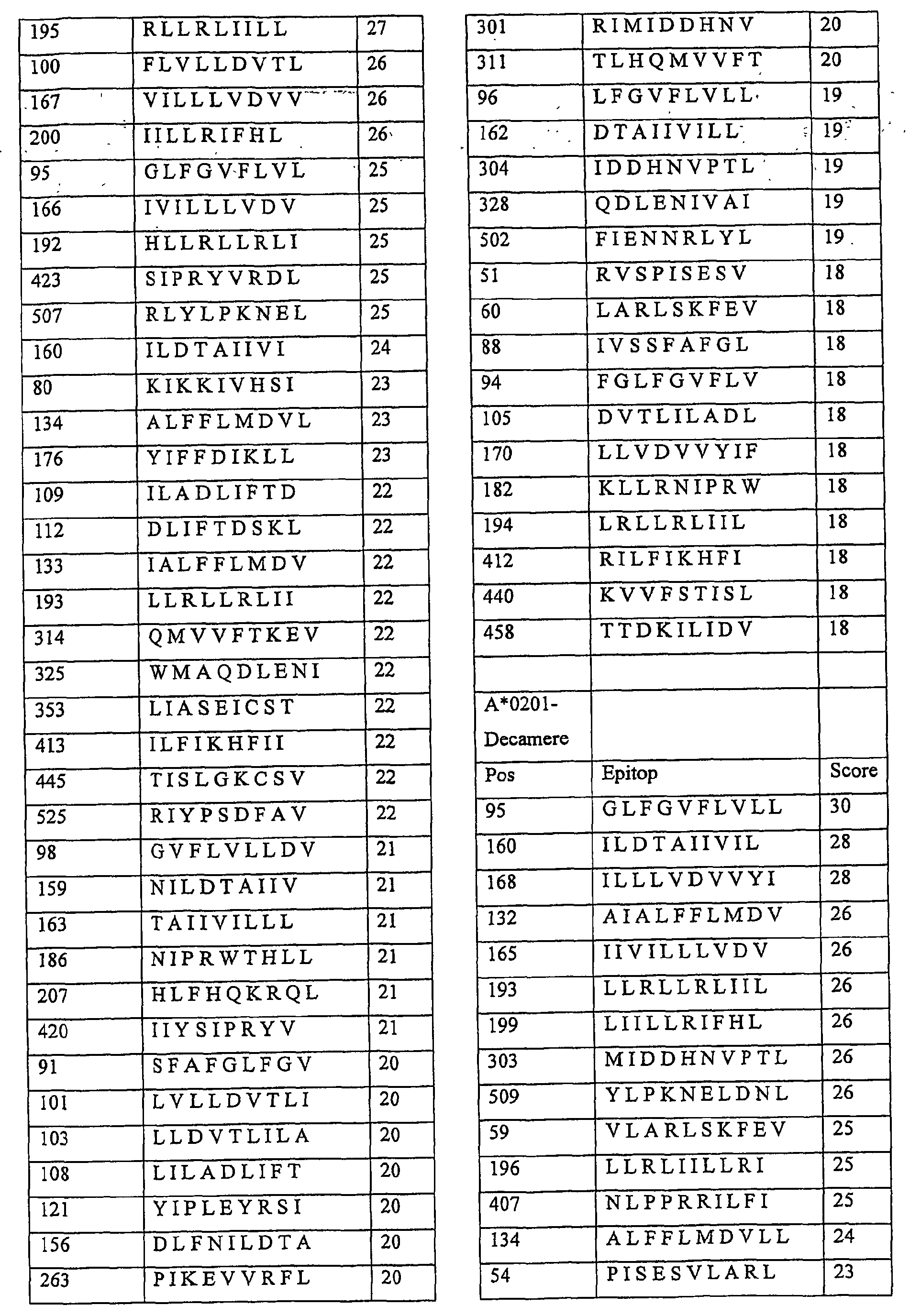

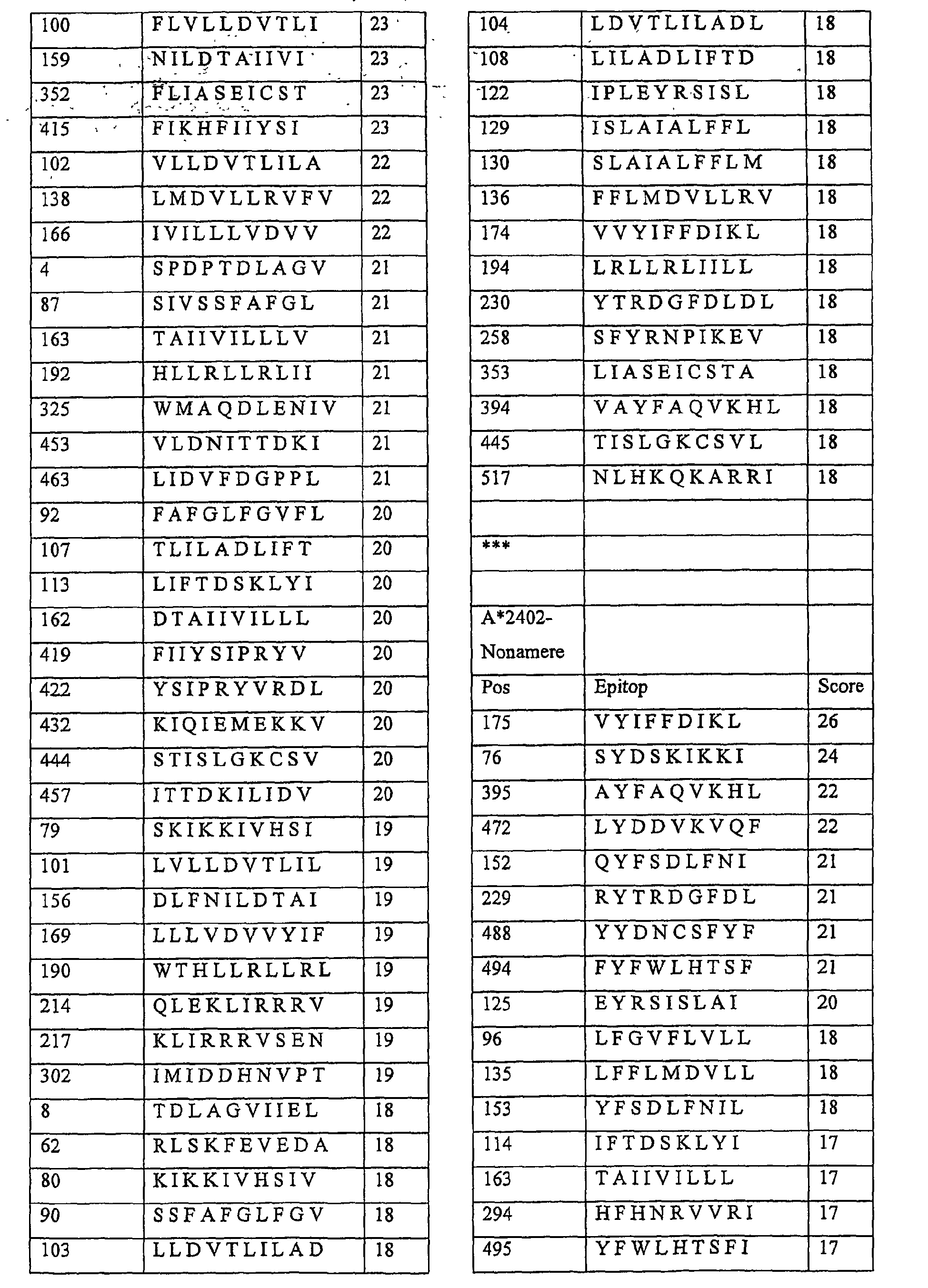

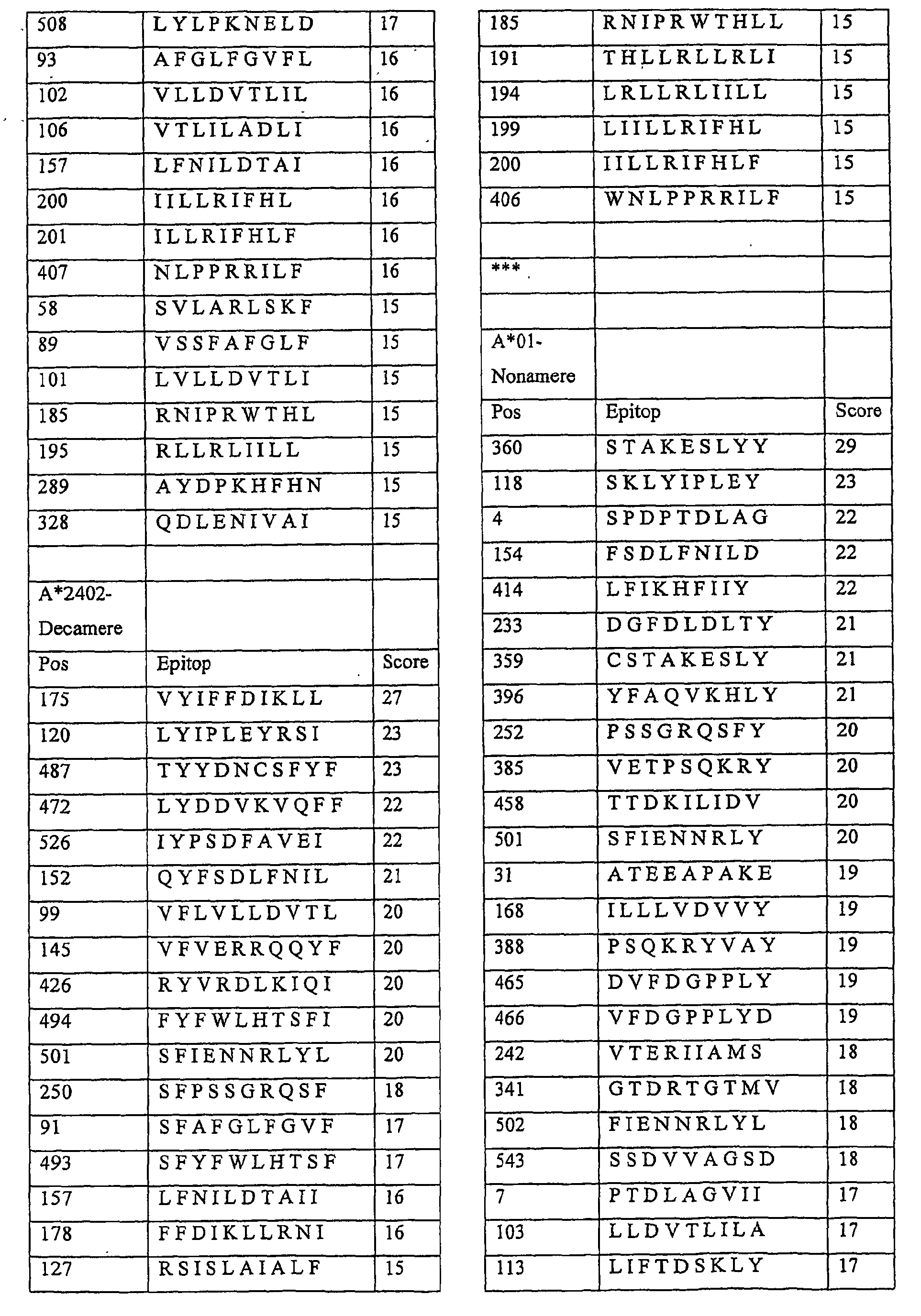

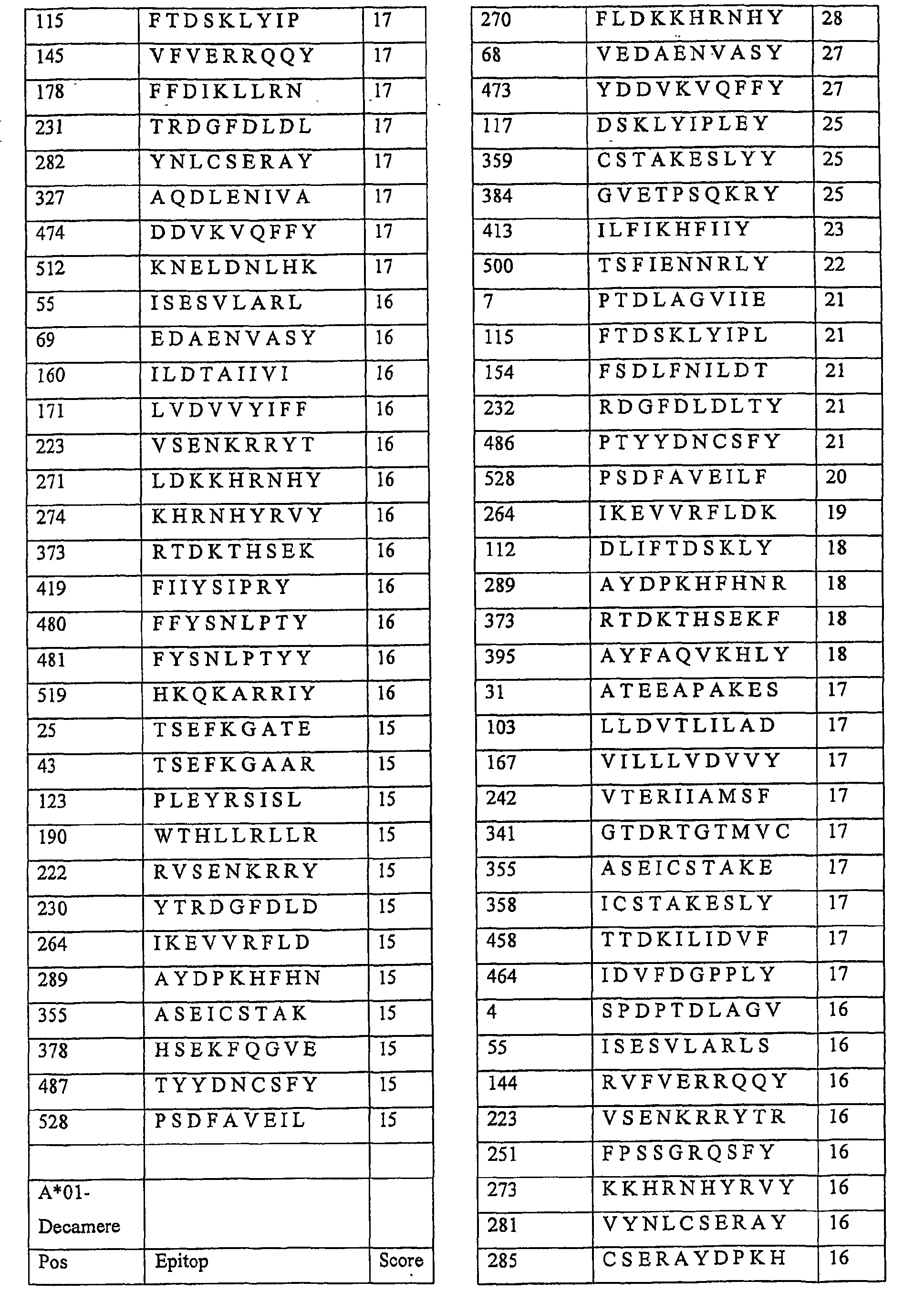

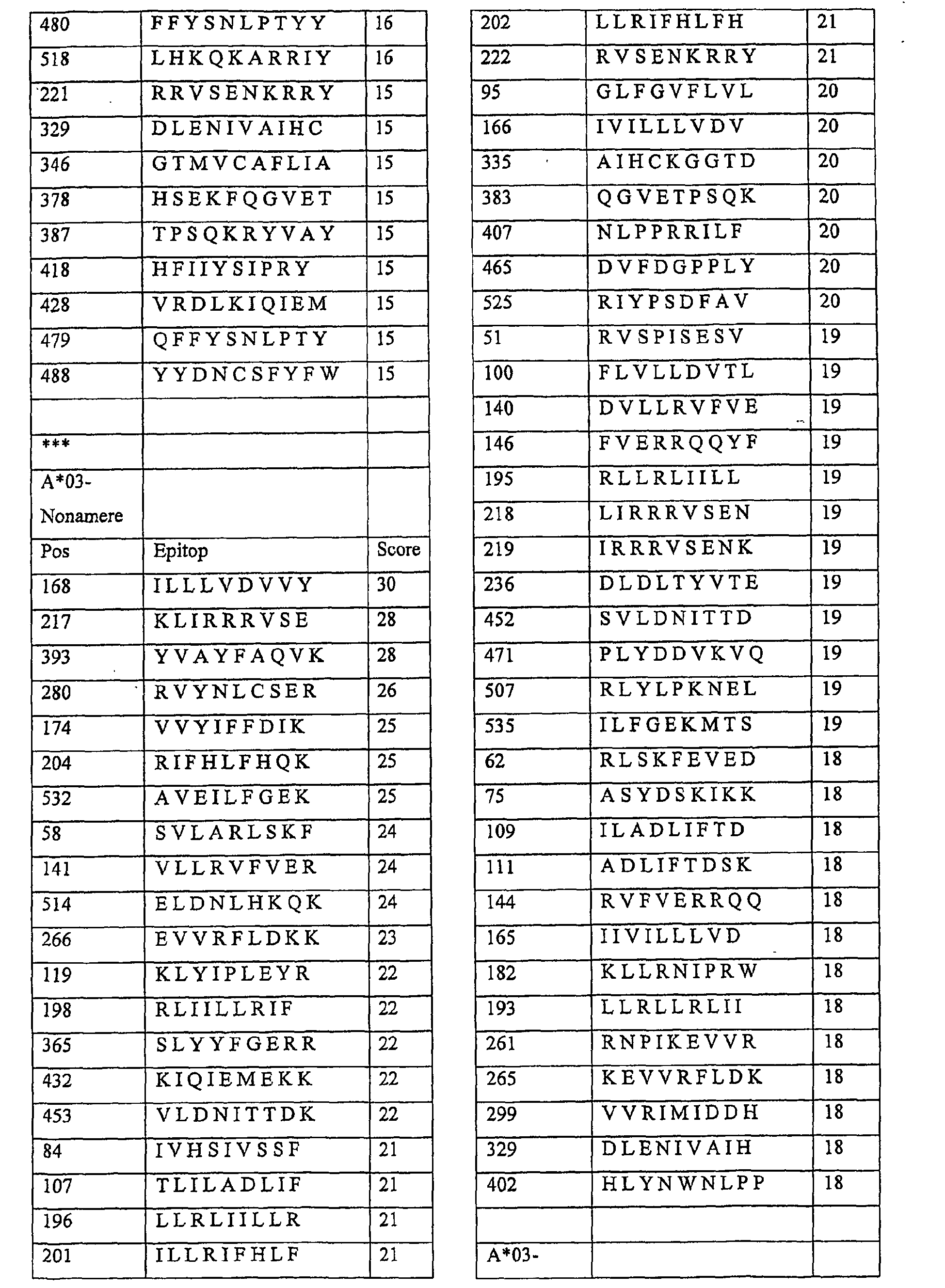

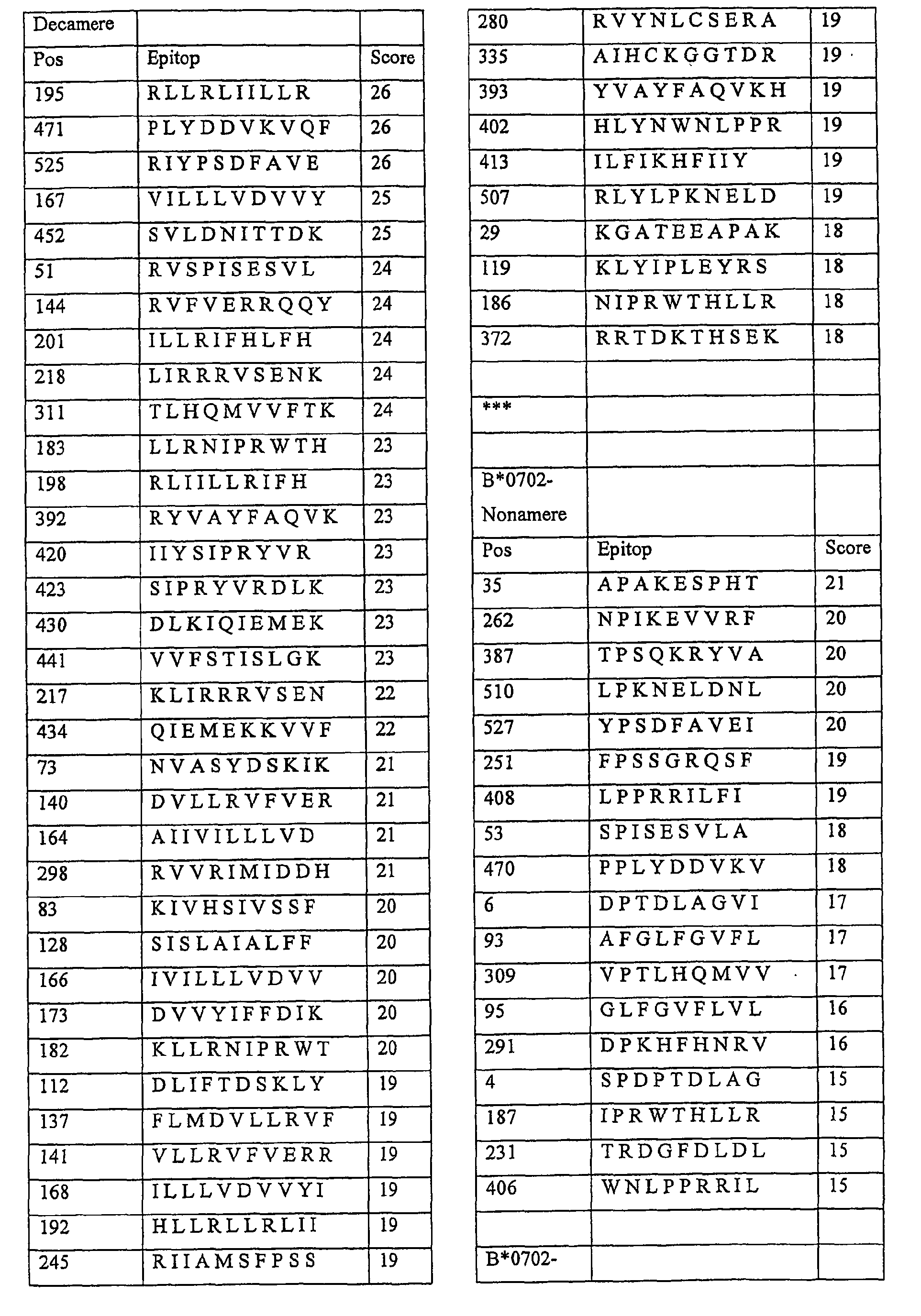

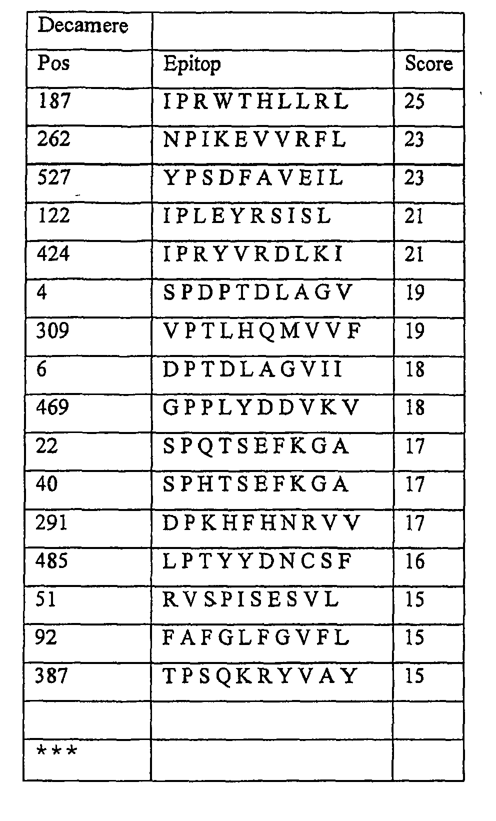

Ein Teil oder ein Fragment einer Nukleinsäure, die für ein Tumor-assoziiertes Antigen kodiert, betrifft erfindungsgemäß den Teil der Nukleinsäure, der zumindest für das Tumorassoziierte Antigen kodiert und/oder für einen Teil oder ein Fragment des Tumor-assoziierten Antigens wie vorstehend definiert kodiert.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch erfolgen durch ein Screening von T-Zellen des Patienten auf Spezifität für das entsprechende tumor-assoziierte Antigen.

Die vorliegende Erfindung ermöglicht auch die Isolierung von Proteinen, die an hier beschriebene Tumor-assoziierte Antigene binden, einschließlich Antikörper und zelluläre Bindepartner der Tumor-assoziierten Antigene.

Erfindungsgemäß werden auch in bestimmten Ausführungsformen "dominant negative" Polypeptide bereitgestellt, die von Tumor-assoziierten Antigenen abgeleitet sind. Ein dominant negatives Polypeptid ist eine inaktive Variante eines Proteins, die durch Interaktion mit der zellulären Maschinerie ein aktives Protein von seiner Interaktion mit der zellulären Maschinerie verdrängt oder mit dem aktiven Protein kompetitiert, wodurch die Wirkung des aktiven Proteins verringert wird. Zum Beispiel kann ein dominant negativer Rezeptor, der einen Liganden bindet, jedoch kein Signal in Reaktion auf die Bindung des Liganden erzeugt, die biologische Wirkung des Liganden verringern. In ähnlicher Weise kann eine dominant negative katalytisch-inaktive Kinase, die normalerweise mit Zielproteinen interagiert, jedoch die Zielproteine nicht phosphoryliert, die Phosphorylierung der Zielproteine in Reaktion auf ein zelluläres Signal verringern. In ähnlicher Weise kann ein dominant negativer Transkriptionsfaktor, der an eine Promotorstelle in der Kontrollregion eines Gens bindet, jedoch die Transkription des Gens nicht erhöht, die Wirkung eines normalen Transkriptionsfaktors durch die Besetzung von Promotorbindestellen ohne eine Erhöhung der Transkription verringern.

Das Ergebnis der Expression eines dominant negativen Polypeptids in einer Zelle ist eine Verringerung der Funktion aktiver Proteine. Der Fachmann kann dominant negative Varianten eines Proteins beispielsweise durch herkömmliche Mutageneseverfahren und Bewerten der dominant negativen Wirkung des Varianten-Polypeptids herstellen.

Erfindungsgemäß umfasst sind auch Stoffe wie Polypeptide, die an Tumor-assoziierte Antigene binden. Solche Bindestoffe können z.B. in Screening-Assays für einen Nachweis von Tumor-assoziierten Antigenen und Komplexen von Tumor-assoziierten Antigenen mit ihren Bindepartnern sowie bei einer Aufreinigung der Tumor-assoziierten Antigene und von Komplexen davon mit ihren Bindepartnern Verwendung finden. Solche Stoffe können auch für eine Hemmung der Aktivität Tumor-assoziierter Antigene beispielsweise durch Bindung an solche Antigene Verwendung finden.

Erfindungsgemäß umfasst sind daher Bindestoffe wie z.B. Antikörper oder Antikörperfragmente, die die Fähigkeit aufweisen, selektiv an Tumor-assoziierte Antigene zu binden. Antikörper umfassen polyklonale und monoklonale Antikörper, die in herkömmlicher Weise hergestellt werden.

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Zum Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Erfindungsgemäß werden auch F(ab')₂-, Fab-, Fv- und Fd-Fragmente von Antikörpern, chimäre Antikörper, bei denen die Fc- und/oder FR- und/oder CDR1- und/oder CDR2-und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden, bereitgestellt. Erfindungsgemäß umfasst sind auch sogenannte einzelkettige Antikörper.

Vorzugsweise ist ein erfindungsgemäß verwendeter Antikörper gegen eine der in SEQ ID NO:14-18, 80-84 oder 101-116 des Sequenzprotokolls dargestellten Sequenzen gerichtet und/oder kann durch Immunisierung mit diesen Peptiden erhalten werden.

Erfindungsgemäß umfasst sind auch Polypeptide, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht-peptidischen synthetischen Resten hergestellt werden.

Phagen-Display kann besonders wirksam bei der Identifizierung erfindungsgemäßer Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "twohybrid-System" aus Hefe kann auch für die Identifizierung von Polypeptiden eingesetzt werden, die an ein Tumor-assoziiertes Antigen binden. Erfindungsgemäß beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluorin-18 und Carbon-11, gamma-Emitter wie Iodin-123, Technitium-99m, Iod-131 und Indium-111, Nuklide für magnetische Kernresonanz wie Fluorin und Gadolinium. Der Begriff "therapeutisch nützlicher Stoff' meint erfindungsgemäß jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabidin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner) beschrieben. Toxine können Proteine wie Pokeweed-antivirales Protein, Choleratoxin, Pertussistoxin, Ricin, Gelonin, Abrin, Diphtherie-Exotoxin oder *Pseudomonas*-Exotoxin sein. Toxinreste können auch Hochenergie-emittierende Radionuklide wie Kobalt-60 sein.

Im Fall von TPTE (vgl. Beispiel 2) konnte erfindungsgemäß gezeigt werden, dass dieses nicht nur ein membranständiges Protein ist, sondern auch zu internalisieren vermag. Daher können die TPTE betreffenden erfingungsgemäß identifizierten Tumor-assoziierten Antigene selbst einem Transport von daran bindenden Substanzen, insbesondere den vorstehend beschriebenen therapeutischen Antikörpern, von der Membran ins Cytoplasma und somit einer Internalisierung dienen, wo diese Substanzen vorzugsweise ihre Wirkung wie eine zellzerstörende Wirkung entfalten können.

Der Begriff "Patient" bedeutet erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "Erkrankung" betrifft erfindungsgemäß jeden pathologischen Zustand, bei dem Tumor-assoziierte Antigene exprimiert oder abnormal exprimiert werden. "Abnormale Expression" bedeutet erfindungsgemäß, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumorassoziierte Antigen nur in Gewebe eines erkrankten Individuums exprimiert, während die Expression bei einem gesunden Individuum reprimiert ist. Ein Beispiel einer solchen Erkrankung ist Krebs, insbesondere Seminome, Melanome, Teratome, Gliome, Kolorektal-, Brust-, Prostata-, Gebärmutter-, Ovarial-, und Lungenkrebs.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Urin, Fäces oder anderen Körperflüssigkeiten.

Der Begriff "immunreaktive Zelle" bedeutet erfindungsgemäß eine Zelle, die in eine Immunzelle (wie B-Zelle, T-Helferzelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht. Die Produktion solcher cytotoxischer T-Lymphozyten *in vitro* ist bekannt. Ein Beispiel für ein Verfahren zur Differenzierung von T-Zellen findet sich in der WO-A-9633265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren den gewünschten Komplex auf ihrer Oberfläche und stimulieren bei einer Kontaktierung mit cytotoxischen T-Lymphozyten deren Vermehrung. Die klonal expandierten autologen cytotoxischen T-Lymphozyten werden sodann an den Patienten verabreicht.

Bei einem anderen Verfahren zur Selektion Antigen-spezifischer cytotoxischer T-Lymphozyten werden fluorogene Tetramere von MHC-Klasse I-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse I-Moleküle werden *in vitro* in Gegenwart von β₂-Mikroglobulin und eines Peptid-Antigens, das an das Klasse I-Molekül bindet, gefaltet. Nach Aufreinigung der MHC/Peptid-Komplexe werden diese mit Biotin markiert. Tetramere werden durch Mischen der biotinylierten Peptid-MHC-Komplexe mit markiertem Avidin (z.B. Phycoerythrin) bei einem molaren Verhältnis von 4:1 gebildet. Tetramere werden sodann mit cytotoxischen T-Lymphozyten wie peripherem Blut oder Lymphknoten in Kontakt gebracht. Die Tetramere binden an cytotoxische T-Lymphozyten, die den Peptid-Antigen/MHC-Klasse I-Komplex erkennen. Zellen, die an die Tetramere gebunden werden, können durch Fluoreszenzgesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen) mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.

Oft lassen sich aus dem T-Zell-Repertoire eines Patienten lediglich niedrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen) mit cytotoxischen T-Lymphozyten von Gesunden kombiniert. Dies führt zu einer Vermehrung hochaffiner spezifischer cytotoxischer T-Lymphozyten, wenn der Spender mit dem spezifischen Komplex bisher keinen Kontakt hatte. Der hochaffine T-Zell-Rezeptor aus diesen vermehrten spezifischen T-Lymphozyten wird kloniert und kann durch Gentransfer z.B. mit retroviralen Vektoren beliebig in T-Zellen von anderen Patienten transduziert werden. Adoptiver Transfer erfolgt dann mit diesen genetisch veränderten T-Lymphozyten (Stanislawski et al., Nat Immunol. 2:962-70, 2001 ; Kessels et al., Nat Immunol. 2:957-61, 2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert werden. Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die erfindungsgemäß anwendbar ist. Cytotoxische T-Lymphozyten können auch *in vivo* in an sich bekannter Weise erzeugt werden. Bei einem Verfahren werden nicht-proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfiziert wurden, die für eine Präsentation des Komplexes notwendig sind (d.h. das Antigene Peptid und das präsentierende HLA-Molekül). Verschiedene Zelltypen können eingesetzt werden. Des weiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhancersequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können nicht-modifizierte extrachromosomale Nukleinsäuren, Plasmide oder virale Genome sein, in die eine Insertion exogener Nukleinsäuren möglich ist. Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des tumorassozüerten Antigenes in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigen-präsentierende Zellen *in vivo* zu ermöglichen. Das tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist. Letzteres ist insbesondere der Fall, wenn diese and HLA-Moleküle binden können. Verabreichungsformen, bei denen das Gesamt-Antigen *in vivo* von einer Dendritischen Zelle prozessiert wird, sind bevorzugt, da hier auch Helfer T-Zell-Antworten entstehen können. Eine effektive Immunantwort benötigt diese (Ossendorp et al, Immunol Lett. 74:75-9, 2000; Ossendorp et al, J. Exp. Med. 187:693-702, 1998). Im allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al, Proc Natl Acad Sci USA 98:3299-303, 2001). Sie kann auch in Kombination mit Reagenzien erfolgen, die eine Aufnahme in Dendritische Zellen erleichtern. Eine in vivo Bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al, J Immunol. 167:787-96, 2001; Appella et al, Biomed Pept Proteins Nucleic Acids 1:177-84, 1995; Wentworth et al, Mol Immunol. 32:603-12, 1995).

Die erfindungsgemäß beschriebenen pharmazeutischen Zusammensetzungen können auch als Vacccinen für die Immunisierung eingesetzt werden. Die Begriffe "Immunisierung" oder "Vaccinierung" bedeuten erfindungsgemäß eine Erhöhung oder Aktivierung einer Immunreaktion gegenüber einem Antigen. Tiermodelle können für ein Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumorassoziierte Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Kreig et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vaccine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Cytokine bei einer Vaccinierung aufgrund ihrer regulatorischen Eigenschaften auf Lymphozyten verwendbar. Solche Cytokine umfassen z.B. Interleukin-12 (IL-12), von dem gezeigt wurde, dass es die schützenden Wirkungen von Vaccinen verstärkt (vgl. Science 268:1432-1434, 1995), GM-CSF und IL-18.

Es gibt eine Reihe von Verbindungen, die eine Immunreaktion verstärken und die daher bei einer Vaccinierung eingesetzt werden können. Diese umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD 152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Natl. Acad Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Th1-Cytokin-Profils in einer T-Zell-Population, was zu einer weiter verstärkten T-Zell-Aktivität führt (Gajewski et al., J. Immuno/. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpern für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nichtprofessionellen Anügen-präsentierenden Zellen (Tumorzellen) präsentiert werden. In diesen Situationen werden T-Helfer- und B7-co-stimulierende Signale nicht bereitgestellt. Dieser Mechanismus könnte im Zusammenhang mit Therapien verwendet werden, die auf Antigengepulsten dendritischen Zellen basieren, oder in Situationen, bei denen T-Helfer-Epitope nicht in bekannten TRA-Vorläufern definiert wurden.

Erfindungsgemäß vorgesehen ist auch eine Verabreichung von Nukleinsäuren, Polypeptiden oder Peptiden. Eine Verabreichung von Polypeptiden und Peptiden kann in an sich bekannter Weise erfolgen. In einer Ausführungsform erfolgt die Verabreichung von Nukleinsäuren durch ex vivo-Verfahren, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Erfindungsgemäß vorgesehen ist auch eine Verabreichung von Nukleinsäuren *in vivo* durch die Verwendung von Vektoren wie Viren und zielgesteuerten Liposomen.

In einer bevorzugten Ausführungsform ist ein viraler Vektor für die Verabreichung einer Nukleinsäure, die für ein Tumor-assoziiertes Antigen kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virusähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um erfindungsgemäß Nukleinsäuren in Zellen in *vitro* oder *in vivo* einzubringen. Solche Verfahren umfassen die Transfektion von Nukleinsäure-CaPO₄-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Bevorzugte Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern und ähnliches.

Die erfindungsgemäßen therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägern, ergänzenden immunitätssteigemden Stoffen wie Adjuvanzien, CpG und Cytokine und gegebenenfalls andere therapeutische Wirkstoffe enthalten.

Die erfindungsgemäßen therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt vorzugsweise durch ein Lungenaerosol. Die Verabreichung von Antisense-Nukleinsäuren erfolgt vorzugsweise durch langsame intravenöse Verabreichung.

Die erfindungsgemäßen Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Krankheit oder eines Zustands kann auch die Verzögerung des Ausbruchs oder eine Verhinderung des Ausbruchs der Krankheit oder des Zustands sein.

Eine wirksame Menge einer erfindungsgemäßen Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die erfindungsgemäßen pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der erfindungsgemäßen Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, vorzugsweise von 10 ng bis 100 µg formuliert und verabreicht. Falls die Verabreichung von Nukleinsäuren (DNA sowie RNA), die für Tumor-assoziierte Antigene kodieren, erwünscht ist, werden Dosen von 1 ng bis 0,1 mg formuliert und verabreicht.

Die erfindungsgemäßen pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nichttoxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine erfmdungsgemäße pharmazeutische Zusammensetzung kann einen pharmazeutisch verträglichen Träger umfassen. Der Begriff "pharmazeutisch verträglicher Träger" betrifft erfindungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der erfindungsgemäßen pharmazeutischen Zusammensetzung sind gewöhnlich derart, dass keine Interaktion auftritt, die die gewünschte pharmazeutische Wirksamkeit wesentlich beeinträchtigt.

Die erfindungsgemäßen pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich, die ebenfalls erfindungsgemäß umfasst sind.

### Abbildungen:

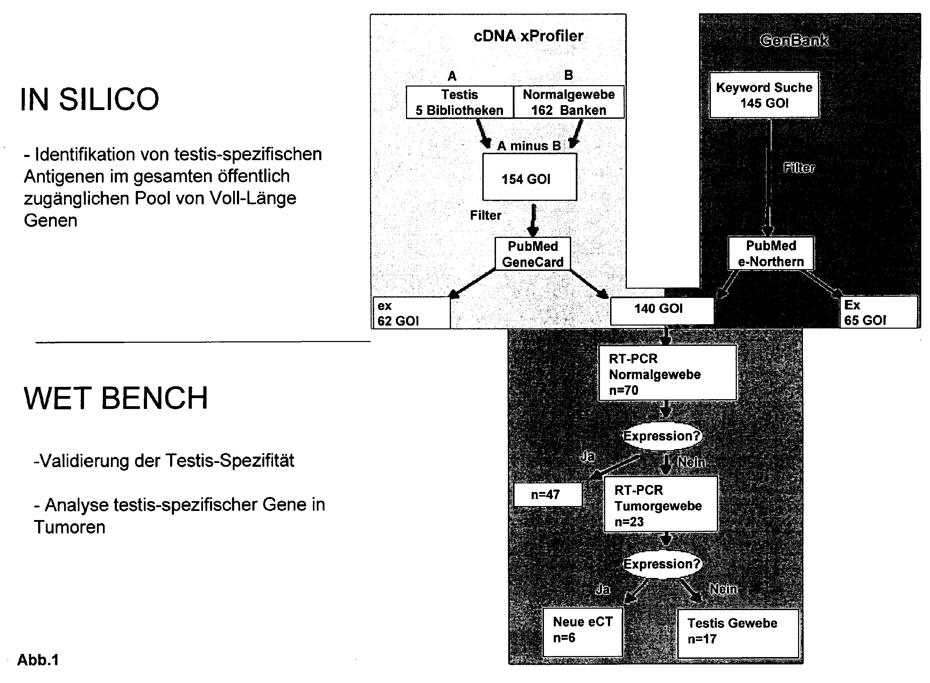

**Abb. 1****: Schematische Darstellung der Klonierung von eCT.** Die Strategie umfasst die Identifizierung von Kandidaten-Genen (GOI = "Genes of interest") in Datenbanken und das Testen dieser Gene durch RT-PCR.

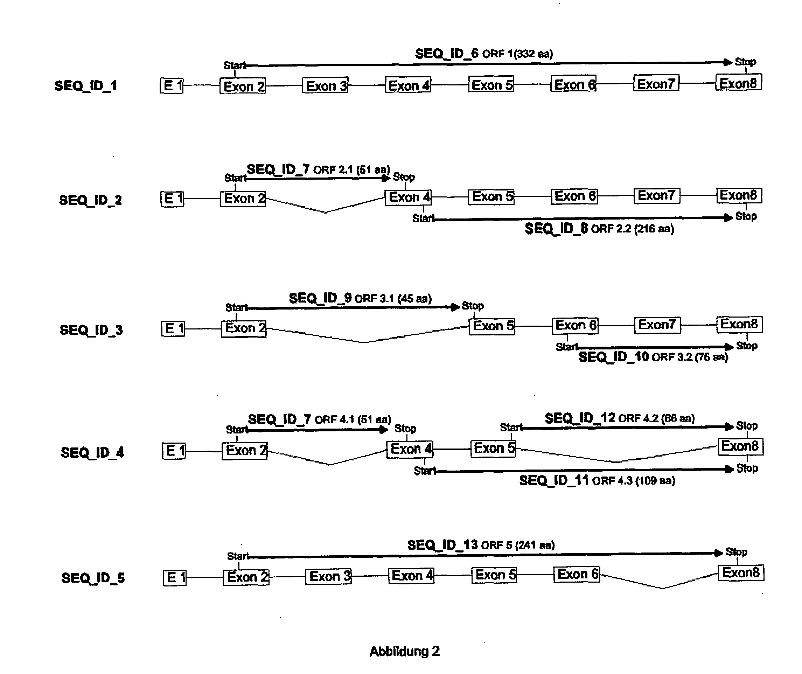

**Abb. 2****: Splicing von LDH C.** Alternative Splicingereignisse führen zum Fehlen von Exon 3 (SEQ ID NO:2), der beiden Exons 3 und 4 (SEQ ID NO:3), der Exons 3, 6 und 7 (SEQ ID NO:4) bzw. von Exon 7 (SEQ ID NO:5). ORF = Offener Leserahmen, aa = Aminosäure.

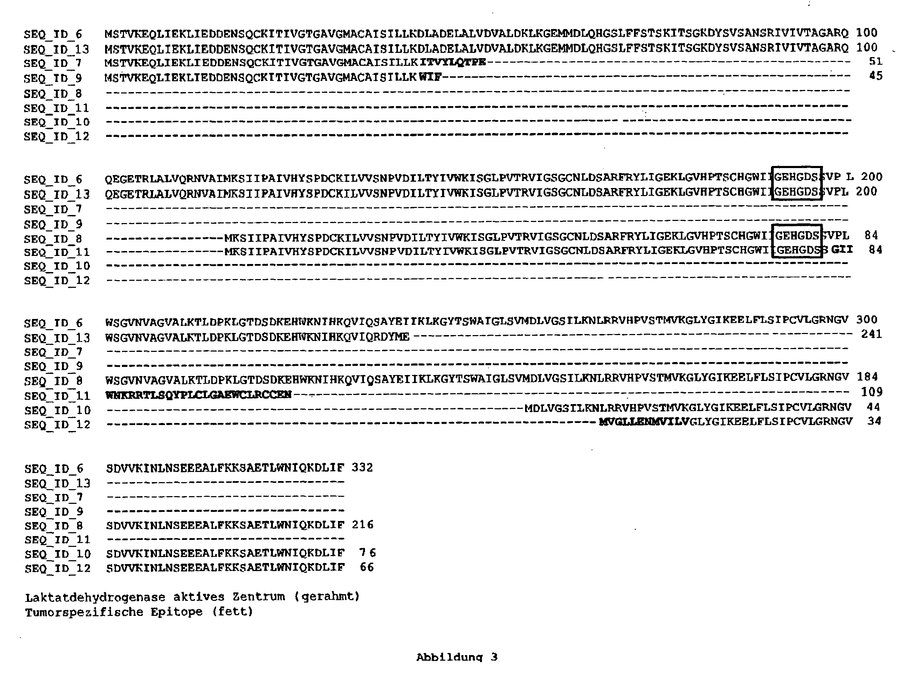

**Abb. 3****: Alignment von möglichen LDH-C-Proteinen.** SEQ ID NO:8 und SEQ ID NO:10 stellen trunkierte Anteile des Prototyp-Proteins (SEQ ID NO:6) dar. Die Proteinsequenzen von SEQ ID NO:7, SEQ ID NO:9, SEQ ID NO:11, SEQ ID NO:12 und SEQ ID NO:13 sind zusätzlich verändert und beinhalten ausschließlich Tumor-spezifische Epitope (fett gedruckt). Das katalytische Zentrum ist gerahmt.

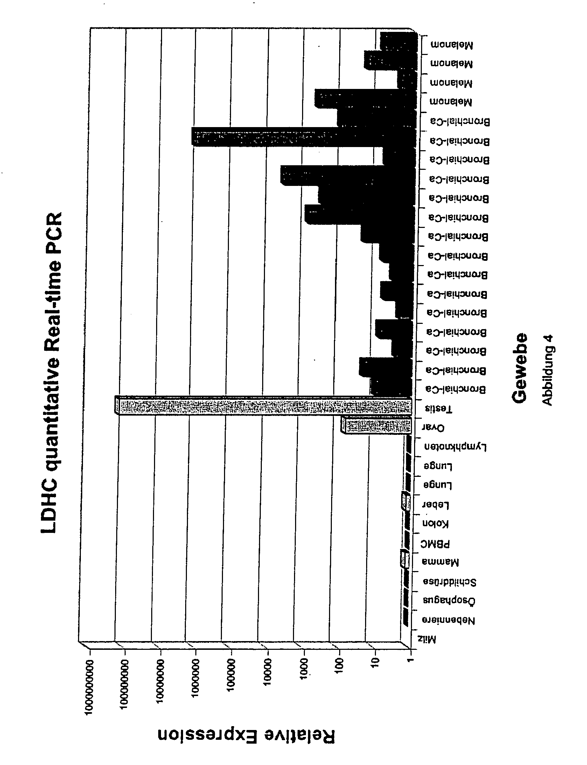

**Abb. 4****: Quantifizierung von LDH C in verschiedenen Geweben durch Real-Time-PCR.** Es wurden keine Transkripte in Normalgeweben außer Testis, allerdings signifikante Expressionslevel in Tumoren nachgewiesen.

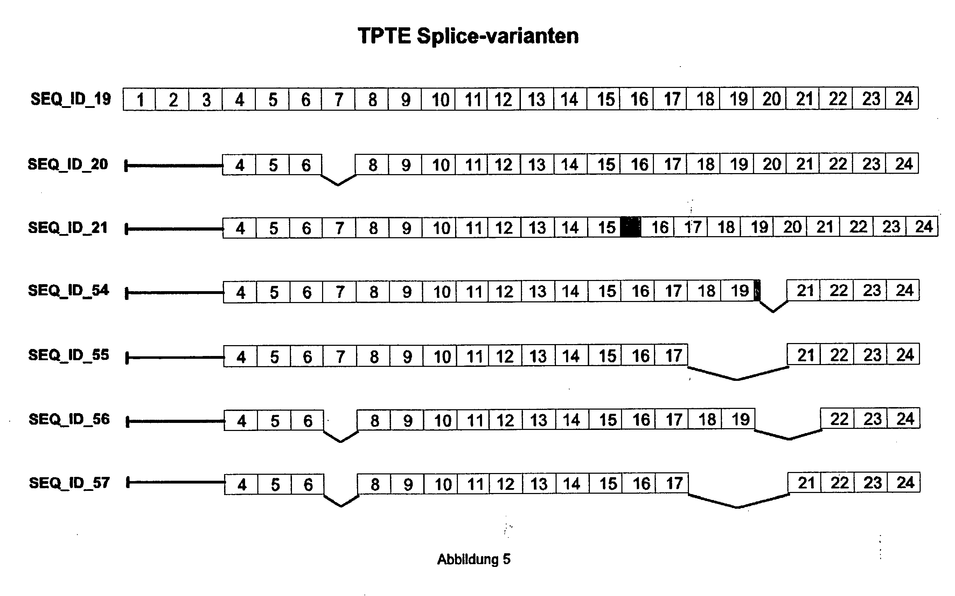

**Abb. 5****: Exon-Zusammensetzung von TPTE-Varianten.** Erfindungsgemäß wurden Splicevarianten identifiziert (SEQ ID NO:20, SEQ ID NO:21, SEQ ID NO:54, SEQ ID NO:55, SEQ ID NO:56, SEQ ID NO:57), die in testikulärem Gewebe und Tumoren exprimiert werden und Leserasterverschiebungen und somit veränderte Sequenzbereiche aufweisen.

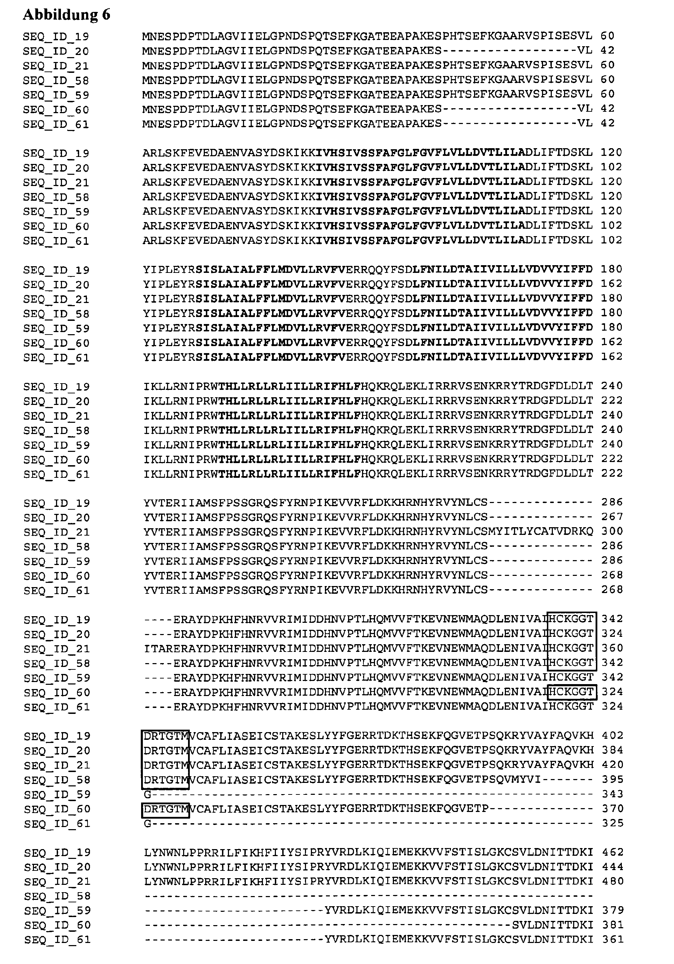

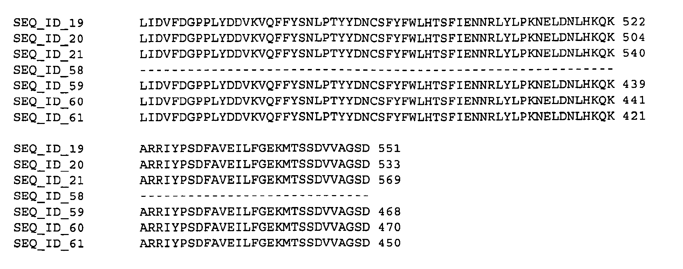

**Abb. 6****: Alignment der möglichen TPTE-Proteine.** Alternative Splicing-Ereignisse resultieren in Veränderungen des kodierten Proteins, wobei das Leseraster grundsätzlich erhalten bleibt. Die putativen transmembrandomänen sind fettgedruckt, die katalytische Domäne ist eingerahmt.

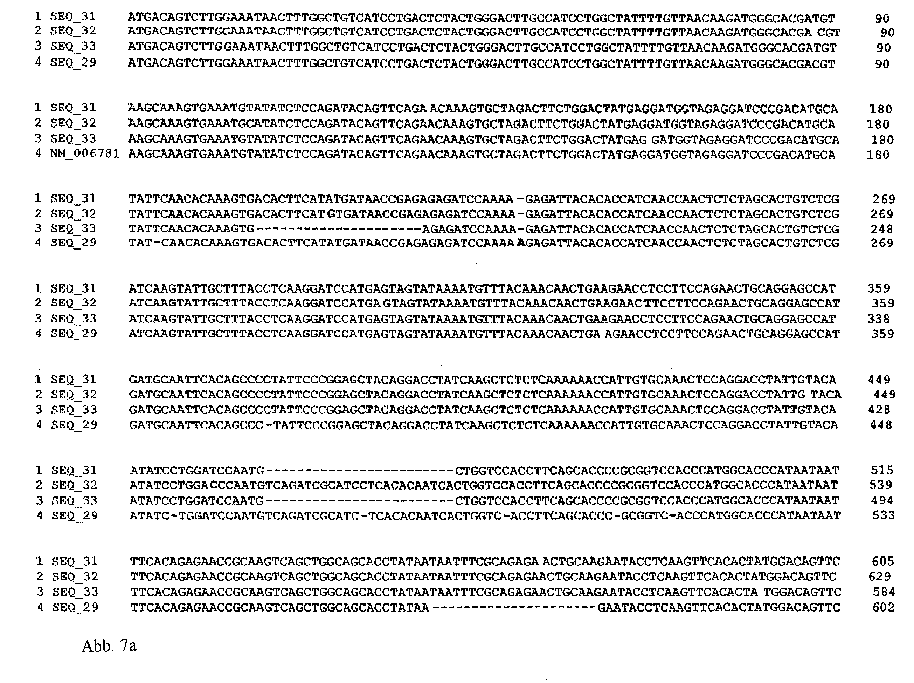

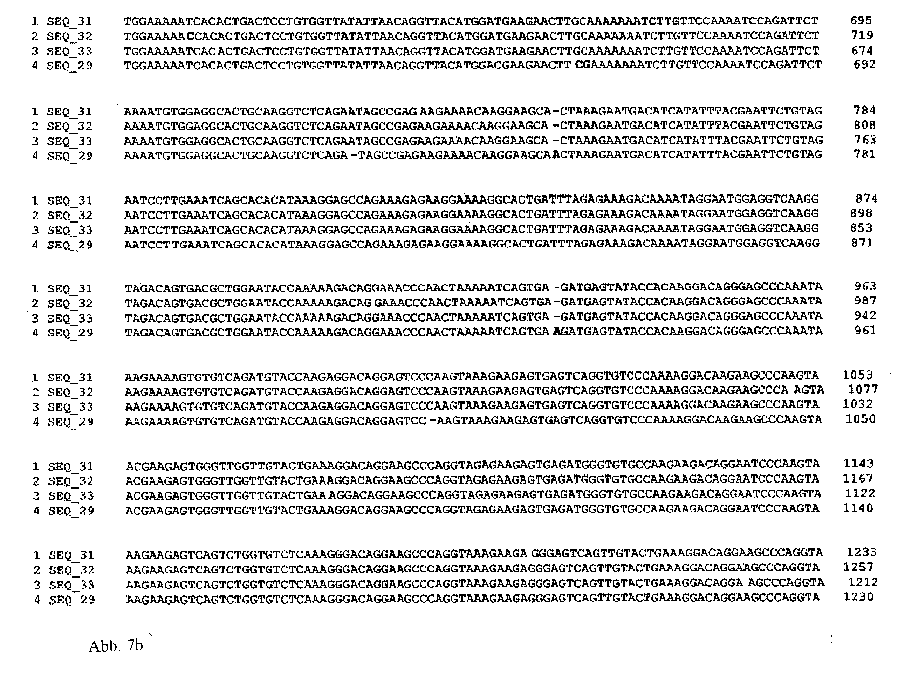

**Abb. 7****: Alignment von TSBP-Varianten auf Nukleotidebene.** Die Unterschiede in den Nukleotidsequenzen der erfindungsgemäß aufgefundenen TSBP-Varianten (SEQ ID NO:31, SEQ ID NO:32, SEQ ID NO:33) zu der bekannten Sequenz (NM_006781, SEQ ID NO:29) sind fett gedruckt.

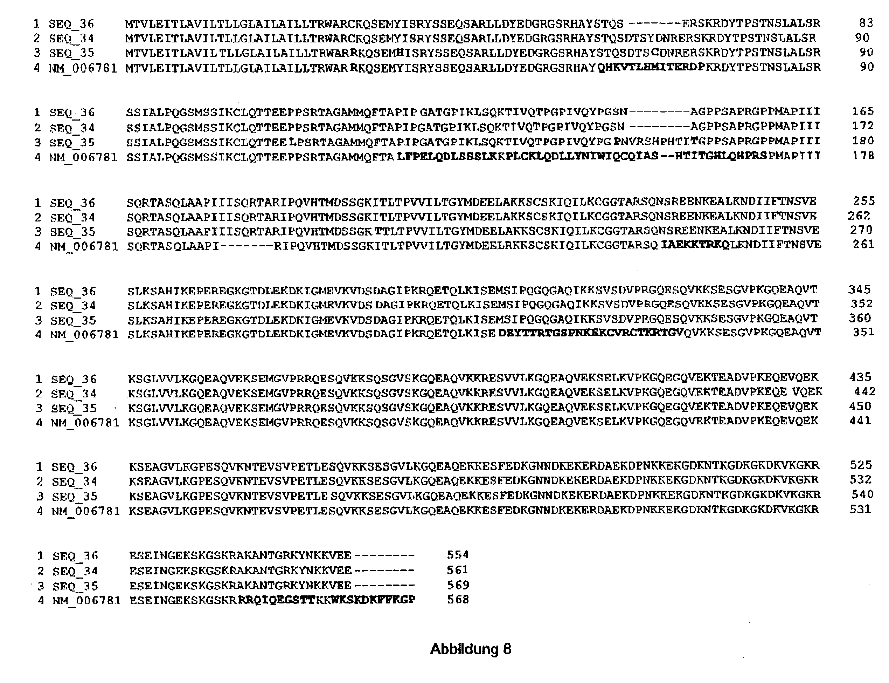

**Abb. 8****: Alignment von TSBP-Varianten auf Proteinebene.** Leserasterverschiebungen führen bei den Proteinen, die durch die erfindungsgemäß aufgefundenen TSBP-Varianten kodiert werden (SEQ ID NO:34, SEQ ID NO:35, SEQ ID NO:36), zu substantiellen Unterschieden zu dem vorbeschriebenen Protein (SEQ ID NO:30, NM_006781) und sind durch Fettdruck gekennzeichnet.

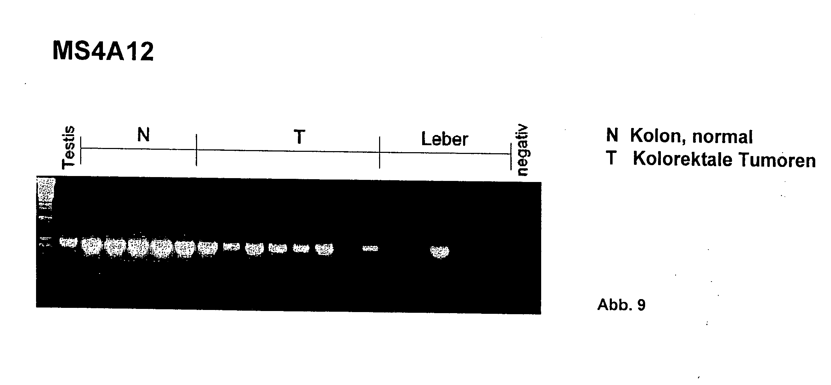

**Abb. 9****: RT-PCR für MS4A12.** In den getesteten Geweben konnte eine Expression nur in Testis, Kolon und kolorektalen Karzinomen (Kolon-Ca's) nachgewiesen werden. Von den gezeigten 6 Lebergewebsproben wurde in einer ein positiver Nachweis für MS4A12 geführt, da diese infiltriert ist durch eine Metastase eines Kolonkarzinoms. Spätere Untersuchungen zeigten auch eine deutliche Expression In Kolonkarzinim-Metastasen

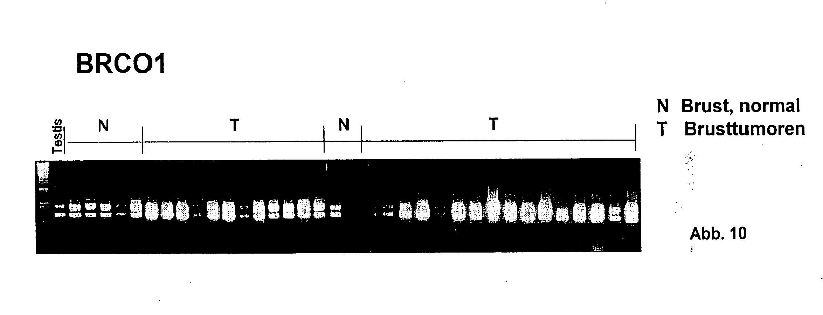

**Abb. 10****: RT-PCR für BRCO1.** In Brusttumoren ist BRCO1 im Vergleich zu der Expression in normalem Brustdrüsengewebe deutlich überexprimiert.

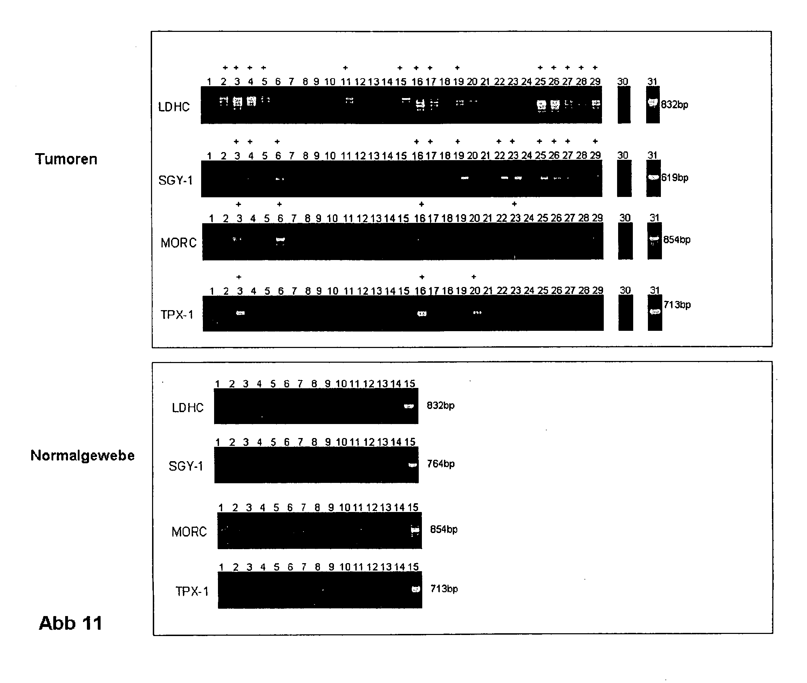

**Abb 11: RT-PCR für MORC, TPX1, LDHC, SGY-1.** Untersuchung verschiedener Normalgewebe zeigt Expression lediglich in Testis (1 Haut, 2 Dünndarm, 3 Kolon, 4 Leber, 5 Lunge, 6 Magen, 7 Brust, 8 Niere, 9 Ovar, 10 Prostata, 11 Schilddrüse, 12 Leukozyten, 13 Thymus, 14 Negativ-Kontrolle, 15 Testis).Die Untersuchung von Tumoren (1-17 Lungentumoren, 18-29 Melanome, 30 Negativ-Kontrolle, 31 Testis) zeigt ektope Expression in diesen in unterschiedlichen Frequenzen für die einzelnen eCT.

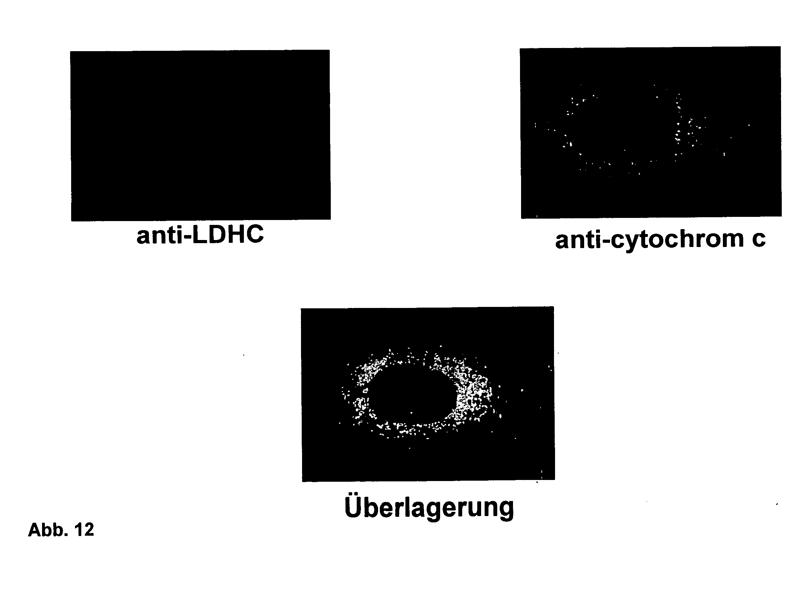

**Abb 12: Mitochondriale Lokalisation von LDHC in der MCF-7 Brustkrebs-Zelllinie.** MCF-7 Zellen wurden transient mit einem LDHC-Expressions-Plasmid transfiziert. Das Antigen wurde mit LDHC-spezifischen Antikörpern detektiert und zeigte eine deutliche Kolokalisation mit dem mitochondrialen Atmungsketten-Enzym Cytochome C-Oxidase.

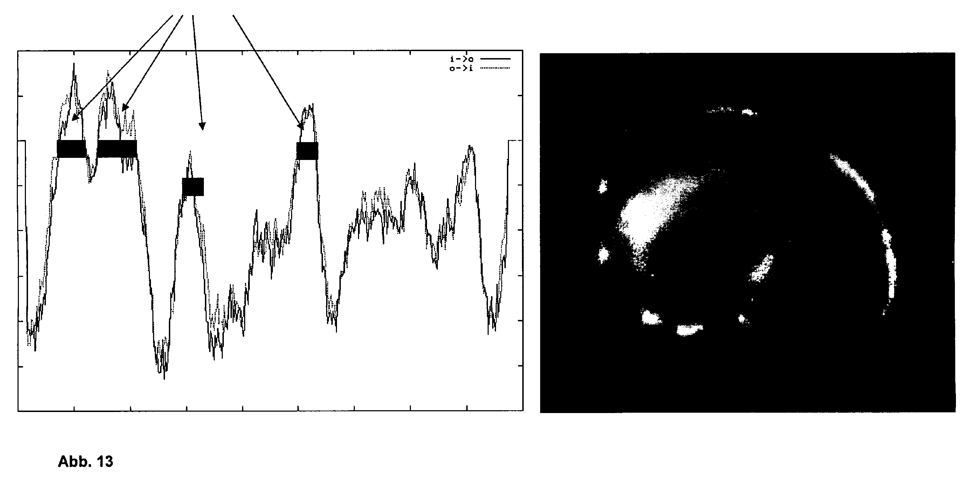

**Abb 13: Prädizierte Topologie von TPTE und subzelluläre Lokalisation an der Zelloberfläche von MCF-7 Zellen**. Das linke Schema zeigt die 4 putativen Trabsmembran-Domänen (Pfeile) von TPTE. MCF-7 Zellen wurden transient mit einem TPTE-Expressions-Plasmid transfiziert. Das Antigen wurde mit TPTE-spezifischen Antikörpern detektiert und zeigte eine deutliche Kolokalisation mit an der Zelloberfläche lokalisierten MHC I Molekülen.

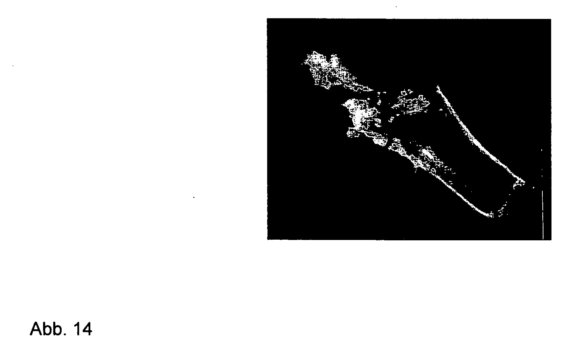

**Abb. 14****: MS4A12 Lokalisation an der Zellmembran.** Tumorzellen wurden transient mit einem GFP-getaggten MS4A12-Konstrukt transfiziert und zeigten in der konfokalen Immunfluoreszenz-Mikrskopie eine komplette Kolokalisation mit Plamamembranmarkern.

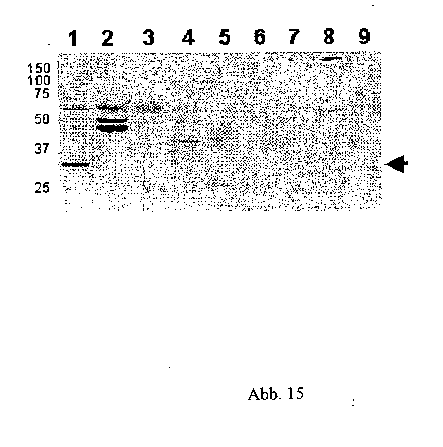

**Abb. 15****: Western Blot Nachweis von LDHC in Normalgeweben.** Die Expression von LDHC ist nur in Testis nachweisbar, alle anderen getesteten Normalgewebe sind negativ. 1-Testis, Normalgewebe, 2-Hautnormalgewebe, 3-Mammanormalgewebe, 4-Lebemormalgewebe, 5-Milznormalgewebe, 6-Kolonnormalgewebe, 7-Lungenormalgewebe, 8-Nierenormalgewebe, 9-Lymphknotennormalgewebe

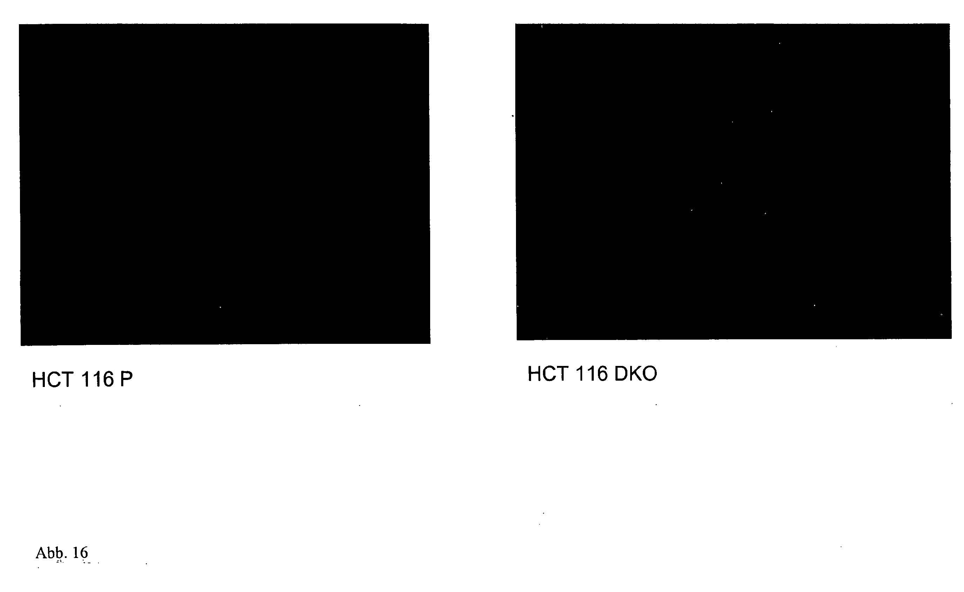

**Abb. 16****: Expression von LDHC in der HCT116 DKO Zellinie.** HCT116 P und HCT116 DKO wurden mit einem LDHC-spezifischen Antikörper gefärbt. Endogene LDHC ist nur in HCT116 DKO Zellen nachweisbar.

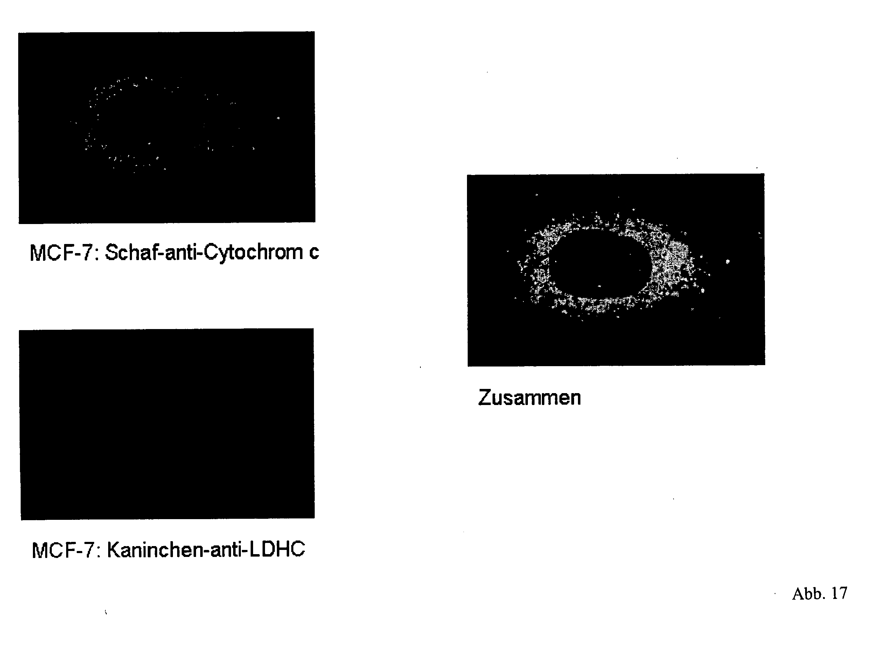

**Abb. 17****: Mitochondriale Lokalisation von LDHC in der MCF-7 Brustkrebs-Zelllinie.** MCF-7 Zellen wurden transient mit einem LDHC-Expressions-Plasmid transfiziert. Das Antigen wurde mit LDHC-spezifischen Antikörpern detektiert und zeigte eine deutliche Kolokalisation mit dem mitochondrialen Atmungsketten-Enzym Cytochome C-Oxidase.

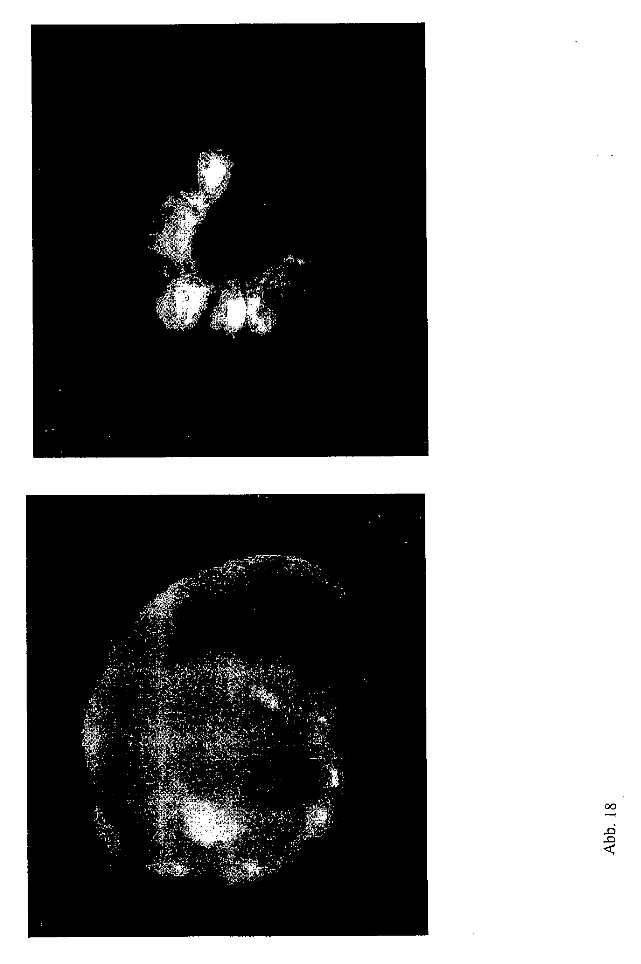

**Abb. 18****: Membranlokalisation von heterolog und endogen exprimierter TPTE in Zellinien.** (Links) NIH3T3 Zellen wurden transient mit einem TPTE-Expressions-Plasmid transfiziert. TPTE wurde mit spezifischen Antikörpern detektiert und zeigte eine deutliche Kolokalisation mit an der Zelloberfläche lokalisierten MHC I Molekülen. (Rechts) Endogene TPTE in SK-Mel 37 Zellen wurde mit einem spezifischen Antikörper detektiert und zeigt deutliche Membranlokalisation.

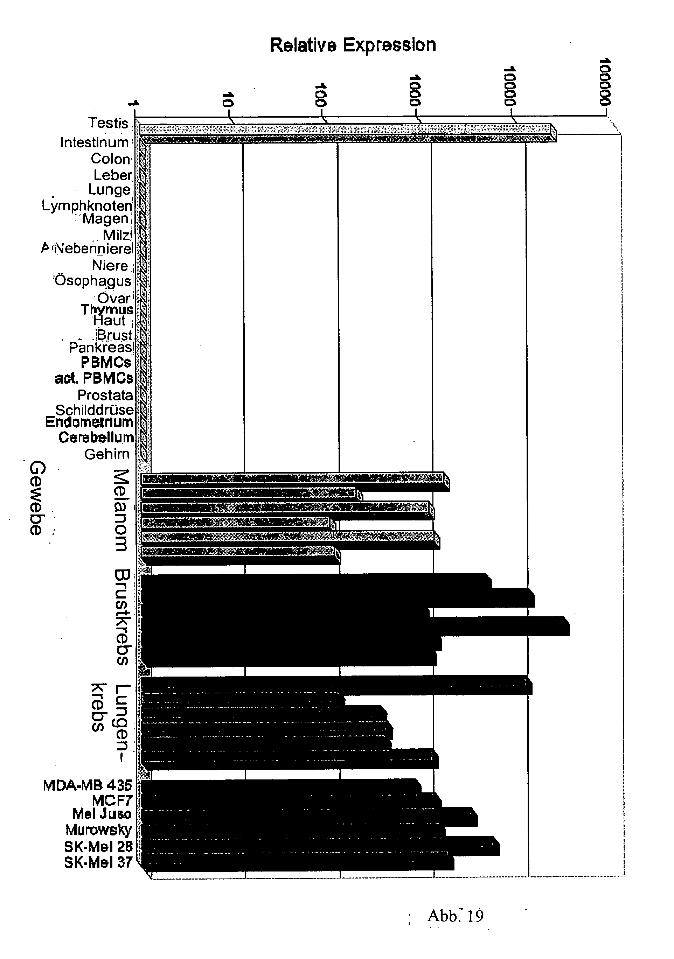

**Abb. 19****: Quantifizierung der TPTE mRNA Expression in verschiedenen Geweben durch Real-Time PCR.** Expression in Normalgeweben ist nur in Testis nachweisbar. Signifikante Expressionslevel finden sich auch in Tumoren und Tumorzellinien.

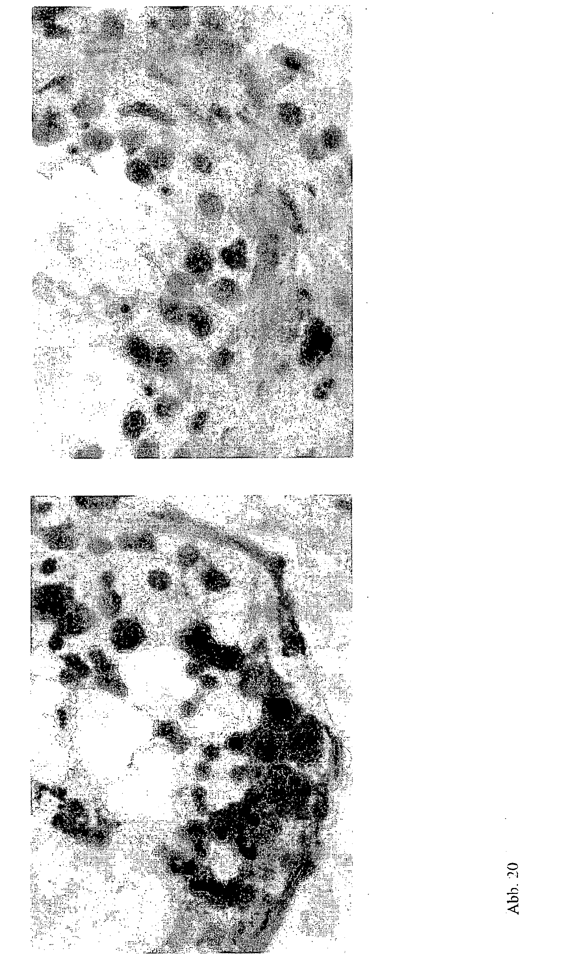

**Abb. 20****: Nachweis der Antiköper-Spezifität.** Schnitte von Testisgewebe wurden mit TPTE-spezifischem Antikörper gefärbt. Der spezifische Nachweis von TPTE wird durch Blocken (rechts) des Antikörpers mit dem zur Immunisierung eingesetzten Peptid verhindert.

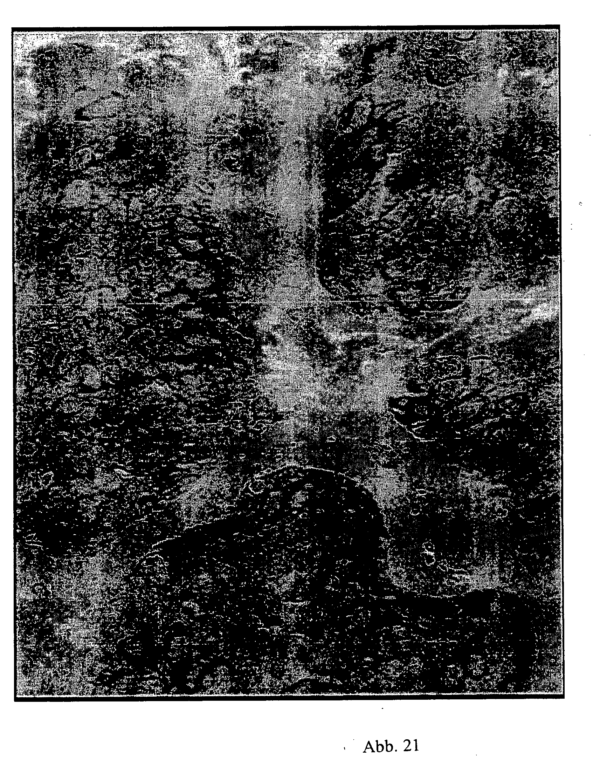

**Abb. 21****: Immunhistochemischer Nachweis von TPTE in Bronchialkarzinomen.** Schnitte von Bronchialkarzinomen wurden mit TPTE-spezifischem Antikörper gefärbt. TPTE wird homogen im gesamten Tumor exprimiert und ist an der Zellmembran lokalisiert.

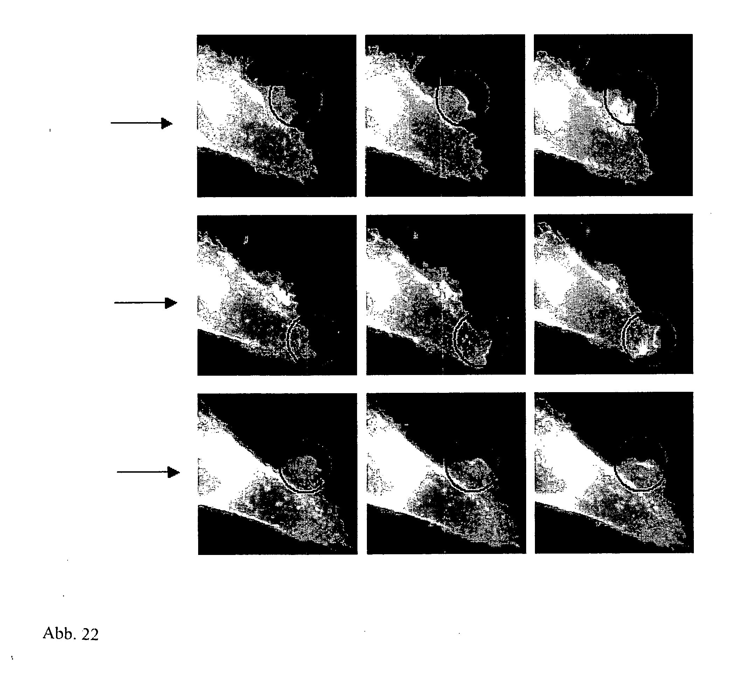

**Abb. 22****: Nachweis der TPTE-Lokalisation in vitalen Zellen.** NIH3T3 Zellen wurden transient mit einem TPTE-Expressions-Plasmid transfiziert und mittels Time-Lapse Mikroskopie analysiert. Die Lokalisation von TPTE an die Membran von Zellprotrusionen und Pseudoposien hat die unmittelbare Retraktion der betreffenden Membranbereiche zur Folge.

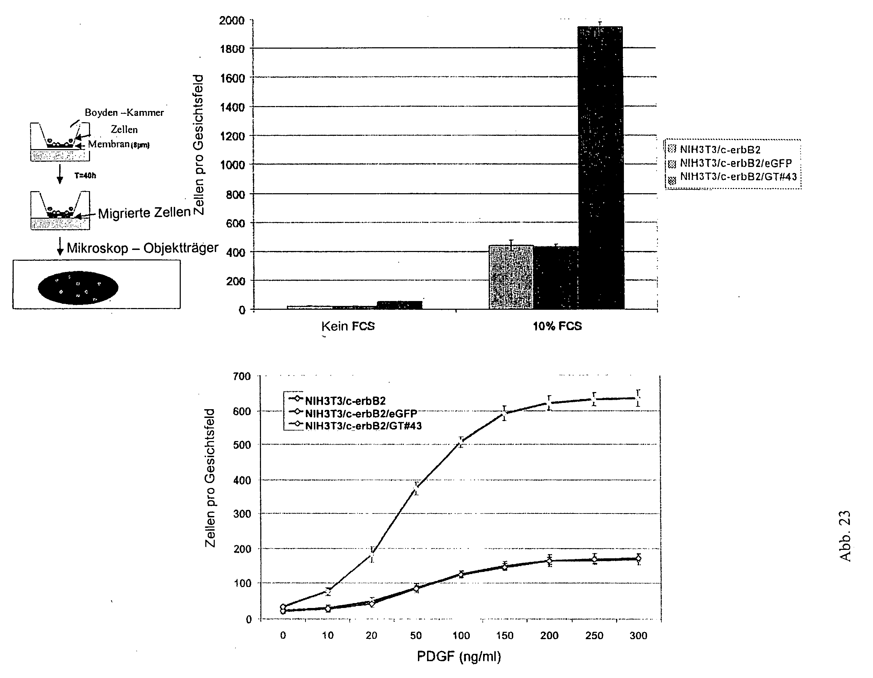

**Abb. 23****: TPTE verstärkt Zellmigration in chemotaktischen Gradienten.** (Links) Schematische Darstellung des Boyden-Kammer Assays. NIH3T3/c-erbB-2 Zellen wurden mit TPTE-eGFP transfiziert und die Migration der Zellen im Vergleich mit nicht-transfizierten und mit Leer-pEGFP-Vektor transfizierten Zellen bestimmt. (Rechts oben) Die Expression von TPTE führt zu einer um den Faktor 4-5 erhöhten Migration der Zellen bei Einsatz von 10%FCS als Chemotaktikum. (Recht unten) Die Expression von TPTE führt schon bei sehr geringen Konzentrationen von PDGF zu einer signifikanten Steigerung der Migration.

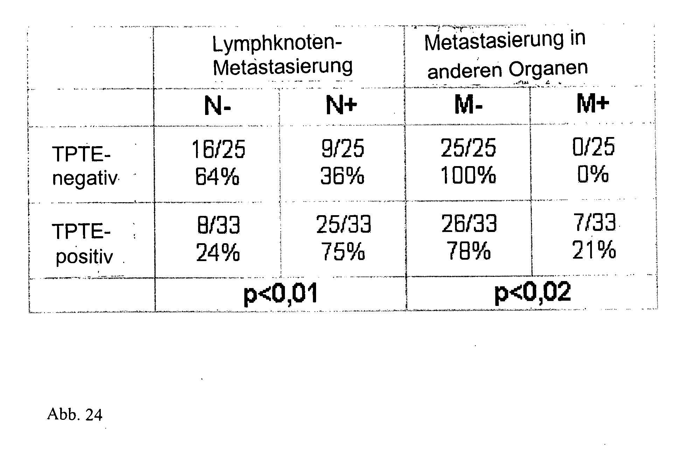

**Abb. 24****: Expression TPTE in Tumoren ist assoziiert mit Metastasierung.** Statistische Auswertung der TNM-Stadien von 58 Tumorproben in Korrelation mit der Expression von TPTE zeigt, dass TPTE positive Tumoren signifankt häufiger lymphogen und hämatogen metastasieren.

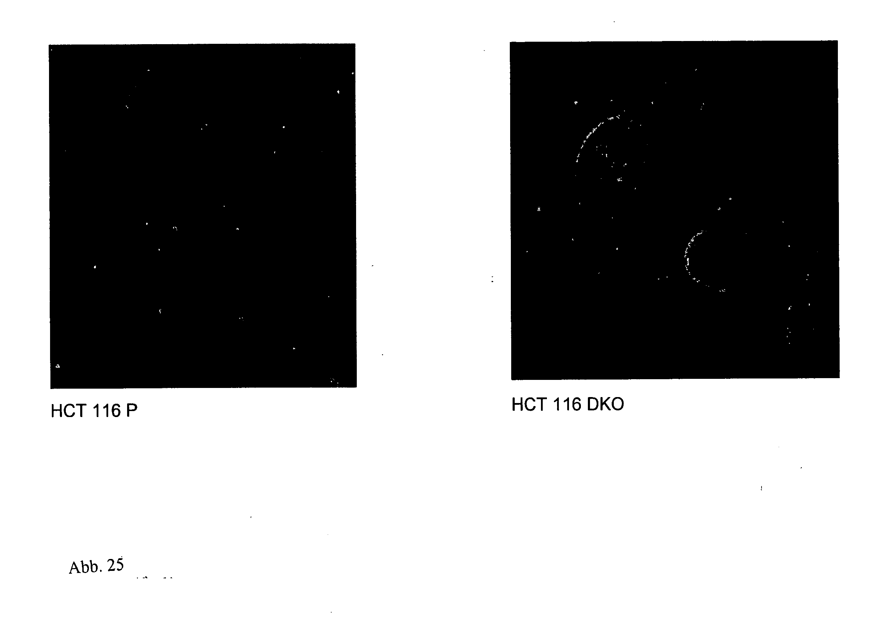

**Abb. 25****: Expression von TSBP in der HCT116 DKO Zellinie.** HCT116 P und HCT116 DKO wurden mit einem TSBP-spezifischen Antikörper gefärbt. Endogenes TSBP ist nur in HCT116 DKO Zellen nachweisbar und ist mit der Kernmembran assoziiert.

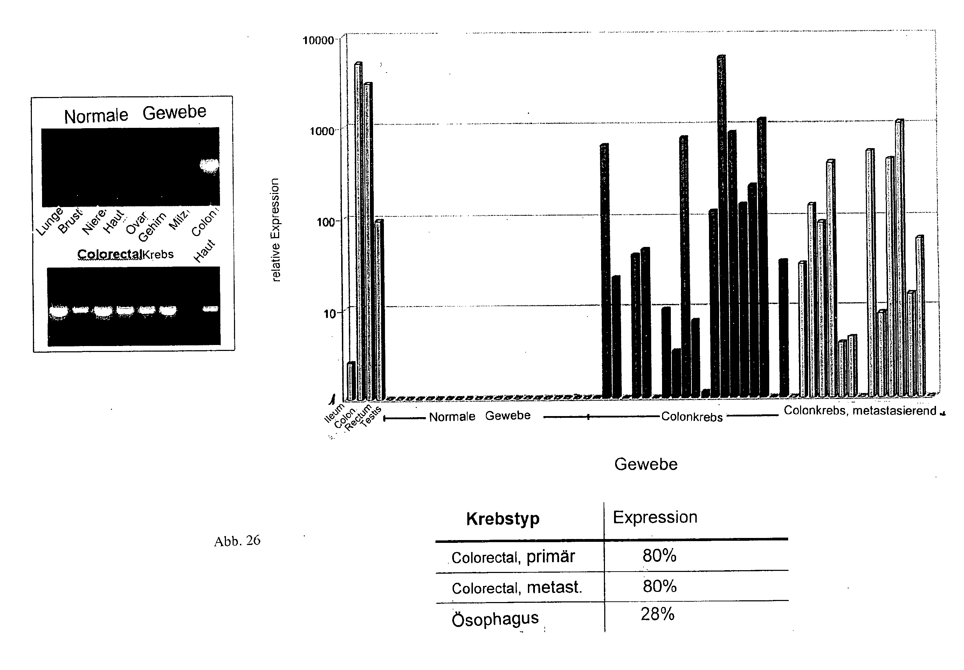

**Abb. 26****: Nachweis der MS4A12 mRNA Expression in Geweben durch RT-PCR und Real-Time** PCR. Die Expression von MS4A12 in Normalgeweben ist beschränkt auf Kolon, Rektum, terminales Ileum und Testis. 80% der Kolonkarzinome und 80% der Kolonkarzinommetastasen zeigen signifikante Expressionslevel von MS4A12.

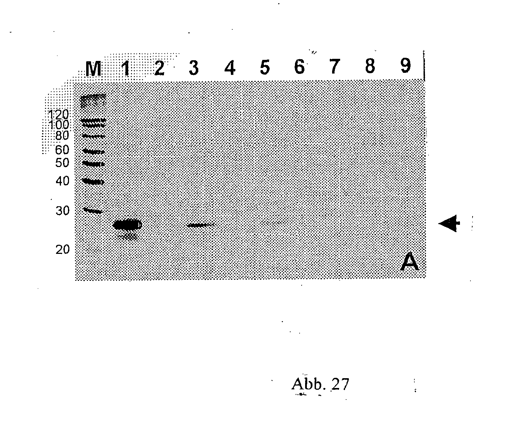

**Abb. 27****: Western Blot Nachweis von MS4A12 in Kolon und Kolonkarzinomen.** Die spezifische Bande ist normalem Kolongewebe und in einigen Kolonkarzinomen nachweisbar. M-MagicMark (Invitrogen), 1-Kolonnormalgewebe, 2-6-Kolontumorgewebe

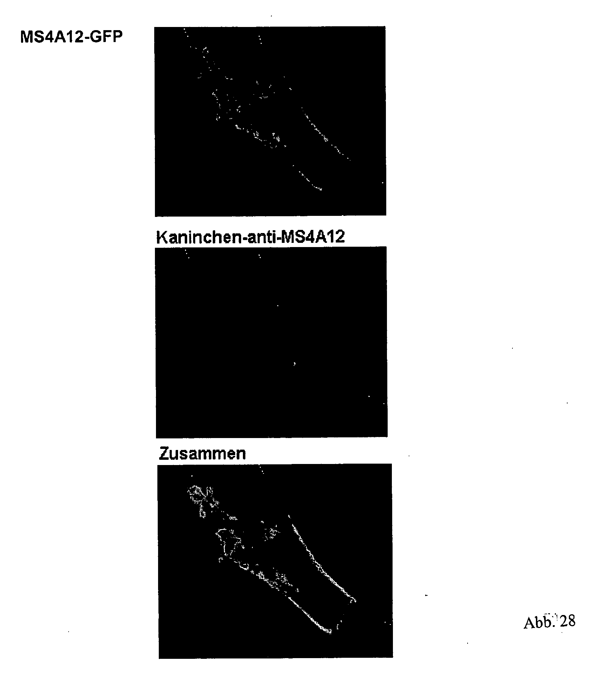

**Abb. 28****: MS4A12 Lokalisation an der Zellmembran.** Tumorzellen wurden transient mit MS4A12-eGFP transfiziert und zeigen nach Färbung mit einem MS4A12-spezifischen Antikörper in der konfokalen Immunfluoreszenz-Mikroskopie Lokalisation an der Plasmamembran.

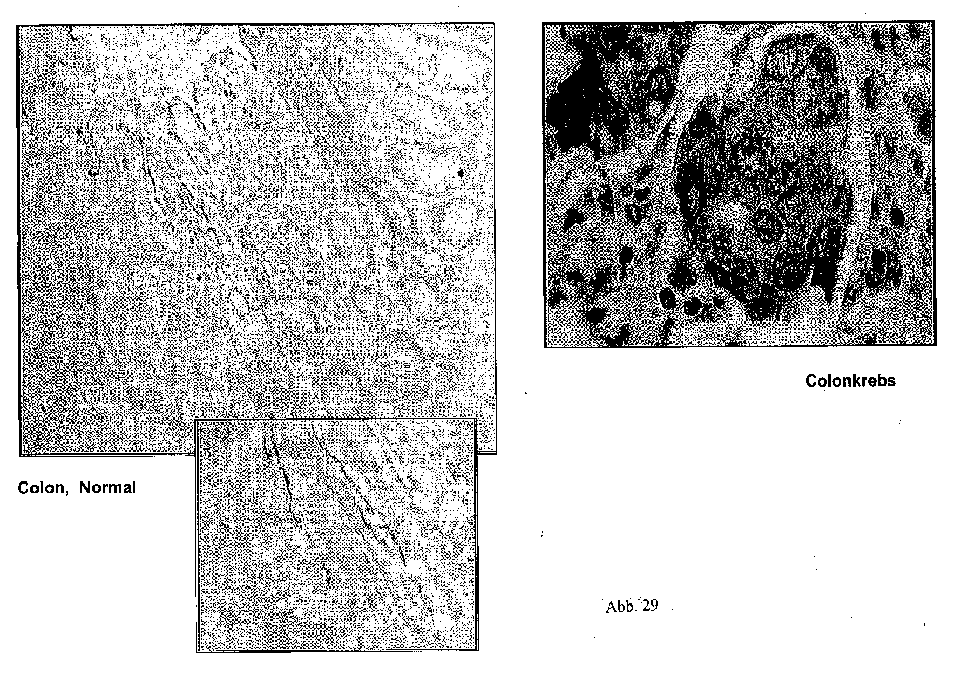

**Abb. 29****: Immunhistochemischer Nachweis von MS4A12 in Kolon und Kolonkarzinomen.** Gewebeschnitte wurden mit einem MS4A12-spezifischen Antikörper gefärbt. In normalem Kolon wird MS4A12 nur in den apikalen Enterozyten exprimiert. In Kolonkarzinomen ist die die Expression von MS4A12 in allen Tumorzellen nachweisbar.

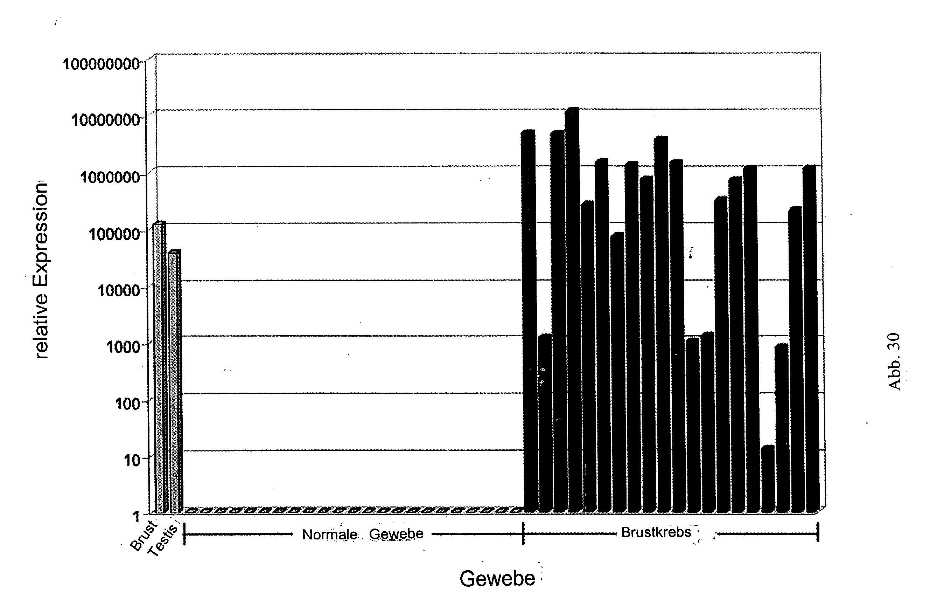

**Abb. 30****: Quantifizierung der BRCO1 mRNA Expression in verschiedenen Geweben durch Real-Time PCR.** Die Expression von BRCO1 in Normalgeweben ist beschränkt auf Brust und Testis. Signifikante Expressionlevel von BRCO1 sind in allen untersuchten Mammakarzinomen nachweisbar. 50% der Tumoren zeigen eine Überexpression von BRCO1 im Vergleich mit exprimierenden Normalgeweben.

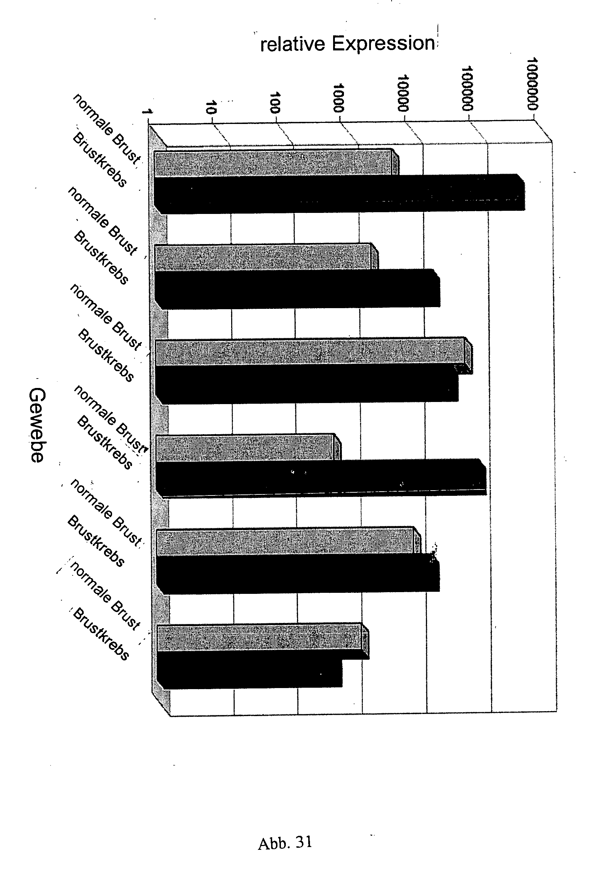

**Abb. 31****: Überexpression von BRCO1 in Mammakarzinomen.** Real-Time PCR Untersuchung von BRCO1 in Mammakarzinomen und dem adjazenten Normalgeweben zeigt eine Überexpression von BRCO1 in 50% der Mammakarzinome.

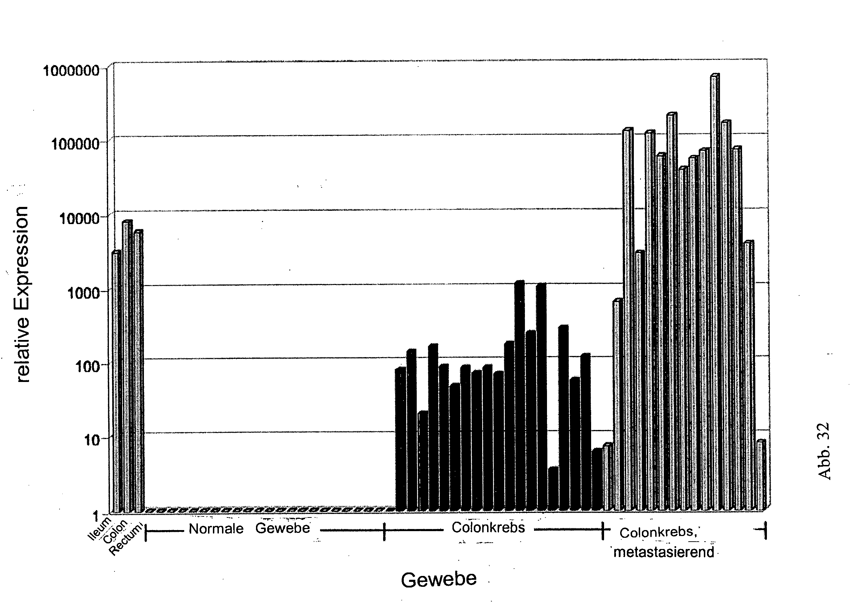

**Abb. 32****: Quantifizierung der PCSC mRNA Expression in verschiedenen Geweben durch Real-Time PCR.** Die Expression von PCSC in Normalgeweben ist beschränkt auf Kolon, Rektum und terminales Ileum. Expression von PCSC ist in allen Kolonkarzinomen nachweisbar. 85% der Kolonkarzinommetastasen zeigen eine signifikante Überexpression von PCSC im Vergleich mit den Primärtumoren.

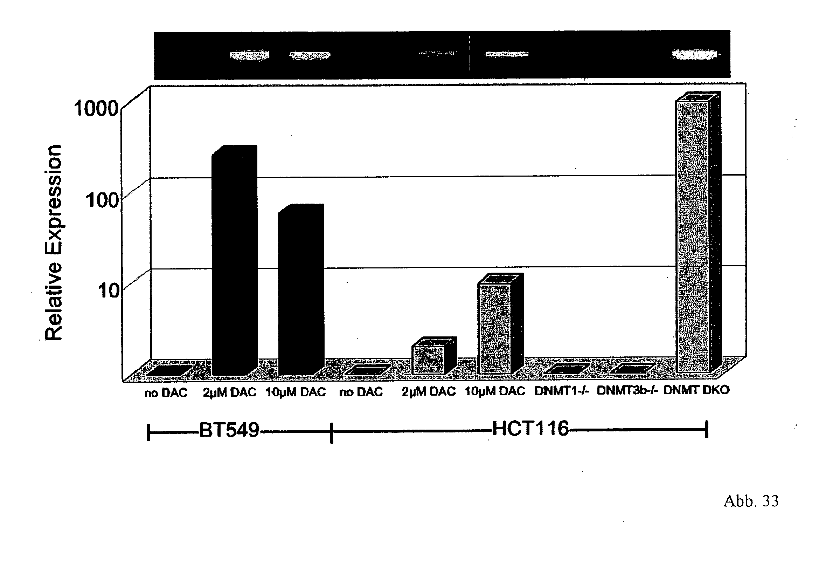

**Abb. 33****: Nachweis der Induzierbarkeit von TPTE durch genomische Demethylierung.** Real-Time RT-PCR Analyse der TPTE-Expression in nicht-exprimierenden Zellinien BT549 und HCT116 nach Behandlung der Zellen mit 2µM bzw. 10µM 5-Aza-2'-deoxycytidin und in DNA-Methyltransferase defizienten HCT116 Zellen.

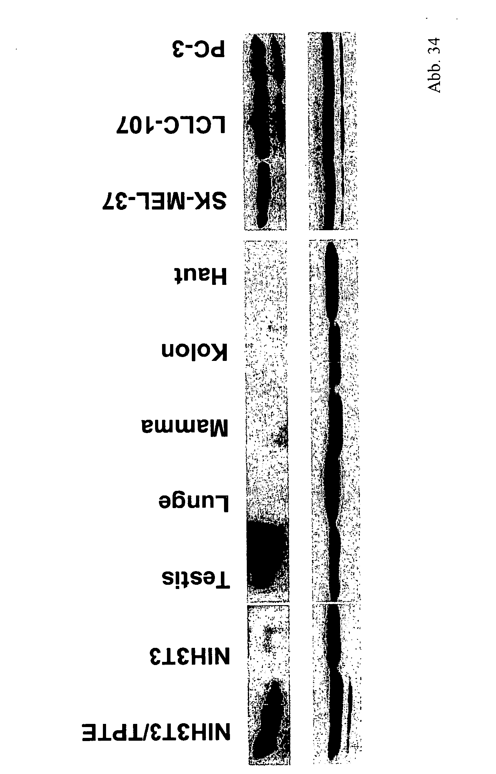

**Abb. 34****: Western Blot Nachweis von TPTE in Normalgeweben und Tumorzellinien.** Die Expression von LDHC ist nur in Testis und Tumorzellinien (SK-Mel-37, LCLC-107, PC-3) nachweisbar, alle anderen getesteten Normalgewebe sind negativ. Die Spezifität des Antikörpers wird durch den Nachweis von TPTE in transfizierten NIH3T3 Zellen (TPTEpcDNA3.1) bestätigt.

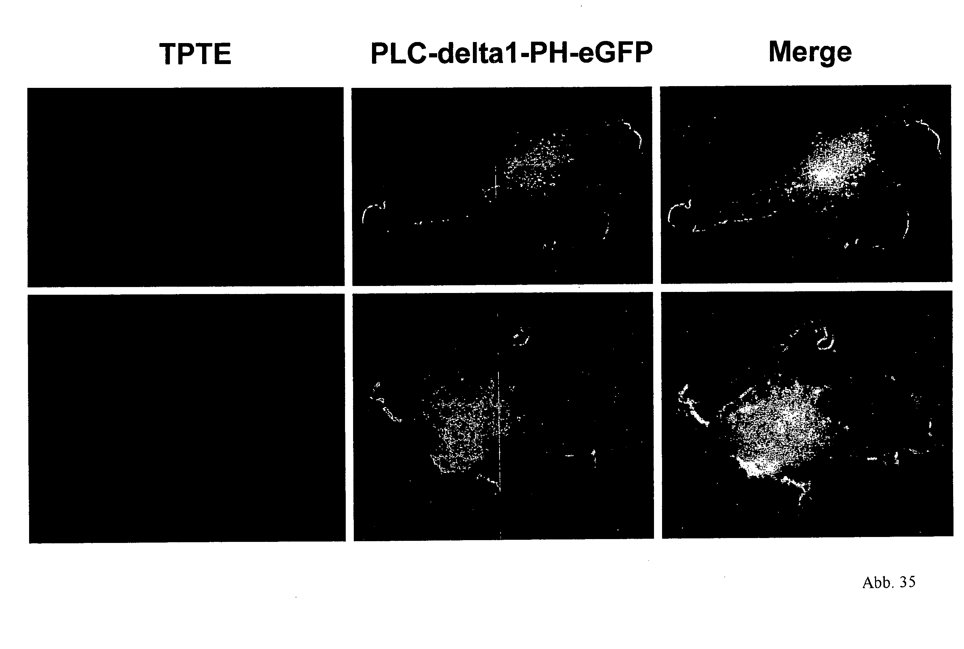

**Abb. 35****: TPTE kolokalisiert mit PIP4,5.** TPTE transfizierte NIH3T3/c-erb-2 Zellen wurden mit PH-eGFP von PLC-deltal kotransfiziert und mit TPTE-spezifischem Antikörper gefärbt. In der Überlagerung zeigt sich eine eindeutige Kolokalisation von TPTE mit PLC-delta1-PH-eGFP als Marker für PIP4,5.

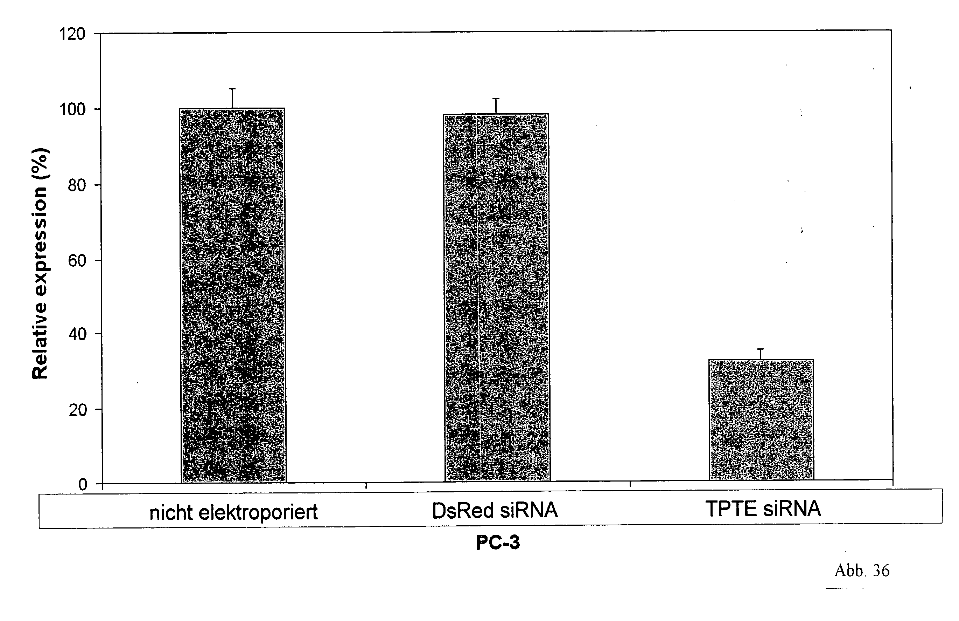

**Abb. 36****: Knock-down der TPTE-Expression in PC-3 Zellen durch RNAi.** PC-3 Zellen wurden mit 1µM siRNA spezifisch für TPTE elektroporiert. Nach 24h wurde die mRNA-Expression mittels Real-Time RT-PCR quantifiziert. Als Kontrolle dienten nichtelektroporierte Zellen und Zellen, die mit DsRed siRNA elektroporiert wurden.

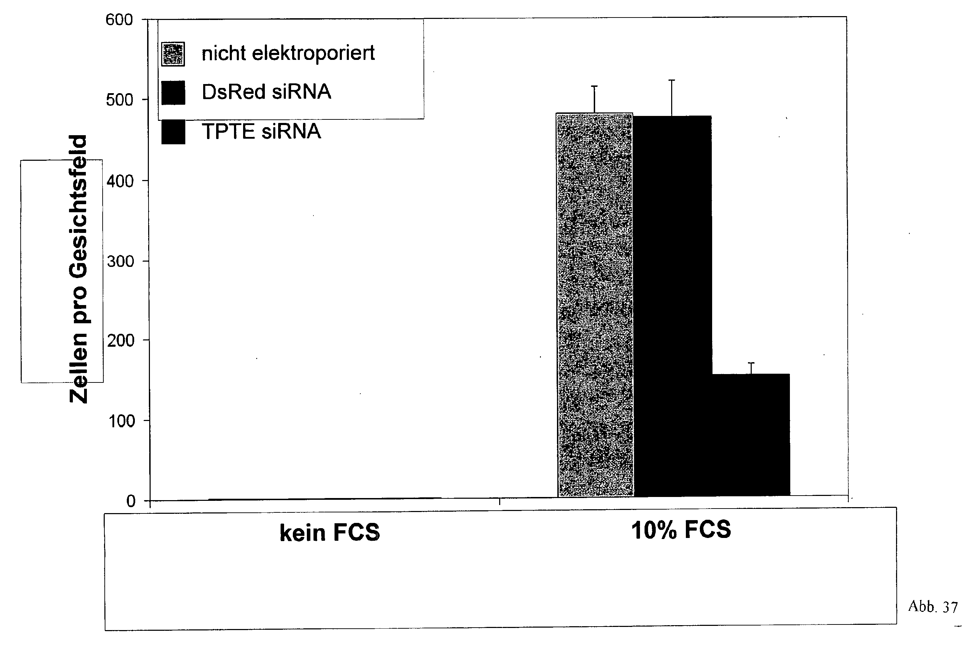

**Abb. 37****: Verminderung der Zellmigration von PC-3 Zellen nach Elektroporation mit TPTE-siRNA.** 48h nach Elektropration mit 1µM TPTE-siRNA zeigt sich eine relevante Abnahme der Zellmigration im Boyden-Kammer Assay.

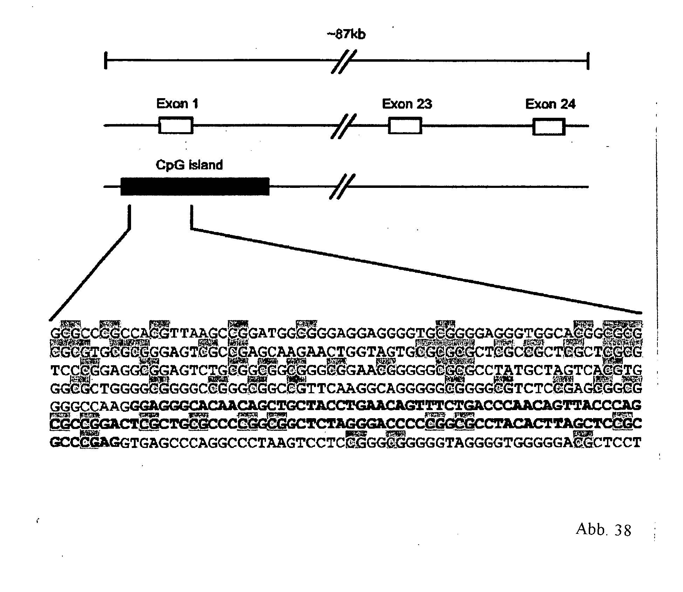

**Abb. 38****: Region von Position 121 bis 540 der in SEQ ID NO:822 des Sequenzprotokolls gezeigten Sequenz.**

### Beispiele:

### Material und Methoden

Die Begriffe "*in silico*", "elektronisch" und "virtuell klonieren" beziehen sich rein auf die Nutzung von auf Datenbanken beruhenden Verfahren, mit denen auch Labor-experimentelle Vorgänge simuliert werden können.

Alle anderen Begriffe und Termini sind, falls nicht explizit anders definiert, so verwendet, wie sie der Fachmann versteht. Die genannten Techniken und Methoden erfolgen in an sich bekannter Weise und sind z.B. in Sambrook et al., Molecular Cloning: A Laboratory Manual, 2. Auflage (1989) Cold Spring Harbor Laboratory Press, Cold Spring Harbor, N. Y beschrieben. Alle Verfahren, die die Verwendung von Kits und Reagenzien einschließen, sind entsprechend den Angaben der Hersteller durchgeführt.

### Datamining-basierte Strategie zur Ermittlung von eCT (elektronisch klonierte Cancer/Testis-Gene)

Zwei *in silico* Strategien nämlich GenBank-Schlagwort-Suche und der cDNAxProfiler wurden kombiniert (Abb. 1). Es wurde unter Nutzung des ENTREZ Search and Retrieval Systems des NCBI (http://www.ncbi.nlm.nih.gov/Entrez) eine Suche nach Kandidaten-Genen in der GenBank durchgeführt, die annotiert sind als spezifisch exprimiert in testikulärem Gewebe (Wheeler et al., Nucleic Acids Research 28:10-14, 2000).

Durch Suchabfragen mit den Schlagworten "testis-specific gene", "sperm-specific gene", "spermatogonia-specific gene" wurden Kandidatengene (GOI, genes of interest) aus den Datenbanken herausextrahiert. Die Suche wurde auf einen Teil der Gesamtinformation dieser Datenbanken eingeschränkt, indem als Limits "homo sapiens" für den Organismus und "mRNA" für die Molekülart eingesetzt wurden.

Die Liste der gefundenen GOI wurde kuratiert, indem unterschiedliche Bezeichnungen für dieselbe Sequenz ermittelt und solche Redundanzen behoben wurden.

Alle Kandidatengene, die sich durch die Schlagwort-Suche ergaben, wurden wiederum durch das Verfahren des "electronic Northern" (eNorthern) bezüglich ihrer Gewebeverteilung untersucht. Der eNorthern basiert darauf, dass die Sequenz eines GOI gegenüber einer EST-(expressed sequence tag) Datenbank (Adams et al., Science 252:1651,1991) abgeglichen wird (http://www.ncbi.nlm.nih.gov/BLAST). Zu jedem EST, das sich als homolog zum eingegebenen GOI ergibt, lässt sich die Gewebeherkunft ermitteln und durch die Summe aller ESTs auf diese Weise eine vorläufige Einschätzung der Gewebeverteilung des GOI erreichen. Nur diejenigen GOI wurden weiteren Untersuchungen zugeführt, die keine Homologien zu EST aus nicht-testikulären Normalgeweben mit Ausnahme von Plazenta und fetalem Gewebe hatten. Für dies Beurteilung wurde auch berücksichtigt, dass es falsch-annotierte cDNA Banken in der öffentlichen Domäne gibt (Scheurle et al., Cancer Res. 60:4037-4043, 2000) (www.fau.edu/cmbb/publications/cancergenes6.htm).

Als zweites Datamining-Verfahren wurde der **cDNA xProfiler** des Cancer Genome Anatomy Projekts des NCBI (http://cgap.nci.nih.gov/Tissues/xProfiler) genutzt (Hillier et al., Genome Research 6:807-828, 1996; Pennisi, Science 276:1023-1024, 1997). Dieser erlaubt Pools von in Datenbanken abgelegten Transkriptomen durch logische Operatoren in Beziehung zueinander zu setzen. Wir haben einen Pool A definiert, dem alle aus Testis hergestellten Expressionsbibliotheken, unter Ausschluss von gemischten Bibliotheken zugeordnet wurden. Dem Pool B wurden alle cDNA-Bibliotheken zugeordnet, die von Normalgeweben mit Ausnahme von Testis, Ovar und Fetalgewebe hergestellt waren. Generell wurden alle cDNA-Banken unabhängig vom zugrundeliegenden Herstellungsverfahren genutzt, allerdings lediglich solche mit einer Mächtigkeit > 1000 zugelassen. Mittels des BUT NOT Operators wurde Pool B digital von Pool A subtrahiert. Auch das Set der auf diese Weise gefundenen GOI wurde eNorthern-Studien unterzogen, sowie durch eine Literaturrecherche abgesichert. Dieses kombinierte Datamining schließt alle etwa 13 000 Vollänge-Gene in der öffentlichen Domäne ein und prädiziert aus diesen insgesamt 140 Gene mit potentieller Testis-spezifischer Expression. Unter diesen waren 25 bereits bekannte Gene der CT-Genklasse, was die Effizienz unserer Strategie unterstreicht.

Alle anderen Gene wurden zunächst durch spezifische RT-PCR in Normalgeweben evaluiert. Alle GOI, die sich als in nicht-testikulären Normalgeweben exprimiert erwiesen, hatten als Falsch-Positive zu gelten und wurden aus weiteren Untersuchungen ausgeschlossen. Die verbliebenen wurden in einem großen Panel an verschiedensten Tumorgeweben untersucht. Die unten dargestellten Antigene erwiesen sich dabei als ektop in Tumorzellen aktiviert.

### RNA-Extraktion, Herstellung von poly-d(T) geprimter cDNA und RT-PCR Analyse

Gesamt-RNA aus nativem Gewebematerial wurde unter Verwendung von Guanidiumisothiocyanate als chaotrophem Agens extrahiert (Chomczynski & Sacchi, Anal. Biochem. 162:156-9, 1987). Nach Extraktion mit saurem Phenol und Fällung mit Isopropanol wurde die RNA in DEPC-behandeltem Wasser gelöst.

Aus 2-4 µg Gesamt-RNA wurde in einem 20µl Reaktionsansatz mittels Superscript II (Invitrogen) entsprechend den Angaben des Herstellers eine Erststrang-cDNA-Synthese durchgeführt. Als Primer wurde ein dT(18) Oligonukleotid verwendet. Integrität und Qualität der cDNA wurden durch Amplifikation von p53 in einer 30 Zyklen-PCR (sense CGTGAGCGCTTCGAGATGTTCCG, antisense CCTAACCAGCTGCCCAACTGTAG, Hybridisierungstemperatur 67°C) überprüft.

Es wurde ein Archiv aus Erststrang-cDNAs aus einer Reihe von Normalgeweben und Tumorentitäten hergestellt. Für Expressionsstudien wurden 0,5 µl dieser cDNAs in einem 30µl Reaktionsansatz mit GOI-spezifischen Primern (siehe unten) und 1 U HotStarTaq DNA Polymerase (Qiagen) amplifiziert. Der Reaktionsansatz enthielt jeweils 0,3 mM dNTPs, je 0,3 µM jeden Primers und 3 µl 10 x Reaktionspuffer.

Die Primer wurden so ausgewählt, dass sie in 2 verschiedenen Exons liegen und die Beseitigung der Interferenz durch kontaminierende genomische DNA als Grund für falsch positive Resultate wurde durch Testen von nicht revers transkribierter DNA als Matritze bestätigt. Nach 15 Minuten bei 95°C zur Aktivierung der HotStarTaq DNA Polymerase wurden 35 Zyklen PCR durchgeführt (1 min 94°C, 1 min jeweilige Hybridisierungstemperatur, 2 min 72°C und abschließende Elongation bei 72°C für 6 min). 20µl dieser Reaktion wurden auf einem mit Ethidiumbromid gefärbten Agarosegel aufgetrennt und analysiert.

Folgende Primer wurden für die Expressionsanalyse der entsprechenden Antigene bei der angegebenen Hybridisierungstemperatur verwendet
LDH-C (67°C)
   sense TGCCGTAGGCATGGCTTGTGC, antisense CAACATCTGAGACACCATTCC
TPTE (64°C)
   sense TGGATGTCACTCTCATCCTTG, antisense CCATAGTTCCTGTTCTATCTG
TSBP (63°C)
   sense TCTAGCACTGTCTCGATCAAG, antisense TGTCCTCTTGGTACATCTGAC
MS4A12 (66°C)
   sense CTGTGTCAGCATCCAAGGAGC, antisense TTCACCTTTGCCAGCATGTAG
BRCO1 (60°C)
   sense CTTGCTCTGAGTCATCAGATG, antisense CACAGAATATGAGCCATACAG
TPX1 (65°C)
   sense TTTTGTCTATGGTGTAGGACC, antisense GGAATGGCAATGATGTTACAG

### Herstellung von Random-Hexamer-geprimter cDNA und quantitative Real-Time-PCR

Die Expression von LDHC wurde mittels Real-Time-PCR quantifiziert.

Das Prinzip der quantitativen Real-Time-PCR mit dem ABI PRISM Sequence Detection System (PE Biosystems, USA) nutzt die 5'-3' Exonuklease-Aktivität der Taq DNA Polymerase zur direkten und spezifischen Detektion von PCR-Produkten durch Freisetzung von Fluoreszenz-Reporterfarbstoffen. Neben Sense- und Antisense-Primern wird bei der PCR eine doppelt fluoreszenz-markierte Sonde (TaqMan-Probe) eingesetzt die an eine Sequenz des PCR-Produkts hybridisiert. Die Sonde ist 5' mit einem Reporterfarbstoff (z.B. FAM) und 3' mit einem Quencherfarbstoff (z.B. TAMRA) markiert. Ist die Sonde intakt, supprimiert die räumliche Nähe von Reporter zu Quencher die Emission der Reporterfluoreszenz. Hybridisiert die Sonde während der PCR an das PCR-Produkt, wird sie durch die 5'-3' Exonuklease-Aktivität der Taq DNA Polymerase gespalten und die Suppression der Reporterfluorszenz aufgehoben. Das Ansteigen der Reporterfluoreszenz als Folge der Zielamplifikation wird nach jedem PCR-Zyklus gemessen und zur Quantifizierung genutzt. Die Expressionsquantifizierung des Zielgens erfolgt absolut oder relativ zur Expression eines Kontrollgens mit konstanter Expression in den zu untersuchenden Geweben. Die Expression von LDHC wurde nach Normalisierung der Proben gegen 18s RNA als sog. Housekeeping-Gen mittels der ΔΔ-Cₜ Methode (PE Biosystems, USA) berechnet Die Reaktionen wurden in Duplex-Ansätzen durchgeführt und in Duplikaten bestimmt. Die Synthese der cDNA erfolgte mit dem High Capacity cDNA Archive Kit (PE Biosystems, USA) unter Verwendung von Hexamer-Primern nach Angaben des Herstellers. Jeweils 5 µl der verdünnten cDNA wurden in 25 µl Gesamtvolumen für die PCR eingesetzt: sense-Primer (GGTGTCACTTCTGTGCCTTCCT) 300 nM; antisense-Primer (CGGCACCAGTTCCAACAATAG) 300 nM; TaqMan-Probe (CAAAGGTTCTCCAAATGT) 250 nM; sense-Primer 18s RNA 50 nM; antisense-Primer 18s RNA 50 nM; 18 s RNA-Probe 250 nM; 12,5 µl TaqMan Universal PCR Master Mix; anfängliche Denaturierung 95°C (10 min); 95°C (15 sec); 60°C (1 min); 40 Zyklen. Durch die Amplifikation eines 128 bp-Produkts über die Grenze von Exon 1 und Exon 2 wurden alle beschriebenen Splicevarianten von LDHC in die Quantifizierung einbezogen.

Die Expression von TPTE, MS4A12, PCSC und BRCO1 wurde ebenfalls mittels Real-Time-PCR quantifiziert, jedoch wurden die PCR-Produkte mit SYBR-Green als Reporterfarbstoff detektiert. Die Reporterfluoreszenz von SYBR-Green ist in Lösung supprimiert und erst nach Bindung an dopplesträngige DNA-Fragmente ist der Farbstoff aktiv. Das Ansteigen der SYBR-Green Fluoreszenz als Folge der spezifischem Amplifikation mittels GOI-spezifische Primern nach jedem PCR Zyklus wird zur Quantifizierung genutzt. Die Expressionsquantifizierung des Zielgens erfolgt absolut oder relativ zur Expression eines Kontrollgens mit konstanter Expression in den zu untersuchenden Geweben. Die Expression von LDHC wurde nach Normalisierung der Proben gegen 18s RNA als sog. Housekeeping-Gen mittels der ΔΔ-Cₜ Methode (PE Biosystems, USA) berechnet. Die Reaktionen wurden in Duplex-Ansätzen durchgeführt und in Triplikaten bestimmt. Verwendet wurde das QuantiTect SYBR-Green PCR Kit (Qiagen, Hilden) nach Angaben des Herstellers. Die Synthese der cDNA erfolgte mit dem High Capacity cDNA Archive Kit (PE Biosystems, USA) unter Verwendung von Hexamer-Primern nach Angaben des Herstellers. Jeweils 5 µl der verdünnten cDNA wurden in 25 µl Gesamtvolumen für die PCR eingesetzt: sense-Primer 300nM, antisense-Primer 300nM; initiale Denaturierung 95° 15 min; 95° 30 sec; Annealing 30 sec; 72° 30 sec; 40 Zyklen.
TPTE (62°)
   sense GAGTCTACAATCTATGCAGTG, antisense CCATAGTTCCTGTTCTATCTG
MS4A12 (65°)
   sense CTGTGTCAGCATCCAAGGAGC, antisense TTCACCTTTGCCAGCATGTAG
PCSC (59°)
   sense AGAATAGAATGTGGCCTCTAG, antisense TGCTCTTACTCCAAAAAGATG
BRCO1 (60°)
   sense CTTGCTCTGAGTCATCAGATG, antisense CACAGAATATGAGCCATACAG

### Klonierung und Sequenzanalyse

Klonierung von Vollängen bzw. Genfragmenten erfolgte nach gängigen Methoden. Zur Ermittlung der Sequenz wurden entsprechende Antigene mittels der Proofreading-Polymerase pfu (Stratagene) amplifiziert. Nach Beendigung der PCR wurde Adenosin mittels HotStarTaq DNA Polymerase an die Enden des Amplikons ligiert, um die Fragmente entsprechend den Angaben des Herstellers in den TOPO-TA Vektor zu klonieren. Die Sequenzierung wurde durch einen kommerziellen Service durchgeführt. Die Sequenzen wurden mittels gängiger Prädiktionsprogramme und Algorithmen analysiert.

### Western blot

Zellen aus Zellkultur (endogene Expression des Zielgens oder Synthese des Zielproteins nach Transfektion eines Expressionsvektors, der das Zielprotein kodiert) oder Gewebeproben, die das Zielprotein enthalten könnten, werden in einer 1%igen SDS Lösung lysiert. Das SDS denaturiert dabei die im Lysat enthaltenen Proteine. Die Lysate eines experimentellen Ansatzes werden abhängig von der erwarteten Proteingröße auf 8-15 %igen denaturierenden Polyacrylamidgelen (enthalten 1% SDS) der Größe nach elekrophoretisch aufgetrennt (SDS-Polyacrylamid Gelelektrophorese, SDS-PAGE). Anschließend werden die Proteine durch das semi-dry Elektroblot Verfahren (Biorad) auf Nitrozellulose Membran (Schleicher & Schüll) transferiert, auf der das gewünschte Protein nachgewiesen werden kann. Dazu wird die Membran zunächst blockiert (z.B. mit Milchpulver) und anschließend mit dem spezifischen Antikörper in einer Verdünnung von 1:20-1:200 (je nach Spezifität des Antikörpers) für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. Nach einem weiteren Waschschritt wird anschließend das Zielprotein in einer Farb- oder Chemilumineszenz-Reaktion auf der Membran mittels einer Enzymreaktion sichtbar gemacht (z.B. ECL, Amersham Bioscience). Das Ergebnis wird durch Aufnahme mit einer geeigneten Kamera dokumentiert.

### Immunfluoreszenz

Es werden Zellen etablierter Zelllinien benutzt, die entweder das Zielprotein endogen synthetisieren (Nachweis der RNA in der RT-PCR oder des Proteins im Western blot) oder aber vor der IF mit Plasmid-DNA transfiziert worden sind. Zur Transfektion von Zelllinien mit DNA sind die verschiedensten Methoden (z.B. Elektroporation, Liposomen basierte Transfektion, Calciumphosphatpräzipitation) gut etabliert (z.B. Lemoine et al. Methods Mol Biol. 1997; 75: 441-7). Das transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen. Zellen, die das Zielprotein synthetisieren, werden mit Paraformaldehyd; Saponin oder Methanol fixiert. Anschließend können die Zellen bei Bedarf durch Inkubation mit Detergenzien (z.B. 0,2% Triton X-100) permeabilisiert werden. Nach der Fixierung/Permeabilisierung werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, dass direkt angeregt werden kann und selbst fluoresziert, so dass zum Nachweis keine Antikörper benötigt werden.

### Immunhistochemie

Die IHC dient im einzelnen dazu, um (1) die Menge an Zielprotein in Tumor und Normalgeweben abschätzen zu können, (2) zu analysieren, wie viele Zellen in Tumor und gesundem Gewebe das Zielgen synthetisieren, (3) den Zelltyp in einem Geweben (Tumor, gesunde Zellen) zu definieren, in dem das Zielprotein nachweisbar ist.

Je nach individuellem Antikörper müssen unterschiedliche Protokolle verwendet werden (z.B. "Diagnostic Immunohistochemistry by David J., MD Dabbs ISBN: 0443065667" oder in "Microscopy, Immunohistochemistry, and Antigen Retrieval Methods: For Light and Electron Microscopy ISBN: 0306467704").

Formalin fixierte (andere Fixierungen: z.B. Methanol) und in Paraffin eingebettete Gewebestücke mit einer Dicke von ca. 4µm werden auf einem Glasträger aufgebracht und z.B. mit Xylene deparaffiniert. Die Proben werden mit TBS-T gewaschen in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60 minütige Inkubation mit einem zweiten mit Alkalischer Phosphatase (alternativ: z.B. Peroxidase) gekoppelten Antikörper, der gegen den ersten Antikörper gerichtet ist. Anschließend erfolgt eine Farbreaktion unter Verwendung der Alkalischen Phosphatase (Referenzen: Shi et al. J Histochem Cytochem 1991 39: 741-748; Shin et al. Lab Invest. 1991 64: 693-702). Zum Nachweis der Antikörperspezifität kann die Reaktion durch vorherige Zugabe des Immunogens blockiert werden.

### Immunisierung

(Siehe auch Monoclonal Antibodies: A Practical Approach by Philip Shepherd, Christopher Dean isbn 0-19-963722-9; Antibodies: A Laboratory Manualby Ed Harlow, David Lane ISBN: 0879693142; Using Antibodies : A Laboratory Manual : Portable Protocol NO. by Edward Harlow, David Lane, Ed Harlow ISBN: 0879695447).

Im Folgenden wird der Herstellungsprozess von Antikörpern kurz beschrieben, Details sind den zitierten Publikationen zu entnehmen. Zunächst werden Tiere (z.B. Kaninchen) durch eine erste Injektion des gewünschten Zielproteins immunisiert. Durch eine zweite oder dritte Immunisierung innerhalb eines definierten Zeitraums (ca. 2-4 Wochen nach der vorangegangenen Immunisierung) lässt sich die Immunantwort des Tieres gegen das Immunogen verstärken. Wiederum nach verschiedenen definierten Zeitabständen (1. Blutung nach 4 Wochen, anschließend ca. alle 2 Wochen mit insgesamt bis zu 5 Entnahmen) wird den Tieren Blut entnommen und daraus ein Immunserum gewonnen.

Die Immunisierung der Tiere erfolgt in der Regel über eines von vier gut etablierte Verfahren, wobei auch andere Verfahren existieren. Immunisiert werden kann dabei mit Peptiden, die für das Zielprotein spezifisch sind, dem gesamten Protein, mit extrazellulären Teilsequenzen eines Proteins, das experimentell oder über Prediktionsprogramme identifiziert werden kann.
(1) Im ersten Fall werden an KLH (keyhole limpet hemocyanin) konjugierte-Peptide (Länge: 8-12 Aminosäuren) über ein standardisiertes in vitro Verfahren synthetisiert und diese Peptide zur Immunisierung verwendet. In der Regel erfolgen 3 Immunisierungen mit einer Konzentration von 5-1000 µg/Immunisierung. Die Durchführung der Immunisierung kann auch als Service von Dienstleistern erfolgen.
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei in vitro, in Bakterien (z.B. E. coli), in Hefe (z.B. S. pombe), in Insektenzellen oder in mammalen Zellen synthetisiert. Nach Synthese in einem der Systeme wird das Zielprotein aufgereinigt, die Aufreinigung erfolgt dabei in der Regel über standardisierte chromatografische Methoden. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; Gst Fusionsproteine). Eine Vielzahl von Protokollen befinden sich z.B. in den "Current Protocols in Molecular Biology (John Wiley & Sons Ltd, Wiley InterScience).
(3) Falls eine Zelllinie zur Verfügung steht, die das gewünschte Protein endogen synthetisiert, kann auch diese Zelllinie zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV Promotor). Anschließend werden 5-100 µg DNA als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Zielgen wird exprimiert und das Tier entwickelt schließlich eine Immunantwort gegen das Zielgen (Jung et al., Mol Cells 12: 41-49, 2001; Kasinrerk et a., Hybrid Hybridomics 21: 287-293, 2002).

### Affinitätsreinigung

Die Reinigung der polyklonalen Seren erfolgte im Fall der Peptidantikörper gänzlich oder im Fall der Antikörper gegen rekombinante Proteine teilweise als Service durch die beauftragten Firmen. Hierzu wurde in beiden Fällen das entsprechende Peptid bzw. rekombinante Protein kovalent an eine Matrix gebunden, diese nach der Kopplung mit einem nativen Puffer (PBS: phosphate buffered saline) äquilibriert und im Anschluß mit dem Rohserum inkubiert. Nach einem weiteren Waschschritt mit PBS wurde der Antikörper mit 100 mM Glycin pH 2,7 eluiert und das Eluat sogleich in 2 M TRIS pH 8 neutralisiert. Die so gereinigten Antikörper konnten dann zur spezifischen Detektion der Zielproteine sowohl durch Westernblotting als auch durch Immunfluoreszenz eingesetzt werden.

### Boydenkammer Migrationsassay

Die Boydenkammer dient der Quantifizierung der Zellmigration in Reaktion auf chemotaktische Stimuli. Die Kammer besteht aus zwei Kompartimenten die durch eine Micropore-Membran voneinander getrennt sind. In das obere Kompartment gibt man die zu untersuchenden Zellen in Minimalmedium, währen das untere Kompartment mit Medium gefüllt wird welches das entsprechende Chemotaktikum enthält. Die Zellen wandern entsprechend des Gradienten durch die Membran und adhärieren an die Unterseite der Membran. Nach Fixation können die transmigrierten Zellen unter dem Mikroskop ausgezählt werden.

Der Migrationsassay wurde zur Bestimmung des promigratorischen Potentials von TPTE durchgeführt. Eingesetzt wurden mit TPTE-eGFP transfizierte, c-erbB2 transformierte NIH3T3 Fibroblasten. Als Kontrollzellen wurden nicht-transfizierte Zellen und mit eGFP-N3 Leervektor transfizierte Zellen eingesetzt. Für den Assay wurden Transwell Kammern (Becton Dickinson) mit einer Porengrösse von 8,0 µM in der Membran verwendet Jeweils 4x10⁴ Zellen in 400 µl Serum-freiem DMEM Medium wurden in das obere Kompartiment gegeben. Das untere Kompartiment wurde mit 800 µl DMEM Medium gefüllt, dem 10%FCS oder PDGF-BB in aufsteigenden Konzentrationen (10-300ng/µl) zugefügt wurde. Die Kammern wurden für 40h bei 37° inkubiert. Anschliessend wurden die transmigrierten Zellen in eiskaltem Methanol fixiert, die Membranen wurden ausgeschnitten und auf Objektträgern mit Hoechst Kernfarbstoff (DAKO) zur Fluoreszenzmikroskopie eingedeckt. Zellen in fünf Gesichtsfeldern (Vergrösserung 20fach) pro Membran wurden ausgezählt. Alle Experimente wurden in Triplikaten durchgeführt.

### RNA-Interferenz (RNAi)

Die siRNA-Oligos wurden entsprechend der Tuschl-Kriterien ausgewählt (Elbashir et al., Nature 411(6836):428-9, 2001). TPTE siRNA-Oligos (sense 5'-CCCUGCCACAUGUUCAUAUdTdT-3'; antisense 5'-AUAUGAACAUGUGGCAGGGdTdT-3') targetierten die Nukleotide 2043-2061 der TPTE mRNA-Sequenz (NM_013315). Als Kontrolle wurden siRNA-Oligos spezifisch für das irrelevante DsRed-Fluoreszenzprotein (AF506025) eingesetzt (sense 5'-AGUUCCAGUACGGCUCCAAdTdT-5'; antisense 5'-UUGGAGCCGUACUGGAACUdTdT-3'). Zur Generierung der siRNA-Duplexe wurden jeweils 200µM der entsprechenden sense- und antisense-Oligos in Hybridisierungspuffer (30mM HEPES (pH 7,4), 100mM Natrium-Acetat, 2mM Magnesium-Acetat) nach initialer Denaturierung (1 min. bei 90°C) für 1h bei 37°C inkubiert.

### siRNA-Elektroporation

5x10⁶ Zellen wurden in 250µl Serum-freiem X-VIVO 15 Medium aufgenommen und mit 1µM der entsprechenden siRNA-Duplexe elektroporiert (200V, 250µF). Die TPTE mRNA-Expression wurde 24h später mittels Real-Time RT-PCR quantifiziert.

### Methylierungsstudien

Zur Untersuchung der Induzierbarkeit der TPTE-Expression durch genomische Demethylierung wurden die TPTE-negative Zellinien BT549 (Mamma-Ca) und HCT116 (Kolon-Ca) für 72h mit 2µM bzw. 10µM 5-Aza-2'-deoxycytidin kultiviert. Anschliessend wurde die TPTE-Expression mittels Real-Time RT-PCR quantifiziert. Desweiteren wurde die TPTE-Expression in DNA-Methyltransferase (DNMT) defizienten HCT116 Zellen quantifiziert. Während DNMT1 (HCT116^{DNMT1-/-}) bzw. DNMT3b (HCT116^{DNMT3b-/-}) defiziente Zellen ein nahezu unverändertes Methylierungsmuster im Vergleich zur parentalen Zellinie aufweisen (HCT116^{par}), sind Zellen in denen beide DNMTs gemeinsam deletiert sind (HCT116^{DKO}) durch fast vollständige Demethylierung der genomischen DNA gekennzeichnet.

### Time-Lapse Mikroskopie

Die Membranlokalisation von TPTE und die Beteilung an der Regulation von Membrandynamiken in vitalen Zellen wurde mittels Time-Lapse Mikroskopie untersucht. Hierfür wurden TPTE-eGFP transfizierte Zellen für 12h in serum-freiem DMEM Medium inkubiert. Zur Induktion von spontanen Zellmemdynamiken in Form von Membranprotrusionen, Pseudopodien und Filopodien wurden die Zellen vor der Analyse durch Zugabe von FCS stimuliert. Bilder der vitalen Zellen wurden in 30sec Abständen mit einem Invert Olympus Mikroskop (IX70) mit einer TILL IMAGO-VGA CCD Kamera aufgenommen.

### Herstellung EGFP-Transfektanten

Für die Immunfluoreszenz-Mikroskopie von heterolog exprimierter TPTE wurde der komplette ORF von TPTE in pEGFP-C1 und pEGFP-N3 Vektoren (Clontech) kloniert. Auf Objektträgern kultivierte CHO und NIH3T3 Zellen wurden mit den entsprechenden Plasmidkonstrukten unter Verwendung von Fugene Transfektionsreagenz (Roche) nach Herstellerangaben transfiziert und nach 12-24h mitels Immunfluoreszenz-Mikroskopie anlysiert.

### Prädiktion von Peptidepitopen für MHC Klasse I

Peptidepitope aus jeder der Proteinsequenzen mit Bindungseigenschaften für die polymorphen HLA-Allele A*0201, A*2402, A*0101, A*0301, B*0702 wurden unter Nutzung des unter http://syfpeithi.bmi-heidelberg.com/ abgelegten Prädiktionalgorithmus ermittelt. Als Peptidlänge wurden 8, 9 und 10mere vorgegeben und ein Cutoff bei einem Score von 15 gesetzt. Ergab die Suche weniger als 10 Peptide für die jeweilige Länge, wurden alternativ die 10 besten Peptide ausgewählt

### Beispiel 1: Identifizierung von LDH C als neues Tumorantigen

LDH C (SEQ ID NO:1) und sein Translationsprodukt (SEQ ID NO:6) wurden als Testisspezifisches Isoenzym der Laktatdehydrogenase-Familie beschrieben. Die Sequenz ist in GenBank unter der Zugangsnummer NM_017448 veröffentlicht. Das Enzym bildet ein Homotetramer mit einem Molekulargewicht von 140 kDa (Goldberg, E. et al., Contraception 64(2):93-8, 2001; Cooker et al., Biol. Reprod. 48(6):1309-19, 1993; Gupta, G.S., Crit. Rev. Biochem. Mol. Biol. 34(6):361-85, 1999).

RT-PCR Untersuchungen zur Expressionsanalyse mit einem Primerpaar (5'-TGCCGTAGGCATGGCTTGTGC-3', 5'-CAACATCTGAGACACCATTCC-3'), das die verwandten und ubiquitär exprimierten Isoenzyme LDH A und LDH B nicht kreuzamplifiziert und auf der LDH C Prototypsequenz NM_017448, die als Testis-spezifisch vorbeschrieben ist, basiert, bestätigten erfindungsgemäß das Fehlen von Expression in allen getesteten Normalgeweben, zeigten jedoch, dass die stringente transkriptionelle Repression dieses Antigenes in somatischen Zellen bei Tumoren aufgehoben ist; vgl. Tabelle 1. Wie klassischerweise für CT-Gene beschrieben, wird LDH C in einer Reihe von Tumorentitäten exprimiert

**Tabelle 1. Expression von LDHC in Tumoren**

| **Gewebe** | **insgesamt getestet** | **Positiv** | **%** |
|---|---|---|---|
| **Melanom** | **16** | **7** | **44** |
| **Mammakarzinome** | **20** | **7** | **35** |
| **Kolorektale Tumoren** | **20** | **3** | **15** |
| **Prostatakarzinome** | **8** | **3** | **38** |
| **Bronchialkarzinome** | **17** | **8** | **47** |
| **Nierenzellkarzinome** | **7** | **4** | **57** |
| **Ovarialkarzinome** | **7** | **3** | **43** |
| **Schilddrüsenkarzinome** | **4** | **1** | **25** |
| **Zervixkarzimome** | **6** | **5** | **83** |
| **Melanom Zellinien** | **8** | **5** | **63** |
| **Bronchialkarzinom Zellinien** | **6** | **2** | **33** |

Die erwartete Größe des Amplifikationsproduktes mit vorstehend genannten PCR-Primern beträgt 824 bp. Erfindungsgemäß wurde allerdings in Tumoren, jedoch nicht in Testis, die Amplifikation von multiplen zusätzlichen Banden beobachtet. Da dies indikativ für das Vorhandensein alternativer Splice-Varianten ist, wurde mit LDH-C spezifischen Primern (5'-TAGCGCCTCAACTGTCGTTGG-3', 5'-CAACATCTGAGACACCATTCC-3') das gesamte offene Leseraster amplifiziert und unabhängige Vollänge-Klone sequenziert. Abgleiche mit dem Prototyp-ORF der beschriebenen Sequenz von LDH C (SEQ ID NO:1) und der genomischen Sequenz auf Chromosom 11 bestätigen zusätzliche Splice-Varianten (SEQ ID NO:2-5). Die alternativen Splicingereignisse führen zum Fehlen von Exon 3 (SEQ ID NO:2), der beiden Exons 3 und 4 (SEQ ID NO:3), der Exons 3, 6 und 7 (SEQ ID NO:4) bzw. von Exon 7 (SEQ ID NO:5) (vgl. Abb. 2).

Diese neuen Splicevarianten werden ausschließlich in Tumoren aber nicht im Testis generiert. Das alternative Splicing führt zu Veränderungen im Leseraster und resultiert in neuen möglichen ORF, die die in SEQ ID NO:7-13 gezeigten Aminosäuresequenzen kodieren (ORF für SEQ ID NO:7: Nukleotidposition 59-214 von SEQ ID NO:2 bzw. SEQ ID NO:4; ORF für SEQ ID NO:8: Nukleotidposition 289-939 von SEQ ID NO:2; ORF für SEQ ID NO:9: Nukleotidposition 59-196 von SEQ ID NO:3; ORF für SEQ ID NO:10: Nukleotidposition 535-765 von SEQ ID NO:3; ORF für SEQ ID NO:11: Nukleotidposition 289-618 von SEQ ID NO:4; ORF für SEQ ID NO:12: Nukleotidposition 497-697 von SEQ ID NO:4; ORF für SEQ ID NO:13: Nukleotidposition 59-784 von SEQ ID NO:5) (Abb. 2, 3). Neben einer vorzeitigen Termination ist auch die Nutzung von alternativen Startcodons möglich, so dass die kodierten Proteine N- wie auch C-terminal verkürzt sein können.

Während SEQ ID NO:8 und SEQ ID NO:10 trunkierte Anteile des Prototyp-Proteins darstellen, sind die Proteinsequenz von SEQ ID NO:7, SEQ ID NO:9, SEQ ID NO:11, SEQ ID NO:12 und SEQ ID NO:13 zusätzlich verändert und beinhalten ausschließlich Tumor-spezifische Epitope (fett gedruckt in Abb. 3). Peptidregionen, die zu Tumor-spezifischen Epitopen führen könnten sind wie folgt (der streng Tumor-spezifische Anteil, der durch Leserasterverschiebungen entsteht ist unterstrichen):
SEQ ID NO:14: GAVGMACAISILLK**ITVYLQTPE** (aus SEQ ID NO:7)
SEQ ID NO:15: GAVGMACAISILLK**WIF** (aus SEQ ID NO:9)
SEQ ID NO:16: GWIIGEHGDSS**GGIIWNKRRTLSQYPLCLGAEWCLRCCEN** (aus SEQ ID NO:11)
SEQ ID NO:17: **MVGLLENMVILV**GLYGIKEELFL (aus SEQ ID NO:12)
SEQ ID NO:18: EHWKNIHKQVIQ**RDYME** (aus SEQ ID NO:13)

Diese Regionen können potentiell Epitope enthalten, die auf MHC I bzw. MHC II Molekülen von T-Lymphozyten erkannt werden können und zu einer strikt Tumor-spezifischen Antwort führen.

Nicht alle der prädizierten Proteine weisen die katalytische Laktatdehydrogenase-Domäne für die NADH-abhängige Metabolisierung von Pyruvat zu Laktat auf, die den letzten Schritt der anaeroben Glycolyse darstellt. Diese Domäne wäre für die enzymatische Funktion als Lactatdehydrogenase notwendig (gerahmt in Abb. 3). Weiterführende Analysen z.B. mit Algorithmen wie TMpred und pSORT (Nakai & Kanehisa, 1992) prädizieren unterschiedliche subzelluläre Lokalisationen für die putativen Proteine.

Erfindungsgemäß wurde durch Real-Time-PCR mit einem spezifischen Primer-Probe-Set Expressionslevel quantifiziert. Das Amplicon liegt im Übergang zwischen Exon 1 and 2 und detektiert damit alle Varianten (SEQ ID NO:1-5). Auch diese Untersuchungen detektieren keine Transkripte in Normalgeweben außer Testis. Sie bestätigen signifikante Expressionslevel in Tumoren (Abb. 4).

Es wurden Antikörper durch Immunisierung von Kaninchen mit Peptiden hergestellt, die jeweils verschiedene der oben genannten Varianten abdecken:
SEQ ID NO:80: MSTVKEQLIEKLIEDDENSQ (aa 1-19)
SEQ ID NO:101: RNGVSDVVKINLNSE (C-terminus)
SEQ ID NO:102: GIIWNKRRTLSQYPL (Variante 5.3 spezifisch)

Beispielhaft sollen die Daten dargestellt werden für Antikörper, die gegen SEQ ID NO:80 gerichtet sind. Diese erkennen in der Westernblot Untersuchung LDHC Protein in TestisGewebe aber nicht in anderen Normälgeweben (Abb. 15) und bestätigen somit die RT-PCR Daten auf Transkriptebene. Der spezifische Antikörper lässt sich auch unter verschiedenen Fixationsbedigungen für Immunfluoreszenz-Untersuchungen nutzen. So ergaben RT-PCR Untersuchungen, dass die Kolonkarzinom Zellinie HCT 116 P kein LDHC exprimiert. Ihre DNA Methyltransferase deletierte Variante HCT 116 DKO allerdings positiv für LDHC ist. Vergleichende Färbungen beider Zellinien mit dem oben genannten Antikörper detektierten das entsprechende Protein in gut nachweisbarer Menge spezifisch in den als positiv typisierten Zellinien (Abb. 16). Entsprechend qualifiziert sich dieser Antikörper auch zu immunhistochemischen Färbung von Normal- und Tumorgewebsschnitten durch den kundigen Fachmann. Die Färbung von Testisgewebe z.B. zeigt eine klare Positivität von Keimzellen (Abb. 16).

Laktatdehydrogenasen katalysieren die Interkonversion von Pyruvat und Laktat in der Glykolyse. Unter aeroben Bedingungen wird Pyruvat als Endprodukt der Glykolyse in die Mitochondrien transportiert, wo es als Substrat für den Zitratzyklus zur Verfügung steht. Der Krebs-Zyklus dient der Generierung von Reduktionsäquivalenten in Form von NADH, die für die ATP-Gewinnung im Rahmen der Atmungskette (oxydative Phosphorylierung) verbraucht werden. Unter anaeroben Bedingungen kann ATP nicht über die Atmungskette generiert werden. Die Zelle reguliert ihren Energiehaushalt unter diesen Bedingungen fast ausschliesslich über die Glykolyse. Pyruvat, das unter diesen Bedingungen nicht verstoffwechselt werden kann, wird durch Laktatdehydrogenasen zu Laktat reduziert.

Die verschiedene Laktatdehydrogenase-Isoformen unterscheiden sich in erster Linie durch ihre Gewebsverteilung und unterschiedliche Substratspezifitäten und -affinitäten. Die keimzell-spezifische LDHC katalysiert bevorzugt die Oxidation von Laktat zu Pyruvat und wird auch durch hohe Konzentrationen von Laktat nicht in ihrer Aktivität gehemmt (Goldberg E. Exp. Clin. Immunogenet. 2:120-4, 1985). Diese Eigenschaften sind physiologisch sinnvoll da sich die Spermatiden in einem Milieu entwickeln das durch hohe Laktatkonzentrationen gekennzeichnet ist. Spermatiden bevorzugen Laktat als Energiequelle vor Glucose, Fructose oder Pyruvat (Mita M. Biol. Reprod. 26:445-55, 1982). Die Oxidation von Laktat durch die LDHC stellt Pyruvat als Ausgangssubstrat für den Zitratzyklus zur Verfügung. Demgemäss nutzen Spermatiden bevorzugt den Zitratzyklus als Hauptquelle zur Energiegewinnung (Storey B.T. Biol. Reprod. 16:549-556, 1977).

Es ist lange bekannt dass Tumorzellen häufig ihren Energiebedarf über die anaerobe Glykolyse decken, d.h. vermehrt Laktat bilden, und den Zitratzyklus und die Atmungskette auch unter aeroben Bedingungen nicht nutzen. Die molekulare Grundlagen dieses als Warburg-Effekt beschriebenen Phänomens sind bis heute nicht aufgeklärt, ursächlich werden Enzymdefekte in den Mitochondrien und die Überexpression von Schlüsselenzymen der Glykolyse dikutiert. In diesem Zusammenhang könnte die Expression von LDHC, die durch hohe Konzentrationen von Laktat in ihrer Funktion nicht gehemmt wird von Vorteil für den Energiehaushalt der Tumorzelle sein, indem Laktat zur Gewinnung von ATP genutzt werden kann. Mitochondriendefekte, insbesondere Enzymdefekte des Zitratzyklus sind bekannte Alterationen in Tumorzellen. Durch einen Ausfall des Zitratzyklus können keine Reduktionsäquivalente für die Atmungskette generiert werden. Eine mitochondriale Laktatdehydrogenase mit hoher Affinität für Lactat wie die LDHC könnte eine Umgehung für einen defekten Zitratzyklus darstellen. Lactat das über Monocarboxylate-Transporter (MCT) in Mitochondrien transportiert wird könnte intramitochondrial zu Pyruvat oxidiert werden und somit der Generierung von Reduktionsäquivalenten dienen die in der Atmungskette zur ATP-Gewinnnung verbraucht werden. Zur Analyse der subzellulären Lokalisation von von LDHC transfizierten wir die LDHC negative Brusttumorzellinie MCF-7 mit einem Fusionskonstrukt aus LDHC und Green-Fluorescent-Protein und färbten diese Transfektante mit einem Antikörper gegen den mitochondrialen Marker Cytochrom C. Die Ko-Lolokalisation der beiden Signale in der konfokalen Lasermikroskopie (Abb. 17) beweist die Präsenz von LDHC in Mitochondrien. Somit wäre denkbar dass die Expression LDHC in Tumorzellen tatsächlich einen benefitären Effekt für den Energiehaushalt der Zelle darstellt und eine spezifische Hemmung der LDHC-Aktivität in Tumoren zur Therapie von Tumorerkrankungen genutzt werden könnte.

### Beispiel 2: Identifizierung von TPTE als neues Tumorantigen

Die Sequenzen des Transkripts von TPTE (SEQ ID NO:19) und seines Translationsprodukt (SEQ ID NO:22) sind in GenBank unter der Zugangsnummer NM_013315 veröffentlicht (Walker, S.M. et al., Biochem. J. 360(Pt 2):277-83, 2001; Guipponi M. et al., Hum. Genet. 107(2):127-31, 2000; Chen H. et al., Hum. Genet. 105(5):399-409, 1999). TPTE wurde als Gen, das für eine mögliche transmembrane Tyrosinphosphatase kodiert, mit Testis-spezifischer Expression beschrieben, die in der perizentromeren Region der Chromosomen 21, 13, 15, 22, and Y liegt (Chen, H. et al., Hum. Genet. 105:399-409, 1999). Erfindungsgemäße Abgleichuntersuchungen zeigen zusätzlich homologe genomische Sequenzen auf den Chromosomen 3 und 7.

Die Membranlokalisation, die z.B. für die Erreichbarkeit von therapeutischen Antikörpern Voraussetzung ist, konnte zweifelsfrei nachgewiesen werden durch Transfektion von TPTE-negativen Zellen mit einem Fusionskonstrukt aus TPTE und Green-Fluorescent-Protein (Abb. 18, rechts). Dieses reichert sich an der Membranoberfläche an und lässt sich dort als kolokalisierend mit anderen bekannten Membranmarkern wie HLA-Molekülen nachweisen.

Basierend auf der Sequenz von TPTE (SEQ ID NO:19) wurden erfindungsgemäß PCR-Primer generiert (5'-TGGATGTCACTCTCATCCTTG-3' and 5'-CCATAGTTCCTGTTCTATCTG3'-) und für RT-PCR-Analysen (95° 15min; 94° 1 min; 63° 1 min; 72° 1 min; 35 Zyklen) in einer Reihe von humanen Geweben eingesetzt. Es zeigte sich, dass die Expression in Normalgeweben auf Testis beschränkt ist. Wie für die anderen eCT beschrieben, konnte erfindungsgemäß gezeigt werden, dass TPTE-Varianten ektop in einer Reihe von Tumorgeweben aktiviert werden; vgl. Tabelle 2.

**Tabelle 2. Expression von TPTE in Tumoren**

| **Gewebe** | **insgesamt getestet** | **Positiv** | **%** |
|---|---|---|---|
| **Melanom** | **18** | **9** | **50** |
| **Mammakarzinome** | **36** | **17** | **47** |
| **Kolorektale Tumoren** | **20** | **0** | **0** |
| **Prostatakarzinome** | **8** | **3** | **38** |
| **Bronchialkarzinome** | **45** | **25** | **55** |
| **Nierenzellkarzinome** | **7** | **2** | **29** |
| **Ovarialkarzinome** | **7** | **2** | **29** |
| **Schilddrüsenkarzinome** | **4** | **0** | **0** |
| **Zervixkarzimome** | **6** | **1** | **17** |
| **Melanom Zellinien** | **8** | **5** | **62** |
| **Bronchialkarzinom Zellinien** | **6** | **2** | **33** |
| **Mammakarzinom Zellinien** | **5** | **4** | **80** |

Es wurde eine quantitative Realtime-PCR (40 Zyklen, initiale Denaturierung 15 min 95°C, 30 sek 94°C, 30 sek 62°C, und 30 sek 72°C) mit den spezifischen Primern (sense GAGTCTACAATCTATGCAGTG; antisense CCATAGTTCCTGTTCTATCTG) durchgeführt (Abb. 19). Diese bestätigt nicht nur das Fehlen von TPTE in nicht-testikulärem Normalgewebe und die ektope Expession in Tumoren, sondern deckt hohe Transkriptmengen in den Tumoren auf, die mit der physiologischen expression in Testisgewebe durchaus vergleichbar sind. Die ektope Expression vieler bekannter CT-Gene in Tumoren wird durch Promotordemethylierung induziert. Zur Untersuchung ob dieser Mechanismus auch für die ektope Aktivierung von TPTE effektiv ist, wurden die nicht-exprimierenden Zellinien BT549 (Mamma-Ca) und HCT116 (Kolon-Ca) mit 5-Aza-2'-deoxycytidin, einem pharmakologischen Inhibitor der DNA-Methylierung, kultiviert. In beiden Fällen resultierte die Demethylierung der DNA in einer starken Induktion der TPTE-Expression (Abb. 33). Der gleiche Effekt zeigt sich auch in DNA-Methyltransferase DNMT1 und DNMT3b defizienten HCT116 Zellen, die durch fast vollständigen Verlust der DNA-Methylierung charakterisiert sind. Diese Ergebnisse belegen dass die Promoterdemethylierung des Genlokus auch für die ektope Induktion der TPTE-Expression effektiv ist.

Zum Nachweis von TPTE Protein wurden Antikörper durch Immunisieren von Kaninchen hergestellt. Folgende Peptide bzw. ein rekombinant in E. coli exprimiertes und gereinigtes Protein wurden zur Propagierung dieser Antikörper genutzt:
SEQ ID NO:103: FTDSKLYIPLEYRSC (aa 116 -128)
SEQ ID NO:104: CFDIKLLRNIPRWT (aa 179 -191)
SEQ ID NO: 105: MNESPDPTDLAGVIIELGPNDSPQTSEFKGATEEAPAKESPHTS EFKGAARVSP (rek.Prot. aa 1-54)

Beispielhaft sollen die Daten dargestellt werden für Antikörper, die gegen SEQ ID NO:105 gerichtet sind. Der spezifische Antikörper lässt sich unter verschiedenen Fixationsbedigungen für Immunfluoreszenz-Untersuchungen nutzen. Bei vergleichenden Färbungen von RT-PCR positiven wie auch negativen Zellinien ist das entsprechende Protein in gut nachweisbarer Menge spezifisch in den als positiv typisierten Zellinien detektierbar (Abb. 18, rechts). Das endogene Protein ist membranlokalisiert. Dieser Antikörper wurde des weiteren eingesetzt zur immunhistochemischen Färbung von Gewebsschnitten. Wie erwartet färbt der Antikörper Testisgewebe spezifisch an. Insbesondere sind es Keimzellen, die angefärbt werden (Abb. 20). Auch Gewebeschnitte von Lungenkarzinomen lassen sich anfärben. TPTE Protein lässt sich dabei homogen und in grosser Menge in allen Zellen eines Tumors detektieren. Auch hier bestätigt sich die Lokalisation des Proteins an der Membran der Zellen (Abb. 21). Die Spezifität der Färbung wurde durch Kompetitionsexperimente bestätigt. Der spezifische Antikörper wurde zunächst mit rekombinantem TPTE Protein vor-inkubiert und danach auf die Gewebeschnitte (z.B. Testis als Positivkontrolle, Abb. 20) verbracht. Hierdurch lässt sich die Reaktivität erfolgreich blockieren. Mit diesem Antikörper lassen sich viele verschiedene primäre Tumortypen (auch Brusttumoren, Melanome etc.) aber nicht das dazugehörige Normalgewebe erfolgreich anfärben. Beispielhaft ist auch dargestellt eine Färbung von Prostatagewebe. Normales Prostatagewebe ist negativ, während invasive wie auch noch nicht invadierte Prostatatumoren positiv für dieses Molekül sind. Auch eine einfache Hypertrophie von Prostata-Epithel färbt sich nicht an, allerdings innerhalb dieser gutartigen Veränderung bereits vorliegende premaligne Bereiche, die in der Folge transformieren werden sind TPTE positiv. Entsprechend kann ein solcher spezifischer Antikörper gegen TPTE dazu dienen, diagnostisch eine bestehende Anlage für Prosatakarzinome früh zu detektieren. Des weiteren wurde der Antikörper zum Expressionsnachweis von TPTE im Westem-Blot eingesetzt. Proteinexpression konnte in Lysaten aus Testisgewebe und spontan exprimierenden Zellinien nachgewiesen werden, Normalgewebe zeigten wie erwartet keine Expression (Abb. 34).

Erfindungsgemäß wurden für TPTE weitere Splicevarianten identifiziert (SEQ ID NO:20, SEQ ID NO:21, SEQ ID NO:54, SEQ ID NO:55, SEQ ID NO:56, SEQ ID NO:57), die in testikulärem Gewebe und Tumoren exprimiert werden und Leserasterverschiebungen und somit veränderte Sequenzbereiche aufweisen (Abb. 5).

Die genomische Sequenz von TPTE besteht aus 24 Exons (Zugangsnummer NT_029430). Das in SEQ ID NO:19 gezeigte Transkript enthält alle diese Exons. Die in SEQ ID NO:20 gezeigte Splicevariante entsteht durch heraussplicen von Exon 7. Die in SEQ ID NO:21 gezeigte Splicevariante zeigt eine partielle Inkorporation eines Introns, das auf Exon 15 folgt. Wie aus den Varianten SEQ ID NO:54, SEQ ID NO:55, SEQ ID NO:56, SEQ ID NO:57 ersichtlich, können alternativ auch Exons 18, 19, 20 und 21 herausgesplict werden.

Diese alternativen Splicing-Ereignisse resultieren in Veränderungen des kodierten Proteins, wobei das Leseraster grundsätzlich erhalten bleibt (Abb. 6). Beispielsweise weist das Translationsprodukt, das von der in SEQ ID NO:20 gezeigten Sequenz kodiert wird (SEQ ID NO:23) eine Deletion von 13 Aminosäuren im Vergleich zu der in SEQ ID NO:22 gezeigten Sequenz auf. Das Translationsprodukt, das von der in SEQ ID NO:21 gezeigten Sequenz kodiert wird (SEQ ID 24) trägt eine zusätzliche Insertion im zentralen Bereich des Moleküls und unterscheidet sich dadurch um 14 Aminosäuren von den anderen Varianten.

Die Translationsprodukte der Varianten SEQ ID NO:54, SEQ ID NO:55, SEQ ID NO:56, SEQ ID NO:57 nämlich die Proteine SEQ ID NO:58, SEQ ID NO:59, SEQ ID NO:60, SEQ ID NO:61 sind ebenfalls verändert.

Analysen zur Prädiktion funktioneller Domänen ergeben für SEQ ID NO:22, SEQ ID NO:23, SEQ ID NO:24, SEQ ID NO:58, SEQ ID NO:60, aber nicht für SEQ ID NO:59, SEQ ID NO:61, das Vorhandensein einer Tyrosinphosphatase-Domäne. Für alle Varianten werden 3-4 Transmembrandomänen prädiziert (Abb. 6).

Die Analyse der TPTE-Antigen-Expression mit spezifischen Antikörpern bestätigte die selektive Expression in Testis sowie in einer Reihe von unterschiedlichen Tumoren. Durch Kolokalisationsuntersuchungen zeigte sich zudem, dass TPTE erfindungsgemäß mit Klasse I Histokompatibilitätsantigenen an der Zelloberfläche von Tumorzellen lokalisiert ist. Bisher war TPTE lediglich als Golgi-assoziiertes Protein beschrieben worden. Aufgrund der Expression von TPTE auf der Zelloberfläche von Tumorzellen eignet sich dieses Tumorantigen erfindungsgemäß als hervorragendes Target für die Entwicklung diagnostischer und therapeutischer monoklonaler Antikörper. Aufgrund der prädizierten Membrantopologie von TPTE eignen sich für diesen Zweck erfindungsgemäß insbesondere die extrazellulär exponierten Bereiche. Dies umfasst erfindungsgemäß die Peptide FTDSKLYIPLEYRS (SEQ ID NO:81) und FDIKLLRNIPRWT (SEQ ID NO:82). Darüber hinaus konnte gezeigt werden, dass TPTE die Migration von Tumorzellen fördert.

Zellen die für mehrere Stunden in Minimalmedium ohne FCS kultiviert werden neigen zur spontanen Ausbildung von Pseudopodien und Membranprotrusionen. Sowohl in heterolog exprimierenden Zellen (TPTE-eGFP) als auch in spontan exprimierenden Zellinien (AK-Färbung) konnte eine ausgeprägte Anreicherung des Proteins an der Membran dieser Struktur nachgewiesen werden. Ferner ergab eine Ko-Färbung mit Rhodamine-Phalloidin eine eindeutige Ko-Lokalisation von TPTE mit F-Aktin in diesen Bereichen. Die durch Second Messenger wie Phosphoinisitide und Kalzium vermittelte Aktin-Polymerisierung ist ein kritischer Faktor für die Initiierung der Zellmigration in Reaktion auf extrazelluläre Gradienten von Chemotaktika. Die Signaltransduktion über diese Second Messenger wird hauptsächlich durch Rezeptor-Tyrosinkinasen (RTK) initiiert. Einer der wichtigsten Faktoren in der Regulation des Second Messenger Signalling über Phosphoinositide und der daraus folgenden Modulation des Aktin-Zytoskeletts ist die RTK c-erbB-2 (HER2), die in verschiedenen Tumortypen frequent überexprimiert wird. Diese Ergebnisse führten zur Überlegung dass TPTE als zellmembranständige Lipidphosphatase mit Substratspezifität für PIP₃,₄,₅ eine regulierende Funktion auf die RTK-vermittelte Signaltransduktion hat und somit modulierende Effekt auf die Membrandynamik in Tumorzellen ausübt. Für eine detailierte Analyse wurde TPTE-eGFP in NIH3T3 Fibroblasten exprimiert die durch Transformation mit c-erbB-2 ein konstitutiv aktiviertes PI-3 Kinase Signalling und somit eine Überproduktion des Second Messengers PIP₃,₄,₅ aufweisen. Time-Lapse Mikroskopie dieser Zellen nach mehrstündiger Kultivierung in Minimalmedium zeigte, dass die Lokalisation von TPTE an der Membran von Pseudopodien und Protrusionen unverzüglich zur Retraktion des entsprechenden Memranbereichs führt. Eine Erklärung für diese Beobachtung bietet der PI-3 Kinase antagonistische Effekt der TPTE der durch die Termination der RTK-vermittelten Signaltransduktion die Aktin-Polimerisation unter dem betroffene Membranbereich beendet. Diese PI-3K antagonistischen Effekte von TPTE werden durch Dephosphorylierung des Second Messengers PIP₃,₄,₅ zu PIP₄,₅ vermittelt. PIP₄,₅ reguliert die kortikale Aktinpolymerisation und vermittelt die Adhäsion des Zytoskeletts an die Plasmamembran. Zum Nachweis der Kolokalisation von TPTE und PIP_{4,5} and wurden TPTE transfizierte NIH3T3/c-erbB2 Zellen mit der eGFP gekoppelten PH-Domäne von Phospholipase C-δ1 (PLC-δ1) kotransfiziert. Die PH-Domäne vermittelt die spezifische Bindung von PLC-δ1 an ihr physiologisches Substrat PIP_{4,5}, daher ist sie für die Darstellung der PIP₄,₅ Verteilung and der Plasmamembran hervorragend geeignet. Erwartungsgemäss zeigte sich in den kotransfizierten Zellen eine eindeutige Kolokalisation von TPTE und PIP₄,₅ an der Plasmamembran (Abb. 35). Dies konnte in weiteren Untersuchungen durch Kofärbung mit einem PIP_{4,5}-spezifischen Antikörper nochmals bestätigt werden. Kürzlich wurde gezeigt, dass das zu TPTE homologe PTEN das migratorische Potential von Zellen positiv reguliert. Zellmigration ist ein direktionaler Prozess für den die Wahrnehmung extrazellulärer Gradienten von Chemotaktika von essentieller Bedeutung ist. Gerichtete Zellmigration bedingt, dass die Zelle nach Detektion des Chemotaktikums in Richtung des Gradienten polarisiert. PTEN vermittelt die Polarisation der Zelle durch Ausbildung eines intrazellulären Gradienten von Second Messenger PIP₃,₄,₅ in Richtung des extrazellulären Gradienten und somit eine gerichtete Aktin-Polimerisation und Migration. Zur Untersuchung, ob TPTE ähnliche promigratorische Eigenschaften besitzt wurde ein Transwell-Migrationsassay durchgeführt. TPTE transfizierte NIH3T3/c-erbB-2 Zellen und entsprechende Kontrollzellen (nicht-transfizierte NIH3T3/c-erbB-2 Zellen und mit Leer-peGFP-Vektor transfizierte NIH3T3/c-erbB-2 Zellen) wurden mit verschiedenen Mengen an chemischen Lockstoffen getestet. Zellen, die TPTE exprimierten, zeigten eine signifikant erhöhte Migrationsrate von mehr als vierfach im Vergleich zu den Kontrollzellen in Reaktion auf 10% FCS (Abb. 23). Durch den Einsatz von RNA-Interferenz (RNAi) konnte der Nachweis erbracht werden dass auch die spontane Expression von TPTE in Tumorzellinien die Chemotaxis dieser Zellen verstärkt. Hierzu wurden TPTE-positive PC-3 Prostatakarzinomzellen mit TPTE-spezifischen doppelsträngigen RNA-Oligos (sense 5'-CCCUGCCACAUGUUCAUAUdTdT-3' und antisense 5'-AUAUGAACAUGUGGCAGGGdTdT-3') elektroporiert. Nach 24 Stunden konnte mittels quantitativer Real-Time RT-PCR eine Abnahme der TPTE-Expression um 70% nachgewiesen werden (Abb. 36). Im Transwell-Migrationsassay zeigte sich eine deutliche Reduktion der Migrationsrate (70%) im Vergleich zu den entsprechenden Kontrollen (unbehandelte PC-3 Zellen und PC-3 Zellen die mit irrelevanter siRNA elektroporiert wurden) (Abb. 37). Während PTEN ein cytoplasmatisches Protein ist, das mittels PH-Domänen-rekrutierenden Second Messengern wie PIP₃,₄,₅ zu der Plasmamembran gelenkt werden kann, könnte TPTE im Gegensatz dazu eine Wahrnehmung von chemotaktischen Gradienten direkt an der Membran durch eine Blockierung der Signaltransduktion von aktivierten RTKs modulieren, die häufig in Krebszellen überexprimiert sind, und dadurch eine Wahrnehmung eines räumlichen Gradienten vermitteln.

Phosphatasen sind oft bei der Zellmotilität und Migration involviert. Um die Bedeutung der TPTE bei Zellbewegung zu prüfen, wurde TPTE als Fusionsprotein mit Green-Fluorescence Protein in eine TPTE-negative Zellinie eingebracht und die Verteilung dieses Proteins in vivo mittels Real-time Mikroskopie beobachtet. Zellen bilden Membranausläufer (Protrusionen) als Vorstufe zur Migration. Nach Ausbildung einer solchen Protrusion sammelt sich TPTE an diesem Membranbereich an (Abb. 22). Bei beginnender Abflachung der Protrusion wird TPTE von der Membran zurückgezogen und internalisiert und ist offensichtlich in den Ablauf von Motilitätsereignissen der Zelle involviert. Gleichsam ergibt der Vergleich der Wandereigenschaften der mit TPTE- versus Kontrollplasmid-transfizierten Zellen in der Migrationskammer (BOYDEN Chamber), dass TPTE zu einer signifikanten Erhöhung der Migration entlang chemotaktischer Gradienten von Wachstumsfaktoren (hier z.B. PDGF=Platelet-derived Growth Factor, oder FCS=Fetales Kalbsserum) führt (Abb. 23).

Da TPTE einer Zelle offensichtlich promigratorische Eigenschaften vermittelt, wurden Brust- bzw. Lungentumoren von insgesamt 58 Patienten auf Expression von TPTE untersucht und statistisch mit derm Krankheitsverlauf der Patienten verglichen. Diejenigen Tumoren, die TPTE exprimieren, neigen signifikant häufiger zu Lymphknoten- aber auch zu Fernmetastasen als die TPTE-negativen (Abb. 24).

Diese funktionellen Daten deuten darauf hin, dass TPTE bei der Metastasierung von Tumoren eine wesentliche Rolle spielt. Entsprechend kann TPTE als Marker für prognostisch schlechteren Verlauf und Metastasenneigung gelten. Ferner können Verfahren, die erfindungsgemäß die endogene Aktivität von TPTE in Tumorzellen inhibieren, z.B. durch den Einsatz von Antisene RNA, unterschiedlicher Methoden der RNA-Interferenz (RNAi) mittels Expressionsvektoren oder Retroviren, sowie durch den Einsatz von kleinen Moleküle, zu einer reduzierten Meatstasierung führen und somit therapeutisch sehr bedeutsam sein.

### Beispiel 3: Identifizierung von TSBP als neues Tumorantigen

Das erfindungsgemäß eingesetzte elektronische Klonierungsverfahren ergab TSBP (SEQ ID NO:29) und das davon abgeleitete Protein (SEQ ID NO:30). Das Gen ist als Testis-spezifisch reguliert vorbeschrieben (Zugangsnummer NM_006781). Es wurde prädiziert, dass das Gen ein basisches Protein kodiert und auf Chromosom 6 in der Nähe einer für einen MHC-Komplex kodierenden Sequenz (C6orf10) lokalisiert ist (Stammers M. et al., Immunogenetics 51(4-5):373-82, 2000). Erfindungsgemäß wurde gezeigt, dass die vorbeschriebene Sequenz fehlerhaft ist. Die erfindungsgemäße Sequenz weist signifikante Sequenzunterschiede zu der bekannten Sequenz auf. Erfindungsgemäß wurden 3 verschiedene Splicingvarianten kloniert. Die Unterschiede in den Nukleotidsequenzen der erfindungsgemäß aufgefundenen TSBP-Varianten (SEQ ID NO:31, SEQ ID NO:32, SEQ ID NO:33) zu der bekannten Sequenz (NM_006781, SEQ ID NO:29) sind in Abb. 7 gezeigt (Unterschiede fett dargestellt). Sie führen zu Leserasterverschiebungen, so dass sich die Proteine, die durch die erfindungsgemäß aufgefundenen TSBP-Varianten kodiert werden (SEQ ID NO:34, SEQ ID NO:35, SEQ ID NO:36) substantiell von dem vorbeschriebenen Protein (SEQ ID NO:30) unterscheiden (Abb. 8).

Für dieses Antigen wurde erfindungsgemäß eine strikte transkriptionale Repression in Normalgeweben bestätigt (PCR-primer 5'-TCTAGCACTGTCTCGATCAAG-3' und 5'-TGTCCTCTTGGTACATCTGAC-3'). Allerdings wurde in 25 untersuchten Normalgeweben außer in Testis auch in normalem Lymphknotengewebe TSBP exprimiert. Erfindungsgemäß wurde auch für TSBP eine ektope Aktivierung in Tumoren nachgewiesen, weshalb es sich als Tumormarker bzw. Tumor-assoziiertes Antigen qualifiziert (Tabelle 3).

TSBP-Expression findet sich zwar in primärem Tumorgewebe aber nicht in permanenten Zellinien aus entsprechenden Tumorentitäten. Außerdem liegt das Gen in direkter Nachbarschaft zu Notch 4, das eine spezifische Expression in Arterien aufweißt und in die vaskuläre Morphogenese involviert ist. Dies sind signifikante Hinweise dafür, dass es sich hier um einen Marker für spezifische Endothelzellen handelt. TSBP kann daher als potentieller Marker für Tumorendothelien und für neovaskuläres Targeting dienen.

Mithin kann der Promotor von TSBP an ein anderes Genprodukt kloniert werden, dessen selektive Expression im Lymphknoten erwünscht ist.

Die Analyse der TSBP-Antigen-Expression mit spezifischen Antikörpern bestätigte die selektive Lokalisation des Proteins in Testis und Lymphknoten sowie in Melanomen und Bronchialkarzinomen. Darüber hinaus zeigten immunhistologische Untersuchungen mit GFPgetaggtem TSBP eine ausgeprägte perinukleäre Akkumulation.

**Tabelle 3. Expression von TSBP in Tumoren**

| **Gewebe** | **insgesamt getestet** | **Positiv** | **%** |
|---|---|---|---|
| **Melanom** | **12** | **2** | **16** |
| **Mammakarzinome** | **15** | **0** | **-** |
| **Kolorektale Tumoren** | **15** | **0** | **-** |
| **Prostatakarzinome** | **8** | **0** | **-** |
| **Bronchialkarzinome** | **7** | **17** | **41** |
| **Nierenzellkarzinome** | **7** | **0** | **-** |
| **Ovarialkarzinome** | **7** | **0** | **-** |
| **Schilddrüsenkarzinome** | **4** | **0** | **-** |
| **Zervixkarzimome** | **6** | **0** | **-** |
| **Melanom Zellinien** | **8** | **0** | **-** |
| **Bronchialkarzinom Zellinien** | **6** | **0** | **-** |

Zum Nachweis von TSBP Protein wurden Antikörper durch Immunisieren von Kaninchen hergestellt. Folgende Peptide bzw. ein rekombinant in E. coli exprimiertes und gereinigtes Protein wurden zur Propagierung dieser Antikörper genutzt:
SEQ ID NO:106: CYQHKVTLHMITERDP (aa 60 - 74)
SEQ ID NO:107: CRIPQVHTMDSSGKI (aa 191- 204)
SEQ ID NO:108:

RRKQSEMHISRYSSEQSARLLDYEDGRGSRHAYSTQSDTSCDNRERSKRDYTPSTNSL
ALSRSSIALPQGSMSSIKCLQTTEELPSRTAGAMMQFTAPIPGATGPIKLSQKTIVQTPG
PIVQYPGPNVRSHPHTITGPPSAPRGPPMAPIIISQRTASQLAAPIIISQRTARIPQVHTM
DSSGKTTLTPVVILTGYMDEELAKKSCSKIQILKCGGTARSQNSREENKEALKNDIIFT
NSVESLKSAHIKEPEREGKGTDLEKDKIGMEVKVDSDAGIPKRQETQLKISEMSIPQG
QGAQIKKSVSDVPRGQESQVKKSESGVPKGQEAQVTKSGLVVLKGQEAQVEKSEMG
VPRRQESQVKKSQSGVSKGQEAQVKKRESVVLKGQEAQVEKSELKVPKGQEGQVE
KTEADVPKEQEVQEKKSEAGVLKGPESQVKNTEVSVPETLESQVKKSESGVLKGQEA
QEKKESFEDKGNNDKEKERDAEKDPNKKEKGDKNTKGDKGKDKVKGKRESEINGE
KSKGSKRAKANTGRKYNKKVEE (rek.Prot. 29 – 569)

Beispielhaft sollen die Daten dargestellt werden für Antikörper, die gegen SEQ ID NO:108 gerichtet sind. Der spezifische Antikörper lässt sich unter verschiedenen Fixationsbedigungen für Immunfluoreszenz-Untersuchungen nutzen. So ergaben RT-PCR Untersuchungen, dass die Kolonkarzinom Zellinie HCT 116 P kein TSBP exprimiert. Ihre DNA Methyltransferase deletierte Variante HCT 116 DKO allerdings positiv für TSBP ist. Vergleichende Färbungen beider Zellinien mit dem oben genannten Antikörper detektierten das entsprechende Protein in gut nachweisbarer Menge spezifisch in den als positiv typisierten Zellinien (Abb. 25). Das endogene Protein ist in erster Linie an der Kern- und ER-Membran lokalisiert mit einer leichten Anfärbung der Plasmamembran.

Dieser Antikörper wurde des weiteren eingesetzt zur immunhistochemischen Färbung von Gewebsschnitten. Wie erwartet färbt der Antikörper Testisgewebe spezifisch an. Insbesondere sind es Keimzellen, die angefärbt werden (Abb. 25).

In der bioinformatischen Analyse zeigt sich dass TSBP ein stark basisches Protein mit nukleärem Lokalisationssignal ist. Dementsprechend wird die subzelluläre Lokalisation des Proteins eindeutig nukleär prädiziert, während das endogen exprimierte Protein wie oben beschrieben an der Kernmembran lokalisiert ist. Der N-Terminus enthält eine Transmembrandomäne gefolgt von einer proteolytischen Spaltstelle, was darauf schliessen lässt dass die Transmembrandomäne im Verlauf der Proteinprozessierung durch Proteasen abgespalten wird. Diese Kombination von nukleären Lokalisationssignalen, Lokalisation des Proteins an Kern-, ER- oder Plasma-Membranen und Transmembrandomänen gefolgt von proteolytischen Spaltstellen findet sich bei einer distinkten Klasse von Transkriptionsfaktoren von denen Mitglieder der NOTCH-Familie und SREBP am besten charakterisiert sind (Weinmaster G. Curr. Opin. Genet. Dev. 10(4):363-9, 2000; Hoppe T. et al. Curr. Opin. Cell. Biol. 13(3):344-8, 2001)). Die biologische Aktivität dieser Faktoren wird durch den Mechanismus der Regulierten Intramembran Proteolyse (RIP) reguliert. Nach der Translation werden die Proteine über die Transmembrandomäne in die Kern-, ER- oder Plasmamembran integriert. Im Falle von NOTCH induziert die Bindung eines spezifischen Liganden die proteolytische Abspaltung unterhalb der Membrandomäne so dass das Protein an den Ort der Wirkung gelangt. Transkriptionsprozesse können auf diese Weise z.B. im Laufe der Organentwicklung milieuabhängig reguliert werden. Aufgrund seiner strukturellen Merkmale und der membranständigen Lokalisation könnte TSBP in diese Klasse eingeordnet werden. Zwar enthält die TSBP-Sequenz keine spezifischen DNA-bindenden Strukturelemente, doch macht die hohe Basizität des Proteins eine Bindung an DNA wahrscheinlich, so dass es sich bei TSBP um einen Transkriptionsfaktor bzw. transkriptionsmodulierenden Faktor handeln könnte.

### Beispiel 4: Identifizierung von MS4A12 als neues Tumorantigen

MS4A12 (SEQ ID NO:37, Zugangsnummer NM_017716) und sein Translationsprodukt (SEQ ID NO:38) sind als Mitglieder einer Multigen-Familie mit Verwandschaft zum B-Zell spezifischen Antigen CD20, dem hämatopoesezell-spezifischen Protein HTm4, und der β-Kette des high-affinity IgE-Rezeptors vorbeschrieben. Als Charakteristikum besitzen alle Familienmitglieder mindestens vier potentielle Transmembrandomänen, sowohl der C- als auch der N-Terminus sind cytoplasmatisch (Liang Y. et al., Immunogenetics 53(5):357-68, 2001; Liang Y. & Tedder, Genomics 72(2):119-27, 2001). Erfindungsgemäß wurden RT-PCR Untersuchungen für MS4A12 durchgeführt. Die Primer wurden basierend auf der veröffentlichten MS4A12-Sequenz (NM_017716) ausgewählt (sense: CTGTGTCAGCATCCAAGGAGC, antisense: TTCACCTTTGCCAGCATGTAG). In den getesteten Geweben konnte eine Expression nur in Testis, Kolon (6/8) und kolorektalen Karzinomen (Kolon-Ca's) (16/20) sowie in Kolon-Metastasen nachgewiesen (12/15) werden (Abb. 9).

Die hohe Inzidenz in Kolon-Metastasen machen TSBP zu einem attraktiven diagnostischen und therapeutischen Target. Für die Herstellung von monoklonalen Antikörpern und kleiner chemischer Inhibitoren eignet sich erfindungsgemäß besonders der pädizierte extrazelluläre Bereich der die Proteinsequenz GVAGQDYWAVLSGKG (SEQ ID NO:83) umfasst. Die intrazelluläre Lokalisation des MS4A12-Proteins an der Zellmembran wurde erfindungsgemäß auch durch Fluoreszenzüberlagerung mit Plasmamembranmarkern in der konfokalen Immunfluoreszenz bestätigt.

**Tabelle 4. Expression von MS4A12 in Normalgeweben und kolorektalen Karzinomen und Metastasen**

| | |
|---|---|
| **Ileum** | **+** |
| **Kolon** | **+** |
| **Leber** | **-** |
| **Lunge** | **-** |
| **Lymphknoten** | **-** |
| **Magen** | **-** |
| **Milz** | **-** |
| **Nebenniere** | **-** |
| **Niere** | **-** |
| **Ösophagus** | **-** |
| **Ovar** | **-** |
| **Rektum** | **+** |
| **Testis** | **+** |
| **Thymus** | **-** |
| **Haut** | **-** |
| **Mamma** | **-** |
| **Pankreas** | **-** |
| **PBMC** | **-** |
| **PBMC akt.** | **-** |
| **Prostata** | **-** |
| **Schilddrüse** | **-** |
| **Tube** | **-** |
| **Uterus** | **-** |
| **Grosshirn** | **-** |
| **Kleinhirn** | **-** |
| **Kolorektale Tumoren** | **16/20** |
| **Kolorektale Tumoren Metastasen** | **12/15** |

Somit ist MS4A12 ein zellmembranständiges Differenzierungsantigen für normales Kolon-Epithel, das auch in kolorektalen Tumoren und Metastasen exprimiert wird.

Es wurde eine quantitative Realtime-PCR (40 Zyklen, initiale Denaturierung 15 min 95°C, 30 sek 94°C, 30 sek 62°C, und 30 sek 72°C) mit den spezifischen Primern (CTGTGTCAGCATCCAAGGAGC und TTCACCTTTGCCAGCATGTAG) durchgeführt (Abb. 26). Diese bestätigt nicht nur das Fehlen von MS4A12 in der Mehrheit der Normalgewebe ausser Testis und colorektalen Normalgeweben. Sondern sie deckt hohe Transkriptmengen in z.B. primären Darmtumoren und ihren Metastasen auf. Zum Nachweis von MS4A12 Protein wurden Antikörper durch Immunisieren von Kaninchen hergestellt. Folgende Peptide bzw. ein rekombinant in E.Coli exprimiertes und gereinigtes Protein wurden zur Propagierung dieser Antikörper genutzt:
SEQ ID NO: 109: MMSSKPTSHAEVNETC (aa 1-15)
SEQ ID NO:110: CGVAGQDYWAVLSGKG (aa 64 - 73)
SEQ ID NO:111: MMSSKPTSHAEVNETIPNPYPPGSFMAPGFQQPLGSINLENQAQGAQRAQPYGITSPGI FASS (rek.Prot. aa 1-63)

Beispielhaft sollen die Daten dargestellt werden für Antikörper, die gegen SEQ ID NO:111 gerichtet sind. Im Westernblot ist auf erwarteter Höhe in normalem Darm, in Kolonkarzinomen aber nicht in anderen Normalgeweben eine spezifischen Bande nachweisbar (Abb. 27). Der spezifische Antikörper lässt sich unter verschiedenen Fixationsbedigungen für Immunfluoreszenz-Untersuchungen nutzen. Bei Färbungen von RT-PCR positiven wie auch negativen Zellinien ist das entsprechende Protein in gut nachweisbarer Menge spezifisch in den als positiv typisierten Zellinien detektierbar und auch eukaryontisch rekombinant exprimiertes MS4A12 wird spezifisch erkannt (Abb. 28). Auch diese Experimente bestätigen die Membranlokalisation. Dieser Antikörper wurde des weiteren eingesetzt zur immunhistochemischen Färbung von Gewebsschnitten. Wie erwartet färbt der Antikörper gesundes Darmgewebe. Allerdings sind es lediglich die apikalen Epithelzellen der Kolonmukosa (Abb. 29, links). Tumorzellen von Darmtumoren werden homogen und ebenfalls an der Membran angefärbt (Abb. 29, rechts).

### Beispiel 5: Identifizierung von BRCO1 als neues Tumorantigen

BRCO1 und sein Translationsprodukt sind nicht vorbeschrieben. Das erfindungsgemäße Datamining-Verfahren ergab das EST (expressed sequence tag) AI668620. Mit spezifischen Primern (sense: CTTGCTCTGAGTCATCAGATG, antisense: CACAGAATATGAGCCATACAG) wurden RT-PCR Studien zur Expressionsanalyse durchgeführt. Erfindungsgemäß wurde spezifische Expression in testikulärem Gewebe, sowie zusätzlich in normaler Brustdrüse gefunden (Tabelle 5). In allen anderen Geweben ist dieses Antigen transkriptionell reprimiert. In Brustdrüsentumoren wird es ebenfalls (20 von 20) nachgewiesen. In Brusttumoren ist BRCO1 im Vergleich zu der Expression in normalem Brustdrüsengewebe deutlich überexprimiert (Abb. 10).

Durch elektronische Vollängeklonierung unter Nutzung von EST-contigs (folgende EST wurden inkorporiert AW137203, BF327792, BF327797, BE069044, BF330665) gelang erfindungsgemäß die Klonierung von mehr als 1500 bp dieses Transkriptes (SEQ ID NO:39). Die Sequenz kartiert auf Chromosom 10p11-12. In der gleichen Region in unmittelbarer Nähe ist bereits das Gen für ein Mamma-DifferenzierungsAntigen NY-BR-1 beschrieben worden (NM_052997; Jager, D. et al., Cancer Res. 61(5):2055-61, 2001).

**Tabelle 5. Expression von BRCO1 in Normalgeweben und Brusttumoren**

| | |
|---|---|
| **Ileum** | **-** |
| **Kolon** | **-** |
| **Leber** | **-** |
| **Lunge** | **-** |
| **Lymphknoten** | **-** |
| **Magen** | **-** |
| **Milz** | **-** |
| **Nebenniere** | **-** |
| **Niere** | **-** |
| **Ösophagus** | **-** |
| **Ovar** | **-** |
| **Rektum** | **-** |
| **Testis** | **+** |
| **Thymus** | **-** |
| **Haut** | **-** |
| **Mamma** | **+** |
| **Pankreas** | **-** |
| **PBMC** | **-** |
| **PBMC akt.** | **-** |
| **Prostata** | **-** |
| **Schilddrüse** | **-** |
| **Tube** | **-** |
| **Uterus** | **-** |
| **Grosshirn** | **-** |
| **Kleinhirn** | **-** |
| **Mammakarzinome** | **++ (20/20)** |

In matched pair (Mama-Karzinom und benachbartes Normalgewebe) Untersuchungen zeigte sich erfindungsgemäß in 70 % der Mama-Karzinome eine BRCO1 Überexpression im Vergleich zum Normagewebe.

Somit ist BRCO1 ein neues DifferenzierungsAntigen für normales Brustdrüsenepithel, das in Brusttumoren überexprimiert wird.

Dies bestätigt auch die quantitative Realtime-PCR (40 Zyklen, initiale Denaturierung 15 min 95°C, 30 sek 94°C, 30 sek 60°C, und 30 sek 72°C) mit spezifischen Primern (5'-CTTGCTCTGAGTCATCAGATG-3'; 5'-CACAGAATATGAGCCATACAG-3') (Abb. 30 & 31). Diese bestätigte nicht nur das Fehlen von BRCO1 in der Mehrheit der Normalgewebe außer Testis und Brust-Normalgeweben, sondern deckte auch eine Überexpression in Brusttumoren auf.

Zum Nachweis von BRCO1 Protein wurden Antikörper durch Immunisieren von Kaninchen hergestellt. Folgende Peptide wurden zur Propagierung dieser Antikörper genutzt:
SEQ ID NO:115: IAPNTRGQQTIVL
SEQ ID NO:116: VWKSNGKSILKMPF

### Beispiel 6: Identifizierung von TPX1 als neues Tumorantigen

Die Sequenz von TPX1 (Acc-Nr NM_003296; SEQ ID NO:40) und seines Translationsprodukts (SEQ ID NO:41) sind bekannt. Das Antigen ist bisher als lediglich Testis-spezifisch beschrieben worden und zwar als Element der äußeren Fibern und des Akrsosoms von Spermien. Ihm wurde bisher eine Rolle als Adhäsionsmolekül bei der Anheftung von Spermien an Sertoli-Zellen zugeschrieben (O'Bryan, M.K. et al., Mol. Reprod. Dev. 58(1):116-25, 2001; Maeda, T. et al., Dev. Growth Differ. 41(6):715-22, 1999). Erfindungsgemäß wurde erstmals die aberrante Expression von TPX1 in soliden Tumoren gezeigt (Tabelle 6). Aufgrund der ausgeprägten Aminosäure Homologie zwischen TPX1 und dem Neutrophilen-spezifischen Matrix -Glykoprotein SGP 28 (Kjeldsen et al., FEBS Lett 380:246-259, 1996) eignen sich für die Herstellung diagnostischer und therapeutischer Moleküle erfindungsgemäß TPX1-spezifische Proteinsequenzen, die das Peptid SREVTTNAQR (SEQ ID NO:84) umfassen.

**Tabelle 6. Expression von TPX1 in Tumoren**

| **Gewebe** | **insgesamt getestet** | **Positive** | **%** |
|---|---|---|---|
| **Melanom** | **16** | **1** | **6** |
| **Mammakarzinome** | **20** | **3** | **15** |
| **Kolorektale Tumoren** | **20** | **0** | **0** |
| **Prostatakarzinome** | **8** | **3** | **37** |
| **Bronchialkarzinome** | **17** | **2** | **11** |
| **Nierenzellkarzinome** | **7** | **1** | **14** |
| **Ovarialkarzinome** | **7** | **1** | **14** |
| **Schilddrüsenkarzinome** | **4** | **0** | **0** |
| **Zervixkarzimome** | **6** | **1** | **16** |
| **Melanom Zellinien** | **8** | **2** | **25** |
| **Bronchialkarzinom Zellinien** | **6** | **1** | **16** |

### Beispiel 7: Identifizierung von BRCO2 als neues Tumor-Genprodukt

BRCO2 und sein Translationsprodukt sind nicht vorbeschrieben. Das erfindungsgemäße Verfahren ergab die ESTs (expressed sequence tag) BE069341, BF330573 und AA601511. Mit spezifischen Primern (sense: AGACATGGCTCAGATGTGCAG, antisense: GGAAATTAGCAAGGCTCTCGC) wurden RT-PCR Studien zur Expressionsanalyse durchgeführt. Erfindungsgemäß wurde spezifische Expression in testikulärem Gewebe, sowie zusätzlich in normaler Brustdrüse gefunden (Tabelle 7). In allen anderen Geweben ist dieses Genprodukt transkriptionell reprimiert. In Brustdrüsentumoren wird es ebenfalls nachgewiesen. Durch elektronische Vollängeklonierung unter Nutzung von EST-contigs (folgende EST wurden inkorporiert BF330573, AL 044891 und AA601511) gelang erfindungsgemäß die Klonierung von 1300 bp dieses Transkriptes (SEQ ID 62). Die Sequenz kartiert auf Chromosom 10p11-12. In der gleichen Region in unmittelbarer Nähe ist bereits das Gen für ein Mamma-DifferenzierungsGenprodukt NY-BR-1 beschrieben worden (NM_052997; Jager, D. et al., Cancer Res. 61(5):2055-61, 2001) und hier liegt das unter Beispiel 6 vorbeschriebene BRCO1 Weiterführende genetische Analysen zeigten erfindungsgemäß, dass die unter SEQ ID NO:62 aufgeführte Sequenz den bisher nicht beschriebenen 3' untranslatierten Bereich des NY-BR-1 Gens repräsentiert.

**Tabelle 7. Expression von BRCO2 in Normalgeweben und Brusttumoren**

| **Gewebe** | **Expression** |
|---|---|
| **Testis** | **+** |
| **Mamma** | **+** |
| **Haut** | **-** |
| **Leber** | **-** |
| **Prostata** | **-** |
| **Thymus** | **-** |
| **Hirn** | **-** |
| **Lunge** | **-** |
| **Lymphknoten** | **-** |
| **Milz** | **-** |
| **Nebenniere** | **-** |
| **Ovar** | **-** |
| **Leukozyten** | **-** |
| **Kolon** | **-** |
| **Ösophagus** | **-** |
| **Uterus** | **-** |
| **Skelettmuskel** | **-** |
| **Nebenhoden** | **-** |
| **Harnblase** | **-** |
| **Niere** | **-** |
| **Mamma-Ca** | **+** |

BRCO2 ist ein neues Differenzierungs-Genprodukt für normales Brustdrüsenepithel, das auch in Brusttumoren exprimiert wird.

### Beispiel 8: Identifizierung von PCSC als neues Tumor-Genprodukt

PCSC (SEQ_ID_63) und sein Translationsprodukt sind nicht vorbeschrieben. Das erfindungsgemäße Datamining-Verfahren ergab das EST (expressed sequence tag) BF064073. Mit spezifischen Primern (sense: TCAGGTATTCCCTGCTCTTAC, antisense: TGGGCAATTCTCTCAGGCTTG) wurden RT-PCR Studien zur Expressionsanalyse durchgeführt. Erfindungsgemäß wurde spezifische Expression in normalem Kolon, sowie zusätzlich in Kolonkarzinomen gefunden (Tabelle 5). In allen anderen Geweben ist dieses Genprodukt transkriptionell reprimiert. PCSC kodiert für drei putative ORFs (SEQ_ID_64, SEQ_ID_65 und SEQ_ID_117). Sequenzanalyse von SEQ_ID_64 ergab eine strukturelle Homologie zu CXC-Cytokinen. Darüber hinaus wurden 4 alternative PCSC- cDNA-Fragmente kloniert (SEQ ID NO:85-88). Dabei enthält erfindungsgemäß jede cDNA 3 putative ORFs, die für die in SEQ ID NO:89-100 gezeigten Polypeptide kodieren.

**Tabelle 8. Expression von PCSC in Normalgeweben und kolorektalen Karzinomen**

| | |
|---|---|
| **Ileum** | **+** |
| **Kolon** | **+** |
| **Leber** | **-** |
| **Lunge** | **-** |
| **Lymphknoten** | **-** |
| **Magen** | **-** |
| **Milz** | **-** |
| **Nebenniere** | **-** |
| **Niere** | **-** |
| **Ösophagus** | **-** |
| **Ovar** | **-** |
| **Rektum** | **+** |
| **Testis** | **-** |
| **Thymus** | **-** |
| **Haut** | **-** |
| **Mamma** | **-** |
| **Pankreas** | **-** |
| **PBMC** | **-** |
| **PBMC akt.** | **-** |
| **Prostata** | **-** |
| **Schilddrüse** | **-** |
| **Tube** | **-** |
| **Uterus** | **-** |
| **Grosshirn** | **-** |
| **Kleinhirn** | **-** |
| **Kolorektale Tumoren** | **19/20** |
| **Kolorektale Tumoren Metastasen** | **15/15** |

Somit ist PCSC ein Differenzierungsantigen für normales Kolon-Epithel, das auch in kolorektalen Tumoren sowie in allen untersuchten Kolon-Metastasen exprimiert wird. Die erfindungsgemäß in allen kolorektalen Metastasen nachgewiesene PCSC-Expression machen dieses Tumorantigen zu einem sehr interessanten Target für die Prophylaxe und Behandlung von metastasiernden Kolon- Tumoren.

Es wurde eine quantitative Realtime-PCR (40 Zyklen, initiale Denaturierung 15 min 95°C, 30 sek 94°C, 30 sek 59°C, und 30 sek 72°C) mit den spezifischen Primern (5'-AGAATAGAATGTGGCCTCTAG-3'; 5'-TGCTCTTACTCCAAAAAGATG-3') durchgeführt (Abb. 32). Diese bestätigt nicht nur das Fehlen von PCSC in der Mehrheit der Normalgewebe außer Testis und kolorektalen Normalgeweben. Sondern es deckt hohe Transkriptmengen in z.B. Darmtumoren auf. Dabei haben Metastasen von Darmtumoren eine signifikant höhere Expression als Primärtumoren. Dies und der Umstand, dass PCSC Homologien mit der Familie der Chemokine untermauern eine Rolle bei erhöhter Zellmotilität und Metastasierungsvorgängen.

Zum Nachweis von PCSC Protein wurden Antikörper durch Immunisieren von Kaninchen hergestellt. Folgende Peptide wurden zur Propagierung dieser Antikörper genutzt:
SEQ ID NO:112: GHGPGHPPPGPHH
SEQ ID NO:113: KPERIAQLTWNEA
SEQ ID NO:114: PRSPTPWSTSLRK

### Beispiel 9: Identifizierung von SGY-1 als neues Tumorantigen

Die Sequenzen des Transkripts von SGY-1 (SEQ ID NO:70) und seines Translationsprodukt (SEQ ID NO:71) sind in GenBank unter der Zugangsnummer AF177398 veröffentlicht (Krupnik et al., Gene 238, 301-313, 1999). Soggy-1 ist vorbeschrieben als Mitglied der Dickkopf-Protein Familie, welche als Inhibitoren und Antagonisten der Wnt-Familie von Proteinen wirken. Die Wnt-Proteine wiederum haben wichtige Funktionen bei der Embryonalentwicklung. Basierend auf der Sequenz von SGY-1 (SEQ ID NO:70) wurden erfindungsgemäß PCR-Primer generiert (5'-CTCCTATCCATGATGCTGACG-3' und 5'-CCTGAGGATGTACAGTAAGTG-3') und für RT-PCR-Analysen (95° 15min; 94° 1 min; 63° 1 min; 72° 1 min; 35 Zyklen) in einer Reihe von humanen Geweben eingesetzt. Es zeigte sich, dass die Expression in Normalgeweben auf Testis beschränkt ist. Wie für die anderen eCT beschrieben, konnte erfindungsgemäß gezeigt werden, dass SGY-1 ektop in einer Reihe von Tumorgeweben aktiviert wird; vgl. Tabelle 9.

**Tabelle 9. Expression von SGY-1 in Tumoren**

| **Gewebe** | **insgesamt getestet** | **Positiv** | **%** |
|---|---|---|---|
| **Melanom** | **16** | **4** | **25** |
| **Mammakarzinome** | **20** | **4** | **20** |
| **Kolorektale Tumoren** | **20** | **0** | **0** |
| **Prostatakarzinome** | **8** | **1** | **13** |
| **Bronchialkarzinome** | **32** | **3** | **18** |
| **Nierenzellkarzinome** | **7** | **0** | **0** |
| **Ovarialkarzinome** | **7** | **4** | **57** |
| **Schilddrüsenkarzinome** | **4** | **0** | **0** |
| **Zervixkarzimome** | **6** | **2** | **33** |
| **Melanom Zellinien** | **8** | **2** | **25** |
| **Bronchialkarzinom Zellinien** | **6** | **2** | **33** |
| **Mammakarzinom Zellinien** | | | |

### Beispiel 10: Identifizierung von MORC als neues Tumorantigen

Die Sequenzen des Transkripts von MORC (SEQ ID NO:74) und seines Translationsprodukt (SEQ ID NO:75) sind in GenBank unter der Zugangsnummer XM_037008 veröffentlicht (Inoue et al., Hum Mol Genet. Jul; 8(7):1201-7, 1999*).*

MORC ist ursprünglich beschrieben als involviert in die Spermatogenese. Mutation dieses Proteins im Maussystem führt zur Gonadenunterentwicklung.

Basierend auf der Sequenz von MORC (SEQ ID NO:74) wurden erfindungsgemäß PCR-Primer generiert (5'-CTGAGTATCAGCTACCATCAG-3' und 5'-TCTGTAGTCCTTCACATATCG-3') und für RT-PCR-Analysen (95° 15min; 94° 1 min; 63° 1 min; 72° 1 min; 35 Zyklen) in einer Reihe von humanen Geweben eingesetzt. Es zeigte sich, dass die Expression in Normalgeweben auf Testis beschränkt ist. Wie für die anderen eCT beschrieben, konnte erfindungsgemäß gezeigt werden, dass MORC ektop in einer Reihe von Tumorgeweben aktiviert wird; vgl. Tabelle 10.

**Tabelle 10. Expression von MORC in Tumoren**

| **Gewebe** | **insgesamt getestet** | **Positiv** | **%** |
|---|---|---|---|
| **Melanom** | **16** | **3** | **18** |
| **Mammakarzinome** | **20** | **0** | **0** |
| **Kolorektale Tumoren** | **20** | **0** | **0** |
| **Prostatakarzinome** | **8** | **0** | **0** |
| **Bronchialkarzinome** | **17** | **3** | **18** |
| **Nierenzellkarzinome** | **7** | **0** | **0** |
| **Ovarialkarzinome** | **7** | **1** | **14** |
| **Schilddrüsenkarzinome** | **4** | **0** | **0** |
| **Zervixkarzimome** | **6** | **0** | **0** |
| **Melanom Zellinien** | **8** | **1** | **12** |
| **Bronchialkarzinom Zellinien** | **6** | **1** | **17** |

## Patentansprüche

1. Pharmazeutische Zusammensetzung umfassend einen oder mehrer Bestandteile, die aus der Gruppe ausgewählt sind, bestehend aus:
(i) einem Tumor-assoziierten Antigen oder einem Teil davon,
(ii) einer Nukleinsäure, die für ein Tumor-assoziiertes Antigen oder einen Teil davon kodiert,
(iii) einem Antikörper, der an ein Tumor-assoziiertes Antigen oder einen Teil davon bindet,
(iv) einer Antisense-Nukleinsäure, die spezifisch mit einer Nukleinsäure, die für ein Tumor-assoziiertes Antigen kodiert, hybridisiert,
(v) einer Wirtszelle, die ein Tumor-assoziiertes Antigen oder einen Teil davon exprimiert, und
(vi) isolierten Komplexen zwischen einem Tumor-assoziierten Antigen oder einem Teil davon und einem HLA-Molekül,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

2. Pharmazeutische Zusammensetzung nach Anspruch 1, wobei die Wirtszelle das Tumor-assoziierte Antigen oder den Teil davon sekretiert, auf der Oberfläche exprimiert oder ein HLA-Molekül exprimiert, das an das Tumor-assoziierte Antigen oder den Teil davon bindet.

3. Pharmazeutische Zusammensetzung nach Anspruch 1 oder 2 zur Behandlung einer Erkrankung, die sich durch die Expression oder abnormale Expression eines Tumorassoziierten Antigens auszeichnet.

4. Verfahren zur Diagnose einer Erkrankung, die sich durch die Expression oder abnormale Expression eines Tumor-assoziierten Antigens auszeichnet, umfassend
(i) den Nachweis einer Nukleinsäure, die für das Tumor-assoziierte Antigen kodiert, oder eines Teils davon, und/oder
(ii) den Nachweis des Tumor-assoziierten Antigens oder eines Teils davon, und/oder
(iii) den Nachweis eines Antikörpers gegen das Tumor-assoziierte Antigen oder eines Teils davon und/oder
(iv) den Nachweis von cytotoxischen oder Helfer-T-Lymphozyten, die für das Tumorassoziierte Antigen oder einen Teil davon spezifisch sind,
in einer aus einem Patienten isolierten biologischen Probe,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

5. Verfahren zur Bestimmung der Regression, des Verlaufs oder des Ausbruchs einer Erkrankung, die sich durch die Expression oder abnormale Expression eines Tumorassoziierten Antigens auszeichnet, umfassend die Überwachung einer Probe aus einem Patienten, der die Erkrankung aufweist oder in Verdacht steht, an der Erkrankung zu erkranken, in Bezug auf einen oder mehrere Parameter, ausgewählt aus der Gruppe, bestehend aus:
(i) der Menge der Nukleinsäure, die für das Tumor-assoziierte Antigen kodiert, oder eines Teils davon,
(ii) der Menge des Tumor-assoziierten Antigens oder eines Teils davon,
(iii) der Menge an Antikörpern, die an das Tumor-assoziierte Antigen oder einen Teil davon binden, und
(iv) der Menge an cytolytischen oder Cytokin-ausschüttenden T-Zellen, die für einen Komplex zwischen dem Tumor-assoziierten Antigen oder einem Teil davon und einem MHC-Molekül spezifisch sind,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

6. Antikörper, der spezifisch an ein Protein oder Polypeptid oder an einen Teil davon bindet, wobei das Protein oder Polypeptid von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

7. Pharmazeutische Zusammensetzung nach Anspruch 1 oder Antikörper nach Anspruch 6, wobei der Antikörper mit einem therapeutischen oder diagnostischen Mittel gekoppelt ist.

8. Antikörper nach Anspruch 7 zur Verwendung in einem Verfahren zur Behandlung, Diagnose oder Überwachung einer Erkrankung, die sich durch die Expression oder abnormale Expression eines Tumor-assoziierten Antigens auszeichnet, wobei das Verfahren die Verabreichung des Antikörpers umfasst und das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

9. Antikörper nach Anspruch 7 zur Verwendung in einem Verfahren zur Behandlung Diagnose oder Überwachung eines metastasierenden Tumors, der sich durch die Expression oder abnormale Expression von TPTE auszeichnet, wobei das Verfahren die Verabreichung des Antikörpers umfasst, der Antikörper an TPTE oder einen Teil davon bindet und das TPTE eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe, bestehend aus SEQ ID NO:19, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

10. Pharmazeutische Zusammensetzung nach Anspruch 1 oder 7 oder Antikörper nach einem der Ansprüche 6 bis 9, wobei der Antikörper ein monoklonaler, chimärer oder humanisierter Antikörper oder ein Fragment eines Antikörpers ist.

11. Cytolytische oder Cytokine-ausschüttende T-Zellen zur Verwendung in einem Verfahren zur Behandlung eines Patienten mit einer Erkrankung, die sich durch die Expression oder abnormale Expression eines Tumor-assoziierten Antigens auszeichnet, umfassend:
(i) die Entfernung einer Probe mit immunreaktiven Zellen aus dem Patienten,
(ii) die Kontaktierung der Probe mit einer Wirtszelle, die das Tumor-assoziierte Antigen oder einen Teil davon exprimiert, unter Bedingungen, die eine Produktion cytolytischer oder Cytokine-ausschüttender T-Zellen gegen das Tumor-assoziierte Antigen oder einen Teil davon begünstigen, und
(iii) das Einbringen der cytolytischen oder Cytokine-ausschüttenden T-Zellen in den Patienten in einer Menge, die geeignet ist, Zellen zu lysieren, die das Tumor-assoziierte Antigen oder einen Teil davon exprimieren, wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

12. Cytolytische oder Cytokine-ausschüttende T-Zellen nach Anspruch 11, wobei die Wirtszelle ein HLA-Molekül rekombinant oder endogen exprimiert, das an das Tumorassoziierte Antigen oder einen Teil davon bindet.

13. Pharmazeutische Zusammensetzung nach Anspruch 3, Verfahren nach Anspruch 4 oder 5, Antikörper nach einem der Ansprüche 8 bis 10, oder cytolytische oder Cytokine-ausschüttende T-Zellen nach Anspruch 11 oder 12, wobei die Erkrankung Krebs ist.

14. Pharmazeutische Zusammensetzung nach Anspruch 3, Verfahren nach Anspruch 4 oder 5, Antikörper nach einem der Ansprüche 8 bis 10, oder cytolytische oder Cytokine-ausschüttende T-Zellen nach Anspruch 11 oder 12, wobei die Erkrankung ein Lungentumor, ein Brusttumor, ein Prostatatumor, ein Melanom, ein Colontumor, eine Metastase eines Colontumors, ein Nierenzellkarzinom, ein Zervixkarzinom, ein Coloncarcinom oder ein Mammacarcinom ist.

15. Pharmazeutische Zusammensetzung nach einem der Ansprüche 1 bis 3, 7, 10, 13 und 14, Verfahren nach einem der Ansprüche 4, 5, 13 und 14, Antikörper nach einem der Ansprüche 6 bis 10, 13 und 14, oder cytolytische oder Cytokine-ausschüttende T-Zellen nach einem der Ansprüche 11 bis 14, wobei das Protein oder Polypeptid oder das Tumorassoziierte Antigen eine Aminosäuresequenz umfasst, die aus der Gruppe bestehend aus SEQ ID NOs: 22-24, 58-61, 81, 82, 103-105, 6-13, 14-18, 30, 34-36, 38, 41, 64, 65, 71, 75, 80, 83, 84, 89-100, 101, 102, 106-117, einem Teil oder Derivat davon ausgewählt ist.

16. Nukleinsäure, die für ein Protein oder Polypeptid kodiert, das eine Aminosäuresequenz umfasst, ausgewählt aus der Gruppe, bestehend aus SEQ ID NOs: 23-24, 58-61, 81, 82, 103-105, 7-13, 14-18, 34-36, 64, 65, 80, 83, 84, 89-100, 101, 102, 106-117, einem Teil oder Derivat davon.

17. Nukleinsäure nach Anspruch 16, die aus der Gruppe ausgewählt ist, bestehend aus:
(a) einer Nukleinsäure, die eine Nukleinsäuresequenz umfasst, die aus der Gruppe bestehend aus SEQ ID NOs: 20-21, 54-57, 2-5, 31-33, 37, 39, 62, 63, 85-88, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

18. Wirtszelle, die eine Nukleinsäure nach Anspruch 16 oder 17 umfasst.

19. Protein oder Polypeptid, das von einer Nukleinsäure nach Anspruch 16 oder 17 kodiert wird, oder ein immunogenes Fragment des Proteins oder Polypeptids.

20. Protein oder Polypeptid, das eine Aminosäuresequenz umfasst, ausgewählt aus der Gruppe, bestehend aus SEQ ID NOs: 23-24, 58-61, 81, 82, 103-105, 7-13, 14-18, 34-36, 64, 65, 80, 83, 84, 89-100, 101, 102, 106-117, einem Teil oder Derivat davon, oder ein immunogenes Fragment des Proteins oder Polypeptids.

21. Kit zum Nachweis der Expression oder abnormalen Expression eines Tumorassoziierten Antigens, umfassend Mittel zum Nachweis
(i) der Nukleinsäure, die für das Tumor-assoziierte Antigen kodiert, oder eines Teils davon,
(ii) des Tumor-assoziierten Antigens oder eines Teils davon,
(iii) von Antikörpern, die an das Tumor-assoziierte Antigen oder einen Teil davon binden, und/oder
(iv) von T-Zellen, die für einen Komplex zwischen dem Tumor-assoziierten Antigen oder einem Teil davon und einem MHC-Molekül spezifisch sind,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21, 54-57, 1-5, 29, 31-33, 37, 39, 40, 62, 63, 70, 74, 85-88 einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

22. Pharmazeutische Zusammensetzung, die RNA Interferenz (RNAi) umfasst, die die Expression oder Aktivität des Tumorantigens TPTE, **gekennzeichnet durch** die SEQ ID NOs: 19, 22, hemmt.

23. Pharmazeutische Zusammensetzung nach Anspruch 22, wobei die RNAi eine short hairpin Struktur (shRNA) enthält.
